# EUROPEAN PATENT APPLICATION

(11) **EP 2 357 237 A1**
(43) Date of publication of application: **17.08.2011**
(21) Application number: 10180258.5
(22) Date of filing: 14.05.2004
(51) Int. Cl.: C12N 15/13, C07K 16/18, C12N 15/09, G01N 33/68

(54) **A PROCESS FOR RECOVERING POLYPEPTIDES THAT UNFOLD REVERSIBLY FROM A POLYPEPTIDE REPERTOIRE**

(30) Priority: 14.05.2003 US 470340 P; 17.03.2004 US 554021 P
(62) Divisional of application: 04733030.3
(71) Applicant: Domantis Limited, Cambridge Cambridgeshire, CB4 OWG (GB)
(72) Inventor: Jespers, Laurent S, Cambridge, Cambridgeshire CB4 0WG (GB); Jones, Philip C, Cambridge, Cambridgeshire CB4 0WG (GB); Famm, Kristoffer H J, Cambridge, Cambridgeshire CB4 0WG (GB); Winter, Gregory P, Cambridge, Cambridgeshire CB4 0WG (GB)
(74) Representative: Wilson, Lynn Margaret

(57) **Abstract**

The invention relates to polypeptides that unfold reversibly (e.g., unfolds when heated and refolds when cooled), to repertoires containing polypeptides that unfold reversibly and to libraries that contain polypeptides that unfold reversibly or nucleic acids that encode polypeptides that unfold reversibly. The invention further relates to processes for producing a library enriched in polypeptides that unfold reversibly or nucleic acids encoding polypeptides that unfold reversibly, processes for selecting and/or isolating polypeptides that unfold reversibly, and to methods for producing a polypeptide that unfolds reversibly.

SEQUENCE LISTING

<110> Domantis Limited
      Jespers, Laurent S.
      Jones, Philip C.
      Famm, H.J. Kristoffer
      Winter, Gregory P.

<120> REFOLDABLE POLYPEPTIDES

<130> DB00013D1 EP

<140>
<141>

<150> EP04733030.3
<151> 2004-05-14

<160> 634

<170> FastSEQ for Windows Version 4.0

<210> 1
<211> 348
<212> DNA
<213> Homo Sapiens

<400> 1
gaggtgcagc tgttggagtc tgggggaggc ttggtacagc ctggggggtc cctgcgtctc 60
tcctgtgcag cctccggatt cacctttagc agctatgcca tgagctgggt ccgccaggct 120
ccagggaagg gtctagagtg ggtctcagct attagtggta gtggtggtag cacatactac 180
gcagactccg tgaagggccg gttcaccatc tcccgtgaca attccaagaa cacgctgtat 240
ctgcaaatga acagcctgcg tgccgaggac accgcggtat attactgtgc gaaaagttat 300
ggtgcttttg actactgggg tcagggaacc ctggtcaccg tctcgagc 348

<210> 2
<211> 348
<212> DNA
<213> Homo Sapiens

<400> 2
ctccacgtcg acaacctcag accccctccg aaccatgtcg gaccccccag ggacgcagag 60
aggacacgtc ggaggcctaa gtggaaatcg tcgatacggt actcgaccca ggcggtccga 120
ggtcccttcc cagatctcac ccagagtcga taatcaccat caccaccatc gtgtatgatg 180
cgtctgaggc acttcccggc caagtggtag agggcactgt taaggttctt gtgcgacata 240
gacgtttact tgtcggacgc acggctcctg tggcgccata taatgacacg cttttcaata 300
ccacgaaaac tgatgacccc agtcccttgg gaccagtggc agagctcg 348

<210> 3
<211> 116
<212> PRT
<213> Homo Sapiens

<400> 3
Glu Val Gln Leu Leu Glu Ser Gly Gly Gly Leu Val Gln Pro Gly Gly

 1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ser Tyr Gly Ala Phe Asp Tyr Trp Gly Gln Gly Thr Leu Val
            100                 105                 110
Thr Val Ser Ser
        115

<210> 4
<211> 324
<212> DNA
<213> Homo Sapiens

<400> 4
gacatccaga tgacccagtc tccatcctcc ctgtctgcat ctgtaggaga ccgtgtcacc 60
atcacttgcc gggcaagtca gagcattagc agctatttaa attggtacca gcagaaacca 120
gggaaagccc ctaagctcct gatctatgct gcatccagtt tgcaaagtgg ggtcccatca 180
cgtttcagtg gcagtggatc tgggacagat ttcactctca ccatcagcag tctgcaacct 240
gaagattttg ctacgtacta ctgtcaacag agttacagta cccctaatac gttcggccaa 300
gggaccaagg tggaaatcaa acgg                                         324

<210> 5
<211> 324
<212> DNA
<213> Homo Sapiens

<400> 5
ctgtaggtct actgggtcag aggtaggagg gacagacgta gacatcctct ggcacagtgg 60
tagtgaacgg cccgttcagt ctcgtaatcg tcgataaatt taaccatggt cgtctttggt 120
ccctttcggg gattcgagga ctagatacga cgtaggtcaa acgtttcacc ccagggtagt 180
gcaaagtcac cgtcacctag accctgtcta aagtgagagt ggtagtcgtc agacgttgga 240
cttctaaaac gatgcatgat gacagttgtc tcaatgtcat ggggattatg caagccggtt 300
ccctggttcc acctttagtt tgcc                                         324

<210> 6
<211> 108
<212> PRT
<213> Homo Sapiens

<400> 6
Asp Ile Gln Met Thr Gln Ser Pro Ser Ser Leu Ser Ala Ser Val Gly
 1               5                   10                  15
Asp Arg Val Thr Ile Thr Cys Arg Ala Ser Gln Ser Ile Ser Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro

65                  70                  75                  80
Glu Asp Phe Ala Thr Tyr Tyr Cys Gln Gln Ser Tyr Ser Thr Pro Asn
                85                  90                  95
Thr Phe Gly Gln Gly Thr Lys Val Glu Ile Lys Arg
            100                 105

<210> 7
<211> 115
<212> PRT
<213> Unknown

<220>
<223> antibody sequence

<400> 7
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asn Tyr
            20                  25                  30
Leu Ala Trp Tyr Gln Gln Lys Pro Gly Lys Ala Pro Glu Leu Arg Ile
        35                  40                  45
Tyr Asp Ala Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Tyr Gln Asn Leu Pro Leu
                85                  90                  95
Thr Phe Gly Pro Gly Thr Lys Val Asp Ile Lys Arg Thr Val Ala Ala
            100                 105                 110
Pro Ser Val
        115

<210> 8
<211> 214
<212> PRT
<213> Unknown

<220>
<223> antibody sequence

<400> 8
Gln Ile Val Leu Thr Gln Ser Pro Ala Ile Met Ser Ala Ser Pro Gly
1               5                   10                  15
Glu Lys Val Thr Met Thr Cys Ser Ala Ser Ser Ser Val Tyr Tyr Met
            20                  25                  30
Tyr Trp Tyr Gln Gln Lys Pro Gly Ser Ser Pro Arg Leu Leu Ile Tyr
        35                  40                  45
Asp Thr Ser Asn Leu Ala Ser Gly Val Pro Val Arg Phe Ser Gly Ser
    50                  55                  60
Gly Ser Gly Thr Ser Tyr Ser Leu Thr Ile Ser Arg Met Glu Ala Glu
65                  70                  75                  80
Asp Ala Ala Thr Tyr Tyr Cys Gln Gln Trp Ser Ser Tyr Pro Pro Ile
                85                  90                  95
Thr Phe Gly Val Gly Thr Lys Leu Glu Leu Lys Arg Ala Asp Ala Ala
            100                 105                 110
Pro Thr Val Ser Ile Phe Pro Pro Ser Ser Glu Gln Leu Thr Ser Gly

115                 120                 125
Gly Ala Ser Val Val Cys Phe Leu Asn Asn Phe Tyr Pro Lys Asp Ile
    130                 135                 140
Asn Val Lys Trp Lys Ile Asp Gly Ser Glu Arg Gln Asn Gly Val Leu
145                 150                 155                 160
Asn Ser Trp Thr Asp Gln Asp Ser Lys Asp Ser Thr Tyr Ser Met Ser
                165                 170                 175
Ser Thr Leu Thr Leu Thr Lys Asp Glu Tyr Glu Arg His Asn Ser Tyr
            180                 185                 190
Thr Cys Glu Ala Thr His Lys Thr Ser Thr Ser Pro Ile Val Lys Ser
        195                 200                 205
Phe Asn Arg Asn Glu Cys
    210

<210> 9
<211> 212
<212> PRT
<213> Unknown

<220>
<223> antibody sequence

<400> 9
Asp Ile Val Leu Thr Gln Ser Pro Ala Ile Met Ser Ala Ser Pro Gly
 1               5                  10                  15
Glu Lys Val Thr Met Thr Cys Ser Ala Ser Ser Ser Val Asn Tyr Met
            20                  25                  30
Tyr Trp Tyr Gln Gln Lys Ser Gly Thr Ser Pro Lys Arg Trp Ile Tyr
        35                  40                  45
Asp Thr Ser Lys Leu Ala Ser Gly Val Pro Val Arg Phe Ser Gly Ser
    50                  55                  60
Gly Ser Gly Thr Ser Tyr Ser Leu Thr Ile Ser Ser Met Glu Thr Glu
65                  70                  75                  80
Asp Ala Ala Thr Tyr Tyr Cys Gln Gln Trp Gly Arg Asn Pro Thr Phe
                85                  90                  95
Gly Gly Gly Thr Lys Leu Glu Ile Lys Arg Ala Asp Ala Ala Pro Thr
            100                 105                 110
Val Ser Ile Phe Pro Pro Ser Ser Glu Gln Leu Thr Ser Gly Gly Ala
        115                 120                 125
Ser Val Val Cys Phe Leu Asn Asn Phe Tyr Pro Lys Asp Ile Asn Val
    130                 135                 140
Lys Trp Lys Ile Asp Gly Ser Glu Arg Gln Asn Gly Val Leu Asn Ser
145                 150                 155                 160
Trp Thr Asp Gln Asp Ser Lys Asp Ser Thr Tyr Ser Met Ser Ser Thr
                165                 170                 175
Leu Thr Leu Thr Lys Asp Glu Tyr Glu Arg His Asn Ser Tyr Thr Cys
            180                 185                 190
Glu Ala Thr His Lys Thr Ser Thr Ser Pro Ile Val Lys Ser Phe Asn
        195                 200                 205
Arg Asn Glu Cys
    210

<210> 10
<211> 106
<212> PRT
<213> Unknown

<220>
<223> antibody sequence

<400> 10
Gln Ile Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Asn Pro Tyr Thr
                85                  90                  95
Phe Gly Gly Gly Thr Lys Leu Glu Ile Lys
            100                 105


<210> 11
<211> 106
<212> PRT
<213> Unknown

<220>
<223> antibody sequence

<400> 11
Gln Ile Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Asn Pro Tyr Thr
                85                  90                  95
Phe Gly Gly Gly Thr Lys Leu Glu Ile Lys
            100                 105


<210> 12
<211> 214
<212> PRT
<213> Unknown

<220>
<223> antibody sequence

<400> 12
Glu Ile Gln Leu Thr Gln Ser Pro Ser Ser Leu Ser Ala Ser Leu Gly
1               5                   10                  15
Glu Arg Val Ser Leu Thr Cys Arg Thr Ser Gln Glu Ile Ser Gly Tyr

            20                  25                  30
Leu Ser Trp Leu Gln Gln Lys Pro Asp Gly Thr Ile Lys Arg Leu Ile
        35                  40                  45
Tyr Asp Ala Thr Lys Leu Asp Ser Gly Ala Pro Lys Arg Phe Ser Gly
    50                  55                  60
Ser Arg Ser Gly Ser Asp Tyr Ser Leu Thr Ile Ser Ser Leu Glu Ser
65                  70                  75                  80
Glu Asp Phe Ala Asp Tyr Tyr Cys Leu Gln Tyr Ala Ser Phe Pro Arg
                85                  90                  95
Thr Phe Gly Gly Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 13
<211> 108
<212> PRT
<213> Unknown

<220>
<223> antibody sequence

<400> 13
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asp Tyr
            20                  25                  30
Leu Ile Trp Tyr Gln Gln Lys Leu Gly Lys Ala Pro Asn Leu Leu Ile
        35                  40                  45
Tyr Asp Ala Ser Thr Leu Glu Thr Gly Val Pro Ser Arg Phe Ser Gly
    50                  55                  60
Ser Gly Ser Gly Thr Glu Tyr Thr Phe Thr Ile Ser Ser Leu Gln Pro
65                  70                  75                  80
Glu Asp Ile Ala Thr Tyr Tyr Cys Gln Gln Tyr Asp Asp Leu Pro Tyr
                85                  90                  95
Thr Phe Gly Gln Gly Thr Lys Val Glu Ile Lys Arg
            100                 105

<210> 14
<211> 108
<212> PRT
<213> Unknown

<220>

<223> antibody sequence

<400> 14
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asp Tyr
            20                  25                  30
Leu Ile Trp Tyr Gln Gln Lys Leu Gly Lys Ala Pro Asn Leu Leu Ile
        35                  40                  45
Tyr Asp Ala Ser Thr Leu Glu Thr Gly Val Pro Ser Arg Phe Ser Gly
    50                  55                  60
Ser Gly Ser Gly Thr Glu Tyr Thr Phe Thr Ile Ser Ser Leu Gln Pro
65                  70                  75                  80
Glu Asp Ile Ala Thr Tyr Tyr Cys Gln Gln Tyr Asp Asp Leu Pro Tyr
                85                  90                  95
Thr Phe Gly Gln Gly Thr Lys Val Glu Ile Lys Arg
            100                 105


<210> 15
<211> 108
<212> PRT
<213> Unknown

<220>
<223> antibody sequence

<400> 15
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asp Tyr
            20                  25                  30
Leu Ile Trp Tyr Gln Gln Lys Leu Gly Lys Ala Pro Asn Leu Leu Ile
        35                  40                  45
Tyr Asp Ala Ser Thr Leu Glu Thr Gly Val Pro Ser Arg Phe Ser Gly
    50                  55                  60
Ser Gly Ser Gly Thr Glu Tyr Thr Phe Thr Ile Ser Ser Leu Gln Pro
65                  70                  75                  80
Glu Asp Ile Ala Thr Tyr Tyr Cys Gln Gln Tyr Asp Asp Leu Pro Tyr
                85                  90                  95
Thr Phe Gly Gln Gly Thr Lys Val Glu Ile Lys Arg
            100                 105


<210> 16
<211> 104
<212> PRT
<213> Unknown

<220>
<223> antibody sequence

<400> 16
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asp Tyr
            20                  25                  30
Leu Ile Trp Tyr Gln Gln Lys Leu Gly Lys Ala Pro Asn Leu Leu Ile

35                  40                  45
Tyr Asp Ala Ser Thr Leu Glu Thr Gly Val Pro Ser Arg Phe Ser Gly
    50                  55                  60
Ser Gly Ser Gly Thr Glu Tyr Thr Phe Thr Ile Ser Ser Leu Gln Pro
65                  70                  75                  80
Glu Asp Ile Ala Thr Tyr Tyr Cys Gln Gln Tyr Asp Asp Leu Pro Tyr
                85                  90                  95
Thr Phe Gly Gln Gly Thr Lys Val
            100

<210> 17
<211> 95
<212> PRT
<213> Unknown

<220>
<223> antibody sequence

<400> 17
Asp Ile Gln Met Thr Gln Ser Pro Ser Thr Leu Ser Ala Ser Val Gly
 1               5                  10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Asp Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Ser
                85                  90                  95

<210> 18
<211> 96
<212> PRT
<213> Unknown

<220>
<223> antibody sequence

<400> 18
Asp Ile Val Leu Thr Gln Ala Pro Pro Ser Leu Asp Val Ser Gln Gly
 1               5                  10                  15
Arg Ala Thr Ile Ser Cys Arg Thr Ser Lys Ser Val Arg Thr Ser Ser
            20                  25                  30
Tyr Ser Tyr Met His Trp Tyr Gln Gln Lys Pro Gly Gln Pro Pro Lys
        35                  40                  45
Leu Leu Asn Leu Cys Asn Gln Val Ser Arg Val Pro Ala Arg Phe Ser
    50                  55                  60
Gly Ser Gly Ser Gly Thr Asp Phe Thr Leu Lys Ile His Pro Val Glu
65                  70                  75                  80
Glu Glu Asp Ala Ala Thr Tyr Phe Cys Gln Gln Ser Asn Glu Asn Pro
                85                  90                  95

<210> 19

<211> 95
<212> PRT
<213> Unknown

<220>
<223> antibody sequence

<400> 19
Val Ile Trp Met Thr Gln Ser Pro Ser Leu Leu Ser Ala Ser Thr Gly
1               5                   10                  15
Asp Arg Val Thr Ile Ser Cys Arg Met Ser Gln Gly Ile Ser Ser Tyr
            20                  25                  30
Leu Ala Trp Tyr Gln Gln Lys Pro Gly Lys Ala Pro Glu Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Cys Leu Gln Ser
65                  70                  75                  80
Glu Asp Phe Ala Thr Tyr Tyr Cys Gln Gln Tyr Tyr Ser Phe Pro
                85                  90                  95


<210> 20
<211> 92
<212> PRT
<213> Unknown

<220>
<223> antibody sequence

<400> 20
Ala Ile Gln Leu Thr Gln Ser Pro Ser Ser Leu Ser Ala Ser Val Gly
1               5                   10                  15
Asp Arg Val Thr Ile Thr Cys Arg Ala Ser Gln Gly Ile Ser Ser Ala
            20                  25                  30
Tyr Gln Gln Lys Pro Gly Lys Ala Pro Lys Leu Leu Ile Tyr Asp Ala
        35                  40                  45
Ser Ser Leu Glu Ser Gly Val Pro Ser Arg Phe Ser Gly Ser Gly Ser
    50                  55                  60
Gly Thr Asp Phe Thr Leu Thr Ile Ser Ser Leu Gln Pro Glu Asp Phe
65                  70                  75                  80
Ala Thr Tyr Tyr Cys Gln Gln Phe Asn Asn Tyr Pro
                85                  90


<210> 21
<211> 93
<212> PRT
<213> Unknown

<220>
<223> antibody sequence

<400> 21
Ala Ile Gln Leu Thr Gln Ser Pro Ser Ser Leu Ser Ala Ser Val Gly
1               5                   10                  15
Asp Arg Val Thr Ile Thr Cys Arg Ala Ser Gln Gly Ile Ser Ser Ala
            20                  25                  30

Trp Tyr Gln Gln Lys Pro Gly Lys Ala Pro Lys Leu Leu Ile Tyr Asp
        35                  40                  45
Ala Ser Ser Leu Glu Ser Gly Val Pro Ser Arg Phe Ser Gly Ser Gly
    50                  55                  60
Ser Gly Thr Asp Phe Thr Leu Thr Ile Ser Ser Leu Gln Pro Glu Asp
65                  70                  75                  80
Phe Ala Thr Tyr Tyr Cys Gln Gln Phe Asn Asn Tyr Pro
                85                  90

<210> 22
<211> 93
<212> PRT
<213> Unknown

<220>
<223> antibody sequence

<400> 22
Asp Ile Gln Met Thr Gln Ser Pro Ser Ser Leu Ser Ala Ser Val Gly
 1               5                  10                  15
Asp Arg Val Thr Ile Thr Cys Gln Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Asp Asn Leu Glu Thr Gly Val Pro Ser Arg Phe Ser Gly Ser Gly
    50                  55                  60
Ser Gly Thr Asp Phe Thr Phe Thr Ile Ser Ser Leu Gln Pro Glu Asp
65                  70                  75                  80
Ile Ala Thr Tyr Tyr Cys Gln Gln Tyr Asp Asn Leu Pro
                85                  90

<210> 23
<211> 93
<212> PRT
<213> Unknown

<220>
<223> antibody sequence

<400> 23
Asp Ile Gln Met Thr Gln Ser Pro Ser Ser Leu Ser Ala Ser Val Gly
 1               5                  10                  15
Asp Arg Val Thr Ile Thr Cys Gln Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Asp Asn Leu Glu Thr Gly Val Pro Ser Arg Phe Ser Gly Ser Gly
    50                  55                  60
Ser Gly Thr Asp Phe Thr Phe Thr Ile Ser Ser Leu Gln Pro Glu Asp
65                  70                  75                  80
Ile Ala Thr Tyr Tyr Cys Gln Gln Tyr Asp Asn Leu Pro
                85                  90

<210> 24
<211> 95

<212> PRT
<213> Unknown

<220>
<223> antibody sequence

<400> 24
Asp Ile Gln Met Ile Gln Ser Pro Ser Phe Leu Ser Ala Ser Val Gly
1               5                   10                  15
Asp Arg Val Ser Ile Ile Cys Trp Ala Ser Glu Gly Ile Ser Ser Asn
            20                  25                  30
Leu Ala Trp Tyr Leu Gln Lys Pro Gly Lys Ser Pro Lys Leu Phe Leu
        35                  40                  45
Tyr Asp Ala Lys Asp Leu His Pro Gly Val Ser Ser Arg Phe Ser Gly
    50                  55                  60
Arg Gly Ser Gly Thr Asp Phe Thr Leu Thr Ile Ile Ser Leu Lys Pro
65                  70                  75                  80
Glu Asp Phe Ala Ala Tyr Tyr Cys Lys Gln Asp Phe Ser Tyr Pro
                85                  90                  95


<210> 25
<211> 93
<212> PRT
<213> Unknown

<220>
<223> antibody sequence

<400> 25
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Asp Asn Arg Ala Thr Gly Ile Pro Ala Arg Phe Ser Gly Ser Gly
    50                  55                  60
Ser Gly Thr Asp Phe Thr Leu Thr Ile Ser Ser Leu Glu Pro Glu Asp
65                  70                  75                  80
Phe Ala Val Tyr Tyr Cys Gln Gln Arg Ser Asn Trp Pro
                85                  90


<210> 26
<211> 93
<212> PRT
<213> Unknown

<220>
<223> antibody sequence

<400> 26
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Gly Val Ser Ser Tyr
            20                  25                  30
Leu Ala Trp Tyr Gln Gln Lys Pro Gly Gln Ala Pro Arg Leu Leu Ile

35                  40                  45
Tyr Asp Asn Arg Ala Thr Gly Ile Pro Ala Arg Phe Ser Gly Ser Gly
    50                  55                  60
Pro Gly Thr Asp Phe Thr Leu Thr Ile Ser Ser Leu Glu Pro Glu Asp
65                  70                  75                  80
Phe Ala Val Tyr Tyr Cys Gln Gln Arg Ser Asn Trp His
                85                  90

<210> 27
<211> 96
<212> PRT
<213> Unknown

<220>
<223> antibody sequence

<400> 27
Glu Ile Val Leu Thr Gln Ser Pro Ala Thr Leu Ser Leu Ser Pro Gly
 1               5                  10                  15
Glu Arg Ala Thr Leu Ser Cys Gly Ala Ser Gln Ser Val Ser Ser Ser
            20                  25                  30
Tyr Leu Ala Trp Tyr Gln Gln Lys Pro Gly Leu Ala Pro Arg Leu Leu
        35                  40                  45
Ile Tyr Asp Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Pro
                85                  90                  95

<210> 28
<211> 95
<212> PRT
<213> Unknown

<220>
<223> antibody sequence

<400> 28
Gln Ile Val Leu Thr Gln Ser Pro Ala Ile Met Ser Ala Asp Pro Gly
 1               5                  10                  15
Glu Lys Val Thr Met Thr Cys Ser Ala Ser Ser Ser Val Ser Tyr Met
            20                  25                  30
Tyr Trp Tyr Gln Gln Lys Pro Gly Ser Ser Pro Arg Leu Leu Ile Tyr
        35                  40                  45
Asp Thr Ser Asn Leu Ala Ser Gly Val Pro Val Arg Phe Ser Gly Ser
    50                  55                  60
Gly Ser Gly Thr Ser Tyr Ser Leu Thr Ile Ser Arg Met Glu Ala Glu
65                  70                  75                  80
Asp Ala Ala Thr Tyr Tyr Cys Gln Gln Trp Ser Ser Tyr Pro Pro
                85                  90                  95

<210> 29
<211> 94
<212> PRT

<213> Unknown

<220>
<223> antibody sequence

<400> 29
Gln Ile Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Asn Pro
                85                  90


<210> 30
<211> 95
<212> PRT
<213> Unknown

<220>
<223> antibody sequence

<400> 30
Gln Ile Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
Tyr Trp Tyr Gln Gln Lys Pro Gly Ser Ser Pro Arg Leu Trp Ile Tyr
        35                  40                  45
Asp Thr Ser Asn Leu Val Ser Gly Val Pro Ala Arg Phe Ser Gly Ser
    50                  55                  60
Arg Ser Gly Thr Ser Tyr Ser Leu Thr Ile Ser Ser Met Glu Ala Glu
65                  70                  75                  80
Asp Ala Ala Thr Tyr Tyr Cys Gln Gln Tyr Ser Gly Tyr Pro Ser
                85                  90                  95


<210> 31
<211> 95
<212> PRT
<213> Unknown

<220>
<223> antibody sequence

<400> 31
Glu Asn Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Ser Thr Ser Pro Lys Leu Trp Ile Tyr
        35                  40                  45

Asp Thr Ser Lys Leu Ala Ser Gly Val Pro Gly Arg Phe Ser Gly Ser
    50                  55                  60
Gly Ser Gly Asn Ser Tyr Ser Leu Thr Ile Ser Ser Met Glu Ala Glu
65                  70                  75                  80
Asp Val Ala Thr Tyr Tyr Cys Phe Gln Gly Ser Gly Tyr Pro Leu
                85                  90                  95

<210> 32
<211> 94
<212> PRT
<213> Unknown

<220>
<223> antibody sequence

<400> 32
Gln Ile Leu Leu Thr Gln Ser Pro Ala Ile Met Ser Ala Ser Pro Gly
 1               5                  10                  15
Glu Lys Val Thr Met Thr Cys Ser Ala Ser Ser Ser Val Ser Tyr Met
            20                  25                  30
His Trp Tyr Gln Gln Lys Pro Gly Ser Ser Pro Lys Pro Trp Ile Tyr
        35                  40                  45
Asp Thr Ser Asn Leu Ala Ser Gly Phe Pro Ala Arg Phe Ser Gly Ser
    50                  55                  60
Gly Ser Gly Thr Ser Tyr Ser Leu Ile Ile Ser Ser Met Glu Ala Glu
65                  70                  75                  80
Asp Ala Ala Thr Tyr Tyr Cys His Gln Arg Ser Ser Tyr Pro
                85                  90

<210> 33
<211> 94
<212> PRT
<213> Unknown

<220>
<223> antibody sequence

<400> 33
Gln Ile Val Leu Thr Gln Ser Pro Ala Ile Met Ser Ala Ser Pro Gly
 1               5                  10                  15
Glu Lys Val Thr Met Thr Cys Ser Ala Ser Ser Ser Ile Ser Tyr Met
            20                  25                  30
His Trp Tyr Gln Gln Lys Pro Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys His Gln Arg Ser Ser Tyr Pro
                85                  90

<210> 34
<211> 94
<212> PRT
<213> Unknown

<220>
<223> antibody sequence

<400> 34
Asp Ile Gln Met Thr Gln Ser Pro Ala Ser Leu Ser Ala Ser Val Gly
1               5                   10                  15
Glu Thr Val Thr Ile Thr Cys Gln Ala Ser Glu Asn Ile Ala Ser Asp
            20                  25                  30
Leu Ala Trp Tyr Gln Lys Gln Gly Lys Ser Pro Gln Leu Leu Val Tyr
        35                  40                  45
Asp Ala Arg Asn Leu Ala Asp Gly Val Pro Ser Arg Phe Ser Gly Ser
    50                  55                  60
Gly Ser Gly Thr His Tyr Ser Leu Asn Ile His Ser Leu Gln Ser Glu
65                  70                  75                  80
Asp Val Ala Arg Tyr Tyr Cys Gln His Tyr Tyr Gly Thr Pro
                85                  90


<210> 35
<211> 100
<212> PRT
<213> Unknown

<220>
<223> antibody sequence

<400> 35
Ala Gln Val Leu Thr Gln Thr Glu Ser Pro Val Ser Ala Pro Val Gly
1               5                   10                  15
Gly Thr Val Thr Ile Asn Cys Gln Ala Ser Gln Ser Val Tyr Asp Asn
            20                  25                  30
Asn Tyr Leu Ser Trp Tyr Gln Gln Lys Pro Gly Gln Pro Pro Lys Leu
        35                  40                  45
Leu Ile Tyr Asp Ala Ser Lys Leu Ala Ser Gly Val Pro Ser Arg Phe
    50                  55                  60
Ser Gly Ser Gly Ser Gly Thr Gln Phe Thr Leu Thr Ile Ser Gly Val
65                  70                  75                  80
Gln Cys Asp Asp Ala Ala Thr Tyr Tyr Cys Gln Gly Ser Tyr Tyr Ser
                85                  90                  95
Ser Gly Trp Tyr
            100


<210> 36
<211> 100
<212> PRT
<213> Unknown

<220>
<223> antibody sequence

<400> 36
Ala Gln Gly Pro Thr Gln Thr Pro Ser Ser Val Ser Ala Ala Val Gly
1               5                   10                  15
Gly Thr Val Thr Ile Asn Cys Gln Thr Ser Glu Ser Phe Tyr Ser Asn
            20                  25                  30
Asn Ile Leu Ser Trp Tyr Gln Gln Lys Pro Gly Gln Pro Pro Lys Leu

35                  40                  45
Leu Ile Tyr Asp Ala Ser Thr Leu Ala Ser Gly Val Pro Ser Arg Phe
    50                  55                  60
Lys Gly Ser Gly Ser Gly Thr Gln Phe Thr Leu Thr Ile Ser Asp Val
65                  70                  75                  80
Gln Cys Asp Asp Ala Ala Thr Tyr Tyr Cys Gln Gly Ser Tyr His Ser
                85                  90                  95
Ser Gly Trp Tyr
            100

<210> 37
<211> 99
<212> PRT
<213> Unknown

<220>
<223> antibody sequence

<400> 37
Ala Ala Val Leu Thr Gln Thr Pro Ser Pro Val Ser Ala Ala Val Gly
1               5                   10                  15
Gly Thr Val Thr Ile Lys Cys Gln Ser Ser Gln Ser Val Tyr Asn Asn
            20                  25                  30
Asn Leu Leu Ser Trp Tyr Gln Gln Lys Pro Gly Gln Pro Pro Lys Leu
        35                  40                  45
Leu Ile Tyr Asp Ala Ser Asn Leu Ala Ser Gly Val Pro Asp Arg Phe
    50                  55                  60
Ser Gly Ser Gly Ser Gly Thr Gln Phe Thr Leu Thr Ile Ser Gly Val
65                  70                  75                  80
Gln Cys Asp Asp Ala Ala Thr Tyr Tyr Cys Leu Gly Gly Tyr Asp Asp
                85                  90                  95
Asp Ala Asp

<210> 38
<211> 98
<212> PRT
<213> Unknown

<220>
<223> antibody sequence

<400> 38
Ala Val Val Leu Thr Gln Thr Ala Ser Pro Val Ser Ala Ala Val Gly
1               5                   10                  15
Gly Thr Val Thr Ile Asn Cys Gln Ala Ser Gln Ser Ile Ser Thr Ala
            20                  25                  30
Leu Ala Trp Tyr Gln Gln Lys Pro Gly Gln Arg Pro Lys Leu Leu Ile
        35                  40                  45
Tyr Asp Ala Ser Lys Leu Ala Ser Gly Val Pro Ser Arg Phe Lys Gly
    50                  55                  60
Ser Gly Ser Gly Thr Gln Phe Thr Leu Thr Ile Ser Gly Val Gln Cys
65                  70                  75                  80
Asp Asp Ala Ala Thr Tyr Tyr Cys Gln Gln Gly Tyr Ser Ser Ser Ser
                85                  90                  95
Ala Asp

<210> 39
<211> 121
<212> PRT
<213> Unknown

<220>
<223> antibody sequence

<400> 39
Glu Val Lys Val Ile Glu Ser Gly Gly Gly Leu Val Gln Pro Gly Gly
1               5                   10                  15
Ser Leu Lys Leu Ser Cys Ala Ala Ser Gly Phe Asp Phe Ser Arg Tyr
            20                  25                  30
Trp Met Ser Trp Val Arg Gln Ala Pro Gly Lys Gly Leu Glu Trp Ile
        35                  40                  45
Gly Glu Ile Asn Pro Asp Ser Ser Thr Ile Asn Tyr Thr Pro Ser Leu
    50                  55                  60
Lys Asp Lys Phe Ile Ile Ser Arg Asp Asn Ala Lys Asn Thr Leu Tyr
65                  70                  75                  80
Leu Gln Met Ser Lys Val Arg Ser Glu Asp Thr Ala Leu Tyr Tyr Cys
                85                  90                  95
Ala Arg Leu Gly Gly Asp Leu His Tyr Tyr Ala Met Asp Tyr Trp Gly
            100                 105                 110
Gln Gly Thr Ser Val Thr Val Ser Ser
        115                 120


<210> 40
<211> 121
<212> PRT
<213> Unknown

<220>
<223> antibody sequence

<400> 40
Glu Val Lys Val Ile Glu Ser Gly Gly Gly Leu Val Gln Pro Gly Gly
1               5                   10                  15
Ser Leu Lys Leu Ser Cys Ala Ala Ser Gly Phe Asp Phe Ser Arg Tyr
            20                  25                  30
Trp Met Ser Trp Val Arg Gln Ala Pro Gly Lys Gly Leu Glu Trp Ile
        35                  40                  45
Gly Glu Ile Asn Pro Asp Ser Ser Thr Ile Asn Tyr Thr Pro Ser Leu
    50                  55                  60
Lys Asp Lys Phe Ile Ile Ser Arg Asp Asn Ala Lys Asn Thr Leu Tyr
65                  70                  75                  80
Leu Gln Met Ser Lys Val Arg Ser Glu Asp Thr Ala Leu Tyr Tyr Cys
                85                  90                  95
Ala Arg Leu Gly Gly Asp Leu His Tyr Tyr Ala Met Asp Tyr Trp Gly
            100                 105                 110
Gln Gly Thr Ser Val Thr Val Ser Ser
        115                 120


<210> 41

<211> 220
<212> PRT
<213> Unknown

<220>
<223> antibody sequence

<400> 41
Glu Val Lys Leu Leu Glu Ser Gly Gly Gly Leu Val Gln Pro Gly Gly
1               5                   10                  15
Ser Leu Lys Leu Ser Cys Ala Ala Ser Gly Phe Asp Phe Ser Lys Tyr
            20                  25                  30
Trp Met Ser Trp Val Arg Gln Ala Pro Gly Lys Gly Leu Glu Trp Ile
        35                  40                  45
Gly Glu Ile His Pro Asp Ser Gly Thr Ile Asn Tyr Thr Pro Ser Leu
    50                  55                  60
Lys Asp Lys Phe Ile Ile Ser Arg Asp Asn Ala Lys Asn Ser Leu Tyr
65                  70                  75                  80
Leu Gln Met Ser Lys Val Arg Ser Glu Asp Thr Ala Leu Tyr Tyr Cys
                85                  90                  95
Ala Arg Leu His Tyr Tyr Gly Tyr Asn Ala Tyr Trp Gly Gln Gly Thr
            100                 105                 110
Leu Val Thr Val Ser Ala Glu Ser Ala Arg Asn Pro Thr Ile Tyr Pro
        115                 120                 125
Leu Thr Leu Pro Pro Ala Leu Ser Ser Asp Pro Val Ile Ile Gly Cys
    130                 135                 140
Leu Ile His Asp Tyr Phe Pro Ser Gly Thr Met Asn Val Thr Trp Gly
145                 150                 155                 160
Lys Ser Gly Lys Asp Ile Thr Thr Val Asn Phe Pro Pro Ala Leu Ala
                165                 170                 175
Ser Gly Gly Arg Tyr Thr Met Ser Asn Gln Leu Thr Leu Pro Ala Val
            180                 185                 190
Glu Cys Pro Glu Gly Glu Ser Val Lys Cys Ser Val Gln His Asp Ser
        195                 200                 205
Asn Pro Val Gln Glu Leu Asp Val Asn Cys Ser Gly
    210                 215                 220


<210> 42
<211> 135
<212> PRT
<213> Unknown

<220>
<223> antibody sequence

<400> 42
Met Tyr Arg Ser Ala Phe Ser Val Gly Leu Glu Thr Arg Val Thr Val
1               5                   10                  15
Pro Asn Val Pro Ile Arg Phe Thr Lys Ile Phe Tyr Asn Gln Gln Asn
            20                  25                  30
His Tyr Asp Gly Ser Thr Gly Lys Phe Tyr Cys Asn Ile Pro Gly Leu
        35                  40                  45
Tyr Tyr Phe Ser Tyr His Ile Thr Val Tyr Met Lys Asp Val Lys Val
    50                  55                  60
Ser Leu Phe Lys Lys Asp Lys Ala Val Leu Phe Thr Tyr Asp Gln Tyr
65                  70                  75                  80
Gln Glu Lys Asn Val Asp Gln Ala Ser Gly Ser Val Leu Leu His Leu

85                    90                    95
Glu Val Gly Asp Gln Val Trp Leu Gln Val Tyr Gly Asp Gly Asp His
            100                 105                 110
Asn Gly Leu Tyr Ala Asp Asn Val Asn Asp Ser Thr Phe Thr Gly Phe
        115                 120                 125
Leu Leu Tyr His Asp Thr Asn
    130                 135


<210> 43
<211> 135
<212> PRT
<213> Unknown

<220>
<223> antibody sequence

<400> 43
Met Tyr Arg Ser Ala Phe Ser Val Gly Leu Glu Thr Arg Val Thr Val
 1               5                  10                  15
Pro Asn Val Pro Ile Arg Phe Thr Lys Ile Phe Tyr Asn Gln Gln Asn
            20                  25                  30
His Tyr Asp Gly Ser Thr Gly Lys Phe Tyr Cys Asn Ile Pro Gly Leu
        35                  40                  45
Tyr Tyr Phe Ser Tyr His Ile Thr Val Tyr Met Lys Asp Val Lys Val
    50                  55                  60
Ser Leu Phe Lys Lys Asp Lys Ala Val Leu Phe Thr Tyr Asp Gln Tyr
65                  70                  75                  80
Gln Glu Lys Asn Val Asp Gln Ala Ser Gly Ser Val Leu Leu His Leu
                85                  90                  95
Glu Val Gly Asp Gln Val Trp Leu Gln Val Tyr Gly Asp Gly Asp His
            100                 105                 110
Asn Gly Leu Tyr Ala Asp Asn Val Asn Asp Ser Thr Phe Thr Gly Phe
        115                 120                 125
Leu Leu Tyr His Asp Thr Asn
    130                 135


<210> 44
<211> 135
<212> PRT
<213> Unknown

<220>
<223> antibody sequence

<400> 44
Met Tyr Arg Ser Ala Phe Ser Val Gly Leu Glu Thr Arg Val Thr Val
 1               5                  10                  15
Pro Asn Val Pro Ile Arg Phe Thr Lys Ile Phe Tyr Asn Gln Gln Asn
            20                  25                  30
His Tyr Asp Gly Ser Thr Gly Lys Phe Tyr Cys Asn Ile Pro Gly Leu
        35                  40                  45
Tyr Tyr Phe Ser Tyr His Ile Thr Val Tyr Met Lys Asp Val Lys Val
    50                  55                  60
Ser Leu Phe Lys Lys Asp Lys Ala Val Leu Phe Thr Tyr Asp Gln Tyr
65                  70                  75                  80
Gln Glu Lys Asn Val Asp Gln Ala Ser Gly Ser Val Leu Leu His Leu

85                  90                  95
Glu Val Gly Asp Gln Val Trp Leu Gln Val Tyr Gly Asp Gly Asp His
            100                 105                 110
Asn Gly Leu Tyr Ala Asp Asn Val Asn Asp Ser Thr Phe Thr Gly Phe
        115                 120                 125
Leu Leu Tyr His Asp Thr Asn
    130                 135

<210> 45
<211> 135
<212> PRT
<213> Unknown

<220>
<223> antibody sequence

<400> 45
Met Tyr Arg Ser Ala Phe Ser Val Gly Leu Glu Thr Arg Val Thr Val
1               5                   10                  15
Pro Asn Val Pro Ile Arg Phe Thr Lys Ile Phe Tyr Asn Gln Gln Asn
            20                  25                  30
His Tyr Asp Gly Ser Thr Gly Lys Phe Tyr Cys Asn Ile Pro Gly Leu
        35                  40                  45
Tyr Tyr Phe Ser Tyr His Ile Thr Val Tyr Met Lys Asp Val Lys Val
    50                  55                  60
Ser Leu Phe Lys Lys Asp Lys Ala Val Leu Phe Thr Tyr Asp Gln Tyr
65                  70                  75                  80
Gln Glu Lys Asn Val Asp Gln Ala Ser Gly Ser Val Leu Leu His Leu
                85                  90                  95
Glu Val Gly Asp Gln Val Trp Leu Gln Val Tyr Gly Asp Gly Asp His
            100                 105                 110
Asn Gly Leu Tyr Ala Asp Asn Val Asn Asp Ser Thr Phe Thr Gly Phe
        115                 120                 125
Leu Leu Tyr His Asp Thr Asn
    130                 135

<210> 46
<211> 135
<212> PRT
<213> Unknown

<220>
<223> antibody sequence

<400> 46
Met Tyr Arg Ser Ala Phe Ser Val Gly Leu Glu Thr Arg Val Thr Val
1               5                   10                  15
Pro Asn Val Pro Ile Arg Phe Thr Lys Ile Phe Tyr Asn Gln Gln Asn
            20                  25                  30
His Tyr Asp Gly Ser Thr Gly Lys Phe Tyr Cys Asn Ile Pro Gly Leu
        35                  40                  45
Tyr Tyr Phe Ser Tyr His Ile Thr Val Tyr Met Lys Asp Val Lys Val
    50                  55                  60
Ser Leu Phe Lys Lys Asp Lys Ala Val Leu Phe Thr Tyr Asp Gln Tyr
65                  70                  75                  80
Gln Glu Lys Asn Val Asp Gln Ala Ser Gly Ser Val Leu Leu His Leu

85                  90                  95
Glu Val Gly Asp Gln Val Trp Leu Gln Val Tyr Gly Asp Gly Asp His
            100                 105                 110
Asn Gly Leu Tyr Ala Asp Asn Val Asn Asp Ser Thr Phe Thr Gly Phe
        115                 120                 125
Leu Leu Tyr His Asp Thr Asn
    130                 135


<210> 47
<211> 135
<212> PRT
<213> Unknown

<220>
<223> antibody sequence

<400> 47
Met Tyr Arg Ser Ala Phe Ser Val Gly Leu Glu Thr Arg Val Thr Val
 1               5                  10                  15
Pro Asn Val Pro Ile Arg Phe Thr Lys Ile Phe Tyr Asn Gln Gln Asn
            20                  25                  30
His Tyr Asp Gly Ser Thr Gly Lys Phe Tyr Cys Asn Ile Pro Gly Leu
        35                  40                  45
Tyr Tyr Phe Ser Tyr His Ile Thr Val Tyr Met Lys Asp Val Lys Val
    50                  55                  60
Ser Leu Phe Lys Lys Asp Lys Ala Val Leu Phe Thr Tyr Asp Gln Tyr
65                  70                  75                  80
Gln Glu Lys Asn Val Asp Gln Ala Ser Gly Ser Val Leu Leu His Leu
                85                  90                  95
Glu Val Gly Asp Gln Val Trp Leu Gln Val Tyr Gly Asp Gly Asp His
            100                 105                 110
Asn Gly Leu Tyr Ala Asp Asn Val Asn Asp Ser Thr Phe Thr Gly Phe
        115                 120                 125
Leu Leu Tyr His Asp Thr Asn
    130                 135


<210> 48
<211> 217
<212> PRT
<213> Unknown

<220>
<223> antibody sequence

<400> 48
Ala Val Gln Leu Glu Gln Ser Gly Pro Gly Leu Val Arg Pro Ser Gln
 1               5                  10                  15
Thr Leu Ser Leu Thr Cys Thr Val Ser Gly Thr Ser Phe Asp Asp Tyr
            20                  25                  30
Tyr Trp Thr Trp Val Arg Gln Pro Pro Gly Arg Gly Leu Glu Trp Ile
        35                  40                  45
Gly Tyr Val Phe Tyr Thr Gly Thr Thr Leu Leu Asp Pro Ser Leu Arg
    50                  55                  60
Gly Arg Val Thr Met Leu Val Asn Thr Ser Lys Asn Gln Phe Ser Leu
65                  70                  75                  80
Arg Leu Ser Ser Val Thr Ala Ala Asp Thr Ala Val Tyr Tyr Cys Ala

                85                  90                  95
Arg Asn Leu Ile Ala Gly Gly Ile Asp Val Trp Gly Gln Gly Ser Leu
            100                 105                 110
Val Thr Val Ser Ser Ala Ser Thr Lys Gly Pro Ser Val Phe Pro Leu
        115                 120                 125
Ala Pro Ser Ser Lys Ser Thr Ser Gly Gly Thr Ala Ala Leu Gly Cys
    130                 135                 140
Leu Val Lys Asp Tyr Phe Pro Glu Pro Val Thr Val Ser Trp Asn Ser
145                 150                 155                 160
Gly Ala Leu Thr Ser Gly Val His Thr Phe Pro Ala Val Leu Gln Ser
                165                 170                 175
Ser Gly Leu Tyr Ser Leu Ser Ser Val Val Thr Val Pro Ser Ser Ser
            180                 185                 190
Leu Gly Thr Gln Thr Tyr Ile Cys Asn Val Asn His Lys Pro Ser Asn
        195                 200                 205
Thr Lys Val Asp Lys Lys Val Glu Pro
    210                 215

<210> 49
<211> 225
<212> PRT
<213> Unknown

<220>
<223> antibody sequence

<400> 49
Glu Val Gln Leu Val Glu Ser Gly Gly Gly Leu Val Gln Pro Gly Arg
1               5                   10                  15
Ser Leu Arg Leu Ser Cys Val Thr Ser Gly Phe Thr Phe Asp Asp Tyr
            20                  25                  30
Ala Met His Trp Val Arg Gln Ser Pro Gly Lys Gly Leu Glu Trp Val
        35                  40                  45
Ser Gly Ile Ser Trp Asn Thr Gly Thr Ile Ile Tyr Ala Asp Ser Val
    50                  55                  60
Lys Gly Arg Phe Ile Ile Ser Arg Asp Asn Ala Lys Asn Ser Leu Tyr
65                  70                  75                  80
Leu Gln Met Asn Ser Leu Arg Val Glu Asp Thr Ala Leu Tyr Tyr Cys
                85                  90                  95
Ala Lys Thr Arg Ser Tyr Val Val Ala Ala Glu Tyr Tyr Phe His Tyr
            100                 105                 110
Trp Gly Gln Gly Ile Leu Val Thr Val Ser Ser Gly Ser Ala Ser Ala
        115                 120                 125
Pro Thr Leu Phe Pro Leu Val Ser Cys Glu Asn Ser Asn Pro Ser Ser
    130                 135                 140
Thr Val Ala Val Gly Cys Leu Ala Gln Asp Phe Leu Pro Asp Ser Ile
145                 150                 155                 160
Thr Phe Ser Trp Lys Tyr Lys Asn Asn Ser Asp Ile Ser Ser Thr Arg
                165                 170                 175
Gly Phe Pro Ser Val Leu Arg Gly Gly Lys Tyr Ala Ala Thr Ser Gln
            180                 185                 190
Val Leu Leu Pro Ser Lys Asp Val Met Gln Gly Thr Asn Glu His Val
        195                 200                 205
Val Cys Lys Val Gln His Pro Asn Gly Asn Lys Glu Lys Asp Val Pro
    210                 215                 220
Leu
225

<210> 50
<211> 148
<212> PRT
<213> Unknown

<220>
<223> antibody sequence

<400> 50
Asp Val Gln Leu Gln Ala Ser Gly Gly Gly Ser Val Gln Ala Gly Gly
1               5                   10                  15
Ser Leu Arg Leu Ser Cys Ala Ala Ser Gly Tyr Thr Ile Gly Pro Tyr
            20                  25                  30
Cys Met Gly Trp Phe Arg Gln Ala Pro Gly Lys Glu Arg Glu Gly Val
        35                  40                  45
Ala Ala Ile Asn Met Gly Gly Gly Ile Thr Tyr Tyr Ala Asp Ser Val
    50                  55                  60
Lys Gly Arg Phe Thr Ile Ser Gln Asp Asn Ala Lys Asn Thr Val Tyr
65                  70                  75                  80
Leu Leu Met Asn Ser Leu Glu Pro Glu Asp Thr Ala Ile Tyr Tyr Cys
                85                  90                  95
Ala Ala Asp Ser Thr Ile Tyr Ala Ser Tyr Tyr Glu Cys Gly His Gly
            100                 105                 110
Leu Ser Thr Gly Gly Tyr Gly Tyr Asp Ser Trp Gly Gln Gly Thr Gln
        115                 120                 125
Val Thr Val Ser Ser Gly Arg Tyr Pro Tyr Asp Val Pro Asp Tyr Gly
    130                 135                 140
Ser Gly Arg Ala
145

<210> 51
<211> 216
<212> PRT
<213> Unknown

<220>
<223> antibody sequence

<400> 51
Asp Val Gln Leu Gln Gln Ser Gly Pro Glu Leu Glu Lys Pro Gly Ala
1               5                   10                  15
Ser Val Lys Ile Ser Cys Lys Ala Ser Gly Phe Ser Leu Pro Gly His
            20                  25                  30
Asn Ile Asn Trp Ile Val Gln Arg Asn Gly Lys Ser Leu Glu Trp Ile
        35                  40                  45
Gly Asn Ile Asp Pro Tyr Tyr Gly Gly Thr Asn Phe Asn Pro Lys Phe
    50                  55                  60
Lys Gly Lys Ala Thr Leu Thr Val Asp Lys Ser Ser Ser Thr Leu Tyr
65                  70                  75                  80
Met His Leu Thr Ser Leu Gln Ser Glu Asp Ser Ala Val Tyr Tyr Cys
                85                  90                  95
Ala Arg Arg Arg Asp Gly Asn Tyr Gly Phe Thr Tyr Trp Gly Gln Gly
            100                 105                 110
Thr Leu Val Thr Val Ser Ala Ala Lys Thr Thr Pro Pro Ser Val Tyr
        115                 120                 125

Pro Leu Ala Pro Gly Ser Ala Ala Gln Thr Asn Ser Met Val Thr Leu
    130                 135                 140
Gly Cys Leu Val Lys Gly Tyr Phe Pro Glu Pro Val Thr Val Thr Trp
145                 150                 155                 160
Asn Ser Gly Ser Leu Ser Ser Gly Val His Thr Phe Pro Ala Val Leu
                165                 170                 175
Gln Ser Asp Leu Tyr Thr Leu Ser Ser Ser Val Thr Val Pro Ser Ser
            180                 185                 190
Thr Trp Pro Ser Glu Thr Val Thr Cys Asn Val Ala His Pro Ala Ser
        195                 200                 205
Ser Thr Lys Val Asp Lys Lys Ile
    210                 215

<210> 52
<211> 97
<212> PRT
<213> Unknown

<220>
<223> antibody sequence

<400> 52
Gln Asp Thr Leu Lys Glu Tyr Gly Pro Gly Lys Leu Pro Ser Gln Thr
 1               5                  10                  15
Phe Ser Leu Thr Cys Thr Phe Ser Gly Phe Ser Leu Ser Thr Tyr Gly
            20                  25                  30
Met Met Val Ser Trp Met Cys Gln Pro Ser Gly Lys Gly Leu Val Trp
        35                  40                  45
Leu Ala Leu Ile Trp Cys Asn Asn Asp Lys Gly Tyr Asn Pro Phe Leu
    50                  55                  60
Arg Ser Gln Leu Thr Ile Ser Lys Asp Thr Ser Asn Asn Gln Val Phe
65                  70                  75                  80
Leu Lys Ile Thr Ser Val Asp Pro Ala Asp Thr Ala Thr Tyr Tyr Cys
                85                  90                  95
Ala

<210> 53
<211> 101
<212> PRT
<213> Unknown

<220>
<223> antibody sequence

<400> 53
Gln Val Gln Leu Gln Gln Ser Gly Pro Gly Leu Val Lys Pro Ser Gln
 1               5                  10                  15
Thr Leu Ser Leu Thr Cys Ala Ile Ser Gly Asp Ser Val Ser Ser Asn
            20                  25                  30
Ser Ala Ala Trp Asn Trp Ile Arg Gln Ser Pro Ser Arg Gly Leu Glu
        35                  40                  45
Trp Leu Gly Arg Thr Tyr Tyr Arg Ser Lys Trp Tyr Asn Asp Tyr Ala
    50                  55                  60
Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95
Tyr Tyr Cys Ala Arg
            100

<210> 54
<211> 210
<212> PRT
<213> Unknown

<220>
<223> antibody sequence

<400> 54
Asp Ile Gln Met Thr Gln Ser Pro Ala Ile Met Ser Ala Ser Pro Gly
 1               5                  10                  15
Glu Lys Val Thr Met Thr Cys Ser Ala Ser Ser Ser Val Ser Tyr Met
            20                  25                  30
Tyr Trp Tyr Gln Gln Lys Pro Gly Ser Ser Pro Arg Leu Leu Ile Tyr
        35                  40                  45
Asp Ser Thr Asn Leu Ala Ser Gly Val Pro Val Arg Phe Ser Gly Ser
    50                  55                  60
Gly Ser Gly Thr Ser Tyr Ser Leu Thr Ile Ser Arg Met Glu Ala Glu
65                  70                  75                  80
Asp Ala Ala Thr Tyr Tyr Cys Gln Gln Trp Ser Thr Tyr Pro Leu Thr
                85                  90                  95
Phe Gly Ala Gly Thr Lys Leu Glu Leu Lys Arg Ala Asp Ala Ala Pro
            100                 105                 110
Thr Val Ser Ile Phe Pro Pro Ser Ser Glu Gln Leu Thr Ser Gly Gly
        115                 120                 125
Ala Ser Val Val Cys Phe Leu Asn Asn Phe Tyr Pro Lys Asp Ile Asn
    130                 135                 140
Val Lys Trp Lys Ile Asp Gly Ser Glu Arg Gln Asn Gly Val Leu Asn
145                 150                 155                 160
Ser Trp Thr Asp Gln Asp Ser Lys Asp Ser Thr Tyr Ser Met Ser Ser
                165                 170                 175
Thr Leu Thr Leu Thr Lys Asp Glu Tyr Glu Arg His Asn Ser Tyr Thr
            180                 185                 190
Cys Glu Ala Thr His Lys Thr Ser Thr Ser Pro Ile Val Lys Ser Phe
        195                 200                 205
Asn Arg
    210

<210> 55
<211> 210
<212> PRT
<213> Unknown

<220>
<223> antibody sequence

<400> 55
Gln Ile Val Ser Thr Gln Ser Pro Ala Ile Met Ser Ala Ser Pro Gly
 1               5                  10                  15
Glu Lys Val Thr Met Thr Cys Ser Ala Ser Ser Ser Arg Ser Tyr Met
            20                  25                  30

Gln Trp Tyr Gln Gln Lys Pro Gly Thr Ser Pro Lys Arg Trp Ile Tyr
            35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys His Gln Arg Ser Ser Tyr Thr Phe Gly
                85                  90                  95
Gly Gly Thr Lys Leu Glu Ile Lys Arg Thr Val Ala Ala Pro Ser Val
            100                 105                 110
Phe Ile Phe Pro Pro Ser Asp Glu Gln Leu Lys Ser Gly Thr Ala Ser
        115                 120                 125
Val Val Cys Leu Leu Asn Asn Phe Tyr Pro Arg Glu Ala Lys Val Gln
    130                 135                 140
Trp Lys Val Asp Asn Ala Leu Gln Ser Gly Asn Ser Gln Glu Ser Val
145                 150                 155                 160
Thr Glu Gln Asp Ser Lys Asp Ser Thr Tyr Ser Leu Ser Ser Thr Leu
                165                 170                 175
Thr Leu Ser Lys Ala Asp Tyr Glu Lys His Lys Val Tyr Ala Cys Glu
            180                 185                 190
Val Thr His Gln Gly Leu Ser Ser Pro Val Thr Lys Ser Phe Asn Arg
        195                 200                 205
Gly Glu
    210


<210> 56
<211> 214
<212> PRT
<213> Unknown

<220>
<223> antibody sequence

<400> 56
Glu Leu Val Met Thr Gln Ser Pro Ser Ser Leu Ser Ala Ser Val Gly
 1               5                  10                  15
Asp Arg Val Asn Ile Ala Cys Arg Ala Ser Gln Gly Ile Ser Ser Ala
            20                  25                  30
Leu Ala Trp Tyr Gln Gln Lys Pro Gly Lys Ala Pro Arg Leu Leu Ile
        35                  40                  45
Tyr Asp Ala Ser Asn Leu Glu Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Ile Tyr Tyr Cys Gln Gln Phe Asn Ser Tyr Pro Leu
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr

                180                 185                 190
Ala Cys Glu Val Thr His Gln Gly Leu Ser Ser Pro Val Thr Lys Ser
        195                 200                 205
Phe Asn Arg Gly Glu Cys
    210



<210> 57
<211> 214
<212> PRT
<213> Unknown

<220>
<223> antibody sequence

<400> 57
Glu Leu Val Met Thr Gln Ser Pro Ser Ser Leu Ser Ala Ser Val Gly
 1               5                  10                  15
Asp Arg Val Asn Ile Ala Cys Arg Ala Ser Gln Gly Ile Ser Ser Ala
            20                  25                  30
Leu Ala Trp Tyr Gln Gln Lys Pro Gly Lys Ala Pro Arg Leu Leu Ile
        35                  40                  45
Tyr Asp Ala Ser Asn Leu Glu Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Ile Tyr Tyr Cys Gln Gln Phe Asn Ser Tyr Pro Leu
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210



<210> 58
<211> 107
<212> PRT
<213> Unknown

<220>
<223> antibody sequence

<400> 58
Glu Ile Val Met Thr Gln Ser Pro Ala Ser Leu Ser Leu Ser Pro Gly
 1               5                  10                  15
Glu Arg Ala Thr Leu Ser Cys Arg Ala Ser Gln Ser Val Ser Asn Tyr

20                  25                  30
Leu Ala Trp Tyr Gln Gln Lys Pro Gly Gln Ala Pro Arg Leu Leu Ile
        35                  40                  45
His Asp Ala Ser Gly Arg Ala Thr Gly Ile Pro Asp Arg Phe Ser Gly
    50                  55                  60
Ser Thr Asp Phe Thr Leu Thr Ile Ser Arg Leu Glu Pro Glu Asp Phe
65                  70                  75                  80
Ala Val Tyr Tyr Cys Gln Gln Arg Ala Asn Trp Gly Thr Trp Thr Phe
                85                  90                  95
Gly Gln Gly Thr Lys Val Glu Ile Lys Arg Thr
            100                 105

<210> 59
<211> 215
<212> PRT
<213> Unknown

<220>
<223> antibody sequence

<400> 59
Glu Ile Val Leu Thr Gln Ser Pro Ala Thr Leu Ser Leu Ser Pro Gly
 1               5                  10                  15
Glu Arg Ala Thr Leu Ser Cys Gly Ala Ser Gln Ser Val Ser Ser Asn
            20                  25                  30
Tyr Leu Ala Trp Tyr Gln Gln Lys Pro Gly Gln Ala Pro Arg Leu Leu
        35                  40                  45
Ile Tyr Asp Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Pro
                85                  90                  95
Leu Thr Phe Gly Gly Gly Thr Lys Val Glu Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210> 60
<211> 210
<212> PRT
<213> Unknown

<220>

<223> antibody sequence

<400> 60
Asp Ile Gln Met Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
Tyr Trp Tyr Gln Gln Lys Pro Gly Ser Ser Pro Arg Leu Leu Ile Tyr
        35                  40                  45
Asp Ser Thr Asn Leu Ala Ser Gly Val Pro Val Arg Phe Ser Gly Ser
    50                  55                  60
Gly Ser Gly Thr Ser Tyr Ser Leu Thr Ile Ser Arg Met Glu Ala Glu
65                  70                  75                  80
Asp Ala Ala Thr Tyr Tyr Cys Gln Gln Trp Ser Thr Tyr Pro Leu Thr
                85                  90                  95
Phe Gly Ala Gly Thr Lys Leu Glu Leu Lys Arg Ala Asp Ala Ala Pro
            100                 105                 110
Thr Val Ser Ile Phe Pro Pro Ser Ser Glu Gln Leu Thr Ser Gly Gly
        115                 120                 125
Ala Ser Val Val Cys Phe Leu Asn Asn Phe Tyr Pro Lys Asp Ile Asn
    130                 135                 140
Val Lys Trp Lys Ile Asp Gly Ser Glu Arg Gln Asn Gly Val Leu Asn
145                 150                 155                 160
Ser Trp Thr Asp Gln Asp Ser Lys Asp Ser Thr Tyr Ser Met Ser Ser
                165                 170                 175
Thr Leu Thr Leu Thr Lys Asp Glu Tyr Glu Arg His Asn Ser Tyr Thr
            180                 185                 190
Cys Glu Ala Thr His Lys Thr Ser Thr Ser Pro Ile Val Lys Ser Phe
        195                 200                 205
Asn Arg
    210


<210> 61
<211> 27
<212> DNA
<213> Artificial Sequence

<220>
<223> synthetic oligonucleotide

<400> 61
gagcgccgtg cacaggtgca gctgttg                                          27

<210> 62
<211> 33
<212> DNA
<213> Artificial Sequence

<220>
<223> synthetic oligonucleotide

<400> 62
gagtcgactt gcggccgcgc tcgagacggt gac                                   33

<210> 63
<211> 35
<212> DNA

<213> Artificial Sequence

<220>
<223> synthetic oligonucleotide

<400> 63
gagcgccgtg cacagatcca gatgacccag tctcc                                35

<210> 64
<211> 38
<212> DNA
<213> Artificial Sequence

<220>
<223> synthetic oligonucleotide

<400> 64
gagtcgactt gcggccgccc gtttgatttc caccttgg                             38

<210> 65
<211> 65
<212> DNA
<213> Artificial Sequence

<220>
<223> synthetic oligonucleotide

<400> 65
tgcacaggtc cactgcagga ggcggccgca gaacaaaaac tcatctcaga agaggatctg 60
aattc                                                                 65

<210> 66
<211> 65
<212> DNA
<213> Artificial Sequence

<220>
<223> synthetic oligonucleotide

<400> 66
ggccgaattc agatcctctt ctgagatgag tttttgttct gcggccgcga ggacgtcacc 60
tgctg                                                                 65

<210> 67
<211> 36
<212> DNA
<213> Artificial Sequence

<220>
<223> primer

<400> 67
gagtcgactt gcggccgcgc tcgagacggt gaccag                               36

<210> 68
<211> 36
<212> DNA
<213> Artificial Sequence

<220>
<223> primer

<400> 68
gagcgccgtg cacaggtgca gctgttggag tctggg                                36

<210> 69
<211> 24
<212> DNA
<213> Artificial Sequence

<220>
<223> primer

<400> 69
atggttgttg tcattgtcgg cgca                                             24

<210> 70
<211> 24
<212> DNA
<213> Artificial Sequence

<220>
<223> primer

<400> 70
atgaggtttt gctaaacaac tttc                                             24

<210> 71
<211> 35
<212> DNA
<213> Artificial Sequence

<220>
<223> primer

<400> 71
gagcgccgtg cacagatcca gatgacccag tctcc                                 35

<210> 72
<211> 38
<212> DNA
<213> Artificial Sequence

<220>
<223> primer

<400> 72
gagtcgactt gcggccgccc gtttgatttc caccttgg                              38

<210> 73
<211> 27
<212> DNA
<213> Artificial Sequence

<220>
<223> primer

<400> 73
gagcgccgtg cacaggtgca gctgttg                                          27

<210> 74
<211> 33
<212> DNA
<213> Artificial Sequence

<220>
<223> primer

<400> 74
gagtcgactt gcggccgcgc tcgagacggt gac                                   33

<210> 75
<211> 35
<212> DNA
<213> Artificial Sequence

<220>
<223> oligonucleotide

<221> misc_feature
<222> 19, 20
<223> n = A,T,C or G

<221> misc_feature
<222> 19, 20
<223> n = A,T,C or G

<400> 75
gcagcataga tcaggagknn aggggctttc cctgg                                 35

<210> 76
<211> 34
<212> DNA
<213> Artificial Sequence

<220>
<223> oligonucleotide

<221> misc_feature
<222> 21, 22
<223> n = A,T,C or G

<221> misc_feature
<222> 21, 22
<223> n = A,T,C or G

<400> 76
gcaaactgga tgcagcatak nncaggagct tagg                                  34

<210> 77
<211> 34
<212> DNA
<213> Artificial Sequence

<220>
<223> oligonucleotide

<221> misc_feature
<222> 18, 19
<223> n = A,T,C or G

<221> misc_feature
<222> 18, 19
<223> n = A,T,C or G

<400> 77
gcaaactgga tgcagcknng atcaggagct tagg                                34

<210> 78
<211> 34
<212> DNA
<213> Artificial Sequence

<220>
<223> oligonucleotide

<221> misc_feature
<222> 15, 16
<223> n = A,T,C or G

<221> misc_feature
<222> 15, 16
<223> n = A,T,C or G

<400> 78
gcaaactgga tgcknnatag atcaggagct tagg                                34

<210> 79
<211> 31
<212> DNA
<213> Artificial Sequence

<220>
<223> oligonucleotide

<221> misc_feature
<222> 13, 14
<223> n = A,T,C or G

<221> misc_feature
<222> 13, 14
<223> n = A,T,C or G

<400> 79
ttcactctca ccnnkagcag tctgcaacct g                                   31

<210> 80
<211> 28
<212> DNA
<213> Artificial Sequence

<220>

<223> oligonucleotide

<400> 80
ccggaggctg cacaggagag acgcaggg                                          28

<210> 81
<211> 53
<212> DNA
<213> Artificial Sequence

<220>
<223> oligonucleotide

<221> misc_feature
<222> 30, 31
<223> n = A,T,C or G

<221> misc_feature
<222> 30, 31
<223> n = A,T,C or G

<400> 81
ccctgcgtct ctcctgtgca gcctccggan nkacctttag cagctatgcc atg              53

<210> 82
<211> 28
<212> DNA
<213> Artificial Sequence

<220>
<223> oligonucleotide

<400> 82
gaatccggag gctgcacagg agagacgc                                          28

<210> 83
<211> 53
<212> DNA
<213> Artificial Sequence

<220>
<223> oligonucleotide

<221> misc_feature
<222> 28, 29
<223> n = A,T,C or G

<221> misc_feature
<222> 28, 29
<223> n = A,T,C or G

<400> 83
ctctcctgtg cagcctccgg attcaccnnk agcagctatg ccatgagctg ggc              53

<210> 84
<211> 53
<212> DNA
<213> Artificial Sequence

<220>
<223> oligonucleotide

<221> misc_feature
<222> 27, 28
<223> n = A,T,C or G

<221> misc_feature
<222> 27, 28
<223> n = A,T,C or G

<400> 84
ggcggaccca gctcatggca tagctmnnaa aggtgaatcc ggaggctgca cag        53

<210> 85
<211> 27
<212> DNA
<213> Artificial Sequence

<220>
<223> oligonucleotide

<400> 85
atgccatgag ctgggtccgc caggctc                                      27

<210> 86
<211> 58
<212> DNA
<213> Artificial Sequence

<220>
<223> oligonucleotide

<221> misc_feature
<222> 31, 32
<223> n = A,T,C or G

<221> misc_feature
<222> 31, 32
<223> n = A,T,C or G

<400> 86
ggagcctggc ggacccagct catggcatam nngctaaagg tgaatccgga ggctgcac   58

<210> 87
<211> 30
<212> DNA
<213> Artificial Sequence

<220>
<223> oligonucleotide

<400> 87
atgccatgag ctgggtccgc caggctccag                                   30

<210> 88
<211> 28

<212> DNA
<213> Artificial Sequence

<220>
<223> oligonucleotide

<400> 88
gctaaaggtg aatccggagg ctgcacag                                          28

<210> 89
<211> 55
<212> DNA
<213> Artificial Sequence

<220>
<223> oligonucleotide

<221> misc_feature
<222> 28, 29
<223> n = A,T,C or G

<221> misc_feature
<222> 28, 29
<223> n = A,T,C or G

<400> 89
gcagcctccg gattcacctt tagcagcnnk gccatgagct gggtccgcca ggctc          55

<210> 90
<211> 27
<212> DNA
<213> Artificial Sequence

<220>
<223> oligonucleotide

<400> 90
agctgctaaa ggtgaatccg gaggctg                                           27

<210> 91
<211> 52
<212> DNA
<213> Artificial Sequence

<220>
<223> oligonucleotide

<221> misc_feature
<222> 24, 25
<223> n = A,T,C or G

<221> misc_feature
<222> 24, 25
<223> n = A,T,C or G

<400> 91
ccggattcac ctttagcagc tatnnkatga gctgggtccg ccaggctcca gg             52

<210> 92
<211> 58
<212> DNA
<213> Artificial Sequence

<220>
<223> oligonucleotide

<221> misc_feature
<222> 31, 32
<223> n = A,T,C or G

<221> misc_feature
<222> 31, 32
<223> n = A,T,C or G

<400> 92
agacccttcc ctggagcctg gcggacccam nncatggcat agctgctaaa ggtgaatc   58

<210> 93
<211> 30
<212> DNA
<213> Artificial Sequence

<220>
<223> oligonucleotide

<400> 93
gggtccgcca ggctccaggg aagggtctag                                  30

<210> 94
<211> 26
<212> DNA
<213> Artificial Sequence

<220>
<223> oligonucleotide

<400> 94
accactaata gctgagaccc actcta                                      26

<210> 95
<211> 58
<212> DNA
<213> Artificial Sequence

<220>
<223> oligonucleotide

<221> misc_feature
<222> 29, 30
<223> n = A,T,C or G

<221> misc_feature
<222> 29, 30
<223> n = A,T,C or G

<400> 95

agagtgggtc tcagctatta gtggtagtnn kggtagcaca tactacgcag actccgtg 58

<210> 96
<211> 28
<212> DNA
<213> Artificial Sequence

<220>
<223> oligonucleotide

<400> 96
cgcaggctgt tcatttgcag atacagcg 28

<210> 97
<211> 55
<212> DNA
<213> Artificial Sequence

<220>
<223> oligonucleotide

<221> misc_feature
<222> 26, 27
<223> n = A,T,C or G

<221> misc_feature
<222> 26, 27
<223> n = A,T,C or G

<400> 97
gtatctgcaa atgaacagcc tgcgannkga ggacaccgcg gtatattact gtgcg 55

<210> 98
<211> 26
<212> DNA
<213> Artificial Sequence

<220>
<223> oligonucleotide

<400> 98
gcacgcaggc tgttcatttg cagata 26

<210> 99
<211> 51
<212> DNA
<213> Artificial Sequence

<220>
<223> oligonucleotide

<221> misc_feature
<222> 25, 26
<223> n = A,T,C or G

<221> misc_feature
<222> 25, 26
<223> n = A,T,C or G

<400> 99
ctgcaaatga acagcctgcg tgcannkgac accgcggtat attactgtgc g          51

<210> 100
<211> 27
<212> DNA
<213> Artificial Sequence

<220>
<223> oligonucleotide

<400> 100
acgcgtcgac gcaggtgcag ctgttgg                                     27

<210> 101
<211> 33
<212> DNA
<213> Artificial Sequence

<220>
<223> oligonucleotide

<400> 101
ttaggatcct tattagctcg agacggtgac cag                              33

<210> 102
<211> 29
<212> DNA
<213> Artificial Sequence

<220>
<223> oligonucleotide

<400> 102
acgcgtcgac gcagatccag atgacccag                                   29

<210> 103
<211> 35
<212> DNA
<213> Artificial Sequence

<220>
<223> oligonucleotide

<400> 103
ttaggatcct tattaccgtt tgatttccac cttgg                            35

<210> 104
<211> 10
<212> PRT
<213> Homo Sapiens

<400> 104
Gly Phe Thr Phe Ser Ser Tyr Ala Met Ser
1               5                   10

<210> 105
<211> 17
<212> PRT
<213> Homo Sapiens

<400> 105
Ala Ile Ser Gly Ser Gly Gly Ser Thr Tyr Tyr Ala Asp Ser Val Lys
1               5                   10                  15
Gly

<210> 106
<211> 8
<212> PRT
<213> Homo Sapiens

<400> 106
Lys Ser Tyr Gly Ala Phe Asp Tyr
1               5

<210> 107
<211> 10
<212> PRT
<213> Homo Sapiens

<400> 107
Gly Phe Arg Ile Ser Asp Glu Asp Met Gly
1               5                   10

<210> 108
<211> 17
<212> PRT
<213> Homo Sapiens

<400> 108
Ser Ile Tyr Gly Pro Ser Gly Ser Thr Tyr Tyr Ala Asp Ser Val Lys
1               5                   10                  15
Gly

<210> 109
<211> 12
<212> PRT
<213> Homo Sapiens

<400> 109
Ser Ala Leu Glu Pro Leu Ser Glu Pro Leu Gly Phe
1               5                   10

<210> 110
<211> 10
<212> PRT
<213> Homo Sapiens

<400> 110
Gly Asp Met Val Asn Asp Lys Asp Met Ser
1               5                   10


<210> 111
<211> 17
<212> PRT
<213> Homo Sapiens

<400> 111
Ser Ile Ser Thr Glu Asn Gly Ser Thr Tyr Tyr Ala Asp Ser Val Lys
1               5                   10                  15
Gly



<210> 112
<211> 16
<212> PRT
<213> Homo Sapiens

<400> 112
Gly Val Arg Asp Glu Val Ala Met Gly Glu Asn Pro Asp Leu Ser Tyr
1               5                   10                  15


<210> 113
<211> 10
<212> PRT
<213> Homo Sapiens

<400> 113
Gly Phe Arg Phe Ser Ala Glu Asp Met Gly
1               5                   10


<210> 114
<211> 17
<212> PRT
<213> Homo Sapiens

<400> 114
Ser Ile Asp Asn Asp Asp Gly Ser Thr Tyr Tyr Ala Asp Ser Val Lys
1               5                   10                  15
Gly



<210> 115
<211> 16
<212> PRT
<213> Homo Sapiens

<400> 115
Ser Ser Pro Gly Pro Asp Asn Glu Lys Asp Asn Ala Ser Leu Lys Ser
1               5                   10                  15

<210> 116
<211> 10
<212> PRT
<213> Homo Sapiens

<400> 116
Gly Asp Ser Val Ser Asn Lys Val Met Gly
 1               5                   10


<210> 117
<211> 17
<212> PRT
<213> Homo Sapiens

<400> 117
Ala Ile Asp Thr Lys Asp Gly Ser Thr Tyr Tyr Ala Asp Ser Val Lys
 1               5                   10                  15
Gly



<210> 118
<211> 16
<212> PRT
<213> Homo Sapiens

<400> 118
Ser Gly Asp Val Asp Ala Asp Met Ala Trp Glu Glu Glu Val Ser Ser
 1               5                   10                  15


<210> 119
<211> 10
<212> PRT
<213> Homo Sapiens

<400> 119
Gly Asp Thr Leu Thr Ser Asp Asn Met Ala
 1               5                   10


<210> 120
<211> 17
<212> PRT
<213> Homo Sapiens

<400> 120
Thr Ile Thr Glu Ala Gly Gly Ser Thr Tyr Tyr Ala Asp Ser Val Lys
 1               5                   10                  15
Gly



<210> 121
<211> 15

<212> PRT
<213> Homo Sapiens

<400> 121
Thr Tyr Pro Ala Asp Val Ala Glu Cys Ala Ala Glu Val Cys Tyr
1               5                   10                  15


<210> 122
<211> 10
<212> PRT
<213> Homo Sapiens

<400> 122
Gly Val Asn Val Ser His Asp Ser Met Thr
1               5                   10


<210> 123
<211> 17
<212> PRT
<213> Homo Sapiens

<400> 123
Ala Ile Arg Gly Pro Asn Gly Ser Thr Tyr Tyr Ala Asp Ser Val Lys
1               5                   10                  15
Gly



<210> 124
<211> 19
<212> PRT
<213> Homo Sapiens

<400> 124
Ser Gly Ala Arg His Ala Asp Thr Glu Arg Pro Pro Ser Gln Gln Thr
1               5                   10                  15
Met Pro Phe



<210> 125
<211> 10
<212> PRT
<213> Homo Sapiens

<400> 125
Gly Tyr Arg Ile Ser Pro Asp Tyr Met Gly
1               5                   10


<210> 126
<211> 17
<212> PRT
<213> Homo Sapiens

<400> 126

Ser Ile Ser Asn Asn Gly Gly Ser Thr Tyr Tyr Ala Asp Ser Val Lys
1               5                   10                  15
Gly

<210> 127
<211> 15
<212> PRT
<213> Homo Sapiens

<400> 127
Ser Val Asp Ala Ala Glu Ser Gly Ile Asp Ser Asn Val Gly Ser
1               5                   10                  15

<210> 128
<211> 10
<212> PRT
<213> Homo Sapiens

<400> 128
Gly Tyr Arg Val Asn Ala Gln Asp Met Ser
1               5                   10

<210> 129
<211> 17
<212> PRT
<213> Homo Sapiens

<400> 129
Thr Ile Glu Asn Glu Asn Gly Ser Thr Tyr Tyr Ala Asp Ser Val Lys
1               5                   10                  15
Gly

<210> 130
<211> 13
<212> PRT
<213> Homo Sapiens

<400> 130
Ser Cys Thr Arg Gly Gly Cys Tyr Asp Thr Phe Pro Tyr
1               5                   10

<210> 131
<211> 10
<212> PRT
<213> Homo Sapiens

<400> 131
Gly Tyr Asn Ile Thr Asp Glu Asn Met Ala
1               5                   10

<210> 132
<211> 17
<212> PRT
<213> Homo Sapiens

<400> 132
Thr Ile Ala Ala Asp Asn Gly Ser Thr Tyr Tyr Ala Asp Ser Val Lys
1               5                   10                  15
Gly

<210> 133
<211> 14
<212> PRT
<213> Homo Sapiens

<400> 133
Thr Thr Glu Ala Ala Gly Val Glu Glu Asp Asn Val Arg Ser
1               5                   10

<210> 134
<211> 10
<212> PRT
<213> Homo Sapiens

<400> 134
Gly Asp Lys Val Ser Tyr Asn Asn Met Ala
1               5                   10

<210> 135
<211> 17
<212> PRT
<213> Homo Sapiens

<400> 135
Ser Ile Thr Thr Glu Asn Gly Ser Thr Tyr Tyr Ala Asp Ser Val Lys
1               5                   10                  15
Gly

<210> 136
<211> 19
<212> PRT
<213> Homo Sapiens

<400> 136
Gly Asn Arg Asn Ser Pro Val Asp Tyr Arg Glu Leu Gln Ser Thr Pro
1               5                   10                  15
Leu Asp Ser

<210> 137
<211> 10

<212> PRT
<213> Homo Sapiens

<400> 137
Gly Asp Asn Phe Asn Asn Glu Asn Met Gly
1               5                   10


<210> 138
<211> 17
<212> PRT
<213> Homo Sapiens

<400> 138
Thr Ile Ser Asp Thr Asn Gly Ser Thr Tyr Tyr Ala Asp Ser Val Lys
1               5                   10                  15
Gly


<210> 139
<211> 13
<212> PRT
<213> Homo Sapiens

<400> 139
Thr Thr Gly Thr Arg Gln Pro Gln Lys Glu Val Gly Ser
1               5                   10


<210> 140
<211> 10
<212> PRT
<213> Homo Sapiens

<400> 140
Gly Val Asn Val Thr Asp Gln Asp Met Gly
1               5                   10


<210> 141
<211> 17
<212> PRT
<213> Homo Sapiens

<400> 141
Ser Ile Arg Ser Asn Asp Gly Ser Thr Tyr Tyr Ala Asp Ser Val Lys
1               5                   10                  15
Gly


<210> 142
<211> 12
<212> PRT
<213> Homo Sapiens

<400> 142

Gly Arg Ser Ser Gly Arg Thr Asp Ala Val Pro Tyr
1               5                   10

<210> 143
<211> 10
<212> PRT
<213> Homo Sapiens

<400> 143
Gly Asp Ser Ile Ser Asp Asp Tyr Met Ala
1               5                   10

<210> 144
<211> 17
<212> PRT
<213> Homo Sapiens

<400> 144
Ser Ile Asp Asp Lys Asn Gly Ser Thr Tyr Tyr Ala Asp Ser Val Lys
1               5                   10                  15
Gly

<210> 145
<211> 11
<212> PRT
<213> Homo Sapiens

<400> 145
Gly Gly Asp Gly Gln Ala His Lys Val Asp Tyr
1               5                   10

<210> 146
<211> 10
<212> PRT
<213> Homo Sapiens

<400> 146
Gly Asp Met Leu Asn Tyr Lys Val Met Gly
1               5                   10

<210> 147
<211> 17
<212> PRT
<213> Homo Sapiens

<400> 147
Ser Ile Ile Thr Gln Asp Gly Ser Thr Tyr Tyr Ala Asp Ser Val Lys
1               5                   10                  15
Gly

<210> 148
<211> 12
<212> PRT
<213> Homo Sapiens

<400> 148
Gly Ile Pro Leu Asp Arg Ala Asp Asp Ile Glu Tyr
1               5                   10


<210> 149
<211> 10
<212> PRT
<213> Homo Sapiens

<400> 149
Gly Tyr Lys Val Asn Asp Asn Thr Met Ala
1               5                   10


<210> 150
<211> 17
<212> PRT
<213> Homo Sapiens

<400> 150
Ser Ile Asp Thr Thr Asp Gly Ser Thr Tyr Tyr Ala Asp Ser Val Lys
1               5                   10                  15
Gly



<210> 151
<211> 11
<212> PRT
<213> Homo Sapiens

<400> 151
Ala Ser Asp Gln Arg Thr Ala Asp Met Arg Ser
1               5                   10


<210> 152
<211> 10
<212> PRT
<213> Homo Sapiens

<400> 152
Gly Val Thr Val Ser Asp Glu Asn Met Gly
1               5                   10


<210> 153
<211> 17
<212> PRT
<213> Homo Sapiens

<400> 153

Gly Ile Ser Ser Asn Asp Gly Ser Thr Tyr Tyr Ala Asp Ser Val Lys
1               5                   10                  15
Gly

<210> 154
<211> 14
<212> PRT
<213> Homo Sapiens

<400> 154
Arg Asp Tyr Gly Ser Arg Val Asp Gln Gln His Leu Glu Ser
1               5                   10

<210> 155
<211> 10
<212> PRT
<213> Homo Sapiens

<400> 155
Gly Val Thr Leu Asn Asp Glu Tyr Met Gly
1               5                   10

<210> 156
<211> 17
<212> PRT
<213> Homo Sapiens

<400> 156
Ser Ile Asn Asp Arg Asn Gly Ser Thr Tyr Tyr Ala Asp Ser Val Lys
1               5                   10                  15
Gly

<210> 157
<211> 15
<212> PRT
<213> Homo Sapiens

<400> 157
Ser Trp Val Val Pro Gly Arg Lys Ser Ala Glu Pro Met Asp Tyr
1               5                   10                  15

<210> 158
<211> 10
<212> PRT
<213> Homo Sapiens

<400> 158
Gly Tyr Thr Phe Ser Asp Asn Asp Met Ala
1               5                   10

<210> 159
<211> 17
<212> PRT
<213> Homo Sapiens

<400> 159
Gly Ile Thr Ser Asp Ser Gly Ser Thr Tyr Tyr Ala Asp Ser Val Lys
1               5                   10                  15
Gly

<210> 160
<211> 17
<212> PRT
<213> Homo Sapiens

<400> 160
Thr Glu Ser Pro Asn Gly Val Thr Lys Leu Ser Asp Lys Asn Phe Glu
1               5                   10                  15
Ser

<210> 161
<211> 10
<212> PRT
<213> Homo Sapiens

<400> 161
Gly Asp Asn Val Ile Ser Asp Asp Met Ser
1               5                   10

<210> 162
<211> 17
<212> PRT
<213> Homo Sapiens

<400> 162
Thr Ile Asn Gly Pro Ser Gly Ser Thr Tyr Tyr Ala Asp Ser Val Lys
1               5                   10                  15
Gly

<210> 163
<211> 21
<212> PRT
<213> Homo Sapiens

<400> 163
Ala Asn Gly Glu Asp Thr Asp Met Leu Asp Met Trp Gly Asp Arg Ser
1               5                   10                  15
Ala Ala Leu Lys Ser
            20

<210> 164
<211> 10
<212> PRT
<213> Homo Sapiens

<400> 164
Gly Val Lys Phe Asn Asp Glu Asp Met Ser
1               5                   10


<210> 165
<211> 17
<212> PRT
<213> Homo Sapiens

<400> 165
Ser Ile Gly Thr Glu Asn Gly Ser Thr Tyr Tyr Ala Asp Ser Val Lys
1               5                   10                  15
Gly


<210> 166
<211> 14
<212> PRT
<213> Homo Sapiens

<400> 166
Ala Gly Pro Ser Gly His Glu Gly Asn Tyr Asp Ile Asp Ser
1               5                   10


<210> 167
<211> 10
<212> PRT
<213> Homo Sapiens

<400> 167
Gly Asp Lys Ile Thr Ser Lys Asn Met Ser
1               5                   10


<210> 168
<211> 17
<212> PRT
<213> Homo Sapiens

<400> 168
Thr Ile Pro Ala Glu Gly Gly Ser Thr Tyr Tyr Ala Asp Ser Val Lys
1               5                   10                  15
Gly


<210> 169
<211> 12
<212> PRT
<213> Homo Sapiens

<400> 169
Thr Ala Cys Phe Pro Ser Ala Gln His Val Glu Ser
1               5                   10


<210> 170
<211> 10
<212> PRT
<213> Homo Sapiens

<400> 170
Gly Phe Thr Phe Asp Leu Tyr Asp Met Ser
1               5                   10


<210> 171
<211> 17
<212> PRT
<213> Homo Sapiens

<400> 171
Ser Ile Val Asn Ser Gly Val Arg Thr Tyr Tyr Ala Asp Ser Val Lys
1               5                   10                  15
Gly


<210> 172
<211> 12
<212> PRT
<213> Homo Sapiens

<400> 172
Lys Leu Asn Gln Ser Tyr His Trp Asp Phe Asp Tyr
1               5                   10


<210> 173
<211> 10
<212> PRT
<213> Homo Sapiens

<400> 173
Gly Phe Thr Phe Ser Asp Tyr Arg Met Ser
1               5                   10


<210> 174
<211> 17
<212> PRT
<213> Homo Sapiens

<400> 174
Thr Ile Ile Ser Asn Gly Lys Phe Thr Tyr Tyr Ala Asp Ser Val Lys
1               5                   10                  15
Gly

<210> 175
<211> 10
<212> PRT
<213> Homo Sapiens

<400> 175
Lys Gln Asp Trp Met Tyr Met Phe Asp Tyr
1               5                   10


<210> 176
<211> 10
<212> PRT
<213> Homo Sapiens

<400> 176
Gly Phe Thr Phe Ser Lys Tyr Trp Met Ser
1               5                   10


<210> 177
<211> 17
<212> PRT
<213> Homo Sapiens

<400> 177
Ser Ile Asp Phe Met Gly Pro His Thr Tyr Tyr Ala Asp Ser Val Lys
1               5                   10                  15
Gly


<210> 178
<211> 15
<212> PRT
<213> Homo Sapiens

<400> 178
Lys Gly Arg Thr Ser Met Leu Pro Met Lys Gly Lys Phe Asp Tyr
1               5                   10                  15


<210> 179
<211> 10
<212> PRT
<213> Homo Sapiens

<400> 179
Gly Phe Thr Phe Tyr Asp Tyr Asn Met Ser
1               5                   10


<210> 180
<211> 17
<212> PRT
<213> Homo Sapiens

<400> 180
Thr Ile Thr His Thr Gly Gly Val Thr Tyr Tyr Ala Asp Ser Val Lys
1               5                   10                  15
Gly

<210> 181
<211> 10
<212> PRT
<213> Homo Sapiens

<400> 181
Lys Gln Asn Pro Ser Tyr Gln Phe Asp Tyr
1               5                   10

<210> 182
<211> 10
<212> PRT
<213> Homo Sapiens

<400> 182
Gly Phe Thr Phe His Arg Tyr Ser Met Ser
1               5                   10

<210> 183
<211> 17
<212> PRT
<213> Homo Sapiens

<400> 183
Thr Ile Leu Pro Gly Gly Asp Val Thr Tyr Tyr Ala Asp Ser Val Lys
1               5                   10                  15
Gly

<210> 184
<211> 10
<212> PRT
<213> Homo Sapiens

<400> 184
Lys Gln Thr Pro Asp Tyr Met Phe Asp Tyr
1               5                   10

<210> 185
<211> 10
<212> PRT
<213> Homo Sapiens

<400> 185
Gly Phe Thr Phe Trp Lys Tyr Asn Met Ala
1               5                   10

<210> 186
<211> 17
<212> PRT
<213> Homo Sapiens

<400> 186
Thr Ile Leu Gly Glu Gly Asn Asn Thr Tyr Tyr Ala Asp Ser Val Lys
1               5                   10                  15
Gly

<210> 187
<211> 9
<212> PRT
<213> Homo Sapiens

<400> 187
Lys Thr Met Asp Tyr Lys Phe Asp Tyr
1               5

<210> 188
<211> 10
<212> PRT
<213> Homo Sapiens

<400> 188
Gly Phe Thr Phe Asp Glu Tyr Asn Met Ser
1               5                   10

<210> 189
<211> 17
<212> PRT
<213> Homo Sapiens

<400> 189
Thr Ile Leu Pro His Gly Asp Arg Thr Tyr Tyr Ala Asp Ser Val Lys
1               5                   10                  15
Gly

<210> 190
<211> 10
<212> PRT
<213> Homo Sapiens

<400> 190
Lys Gln Asp Pro Leu Tyr Arg Phe Asp Tyr
1               5                   10

<210> 191
<211> 120

<212> PRT
<213> Homo Sapiens

<400> 191
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Glu Trp Tyr
            20                  25                  30
Trp Met Gly Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Ala Ala Val Tyr Tyr Cys
                85                  90                  95
Ala Lys Val Lys Leu Gly Gly Gly Pro Asn Phe Gly Tyr Arg Gly Gln
            100                 105                 110
Gly Thr Leu Val Thr Val Ser Ser
        115                 120


<210> 192
<211> 15
<212> PRT
<213> Homo Sapiens

<400> 192
Cys Ala Ala Ser Gly Phe Thr Phe Glu Trp Tyr Trp Met Gly Trp
1               5                   10                  15


<210> 193
<211> 15
<212> PRT
<213> Homo Sapiens

<400> 193
Cys Ala Ala Ser Gly Asp Thr Phe Glu Trp Tyr Trp Met Gly Trp
1               5                   10                  15


<210> 194
<211> 15
<212> PRT
<213> Homo Sapiens

<400> 194
Cys Ala Ala Ser Gly Gln Thr Phe Glu Trp Tyr Trp Met Gly Trp
1               5                   10                  15


<210> 195
<211> 15
<212> PRT
<213> Homo Sapiens

<400> 195

Cys Ala Ala Ser Gly Phe Thr Phe Glu Trp Asp Trp Met Gly Trp
1               5                   10                  15

<210> 196
<211> 15
<212> PRT
<213> Homo Sapiens

<400> 196
Cys Ala Ala Ser Gly Phe Thr Phe Glu Trp Gln Trp Met Gly Trp
1               5                   10                  15

<210> 197
<211> 15
<212> PRT
<213> Homo Sapiens

<400> 197
Cys Ala Ala Ser Gly Phe Thr Phe Gln Tyr Gly Pro Met Ser Trp
1               5                   10                  15

<210> 198
<211> 15
<212> PRT
<213> Homo Sapiens

<400> 198
Cys Ala Ala Ser Gly Phe Thr Phe Ala Ala Asp Asn Met Asp Trp
1               5                   10                  15

<210> 199
<211> 15
<212> PRT
<213> Homo Sapiens

<400> 199
Cys Ala Ala Ser Gly Phe Thr Phe Pro Thr Asn Glu Met Ser Trp
1               5                   10                  15

<210> 200
<211> 15
<212> PRT
<213> Homo Sapiens

<400> 200
Cys Ala Ala Ser Gly Phe Thr Phe Gly Asn Ala Ser Met Asp Trp
1               5                   10                  15

<210> 201
<211> 15
<212> PRT
<213> Homo Sapiens

<400> 201
Cys Ala Ala Ser Gly Phe Thr Phe Glu Asp Asp Leu Met Asn Trp
1               5                   10                  15

<210> 202
<211> 15
<212> PRT
<213> Homo Sapiens

<400> 202
Cys Ala Ala Ser Gly Phe Thr Phe Gly Gly Asp Glu Met Thr Trp
1               5                   10                  15

<210> 203
<211> 15
<212> PRT
<213> Homo Sapiens

<400> 203
Cys Ala Ala Ser Gly Phe Thr Phe Asp Asp Ser Thr Met Gln Trp
1               5                   10                  15

<210> 204
<211> 15
<212> PRT
<213> Homo Sapiens

<400> 204
Cys Ala Ala Ser Gly Phe Thr Phe Asp Asn Ser Val Met Gly Trp
1               5                   10                  15

<210> 205
<211> 15
<212> PRT
<213> Homo Sapiens

<400> 205
Cys Ala Ala Ser Gly Phe Thr Phe Asp Gln Thr Ala Met His Trp
1               5                   10                  15

<210> 206
<211> 15
<212> PRT
<213> Homo Sapiens

<400> 206
Cys Ala Ala Ser Gly Phe Thr Phe Pro Glu Leu Pro Met Gly Trp
1               5                   10                  15

<210> 207
<211> 15

<212> PRT
<213> Homo Sapiens

<400> 207
Cys Ala Ala Ser Gly Phe Thr Phe Thr Pro Gly Lys Met Asn Trp
1               5                   10                  15


<210> 208
<211> 15
<212> PRT
<213> Homo Sapiens

<400> 208
Cys Ala Ala Ser Gly Phe Thr Phe Glu His Arg Thr Met Gly Trp
1               5                   10                  15


<210> 209
<211> 15
<212> PRT
<213> Homo Sapiens

<400> 209
Cys Ala Ala Ser Gly Phe Thr Phe Pro Asn Ser Asp Met Val Trp
1               5                   10                  15


<210> 210
<211> 15
<212> PRT
<213> Homo Sapiens

<400> 210
Cys Ala Ala Ser Gly Phe Thr Phe Gly Lys Ser Thr Met Ala Trp
1               5                   10                  15


<210> 211
<211> 108
<212> PRT
<213> Unknown

<220>
<223> H1 Sequence

<400> 211
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Asp Ser Tyr
            20                  25                  30
Leu His Trp Tyr Gln Gln Lys Pro Gly Lys Ala Pro Lys Leu Leu Ile
        35                  40                  45
Tyr Ser Ala Ser Glu Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Val Val Trp Arg Pro Phe

                 85                  90                  95
Thr Phe Gly Gln Gly Thr Lys Val Glu Ile Lys Arg
            100                 105

<210> 212
<211> 15
<212> PRT
<213> Unknown

<220>
<223> H1 Sequence

<400> 212
Lys Ala Pro Lys Leu Leu Ile Tyr Ser Ala Ser Glu Leu Gln Ser
 1               5                  10                  15

<210> 213
<211> 15
<212> PRT
<213> Unknown

<220>
<223> H1 Sequence

<400> 213
Lys Ala Pro Lys Leu Leu Ile Asp Ser Ala Ser Glu Leu Gln Ser
 1               5                  10                  15

<210> 214
<211> 15
<212> PRT
<213> Unknown

<220>
<223> H1 Sequence

<400> 214
Lys Ala Pro Lys Leu Leu Ile Asn Ser Ala Ser Glu Leu Gln Ser
 1               5                  10                  15

<210> 215
<211> 9
<212> PRT
<213> Homo Sapiens

<400> 215
Asp Asn Val Ser Asp Lys Thr Met Gly
 1               5

<210> 216
<211> 6
<212> PRT
<213> Homo Sapiens

<400> 216
Gly Ile Glu Ala Gly Ser
1               5


<210> 217
<211> 13
<212> PRT
<213> Homo Sapiens

<400> 217
Ser Asp Asp Val Ile Met Thr Pro Ala Gln Ile Gly Ser
1               5                   10


<210> 218
<211> 9
<212> PRT
<213> Homo Sapiens

<400> 218
Phe Thr Ile Ser Asn Asp Asn Met Gly
1               5


<210> 219
<211> 6
<212> PRT
<213> Homo Sapiens

<400> 219
Ser Ile Glu Ala Ala Asp
1               5


<210> 220
<211> 14
<212> PRT
<213> Homo Sapiens

<400> 220
Gly Gly Tyr Gly Pro Ser Phe Val Ser Asn Asp Val Asn Ser
1               5                   10


<210> 221
<211> 9
<212> PRT
<213> Homo Sapiens

<400> 221
Asp Arg Leu Asn His Gln Asn Met Gly
1               5


<210> 222
<211> 6

<212> PRT
<213> Homo Sapiens

<400> 222
Thr Ile Asp Asn Gly Asn
1               5


<210> 223
<211> 15
<212> PRT
<213> Homo Sapiens

<400> 223
Thr Gln Asp Asp Gly Ala Lys Thr Ser Ser Glu Thr Val Lys Ser
1               5                   10                  15


<210> 224
<211> 9
<212> PRT
<213> Homo Sapiens

<400> 224
Asp Ser Phe Thr Asn Asn Ala Met Gly
1               5


<210> 225
<211> 6
<212> PRT
<213> Homo Sapiens

<400> 225
Gly Ile Asp Thr His Asp
1               5


<210> 226
<211> 12
<212> PRT
<213> Homo Sapiens

<400> 226
Gly Met Arg Phe Lys Pro Asp Asp Gln Val Ser Ser
1               5                   10


<210> 227
<211> 9
<212> PRT
<213> Homo Sapiens

<400> 227
Asp Met Phe Ser Ala Glu Ser Met Gly
1               5

<210> 228
<211> 6
<212> PRT
<213> Homo Sapiens

<400> 228
Ala Ile Asn Val Ala Asn
1               5


<210> 229
<211> 12
<212> PRT
<213> Homo Sapiens

<400> 229
Ser Gly Val Trp Asn Asn Tyr Ser Ala Leu Arg Phe
1               5                   10


<210> 230
<211> 9
<212> PRT
<213> Homo Sapiens

<400> 230
Asp Lys Phe Asn Asn Glu Asn Met Gly
1               5


<210> 231
<211> 6
<212> PRT
<213> Homo Sapiens

<400> 231
Thr Ile Asp Ala Glu Ser
1               5


<210> 232
<211> 12
<212> PRT
<213> Homo Sapiens

<400> 232
Gly Gly Gln Ser Glu Arg Glu Glu Asp Val Thr Tyr
1               5                   10


<210> 233
<211> 9
<212> PRT
<213> Homo Sapiens

<400> 233
Asp Ser Leu Asn Pro Lys Asn Met Ser
1               5

<210> 234
<211> 6
<212> PRT
<213> Homo Sapiens

<400> 234
Gly Ile Gln Glu Pro Asn
1               5


<210> 235
<211> 12
<212> PRT
<213> Homo Sapiens

<400> 235
Thr His Asp Glu Ser Lys Pro Glu Glu Ile Asp Tyr
1               5                   10


<210> 236
<211> 9
<212> PRT
<213> Homo Sapiens

<400> 236
Asp Arg Phe Asn Ser Asn Asp Met Ser
1               5


<210> 237
<211> 6
<212> PRT
<213> Homo Sapiens

<400> 237
Thr Ile Ser Met Glu Asn
1               5


<210> 238
<211> 15
<212> PRT
<213> Homo Sapiens

<400> 238
Arg Arg Asp Thr Gly Phe Thr Gln Ser Ala Tyr Pro Phe Glu Ser
1               5                   10                  15


<210> 239
<211> 9
<212> PRT
<213> Homo Sapiens

<400> 239

Asp Met Val Asn Asp Lys Asp Met Ser
1               5

<210> 240
<211> 6
<212> PRT
<213> Homo Sapiens

<400> 240
Ser Ile Ser Thr Glu Asn
1               5

<210> 241
<211> 16
<212> PRT
<213> Homo Sapiens

<400> 241
Gly Val Arg Asp Glu Val Ala Met Gly Glu Asn Pro Asp Leu Ser Tyr
1               5                   10                  15

<210> 242
<211> 9
<212> PRT
<213> Homo Sapiens

<400> 242
Asp Thr Ile Ser Thr Lys Asp Met Gly
1               5

<210> 243
<211> 6
<212> PRT
<213> Homo Sapiens

<400> 243
Thr Ile Thr Asp Gln Asn
1               5

<210> 244
<211> 12
<212> PRT
<213> Homo Sapiens

<400> 244
Ser Thr Ala Pro Trp Leu Pro Asp Ala Met Asp Ser
1               5                   10

<210> 245
<211> 9
<212> PRT
<213> Homo Sapiens

<400> 245
Val Met Val Ser Asp Gln Asp Met Ala
1               5

<210> 246
<211> 6
<212> PRT
<213> Homo Sapiens

<400> 246
Thr Ile Asp Val Gly Ser
1               5

<210> 247
<211> 15
<212> PRT
<213> Homo Sapiens

<400> 247
Gly Thr Gly Gln Ser Gln Thr Val Glu Thr Asp Ala Leu Asp Ser
1               5                   10                  15

<210> 248
<211> 9
<212> PRT
<213> Homo Sapiens

<400> 248
Phe Arg Phe Ser Ala Glu Asp Met Gly
1               5

<210> 249
<211> 6
<212> PRT
<213> Homo Sapiens

<400> 249
Ser Ile Asp Asn Asp Asp
1               5

<210> 250
<211> 16
<212> PRT
<213> Homo Sapiens

<400> 250
Ser Ser Pro Gly Pro Asp Asn Glu Lys Asp Asn Ala Ser Leu Lys Ser
1               5                   10                  15

<210> 251
<211> 9

<212> PRT
<213> Homo Sapiens

<400> 251
Asp Ser Leu Thr His Asp Asn Met Ser
1               5


<210> 252
<211> 6
<212> PRT
<213> Homo Sapiens

<400> 252
Thr Ile Asn Thr Pro Ser
1               5


<210> 253
<211> 12
<212> PRT
<213> Homo Sapiens

<400> 253
Thr Gln Asp Asp Val Arg His Ala Asp Leu Thr Ser
1               5                   10


<210> 254
<211> 9
<212> PRT
<213> Homo Sapiens

<400> 254
Asp Ser Val Ser Asn Lys Val Met Gly
1               5


<210> 255
<211> 6
<212> PRT
<213> Homo Sapiens

<400> 255
Ala Ile Asp Thr Lys Asp
1               5


<210> 256
<211> 16
<212> PRT
<213> Homo Sapiens

<400> 256
Ser Gly Asp Val Asp Ala Asp Met Ala Trp Glu Glu Glu Val Ser Ser
1               5                   10                  15

<210> 257
<211> 9
<212> PRT
<213> Homo Sapiens

<400> 257
Phe Met Leu Ser Asp Gln Asp Met Gly
 1               5


<210> 258
<211> 6
<212> PRT
<213> Homo Sapiens

<400> 258
Ser Ile Gly Asn Ala Asp
 1               5


<210> 259
<211> 12
<212> PRT
<213> Homo Sapiens

<400> 259
Arg Val Pro Arg Thr Asn Gln Gln Ala Val Asn Ser
 1               5                   10


<210> 260
<211> 9
<212> PRT
<213> Homo Sapiens

<400> 260
Asp Ser Ile Ile Pro Thr Asn Met Gly
 1               5


<210> 261
<211> 6
<212> PRT
<213> Homo Sapiens

<400> 261
Gly Ile Asp Asp Arg Asn
 1               5


<210> 262
<211> 19
<212> PRT
<213> Homo Sapiens

<400> 262
Thr Gly Asp Asp Gly Asp Ile Ser Pro Gly Gly Lys Thr Gln Thr Thr
 1               5                   10                  15

Leu Asp Ser

<210> 263
<211> 9
<212> PRT
<213> Homo Sapiens

<400> 263
Asp Ser Val Ile Asp Gln Asn Met Gly
1               5

<210> 264
<211> 6
<212> PRT
<213> Homo Sapiens

<400> 264
Ser Ile Asn Asn Gln Asp
1               5

<210> 265
<211> 13
<212> PRT
<213> Homo Sapiens

<400> 265
Gly Val Asn Ala Gly Gly His Ala Thr Pro Ile Tyr Ser
1               5                   10

<210> 266
<211> 9
<212> PRT
<213> Homo Sapiens

<400> 266
Asp Arg Val Thr Asp Glu Ile Met Gly
1               5

<210> 267
<211> 6
<212> PRT
<213> Homo Sapiens

<400> 267
Ala Ile Glu Glu His Asp
1               5

<210> 268
<211> 19
<212> PRT
<213> Homo Sapiens

<400> 268
Thr Val Pro Arg Gly Thr Leu Gln Pro Ala Gln Ser His Ser Glu Ser
1               5                   10                  15
Val Lys Ser

<210> 269
<211> 9
<212> PRT
<213> Homo Sapiens

<400> 269
Asp Thr Leu Thr Ser Asp Asn Met Ala
1               5

<210> 270
<211> 6
<212> PRT
<213> Homo Sapiens

<400> 270
Thr Ile Thr Glu Ala Gly
1               5

<210> 271
<211> 15
<212> PRT
<213> Homo Sapiens

<400> 271
Thr Tyr Pro Ala Asp Val Ala Glu Cys Ala Ala Glu Val Cys Tyr
1               5                   10                  15

<210> 272
<211> 9
<212> PRT
<213> Homo Sapiens

<400> 272
Asp Arg Val Ser Ser Asp Asp Met Ala
1               5

<210> 273
<211> 6
<212> PRT
<213> Homo Sapiens

<400> 273
Ser Ile His Asn Asp Asn
1               5

<210> 274
<211> 17
<212> PRT
<213> Homo Sapiens

<400> 274
Arg Val Gly Ser Gln Val Asn Lys His Gly Met Thr Asp Glu Val Ala
1               5                   10                  15
Tyr

<210> 275
<211> 9
<212> PRT
<213> Homo Sapiens

<400> 275
Val Asn Val Ser His Asp Ser Met Thr
1               5

<210> 276
<211> 6
<212> PRT
<213> Homo Sapiens

<400> 276
Ala Ile Arg Gly Pro Asn
1               5

<210> 277
<211> 19
<212> PRT
<213> Homo Sapiens

<400> 277
Ser Gly Ala Arg His Ala Asp Thr Glu Arg Pro Pro Ser Gln Gln Thr
1               5                   10                  15
Met Pro Phe

<210> 278
<211> 9
<212> PRT
<213> Homo Sapiens

<400> 278
Asp Arg Val Ser Asp Glu Val Met Ser
1               5

<210> 279
<211> 6
<212> PRT
<213> Homo Sapiens

<400> 279
Ser Ile Asp Asp Asn Asp
1               5


<210> 280
<211> 15
<212> PRT
<213> Homo Sapiens

<400> 280
Ala Lys Ala Leu Ala Glu Glu Ile His Ser Ser Asn Leu Pro Ser
1               5                   10                  15


<210> 281
<211> 9
<212> PRT
<213> Homo Sapiens

<400> 281
Asp Ser Phe Ser Thr Glu Asp Met Gly
1               5


<210> 282
<211> 6
<212> PRT
<213> Homo Sapiens

<400> 282
Ser Ile His Gly Asn Asp
1               5


<210> 283
<211> 15
<212> PRT
<213> Homo Sapiens

<400> 283
Gly Ala Arg Ala Asn Gly Val Tyr Asp Asp Ala Glu Val Gly Phe
1               5                   10                  15


<210> 284
<211> 9
<212> PRT
<213> Homo Sapiens

<400> 284
Asp Met Leu Asn Ala Gln Asn Met Ser
1               5


<210> 285
<211> 6

<212> PRT
<213> Homo Sapiens

<400> 285
Ser Ile Gly Ala Ala Asp
1               5


<210> 286
<211> 19
<212> PRT
<213> Homo Sapiens

<400> 286
Ala Asp Glu Ala Trp Ser Ser Asp Thr Gly Arg Phe Gln Glu Asp Lys
1               5                   10                  15
Leu Gly Ser



<210> 287
<211> 9
<212> PRT
<213> Homo Sapiens

<400> 287
Phe Lys Ile Ser Ser Lys Thr Met Gly
1               5


<210> 288
<211> 6
<212> PRT
<213> Homo Sapiens

<400> 288
Ser Ile Cys Ala Gly Gly
1               5


<210> 289
<211> 15
<212> PRT
<213> Homo Sapiens

<400> 289
Ala Ser Asn Lys Cys Arg Leu Glu Leu Ser Ala Asp Val Glu Tyr
1               5                   10                  15


<210> 290
<211> 9
<212> PRT
<213> Homo Sapiens

<400> 290
Asp Arg Val Ile Asn His Thr Met Gly
1               5

<210> 291
<211> 6
<212> PRT
<213> Homo Sapiens

<400> 291
Ala Ile Val Gly Thr Asp
 1               5


<210> 292
<211> 19
<212> PRT
<213> Homo Sapiens

<400> 292
Ala Pro Arg Gly Val Ser Glu Glu Asn Val Leu His Trp Glu His Lys
 1               5                   10                  15
Val Pro Ser



<210> 293
<211> 9
<212> PRT
<213> Homo Sapiens

<400> 293
Asp Ser Val Ser Asp Glu Asn Met Gly
 1               5


<210> 294
<211> 6
<212> PRT
<213> Homo Sapiens

<400> 294
Thr Ile Gly Ala Gly Gly
 1               5


<210> 295
<211> 11
<212> PRT
<213> Homo Sapiens

<400> 295
Ser Ala Gly Ser Glu Pro Thr Gln Met Glu Ser
 1               5                   10


<210> 296
<211> 9
<212> PRT
<213> Homo Sapiens

<400> 296
Tyr Met Leu Ser Asp Asp Asp Met Ser
1               5

<210> 297
<211> 6
<212> PRT
<213> Homo Sapiens

<400> 297
Thr Ile Ser Glu Pro Ser
1               5

<210> 298
<211> 13
<212> PRT
<213> Homo Sapiens

<400> 298
Thr Asp Ser Gly Arg Thr Thr Ala Gln Glu Val Pro Tyr
1               5                   10

<210> 299
<211> 9
<212> PRT
<213> Homo Sapiens

<400> 299
Tyr Arg Val Asn Ala Gln Asp Met Ser
1               5

<210> 300
<211> 6
<212> PRT
<213> Homo Sapiens

<400> 300
Thr Ile Glu Asn Glu Asn
1               5

<210> 301
<211> 13
<212> PRT
<213> Homo Sapiens

<400> 301
Ser Cys Thr Arg Gly Gly Cys Tyr Asp Thr Phe Pro Tyr
1               5                   10

<210> 302
<211> 9

<212> PRT
<213> Homo Sapiens

<400> 302
Tyr Asn Ile Thr Asp Glu Asn Met Ala
1               5

<210> 303
<211> 6
<212> PRT
<213> Homo Sapiens

<400> 303
Thr Ile Ala Ala Asn Asp
1               5

<210> 304
<211> 14
<212> PRT
<213> Homo Sapiens

<400> 304
Thr Thr Glu Ala Ala Gly Val Glu Glu Asp Asn Val Arg Ser
1               5                   10

<210> 305
<211> 9
<212> PRT
<213> Homo Sapiens

<400> 305
Asp Thr Val Ser His Lys Thr Met Gly
1               5

<210> 306
<211> 6
<212> PRT
<213> Homo Sapiens

<400> 306
Thr Ile Ser Asp Lys Asp
1               5

<210> 307
<211> 15
<212> PRT
<213> Homo Sapiens

<400> 307
Ser Pro Ser Pro Arg Leu Asp Gly Gly Gln Glu Ser Val Thr Ser
1               5                   10                  15

<210> 308
<211> 9
<212> PRT
<213> Homo Sapiens

<400> 308
Asp Lys Val Ser Tyr Asn Asn Met Ala
1               5


<210> 309
<211> 6
<212> PRT
<213> Homo Sapiens

<400> 309
Ser Ile Thr Thr Glu Asn
1               5


<210> 310
<211> 19
<212> PRT
<213> Homo Sapiens

<400> 310
Gly Asn Arg Asn Ser Pro Val Asp Tyr Arg Glu Leu Gln Ser Thr Pro
1               5                   10                  15
Leu Asp Ser



<210> 311
<211> 9
<212> PRT
<213> Homo Sapiens

<400> 311
Asp Met Val Ser Tyr Glu Asp Met Ala
1               5


<210> 312
<211> 6
<212> PRT
<213> Homo Sapiens

<400> 312
Thr Ile Glu Glu Ala Asn
1               5


<210> 313
<211> 14
<212> PRT
<213> Homo Sapiens

<400> 313

Ala Asn Lys Arg Asp Arg Arg Gln Lys Glu Ser Val Ala Ser
1               5                   10

<210> 314
<211> 9
<212> PRT
<213> Homo Sapiens

<400> 314
Asp Asn Phe Asn Asn Glu Asn Met Gly
1               5

<210> 315
<211> 6
<212> PRT
<213> Homo Sapiens

<400> 315
Thr Ile Ser Asp Thr Asn
1               5

<210> 316
<211> 13
<212> PRT
<213> Homo Sapiens

<400> 316
Thr Thr Gly Thr Arg Gln Pro Gln Lys Glu Val Gly Ser
1               5                   10

<210> 317
<211> 9
<212> PRT
<213> Homo Sapiens

<400> 317
Val Asn Val Thr Asp Gln Asp Met Gly
1               5

<210> 318
<211> 6
<212> PRT
<213> Homo Sapiens

<400> 318
Ser Ile Arg Ser Asn Asp
1               5

<210> 319
<211> 12
<212> PRT
<213> Homo Sapiens

<400> 319
Gly Arg Ser Ser Gly Arg Thr Asp Ala Val Pro Tyr
1               5                   10

<210> 320
<211> 9
<212> PRT
<213> Homo Sapiens

<400> 320
Asp Ser Ile Ser Asp Asp Tyr Met Ala
1               5

<210> 321
<211> 6
<212> PRT
<213> Homo Sapiens

<400> 321
Ser Ile Asp Asp Lys Asn
1               5

<210> 322
<211> 11
<212> PRT
<213> Homo Sapiens

<400> 322
Gly Gly Asp Gly Gln Ala His Lys Val Asp Tyr
1               5                   10

<210> 323
<211> 9
<212> PRT
<213> Homo Sapiens

<400> 323
Asp Met Leu Asn Tyr Lys Val Met Gly
1               5

<210> 324
<211> 6
<212> PRT
<213> Homo Sapiens

<400> 324
Ser Ile Ile Thr Gln Asp
1               5

<210> 325
<211> 12

<212> PRT
<213> Homo Sapiens

<400> 325
Gly Ile Pro Leu Asp Arg Ala Asp Asp Ile Glu Tyr
 1               5                   10


<210> 326
<211> 9
<212> PRT
<213> Homo Sapiens

<400> 326
Tyr Lys Val Asn Asp Asn Thr Met Ala
 1               5


<210> 327
<211> 6
<212> PRT
<213> Homo Sapiens

<400> 327
Ser Ile Asp Thr Thr Asp
 1               5


<210> 328
<211> 11
<212> PRT
<213> Homo Sapiens

<400> 328
Ala Ser Asp Gln Arg Thr Ala Asp Met Arg Ser
 1               5                   10


<210> 329
<211> 9
<212> PRT
<213> Homo Sapiens

<400> 329
Val Thr Val Ser Asp Glu Asn Met Gly
 1               5


<210> 330
<211> 6
<212> PRT
<213> Homo Sapiens

<400> 330
Gly Ile Ser Ser Asn Asp
 1               5

<210> 331
<211> 14
<212> PRT
<213> Homo Sapiens

<400> 331
Arg Asp Tyr Gly Ser Arg Val Asp Gln Gln His Leu Glu Ser
1               5                   10

<210> 332
<211> 9
<212> PRT
<213> Homo Sapiens

<400> 332
Asp Thr Leu Thr Asp Gln Asn Met Ser
1               5

<210> 333
<211> 6
<212> PRT
<213> Homo Sapiens

<400> 333
Thr Ile Thr Asp Pro Asp
1               5

<210> 334
<211> 15
<212> PRT
<213> Homo Sapiens

<400> 334
Thr Lys Val Glu Asp Trp Glu Glu Arg Lys Glu Ser Val Gln Ser
1               5                   10                  15

<210> 335
<211> 9
<212> PRT
<213> Homo Sapiens

<400> 335
Val Thr Leu Asn Asp Glu Tyr Met Gly
1               5

<210> 336
<211> 6
<212> PRT
<213> Homo Sapiens

<400> 336
Ser Ile Asn Asp Arg Asn
1               5

<210> 337
<211> 15
<212> PRT
<213> Homo Sapiens

<400> 337
Ser Trp Val Val Pro Gly Arg Lys Ser Ala Glu Pro Met Asp Tyr
1               5                   10                  15


<210> 338
<211> 9
<212> PRT
<213> Homo Sapiens

<400> 338
Tyr Thr Phe Ser Asp Asn Asp Met Ala
1               5


<210> 339
<211> 6
<212> PRT
<213> Homo Sapiens

<400> 339
Gly Ile Thr Ser Asp Ser
1               5


<210> 340
<211> 17
<212> PRT
<213> Homo Sapiens

<400> 340
Thr Glu Ser Pro Asn Gly Val Thr Lys Leu Ser Asp Lys Asn Phe Glu
1               5                   10                  15
Ser


<210> 341
<211> 9
<212> PRT
<213> Homo Sapiens

<400> 341
Asp Asn Val Ile Ser Asp Asp Met Ser
1               5


<210> 342
<211> 6
<212> PRT
<213> Homo Sapiens

<400> 342
Thr Ile Asn Gly Pro Ser
1               5


<210> 343
<211> 21
<212> PRT
<213> Homo Sapiens

<400> 343
Ala Asn Gly Glu Asp Thr Asp Met Leu Asp Met Trp Gly Asp Arg Ser
1               5                   10                  15
Ala Ala Leu Lys Ser
            20


<210> 344
<211> 9
<212> PRT
<213> Homo Sapiens

<400> 344
Val Lys Phe Asn Asp Glu Asp Met Ser
1               5


<210> 345
<211> 6
<212> PRT
<213> Homo Sapiens

<400> 345
Ser Ile Gly Thr Glu Asn
1               5


<210> 346
<211> 14
<212> PRT
<213> Homo Sapiens

<400> 346
Ala Gly Pro Ser Gly His Glu Gly Asn Tyr Asp Ile Asp Ser
1               5                   10


<210> 347
<211> 9
<212> PRT
<213> Homo Sapiens

<400> 347
Asp Thr Val Ser Pro Glu Asn Met Ala
1               5

<210> 348
<211> 6
<212> PRT
<213> Homo Sapiens

<400> 348
Ser Ile Lys Ser Pro Ser
1               5


<210> 349
<211> 11
<212> PRT
<213> Homo Sapiens

<400> 349
Gly Asn Gln Pro Glu Asn Pro Ser Met Glu Tyr
1               5                   10


<210> 350
<211> 9
<212> PRT
<213> Homo Sapiens

<400> 350
Asp Arg Val Thr Pro Gln Asn Met Gly
1               5


<210> 351
<211> 6
<212> PRT
<213> Homo Sapiens

<400> 351
Thr Ile His Thr Thr Asp
1               5


<210> 352
<211> 17
<212> PRT
<213> Homo Sapiens

<400> 352
Ala Ser Arg Asn Gln Thr Ala Lys Ser Leu Thr Gln Asp Asp Ile Ala
1               5                   10                  15
Ser


<210> 353
<211> 9
<212> PRT
<213> Homo Sapiens

<400> 353

Val Lys Leu Thr Asp Asn Asn Met Ser
1               5

<210> 354
<211> 6
<212> PRT
<213> Homo Sapiens

<400> 354
Ser Ile Asp Asn Arg Asn
1               5

<210> 355
<211> 12
<212> PRT
<213> Homo Sapiens

<400> 355
Gly Thr Asp Asn Ala Ser Asn Asp Ser Val Glu Tyr
1               5                   10

<210> 356
<211> 9
<212> PRT
<213> Homo Sapiens

<400> 356
Phe Ser Val Ser Asp Gln Asp Met Ala
1               5

<210> 357
<211> 6
<212> PRT
<213> Homo Sapiens

<400> 357
Thr Ile Lys Asp Thr Asn
1               5

<210> 358
<211> 17
<212> PRT
<213> Homo Sapiens

<400> 358
Gly Arg Ser Val Pro Ser Ser Asp His Trp Asp Ala Asp Ser Val Glu
1               5                   10                  15
Phe

<210> 359
<211> 9

<212> PRT
<213> Homo Sapiens

<400> 359
Tyr Asn Val Ile Asp Glu Asn Met Gly
1               5


<210> 360
<211> 6
<212> PRT
<213> Homo Sapiens

<400> 360
Ala Ile Pro Asp Glu Gly
1               5


<210> 361
<211> 11
<212> PRT
<213> Homo Sapiens

<400> 361
Ala Leu Ser Pro Ser His Gln Ser Leu Lys Ser
1               5                   10


<210> 362
<211> 9
<212> PRT
<213> Homo Sapiens

<400> 362
Asp Asn Val Thr Ser Glu Thr Met Ala
1               5


<210> 363
<211> 6
<212> PRT
<213> Homo Sapiens

<400> 363
Ala Ile Pro Asn Gln Asp
1               5


<210> 364
<211> 17
<212> PRT
<213> Homo Sapiens

<400> 364
Gly Thr Asp Met Asn Met His Ser Arg Gly Asn Lys Thr Lys Phe Asn
1               5                   10                  15
Tyr

<210> 365
<211> 9
<212> PRT
<213> Homo Sapiens

<400> 365
Asp Thr Leu Asn Asp Tyr Asn Met Ser
1               5


<210> 366
<211> 6
<212> PRT
<213> Homo Sapiens

<400> 366
Thr Ile Asn Asp Pro Asn
1               5


<210> 367
<211> 14
<212> PRT
<213> Homo Sapiens

<400> 367
Gly Ser Lys Ser Arg Gly Gly Pro Pro Glu Glu Leu His Ser
1               5                   10


<210> 368
<211> 9
<212> PRT
<213> Homo Sapiens

<400> 368
Val Asn Ile Asn Asp Asn Val Met Gly
1               5


<210> 369
<211> 6
<212> PRT
<213> Homo Sapiens

<400> 369
Ala Ile Asp Asp Arg Asn
1               5


<210> 370
<211> 16
<212> PRT
<213> Homo Sapiens

<400> 370

Ser Ala Ser His Leu Ala Pro Pro Ser Asn Glu Thr Ala Val Arg Tyr
1               5                   10                  15

<210> 371
<211> 9
<212> PRT
<213> Homo Sapiens

<400> 371
Val Asn Leu Asn Ala Glu Asn Met Ser
1               5

<210> 372
<211> 6
<212> PRT
<213> Homo Sapiens

<400> 372
Thr Ile Gly Met Gly Ser
1               5

<210> 373
<211> 11
<212> PRT
<213> Homo Sapiens

<400> 373
Ala Val Asn Thr Leu Glu Gln Thr Val Pro Tyr
1               5                   10

<210> 374
<211> 9
<212> PRT
<213> Homo Sapiens

<400> 374
Asp Thr Ile Thr Asn Lys Asn Met Gly
1               5

<210> 375
<211> 6
<212> PRT
<213> Homo Sapiens

<400> 375
Thr Ile Thr Ala Pro Asn
1               5

<210> 376
<211> 13
<212> PRT
<213> Homo Sapiens

<400> 376
Ala Pro Val Glu Arg Lys Gly Ser Gln Glu Val Gly Phe
1               5                   10

<210> 377
<211> 9
<212> PRT
<213> Homo Sapiens

<400> 377
Asp Arg Val Thr Asp Asn Asn Met Ala
1               5

<210> 378
<211> 6
<212> PRT
<213> Homo Sapiens

<400> 378
Thr Ile Ala Asp Asp Asn
1               5

<210> 379
<211> 13
<212> PRT
<213> Homo Sapiens

<400> 379
Thr Leu Gln Gly Val Gly Asn Ala Pro Gln Phe Gln Ser
1               5                   10

<210> 380
<211> 9
<212> PRT
<213> Homo Sapiens

<400> 380
Asp Lys Ile Ser Asn Gln Tyr Met Ala
1               5

<210> 381
<211> 6
<212> PRT
<213> Homo Sapiens

<400> 381
Ala Ile Asp Asn Asn Asp
1               5

<210> 382
<211> 18

<212> PRT
<213> Homo Sapiens

<400> 382
Ser Ile Trp Gly Asp Glu Pro Val Ala Asp Ser Arg Asp Pro Glu Val
1               5                   10                  15
Asn Ser

<210> 383
<211> 9
<212> PRT
<213> Homo Sapiens

<400> 383
Asp Thr Ile Ser Thr Gln Asp Met Ala
1               5

<210> 384
<211> 6
<212> PRT
<213> Homo Sapiens

<400> 384
Thr Ile Asp Thr Pro Asn
1               5

<210> 385
<211> 12
<212> PRT
<213> Homo Sapiens

<400> 385
Thr Ala Asn Gly Glu Ile Thr Glu Ser Val Arg Tyr
1               5                   10

<210> 386
<211> 9
<212> PRT
<213> Homo Sapiens

<400> 386
Tyr Met Val Ser Asp Gln Thr Met Gly
1               5

<210> 387
<211> 6
<212> PRT
<213> Homo Sapiens

<400> 387
Ser Ile His Asp Pro Gly
1               5

<210> 388
<211> 11
<212> PRT
<213> Homo Sapiens

<400> 388
Arg Asn Glu Gly Ser Ser Glu Pro Ile Tyr Ser
1               5                   10


<210> 389
<211> 9
<212> PRT
<213> Homo Sapiens

<400> 389
Val Met Ile Thr Asp Glu Ile Met Gly
1               5


<210> 390
<211> 6
<212> PRT
<213> Homo Sapiens

<400> 390
Thr Ile Asp Thr Ala Asp
1               5


<210> 391
<211> 21
<212> PRT
<213> Homo Sapiens

<400> 391
Gly Pro Arg Ala Val Arg Gln Glu Val Ala Asp Gln Gly Ile Gln Tyr
1               5                   10                  15
Glu Asn Leu Pro Ser
            20


<210> 392
<211> 9
<212> PRT
<213> Homo Sapiens

<400> 392
Asp Arg Val Asn Pro Glu Thr Met Gly
1               5


<210> 393
<211> 6
<212> PRT
<213> Homo Sapiens

<400> 393
Ala Ile Thr Asn Arg Gly
1               5


<210> 394
<211> 11
<212> PRT
<213> Homo Sapiens

<400> 394
Thr Pro Thr Leu Gly Glu Thr Glu Leu Gly Tyr
1               5                   10


<210> 395
<211> 9
<212> PRT
<213> Homo Sapiens

<400> 395
Asp Arg Phe Ile Thr Gln Asp Met Gly
1               5


<210> 396
<211> 6
<212> PRT
<213> Homo Sapiens

<400> 396
Thr Ile Asn Thr Gln Asn
1               5


<210> 397
<211> 11
<212> PRT
<213> Homo Sapiens

<400> 397
Arg Gln Lys Asn Gly Ala Asn Asn Val Gly Tyr
1               5                   10


<210> 398
<211> 9
<212> PRT
<213> Homo Sapiens

<400> 398
Asp Ser Val Ser Asn Glu Val Met Ser
1               5


<210> 399
<211> 6

<212> PRT
<213> Homo Sapiens

<400> 399
Thr Ile Asp Thr Thr Asn
1               5


<210> 400
<211> 13
<212> PRT
<213> Homo Sapiens

<400> 400
Arg Gly Glu Gln Leu Asp Gly Ser Ala Pro Val Ala Ser
1               5                   10


<210> 401
<211> 9
<212> PRT
<213> Homo Sapiens

<400> 401
Val Asn Ile Asn Thr Glu Ser Met Ala
1               5


<210> 402
<211> 6
<212> PRT
<213> Homo Sapiens

<400> 402
Ser Ile Asn Asp Thr Asn
1               5


<210> 403
<211> 15
<212> PRT
<213> Homo Sapiens

<400> 403
Gly Asp Val Tyr Pro Trp Asn Ile Gly Asn Glu Thr Leu Pro Ser
1               5                   10                  15


<210> 404
<211> 9
<212> PRT
<213> Homo Sapiens

<400> 404
Asp Met Val Asn Ala Gln Phe Met Gly
1               5

<210> 405
<211> 6
<212> PRT
<213> Homo Sapiens

<400> 405
Thr Ile Ser Thr Asp Asp
1               5


<210> 406
<211> 13
<212> PRT
<213> Homo Sapiens

<400> 406
Ser Ser Ser Ser Arg Gly Leu Glu Ser Gln Leu His Tyr
1               5                   10


<210> 407
<211> 9
<212> PRT
<213> Homo Sapiens

<400> 407
Asp Thr Phe Asn Asp Gln Asp Met Ala
1               5


<210> 408
<211> 6
<212> PRT
<213> Homo Sapiens

<400> 408
Thr Ile Glu Val Pro Ser
1               5


<210> 409
<211> 14
<212> PRT
<213> Homo Sapiens

<400> 409
Gly Asp Leu Val Asp Ser Ser Ala Thr Pro Gln Phe Ala Phe
1               5                   10


<210> 410
<211> 9
<212> PRT
<213> Homo Sapiens

<400> 410
Asp Arg Val Ile Ala Asn Tyr Met Gly
1               5

<210> 411
<211> 6
<212> PRT
<213> Homo Sapiens

<400> 411
Ala Ile Lys Ser Ser Asp
1               5


<210> 412
<211> 13
<212> PRT
<213> Homo Sapiens

<400> 412
Thr Gly Pro Arg Trp Gly Glu Glu Lys Asp Val Glu Phe
1               5                   10


<210> 413
<211> 9
<212> PRT
<213> Homo Sapiens

<400> 413
Val Asn Val Asn Asp Lys Asp Met Ala
1               5


<210> 414
<211> 6
<212> PRT
<213> Homo Sapiens

<400> 414
Thr Ile Ser Gly Arg Asp
1               5


<210> 415
<211> 16
<212> PRT
<213> Homo Sapiens

<400> 415
Ser Asn Leu Ser Pro Glu Tyr Gly Thr Arg Ala Thr Ser Val Asp Ser
1               5                   10                  15


<210> 416
<211> 9
<212> PRT
<213> Homo Sapiens

<400> 416

Asp Thr Val Ile Tyr Glu Tyr Met Gly
 1               5

<210> 417
<211> 6
<212> PRT
<213> Homo Sapiens

<400> 417
Ala Ile Arg Met Thr Asp
 1               5

<210> 418
<211> 14
<212> PRT
<213> Homo Sapiens

<400> 418
Gly Gly Asp Gly Met Ile Asp Ser Ser Thr Ser Val Ser Phe
 1               5                   10

<210> 419
<211> 9
<212> PRT
<213> Homo Sapiens

<400> 419
Asp Thr Phe Asn Thr Gln Thr Met Gly
 1               5

<210> 420
<211> 6
<212> PRT
<213> Homo Sapiens

<400> 420
Thr Ile Asp Val Glu Asn
 1               5

<210> 421
<211> 11
<212> PRT
<213> Homo Sapiens

<400> 421
Thr Cys Cys Asp Asp Ser Pro Ala Ile Asp Asn
 1               5                   10

<210> 422
<211> 9
<212> PRT
<213> Homo Sapiens

<400> 422
Asp Thr Phe Ser Thr Glu Asn Met Ala
 1               5

<210> 423
<211> 6
<212> PRT
<213> Homo Sapiens

<400> 423
Gly Ile Ser Asn Pro Asp
 1               5

<210> 424
<211> 20
<212> PRT
<213> Homo Sapiens

<400> 424
Ala Ser Gly Val Leu Asp Ser Thr Ala Glu Ala His Asp Asn Lys Gln
 1               5                   10                  15
Glu Leu Arg Ser
            20

<210> 425
<211> 9
<212> PRT
<213> Homo Sapiens

<400> 425
Asp Met Ile Asn Ser Lys Asp Met Gly
 1               5

<210> 426
<211> 6
<212> PRT
<213> Homo Sapiens

<400> 426
Thr Ile Asn Glu Pro Asn
 1               5

<210> 427
<211> 14
<212> PRT
<213> Homo Sapiens

<400> 427
Ser Arg Gly Val Asp Ala Leu Gly Glu Ala Pro Val Asp Ser
 1               5                   10

<210> 428
<211> 9
<212> PRT
<213> Homo Sapiens

<400> 428
Phe Asn Val Ser Asp Glu Asp Met Gly
1               5


<210> 429
<211> 6
<212> PRT
<213> Homo Sapiens

<400> 429
Gly Ile Glu Gly Thr Asn
1               5


<210> 430
<211> 11
<212> PRT
<213> Homo Sapiens

<400> 430
Gly Glu Ala Lys Lys Glu Asn Asp Met Asn Phe
1               5                   10


<210> 431
<211> 9
<212> PRT
<213> Homo Sapiens

<400> 431
Asp Arg Ile Thr Asn Gln Asn Met Gly
1               5


<210> 432
<211> 6
<212> PRT
<213> Homo Sapiens

<400> 432
Thr Ile Gly Gly Asp Asn
1               5


<210> 433
<211> 13
<212> PRT
<213> Homo Sapiens

<400> 433
Ser Met Val Pro Arg Gln Gly Pro Gln Glu Leu Asn Tyr
1               5                   10

<210> 434
<211> 9
<212> PRT
<213> Homo Sapiens

<400> 434
Asp Ser Phe Asn Ala Lys Asp Met Gly
1               5


<210> 435
<211> 6
<212> PRT
<213> Homo Sapiens

<400> 435
Thr Ile Asp Gly Asn Asn
1               5


<210> 436
<211> 17
<212> PRT
<213> Homo Sapiens

<400> 436
Arg Asp Val His Arg Ala Asn Gly Gly Leu Asp Gln Ser Glu Leu Glu
1               5                   10                  15
Tyr



<210> 437
<211> 9
<212> PRT
<213> Homo Sapiens

<400> 437
Asp Ser Phe Thr Asp Gln Asp Met Thr
1               5


<210> 438
<211> 6
<212> PRT
<213> Homo Sapiens

<400> 438
Ser Ile Thr Thr Ser Asp
1               5


<210> 439
<211> 13
<212> PRT
<213> Homo Sapiens

<400> 439
Arg Ala Lys Val Pro Ala Arg Thr Glu Glu Leu Glu Ser
1               5                   10

<210> 440
<211> 9
<212> PRT
<213> Homo Sapiens

<400> 440
Val Thr Val Asn Pro Gln Val Met Gly
1               5

<210> 441
<211> 6
<212> PRT
<213> Homo Sapiens

<400> 441
Ser Ile His Thr Pro Asp
1               5

<210> 442
<211> 14
<212> PRT
<213> Homo Sapiens

<400> 442
Gly Lys Glu Glu Ser Ser Ala Glu Thr Pro Ala Val Asp Ser
1               5                   10

<210> 443
<211> 9
<212> PRT
<213> Homo Sapiens

<400> 443
Asp Met Ile Thr Pro Lys Asn Met Ala
1               5

<210> 444
<211> 6
<212> PRT
<213> Homo Sapiens

<400> 444
Thr Ile Ala Asn Pro Asn
1               5

<210> 445
<211> 11

<212> PRT
<213> Homo Sapiens

<400> 445
Ala Pro Ser Gly Met Asn Asp Asp Leu Thr Tyr
1               5                   10


<210> 446
<211> 9
<212> PRT
<213> Homo Sapiens

<400> 446
Asp Thr Ile Asn Asp Asp Ser Met Gly
1               5


<210> 447
<211> 6
<212> PRT
<213> Homo Sapiens

<400> 447
Thr Ile His Asp Lys Asp
1               5


<210> 448
<211> 15
<212> PRT
<213> Homo Sapiens

<400> 448
Thr Arg Arg Asp Gln Ser Phe Gly Thr Ala Pro Ala Leu Lys Ser
1               5                   10                  15


<210> 449
<211> 9
<212> PRT
<213> Homo Sapiens

<400> 449
Asp Met Val Asn Asp Lys Thr Met Ser
1               5


<210> 450
<211> 6
<212> PRT
<213> Homo Sapiens

<400> 450
Ala Ile Asp Asn Gln Ser
1               5

<210> 451
<211> 11
<212> PRT
<213> Homo Sapiens

<400> 451
Ala Gln Glu Arg Val Glu Pro Tyr Val Glu Phe
1               5                   10


<210> 452
<211> 9
<212> PRT
<213> Homo Sapiens

<400> 452
Asp Thr Ile Ser Asp Lys Asp Met Ala
1               5


<210> 453
<211> 6
<212> PRT
<213> Homo Sapiens

<400> 453
Gly Ile Pro Thr Asp Asp
1               5


<210> 454
<211> 13
<212> PRT
<213> Homo Sapiens

<400> 454
Gly Trp Asp Glu Asp Lys Lys Ala Thr Gly Val Ala Tyr
1               5                   10


<210> 455
<211> 9
<212> PRT
<213> Homo Sapiens

<400> 455
Asp Ser Val Thr Thr Gln Asn Met Ser
1               5


<210> 456
<211> 6
<212> PRT
<213> Homo Sapiens

<400> 456
Thr Ile Ser Thr Pro Asn
1               5

<210> 457
<211> 14
<212> PRT
<213> Homo Sapiens

<400> 457
Gly Met Asp Ala Glu Asn Arg Asn Ala Asp Thr Pro Arg Ser
1               5                   10


<210> 458
<211> 9
<212> PRT
<213> Homo Sapiens

<400> 458
Tyr Thr Leu Asn Asn Gln Asn Met Ser
1               5


<210> 459
<211> 6
<212> PRT
<213> Homo Sapiens

<400> 459
Ser Ile Gln Thr Ala Asp
1               5


<210> 460
<211> 18
<212> PRT
<213> Homo Sapiens

<400> 460
Gly Gly Gly Ser Pro Met Leu Ser Gln Ala Val Ser Ser Ser Pro Leu
1               5                   10                  15
Arg Ser


<210> 461
<211> 9
<212> PRT
<213> Homo Sapiens

<400> 461
Asp Asn Val Ser Ser Gln Asp Met Ala
1               5


<210> 462
<211> 6
<212> PRT
<213> Homo Sapiens

<400> 462
Thr Ile Met Asn Ser Asn
1               5


<210> 463
<211> 15
<212> PRT
<213> Homo Sapiens

<400> 463
Gly Ser Pro Asp Ala Ser Ile Gln Glu Ser Ser Asn Leu Asp Tyr
1               5                   10                  15


<210> 464
<211> 9
<212> PRT
<213> Homo Sapiens

<400> 464
Asp Arg Val Asn Ala Gln Asp Met Gly
1               5


<210> 465
<211> 6
<212> PRT
<213> Homo Sapiens

<400> 465
Ser Ile Asn Asp Asp Asp
1               5


<210> 466
<211> 11
<212> PRT
<213> Homo Sapiens

<400> 466
Thr Arg Ser Asp Glu Glu Thr Ser Leu Arg Ser
1               5                   10


<210> 467
<211> 9
<212> PRT
<213> Homo Sapiens

<400> 467
Asp Lys Ile Asn Asp Glu Asp Met Ser
1               5


<210> 468
<211> 6

<212> PRT
<213> Homo Sapiens

<400> 468
Ser Ile Ala Asp Asn Ser
1               5


<210> 469
<211> 13
<212> PRT
<213> Homo Sapiens

<400> 469
Thr Asp Pro Val Asn Gly Leu Ala Ser Asp Val Arg Ser
1               5                   10


<210> 470
<211> 9
<212> PRT
<213> Homo Sapiens

<400> 470
Asp Ser Ile Ser Asn Asn Asn Met Gly
1               5


<210> 471
<211> 6
<212> PRT
<213> Homo Sapiens

<400> 471
Ser Ile Gly Asn Arg Asp
1               5


<210> 472
<211> 11
<212> PRT
<213> Homo Sapiens

<400> 472
Ala Gly Glu Ser Glu Glu Glu Ala Leu Gly Tyr
1               5                   10


<210> 473
<211> 9
<212> PRT
<213> Homo Sapiens

<400> 473
Asp Asn Ile Thr Asn Glu Asn Met Gly
1               5

<210> 474
<211> 6
<212> PRT
<213> Homo Sapiens

<400> 474
Ser Ile Gly Ala Pro Gly
 1               5


<210> 475
<211> 15
<212> PRT
<213> Homo Sapiens

<400> 475
Ser Thr Gln Lys Asn Asn Ala Arg Glu Pro Tyr Thr Leu Asp Ser
 1               5                   10                  15


<210> 476
<211> 9
<212> PRT
<213> Homo Sapiens

<400> 476
Val Met Leu Ser Asp Glu Asn Met Gly
 1               5


<210> 477
<211> 6
<212> PRT
<213> Homo Sapiens

<400> 477
Thr Ile Ser Gly Asp Asn
 1               5


<210> 478
<211> 16
<212> PRT
<213> Homo Sapiens

<400> 478
Arg Glu Ser Asp Trp Tyr Gly Gly Val Glu Ala Lys Glu Leu Asn Tyr
 1               5                   10                  15


<210> 479
<211> 9
<212> PRT
<213> Homo Sapiens

<400> 479
Asp Lys Val Thr His Gln Asp Met Ser
 1               5

<210> 480
<211> 6
<212> PRT
<213> Homo Sapiens

<400> 480
Thr Ile Ala Ser Arg Asp
1               5


<210> 481
<211> 14
<212> PRT
<213> Homo Sapiens

<400> 481
Thr Gly Asp Asp Phe Gly Gly Ala Asp Ala Ser Leu Ala Ser
1               5                   10


<210> 482
<211> 9
<212> PRT
<213> Homo Sapiens

<400> 482
Val Ile Val Asn Asn Asp Asp Met Ser
1               5


<210> 483
<211> 6
<212> PRT
<213> Homo Sapiens

<400> 483
Ser Ile Thr Asn Pro Asp
1               5


<210> 484
<211> 19
<212> PRT
<213> Homo Sapiens

<400> 484
Thr Ala Asn Ser Met Lys Ala Gly Ser Asp Trp Val Gly Glu Glu Ala
1               5                   10                  15
Leu Ala Tyr


<210> 485
<211> 9
<212> PRT
<213> Homo Sapiens

<400> 485
Asp Arg Val Ser Ala Asn Ala Met Ala
1               5

<210> 486
<211> 6
<212> PRT
<213> Homo Sapiens

<400> 486
Thr Ile Cys Val Lys Asp
1               5

<210> 487
<211> 14
<212> PRT
<213> Homo Sapiens

<400> 487
Gly Ser Glu Arg Cys Glu Ser Ser Ala Ser Asn Val Gln Tyr
1               5                   10

<210> 488
<211> 9
<212> PRT
<213> Homo Sapiens

<400> 488
Phe Met Ile Asn Asn Gln Asn Met Ala
1               5

<210> 489
<211> 6
<212> PRT
<213> Homo Sapiens

<400> 489
Gly Ile Gly Asp Ala Ser
1               5

<210> 490
<211> 15
<212> PRT
<213> Homo Sapiens

<400> 490
Gly Met Glu Gly Ser Gln His Gln Ser Glu Pro Asn Val Lys Ser
1               5                   10                  15

<210> 491
<211> 9

<212> PRT
<213> Homo Sapiens

<400> 491
Asp Ser Phe Asn Ala Lys Ala Met Gly
1               5


<210> 492
<211> 6
<212> PRT
<213> Homo Sapiens

<400> 492
Ala Ile Asp Asn Glu Ser
1               5


<210> 493
<211> 14
<212> PRT
<213> Homo Sapiens

<400> 493
Thr Ser Gly Ser Tyr Asp Leu Tyr Asp Glu Asp Leu Arg Phe
1               5                   10


<210> 494
<211> 9
<212> PRT
<213> Homo Sapiens

<400> 494
Phe Arg Val Asn Asp Glu Val Met Ala
1               5


<210> 495
<211> 6
<212> PRT
<213> Homo Sapiens

<400> 495
Ser Ile Glu Asn Glu Asn
1               5


<210> 496
<211> 15
<212> PRT
<213> Homo Sapiens

<400> 496
Gly Pro Gly His Thr Ile Ser Ala Lys Thr Pro Ser Leu Ala Ser
1               5                   10                  15

<210> 497
<211> 9
<212> PRT
<213> Homo Sapiens

<400> 497
Asp Thr Ile Ser Asp Lys Asp Met Ala
1               5


<210> 498
<211> 6
<212> PRT
<213> Homo Sapiens

<400> 498
Gly Ile Pro Thr Asp Asp
1               5


<210> 499
<211> 11
<212> PRT
<213> Homo Sapiens

<400> 499
Gly Trp Asp Glu Asp Lys Lys Gln Val Gly Tyr
1               5                   10


<210> 500
<211> 9
<212> PRT
<213> Homo Sapiens

<400> 500
Asp Arg Leu Ile Asn Asn Asp Met Thr
1               5


<210> 501
<211> 6
<212> PRT
<213> Homo Sapiens

<400> 501
Ser Ile Arg Asp Thr Asp
1               5


<210> 502
<211> 13
<212> PRT
<213> Homo Sapiens

<400> 502
Ala Asn Glu Asn Thr Pro Ala Gln Pro Glu Val Asn Ser
1               5                   10

<210> 503
<211> 9
<212> PRT
<213> Homo Sapiens

<400> 503
Val Thr Ile Cys His His Tyr Met Gly
1               5


<210> 504
<211> 6
<212> PRT
<213> Homo Sapiens

<400> 504
Ala Ile Arg Ser Asn Asp
1               5


<210> 505
<211> 11
<212> PRT
<213> Homo Sapiens

<400> 505
Thr Asp Phe Cys Lys Asp Lys Asn Val Ala Tyr
1               5                   10


<210> 506
<211> 9
<212> PRT
<213> Homo Sapiens

<400> 506
Tyr Asn Phe Asn Asp Glu Asp Met Ala
1               5


<210> 507
<211> 6
<212> PRT
<213> Homo Sapiens

<400> 507
Thr Ile Gly Ser Pro Asn
1               5


<210> 508
<211> 12
<212> PRT
<213> Homo Sapiens

<400> 508

Arg Asp Pro Leu Met Ser Asp Thr Ala Phe Ser Tyr
1               5                   10

<210> 509
<211> 9
<212> PRT
<213> Homo Sapiens

<400> 509
Asp Asn Val Ser Ser Lys Ser Met Ala
1               5

<210> 510
<211> 6
<212> PRT
<213> Homo Sapiens

<400> 510
Ser Ile Ser Asn Glu Asn
1               5

<210> 511
<211> 12
<212> PRT
<213> Homo Sapiens

<400> 511
Thr Gly Pro Gly Asp Tyr Pro Glu Gln Leu Glu Ser
1               5                   10

<210> 512
<211> 9
<212> PRT
<213> Homo Sapiens

<400> 512
Asp Lys Val Ser Ser Glu Ser Met Gly
1               5

<210> 513
<211> 6
<212> PRT
<213> Homo Sapiens

<400> 513
Ala Ile Glu Gly Pro Ser
1               5

<210> 514
<211> 11
<212> PRT
<213> Homo Sapiens

<400> 514
Thr His Lys Gly Arg Glu Asp Ala Ile Glu Ser
1               5                   10


<210> 515
<211> 9
<212> PRT
<213> Homo Sapiens

<400> 515
Val Thr Val Ser Ala Asp Ser Met Gly
1               5


<210> 516
<211> 6
<212> PRT
<213> Homo Sapiens

<400> 516
Ala Ile Glu Ala Pro Asp
1               5


<210> 517
<211> 17
<212> PRT
<213> Homo Sapiens

<400> 517
Thr Gly Pro Val Glu Asp Pro Gly Ser Thr Asp Pro His Gln Met His
1               5                   10                  15
Ser



<210> 518
<211> 9
<212> PRT
<213> Homo Sapiens

<400> 518
Asp Thr Ile Ser Ala Glu Val Met Gly
1               5


<210> 519
<211> 6
<212> PRT
<213> Homo Sapiens

<400> 519
Gly Ile Cys Gly His Ser
1               5

<210> 520
<211> 16
<212> PRT
<213> Homo Sapiens

<400> 520
Arg Gly Val Glu Arg Gly Asp Gly Cys Asn Glu Ser Glu Ile Glu Ser
1               5                   10                  15


<210> 521
<211> 9
<212> PRT
<213> Homo Sapiens

<400> 521
Asp Lys Phe Ser Thr Gln Asn Met Ala
1               5


<210> 522
<211> 6
<212> PRT
<213> Homo Sapiens

<400> 522
Gly Ile Val Ala Ala Asn
1               5


<210> 523
<211> 14
<212> PRT
<213> Homo Sapiens

<400> 523
Thr Pro Gln Leu Asn Gln Arg Arg Gln Asp Glu Leu Gly Ser
1               5                   10


<210> 524
<211> 9
<212> PRT
<213> Homo Sapiens

<400> 524
Asp Thr Leu Asn Asn Glu Asp Met Ala
1               5


<210> 525
<211> 6
<212> PRT
<213> Homo Sapiens

<400> 525
Thr Ile Ala Asn Glu Gly
1               5

<210> 526
<211> 16
<212> PRT
<213> Homo Sapiens

<400> 526
Ala His Gly Gly Ser Val Gly Lys Val Gly Gln Ser Thr Val Asp Ser
1               5                   10                  15


<210> 527
<211> 9
<212> PRT
<213> Homo Sapiens

<400> 527
Asp Met Val Ser Asn Asp Ala Met Gly
1               5


<210> 528
<211> 6
<212> PRT
<213> Homo Sapiens

<400> 528
Ser Ile Gln Asp Ala Asp
1               5


<210> 529
<211> 12
<212> PRT
<213> Homo Sapiens

<400> 529
Thr Gly Asp Met Tyr Lys Glu Pro Pro Val Lys Ser
1               5                   10


<210> 530
<211> 9
<212> PRT
<213> Homo Sapiens

<400> 530
Tyr Thr Val Ser Asn Glu Tyr Met Ser
1               5


<210> 531
<211> 6
<212> PRT
<213> Homo Sapiens

<400> 531

Ser Ile His Asp Ala Asp
1               5


<210> 532
<211> 17
<212> PRT
<213> Homo Sapiens

<400> 532
Thr His Gly Pro Arg Pro Ala Leu Leu Val Gln Ser Ser Glu Val Thr
1               5                   10                  15
Ser




<210> 533
<211> 9
<212> PRT
<213> Homo Sapiens

<400> 533
Asp Thr Ile Ser Asp Gln Ser Met Gly
1               5


<210> 534
<211> 6
<212> PRT
<213> Homo Sapiens

<400> 534
Ser Ile Asp Thr Ser Ser
1               5


<210> 535
<211> 12
<212> PRT
<213> Homo Sapiens

<400> 535
Arg Asp Phe Gly Ile Asn Ala Gln Pro Val Asp Tyr
1               5                   10


<210> 536
<211> 9
<212> PRT
<213> Homo Sapiens

<400> 536
Asp Asn Val Thr Asn Asp Asp Met Gly
1               5


<210> 537
<211> 6

<212> PRT
<213> Homo Sapiens

<400> 537
Thr Ile Asn Thr Pro Gly
 1               5


<210> 538
<211> 14
<212> PRT
<213> Homo Sapiens

<400> 538
Ala Ala Glu Lys Gly Gly Ser Glu Ala Pro Gln Met Thr Ser
 1               5                   10


<210> 539
<211> 9
<212> PRT
<213> Homo Sapiens

<400> 539
Asp Thr Val Ser Tyr Gln Asn Met Gly
 1               5


<210> 540
<211> 6
<212> PRT
<213> Homo Sapiens

<400> 540
Ala Ile Asn Ser Arg Asp
 1               5


<210> 541
<211> 18
<212> PRT
<213> Homo Sapiens

<400> 541
Gly Pro Glu Gly Gly Gln Glu Leu Gly His Ser Ala Asp Pro Lys Leu
 1               5                   10                  15
Gly Ser


<210> 542
<211> 9
<212> PRT
<213> Homo Sapiens

<400> 542
Asp Thr Val Ser Ser Glu Asp Met Gly
 1               5

<210> 543
<211> 6
<212> PRT
<213> Homo Sapiens

<400> 543
Gly Ile Asp Ala Thr Ser
1               5


<210> 544
<211> 14
<212> PRT
<213> Homo Sapiens

<400> 544
Thr Ala Glu Gly Gly Arg Gly Asp Ala Ala Thr Met Asn Ser
1               5                   10


<210> 545
<211> 9
<212> PRT
<213> Homo Sapiens

<400> 545
Asp Met Val Ser His Glu Asp Met Gly
1               5


<210> 546
<211> 6
<212> PRT
<213> Homo Sapiens

<400> 546
Thr Ile Asn Ser Gln Asp
1               5


<210> 547
<211> 11
<212> PRT
<213> Homo Sapiens

<400> 547
Ser Asp Met Thr Glu Asp Glu Thr Leu Asn Tyr
1               5                   10


<210> 548
<211> 9
<212> PRT
<213> Homo Sapiens

<400> 548

Phe Thr Phe Ser Asp Asp Asn Met Gly
1               5

<210> 549
<211> 6
<212> PRT
<213> Homo Sapiens

<400> 549
Ser Ile Asp Ala Arg Asp
1               5

<210> 550
<211> 15
<212> PRT
<213> Homo Sapiens

<400> 550
Gly Gln His Thr Pro Ala Ser Val Leu Pro Asp Gln Val Asn Ser
1               5                   10                  15

<210> 551
<211> 9
<212> PRT
<213> Homo Sapiens

<400> 551
Asp Met Phe Ser Ala Glu Ser Met Gly
1               5

<210> 552
<211> 6
<212> PRT
<213> Homo Sapiens

<400> 552
Ala Ile Asn Val Ala Asn
1               5

<210> 553
<211> 12
<212> PRT
<213> Homo Sapiens

<400> 553
Ser Gly Val Trp Asn Asn Tyr Ser Ala Leu Arg Phe
1               5                   10

<210> 554
<211> 9
<212> PRT
<213> Homo Sapiens

<400> 554
Asp Arg Val Ile Asn Gln Asn Met Gly
1               5

<210> 555
<211> 6
<212> PRT
<213> Homo Sapiens

<400> 555
Ser Ile Glu Glu Asn Asp
1               5

<210> 556
<211> 14
<212> PRT
<213> Homo Sapiens

<400> 556
Thr Cys Ser Ala Val Ala Lys Cys Thr Pro Glu Val Glu Ser
1               5                   10

<210> 557
<211> 9
<212> PRT
<213> Homo Sapiens

<400> 557
Tyr Arg Ile Ser Pro Asp Tyr Met Gly
1               5

<210> 558
<211> 6
<212> PRT
<213> Homo Sapiens

<400> 558
Ser Ile Ser Asn Asn Gly
1               5

<210> 559
<211> 15
<212> PRT
<213> Homo Sapiens

<400> 559
Ser Val Asp Ala Ala Glu Ser Gly Ile Asp Ser Asn Val Gly Ser
1               5                   10                  15

<210> 560
<211> 9

<212> PRT
<213> Homo Sapiens

<400> 560
Asp Lys Ile Thr Ser Lys Asn Met Ser
 1               5


<210> 561
<211> 6
<212> PRT
<213> Homo Sapiens

<400> 561
Thr Ile Pro Ala Glu Gly
 1               5


<210> 562
<211> 12
<212> PRT
<213> Homo Sapiens

<400> 562
Thr Ala Cys Phe Pro Ser Ala Gln His Val Glu Ser
 1               5                   10


<210> 563
<211> 9
<212> PRT
<213> Homo Sapiens

<400> 563
Asp Met Phe Ser Ala Glu Ser Met Gly
 1               5


<210> 564
<211> 6
<212> PRT
<213> Homo Sapiens

<400> 564
Ala Ile Asn Val Ala Asn
 1               5


<210> 565
<211> 12
<212> PRT
<213> Homo Sapiens

<400> 565
Ser Gly Val Trp Asn Asn Tyr Ser Ala Leu Arg Phe
 1               5                   10

<210> 566
<211> 9
<212> PRT
<213> Homo Sapiens

<400> 566
Tyr Thr Leu Ser Asp Glu Tyr Met Gly
1               5


<210> 567
<211> 6
<212> PRT
<213> Homo Sapiens

<400> 567
Ala Ile Asn Asn Lys Asp
1               5


<210> 568
<211> 15
<212> PRT
<213> Homo Sapiens

<400> 568
Ser Trp Lys Ser Ala Val Leu Gly Gly Glu Glu Pro Leu Arg Phe
1               5                   10                  15


<210> 569
<211> 9
<212> PRT
<213> Homo Sapiens

<400> 569
Asp Asn Val Ser Asn Glu Asn Met Gly
1               5


<210> 570
<211> 6
<212> PRT
<213> Homo Sapiens

<400> 570
Ser Ile Asp Met Asp Asp
1               5


<210> 571
<211> 11
<212> PRT
<213> Homo Sapiens

<400> 571
Ala Ser Ser Pro Glu Ala Lys Glu Val Lys Tyr
1               5                   10

<210> 572
<211> 9
<212> PRT
<213> Homo Sapiens

<400> 572
Val Asn Ile Thr Asp Glu Thr Met Gly
1               5


<210> 573
<211> 6
<212> PRT
<213> Homo Sapiens

<400> 573
Gly Ile Glu Thr Ala Ser
1               5


<210> 574
<211> 13
<212> PRT
<213> Homo Sapiens

<400> 574
Gly Gln Lys His Val Phe Gly Asn Tyr Thr Phe Pro Ser
1               5                   10


<210> 575
<211> 9
<212> PRT
<213> Homo Sapiens

<400> 575
Val Lys Leu Asn Tyr Gln Tyr Met Gly
1               5


<210> 576
<211> 6
<212> PRT
<213> Homo Sapiens

<400> 576
Gly Ile Asp Met Gln Asn
1               5


<210> 577
<211> 12
<212> PRT
<213> Homo Sapiens

<400> 577

Ser Gly Asp Asp Cys Phe Tyr Ser Pro Ile Lys Phe
1               5                   10

<210> 578
<211> 9
<212> PRT
<213> Homo Sapiens

<400> 578
Val Arg Leu Asn Asp Gln Asp Met Gly
1               5

<210> 579
<211> 6
<212> PRT
<213> Homo Sapiens

<400> 579
Ser Ile Glu Thr Asp Ser
1               5

<210> 580
<211> 18
<212> PRT
<213> Homo Sapiens

<400> 580
Arg Ala Leu Pro Thr Thr Arg Arg Gly Gly Val Leu Ala Ser Asp Ile
1               5                   10                  15
Gly Ser

<210> 581
<211> 9
<212> PRT
<213> Homo Sapiens

<400> 581
Asp Ser Val Asn His Tyr Asp Met Thr
1               5

<210> 582
<211> 6
<212> PRT
<213> Homo Sapiens

<400> 582
Ser Ile Ala Asp Asn Asn
1               5

<210> 583
<211> 18

<212> PRT
<213> Homo Sapiens

<400> 583
Gly Glu Ser Met Asn Gln Arg Gly Glu Ser Glu Leu Ser Asp Thr Phe
1               5                   10                  15
Arg Phe

<210> 584
<211> 9
<212> PRT
<213> Homo Sapiens

<400> 584
Asp Thr Ile Asn Asp Gln Asn Met Gly
1               5

<210> 585
<211> 6
<212> PRT
<213> Homo Sapiens

<400> 585
Ser Ile Gly Asn Asp Asn
1               5

<210> 586
<211> 19
<212> PRT
<213> Homo Sapiens

<400> 586
Gly Ala Asn Ser Gln Thr Asp Val Leu Val Lys Ala Arg Pro Gln Thr
1               5                   10                  15
Val Glu Ser

<210> 587
<211> 9
<212> PRT
<213> Homo Sapiens

<400> 587
Asp Met Val Asn Pro Gln Thr Met Gly
1               5

<210> 588
<211> 6
<212> PRT
<213> Homo Sapiens

<400> 588

Ala Ile Cys Asp Pro Gly
 1               5


<210> 589
<211> 11
<212> PRT
<213> Homo Sapiens

<400> 589
Gly Gly Val Leu Gln Asp Lys Pro Met Gly Tyr
 1               5                   10


<210> 590
<211> 9
<212> PRT
<213> Homo Sapiens

<400> 590
Val Thr Leu Ser Tyr Thr Asn Met Gly
 1               5


<210> 591
<211> 6
<212> PRT
<213> Homo Sapiens

<400> 591
Ala Ile Glu Thr Gly Ser
 1               5


<210> 592
<211> 14
<212> PRT
<213> Homo Sapiens

<400> 592
Pro Leu Ser Phe Ile Ser Lys Glu Gln Ser Gln Val Asp Ser
 1               5                   10


<210> 593
<211> 9
<212> PRT
<213> Homo Sapiens

<400> 593
Phe Thr Leu Tyr Ser Glu Ile Met Gly
 1               5


<210> 594
<211> 6
<212> PRT
<213> Homo Sapiens

<400> 594
Thr Ile Pro Thr Lys Asn
 1               5


<210> 595
<211> 17
<212> PRT
<213> Homo Sapiens

<400> 595
Ala Gln Gly Gly Gly Gly Val Leu Asp Gln Gly Thr Glu Asp Leu Ser
 1               5                   10                  15
Tyr



<210> 596
<211> 9
<212> PRT
<213> Homo Sapiens

<400> 596
Phe Lys Val Ser Asp Gln Ala Met Ala
 1               5


<210> 597
<211> 6
<212> PRT
<213> Homo Sapiens

<400> 597
Thr Met Ile Asn Thr Asp
 1               5


<210> 598
<211> 11
<212> PRT
<213> Homo Sapiens

<400> 598
Ala Gln Asp Gln Lys Lys Asn Asp Val Lys Ser
 1               5                   10


<210> 599
<211> 9
<212> PRT
<213> Homo Sapiens

<400> 599
Asp Thr Ile Ser Thr Gln Asn Met Gly
 1               5

<210> 600
<211> 6
<212> PRT
<213> Homo Sapiens

<400> 600
Gly Ile His Asp Asp Asp
1               5


<210> 601
<211> 20
<212> PRT
<213> Homo Sapiens

<400> 601
Gly Arg Ile Lys Glu Phe Arg Val Lys Trp Cys Gly Ala Pro Glu Glu
1               5                   10                  15
Gln Val Ser Tyr
            20


<210> 602
<211> 9
<212> PRT
<213> Homo Sapiens

<400> 602
Asp Thr Ile Ile Asp Asp Asn Met Ser
1               5


<210> 603
<211> 6
<212> PRT
<213> Homo Sapiens

<400> 603
Thr Ile Asp Thr Gly Asp
1               5


<210> 604
<211> 17
<212> PRT
<213> Homo Sapiens

<400> 604
Gly Cys Leu Gly Thr Phe Gly Val Asn Gly Leu Pro Ala Asp Leu Gly
1               5                   10                  15
Ser


<210> 605
<211> 9
<212> PRT
<213> Homo Sapiens

<400> 605
Asp Asn Ile Thr Asp Gln Ala Met Gly
1               5

<210> 606
<211> 6
<212> PRT
<213> Homo Sapiens

<400> 606
Gly Ile Pro Asp Gln Ser
1               5

<210> 607
<211> 12
<212> PRT
<213> Homo Sapiens

<400> 607
Gly Tyr Lys Leu Gly Ile Lys Pro His Leu Asp Tyr
1               5                   10

<210> 608
<211> 9
<212> PRT
<213> Homo Sapiens

<400> 608
Val Thr Val Thr Asp Asn Asn Met Gly
1               5

<210> 609
<211> 6
<212> PRT
<213> Homo Sapiens

<400> 609
Thr Ile Arg Asp Asp Asp
1               5

<210> 610
<211> 13
<212> PRT
<213> Homo Sapiens

<400> 610
Gly Ala Asn Pro Thr Gly Lys Ala Ser Arg Leu Ser Ser
1               5                   10

<210> 611
<211> 9

<212> PRT
<213> Homo Sapiens

<400> 611
Asp Lys Leu Asn Asn Lys Val Met Ser
1               5


<210> 612
<211> 6
<212> PRT
<213> Homo Sapiens

<400> 612
Thr Ser Asp Asp Lys Asn
1               5


<210> 613
<211> 13
<212> PRT
<213> Homo Sapiens

<400> 613
Ala Asp Pro Leu Arg Val Arg Gln Gln Pro Val Tyr Ser
1               5                   10


<210> 614
<211> 9
<212> PRT
<213> Homo Sapiens

<400> 614
Asp Lys Val Asn Thr Gln Asn Met Gly
1               5


<210> 615
<211> 6
<212> PRT
<213> Homo Sapiens

<400> 615
Ala Ile Arg Asn Gln Asn
1               5


<210> 616
<211> 14
<212> PRT
<213> Homo Sapiens

<400> 616
Ala Pro Asp Gly Lys Pro Gly Pro Gln His Ser Val Gly Ser
1               5                   10

<210> 617
<211> 9
<212> PRT
<213> Homo Sapiens

<400> 617
Asp Thr Val Ser Thr Asp Ser Met Ser
1               5


<210> 618
<211> 6
<212> PRT
<213> Homo Sapiens

<400> 618
Ser Ile Pro Gly Ser Asn
1               5


<210> 619
<211> 15
<212> PRT
<213> Homo Sapiens

<400> 619
Ser Asn Gly Met Ala Gln Val Gly Gln Asp Glu His Val Arg Ser
1               5                   10                  15


<210> 620
<211> 9
<212> PRT
<213> Homo Sapiens

<400> 620
Asp Thr Val Thr Ala Asp Asn Met Gly
1               5


<210> 621
<211> 6
<212> PRT
<213> Homo Sapiens

<400> 621
Gly Ile Ala Asn Pro Asp
1               5


<210> 622
<211> 16
<212> PRT
<213> Homo Sapiens

<400> 622
Arg Ala Ala Gln Gly Val Gly Asp Phe Gln Pro Ala Thr Ile Ser Tyr
1               5                   10                  15

<210> 623
<211> 9
<212> PRT
<213> Homo Sapiens

<400> 623
Asp Met Val Thr Asp Gln Asn Met Gly
 1               5


<210> 624
<211> 6
<212> PRT
<213> Homo Sapiens

<400> 624
Ser Ile Ser Met Pro Asn
 1               5


<210> 625
<211> 21
<212> PRT
<213> Homo Sapiens

<400> 625
Thr Gln Asp Gly Ile Thr Lys Gly Ile Phe Arg Ala Gly Ala Ala Asp
 1               5                   10                  15
Thr Asn Leu Gln Ser
            20


<210> 626
<211> 9
<212> PRT
<213> Homo Sapiens

<400> 626
Asp Asn Phe Thr Asn His Asp Met Ser
 1               5


<210> 627
<211> 6
<212> PRT
<213> Homo Sapiens

<400> 627
Thr Ile Ser Asp Thr Asp
 1               5


<210> 628
<211> 9
<212> PRT
<213> Homo Sapiens

<400> 628
Ser Ser Asp Ile Gly Thr Arg Arg His
1               5

<210> 629
<211> 9
<212> PRT
<213> Homo Sapiens

<400> 629
Asp Ser Leu Thr Ala Asn Ser Met Asp
1               5

<210> 630
<211> 6
<212> PRT
<213> Homo Sapiens

<400> 630
Gly Ile Asp Thr Ser Asp
1               5

<210> 631
<211> 16
<212> PRT
<213> Homo Sapiens

<400> 631
Ala Ser Gln Arg Gln Ser Gln Pro Ala Leu Glu Tyr Asp Leu Asn His
1               5                   10                  15

<210> 632
<211> 9
<212> PRT
<213> Homo Sapiens

<400> 632
Val Arg Val Asn Asn Gln Asn Met Ser
1               5

<210> 633
<211> 6
<212> PRT
<213> Homo Sapiens

<400> 633
Ala Ile Asp Ser Ala Asn
1               5

<210> 634
<211> 14

<212> PRT
<213> Homo Sapiens

<400> 634
Ala Asp Gly Ala Pro Leu Gly Gly Ser Thr Lys Leu Asp His
 1               5                   10

## Description

### RELATED APPLICATIONS

This application claims the benefit of U.S. Provisional Patent Application No. 60/554,021, filed on March 17, 2004, and of U.S. Provisional Patent Application No. 60/470,340, filed on May 14, 2003. The entire teachings of the above applications are incorporated herein by reference.

### BACKGROUND OF THE INVENTION

Polypeptides have become increasingly important agents in a variety of applications, including use as medical therapeutic and diagnostic agents and in industrial applications. One factor that has hinder further application of polypeptides is their physical and chemical properties. For example, polypeptides generally must retain proper folding to be active. However, polypeptides tend to unfold or denature under storage conditions or conditions where they could find utility (e.g., when exposed to heat, organic solvents). In addition, many polypeptides are produced only with relatively low yield using biological production systems. Accordingly, they can be prohibitively costly to produce.

A key factor that limits further application of polypeptides is the tendency of unfolded or denatured polypeptides to aggregate irreversibly. Aggregation is influenced by polypeptide concentration and is thought to arise in many cases from partially folded or unfolded intermediates. Factors and conditions that favor partially folded intermediates, such as elevated temperature and high polypeptide concentration, promote irreversible aggregation. (Fink, A.L., Folding & Design 3:R1-R23 (1998).) For example, storing purified polypeptides in concentrated form, such as a lyophilized preparation, frequently results in irreversible aggregation of at least a portion of the polypeptides. Also, production of a polypeptide by expression in biological systems, such as *E. coli,* often results in the formation of inclusion bodies which contain aggregated polypeptides. Recovering active polypeptides from inclusion bodies can be very difficult and require adding additional steps, such as a refolding step, to a biological production system.

One approach that has been attempted for preparing polypeptides with improved properties is the selection of polypeptide variants that have improved stability or solubility. (See, e.g., Jung, S. et al., J. Mol. BioL 294:163-180 (1999); Davies, J and Riechmannn, L., Prot. Eng. 9:531-537 (1996); Waldo, G.S., Curr. Opin. Chem. Biol. 7:33-38 (2003).) However, selection for improved stability or solubility does not address the aggregation problem because stability (e.g., thermal stability, thermodynamic stability) and solubility are characteristics of the properly folded polypeptide while aggregation arises from the partially folded or partially denatured state. In addition, there is no recognized correlation between polypeptide stability and aggregation. (Fink, A.L., Folding & Design 3:R1-R23 (1998).)

A need exists for polypeptides with improved properties that can be produced with high yields using biological production systems.

### SUMMARY OF THE INVENTION

The invention relates to polypeptides that unfold reversibly, to repertoires containing polypeptides that unfold reversibly and to libraries that contain polypeptides that unfold reversibly or nucleic acids that encode polypeptides that unfold reversibly. The invention further relates to processes for producing a library enriched in polypeptides that unfold reversibly or nucleic acids encoding polypeptides that unfold reversibly, processes for selecting and/or isolating polypeptides that unfold reversibly, and to methods for producing a polypeptide that unfolds reversibly.

In one aspect, the invention is a process for selecting, isolating and/or recovering a polypeptide that unfolds reversibly from a library or a repertoire of polypeptides (e.g., a polypeptide display system). In one embodiment, the method comprises unfolding a collection of polypeptides (*e.g*., the polypeprides in a library, a repertoire or a polypeptide display system), refolding at least a portion of the unfolded polypeptides, and selecting, isolating and/or recovering a refolded polypeptide. In another embodiment, the method comprises providing a collection of unfolded polypeptides (e.g., the polypeptides in a library, a repertoire or a polypeptide display system), refolding at least a portion of the unfolded polypeptides, and selecting, isolating and/or recovering a refolded polypeptide. In another embodiment, the method comprises providing a polypeptide display system comprising a repertoire, heating the repertoire to a temperature (Ts) at which at least a portion of the displayed polypeptides unfold and cooling the repertoire to a temperature (Tc) that is lower than Ts to produce a cooled repertoire. The cooled repertoire comprises at least a portion of polypeptides that have unfolded and refolded and a portion of polypeptides that have aggregated. The method further comprises recovering at a temperature (Tr) at least one polypeptide that binds a ligand and unfolds reversibly. Preferably the ligand binds folded polypeptide and does not bind aggregated polypeptides, the recovered polypeptide has a melting temperature (Tm), and Ts>Tm>Tc, and Ts>Tm>Tr.

In other aspects, the invention relates to repertoires of polypeptides that unfold reversibly, to libraries of nucleic acids that encode polypeptides that unfold reversibly, and to methods for producing such libraries and repertoires.

In one aspect, the invention is an isolated polypeptide that unfolds reversibly. In some embodiments, the polypeptide that unfolds reversibly is a variant of a parental polypeptide that differs from the parental polypeptide in amino acid sequence (e.g., by one or more amino acid replacements, additions and/or deletions), but qualitatively retains function of the parental polypeptide.

The invention also relates to a process for producing an antibody variable domain library enriched in variable domains that unfold reversibly. In one embodiment, the process comprises (1) providing a phage display system comprising a plurality of displayed antibody variable regions, wherein at least a portion of the displayed variable regions have been unfolded and refolded, (2) selecting phage displaying variable regions that have unfolded, refolded and regained binding function from said phage display system, (3) obtaining nucleic acids encoding CDR1 and /or CDR2 of the variable regions displayed on the recovered phage, and (4) assembling a library of nucleic acids encoding antibody variable domains, wherein said nucleic acids obtained in (3) are operably linked to one or more other nucleic acids to produce a library of constructs that encode antibody variable domains in which CDR1 and/or CDR2 are encoded by the nucleic acid obtained in (3). In particular embodiments, substantially all of the displayed variable regions in (1) have been unfolded by heating to about 80°C and refolded by cooling. In other particular embodiments, library of nucleic acids assembled in (4) encodes an antibody variable domain in which CDR3 is randomized or is not derived from antibody variable regions that has been selected for the ability to unfold reversibly.

The polypeptides that unfold reversibly described herein can be produced as soluble proteins in the supernatant of *E. coli* or yeast cultures with high yield.

### BRIEF DESCRIPTION OF THE DRAWINGS

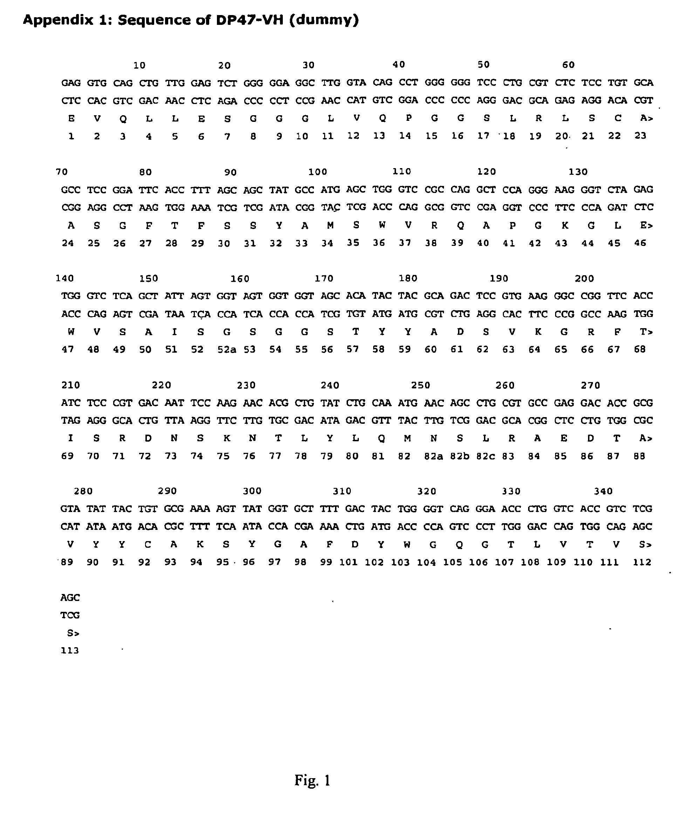

Fig. 1 is an illustration of a nucleic acid encoding human immunoglobulin heavy chain variable region DP47dummy (also referred to herein as DP47d), comprising germline V_{H} gene segment DP47 and germline VJ gene segment JH4b (SEQ ID NO:1, coding strand; SEQ ID NO:2 non-coding strand). Fig. 1 also presents the amino acid sequence of the encoded V_{H} domain (SEQ ID NO:3). The amino acids are numbered according to the system of Kabat. (Kabat, E.A. et al., Sequences of Proteins of Immunological Interest, Fifth Edition, U.S. Department of Health and Human Services, U.S. Government Printing Office (1991).)

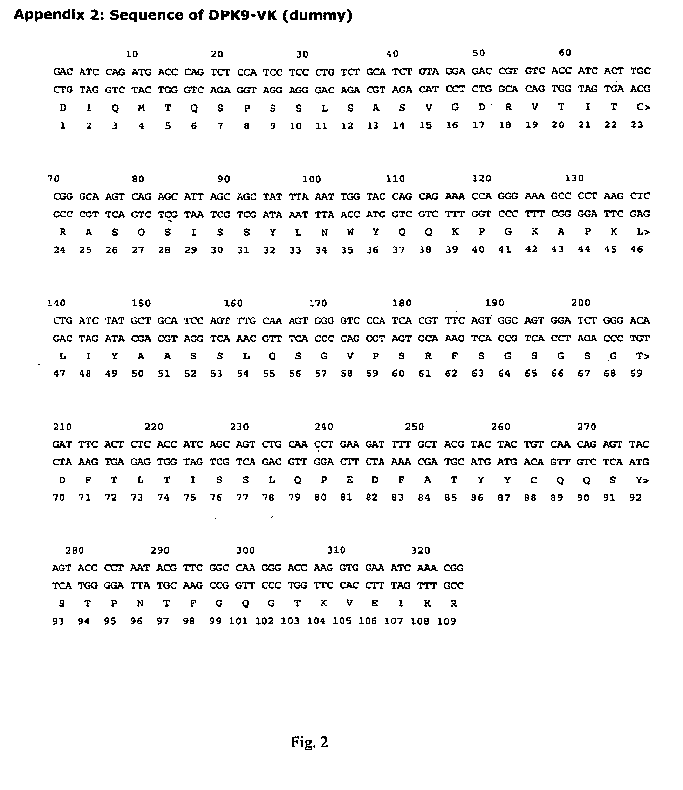

Fig. 2 is an illustration of a nucleic acid encoding human immunoglobulin light chain (κ) variable region DPK9 dummy (also referred to herein as DPK9d), comprising germline V κ gene segment DPK9 and germline JK gene segment JK1 . (SEQ ID NO:4, coding strand; SEQ ID NO:5 non-coding strand). Fig. 2 also presents the amino acid sequence of the encoded V κ domain (SEQ ID NO:6). The amino acids are numbered according to the system of Kabat.

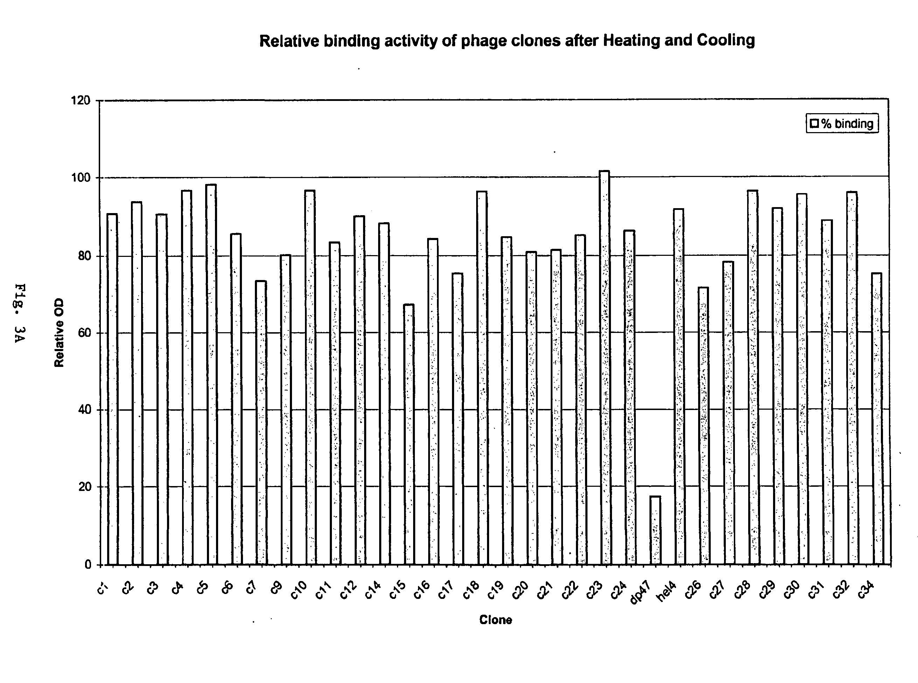

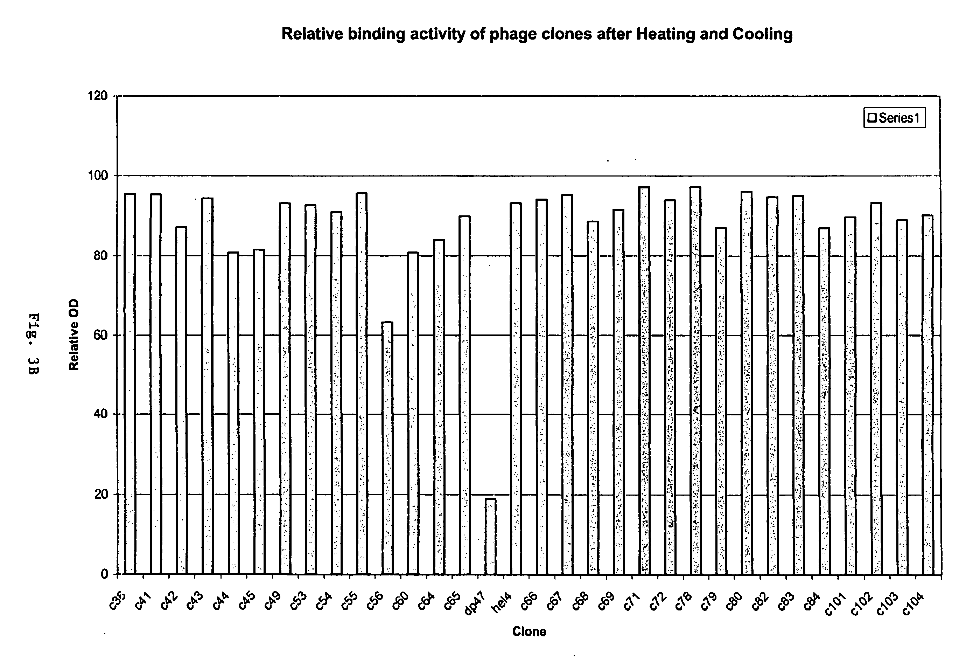

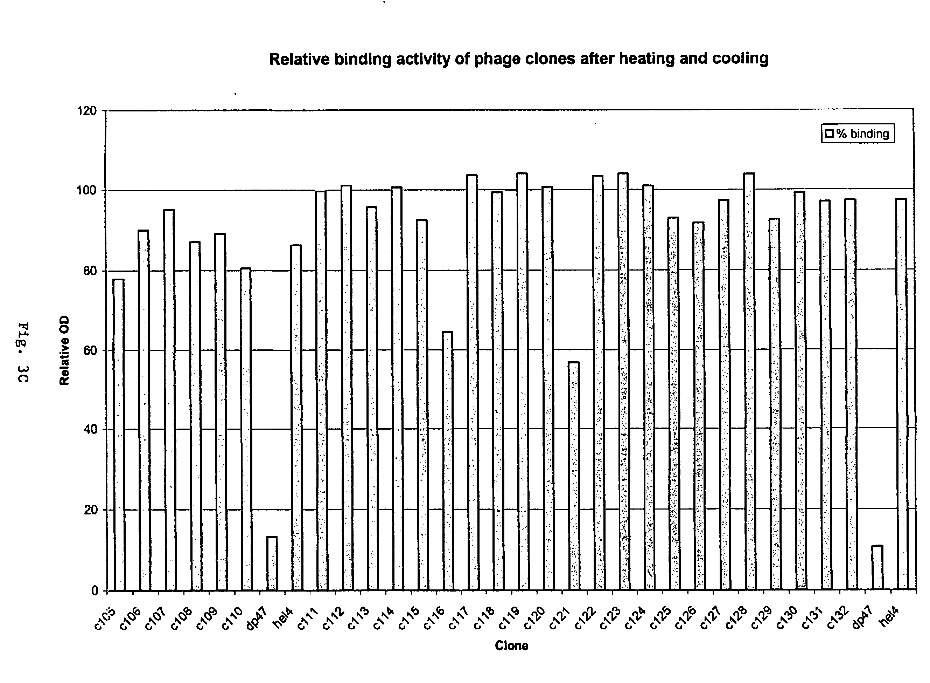

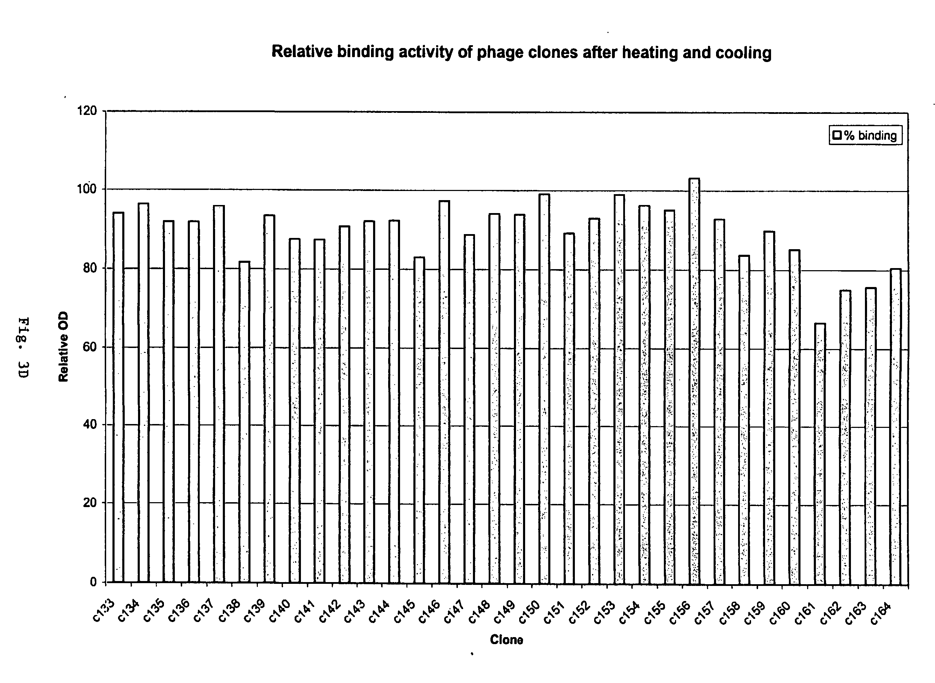

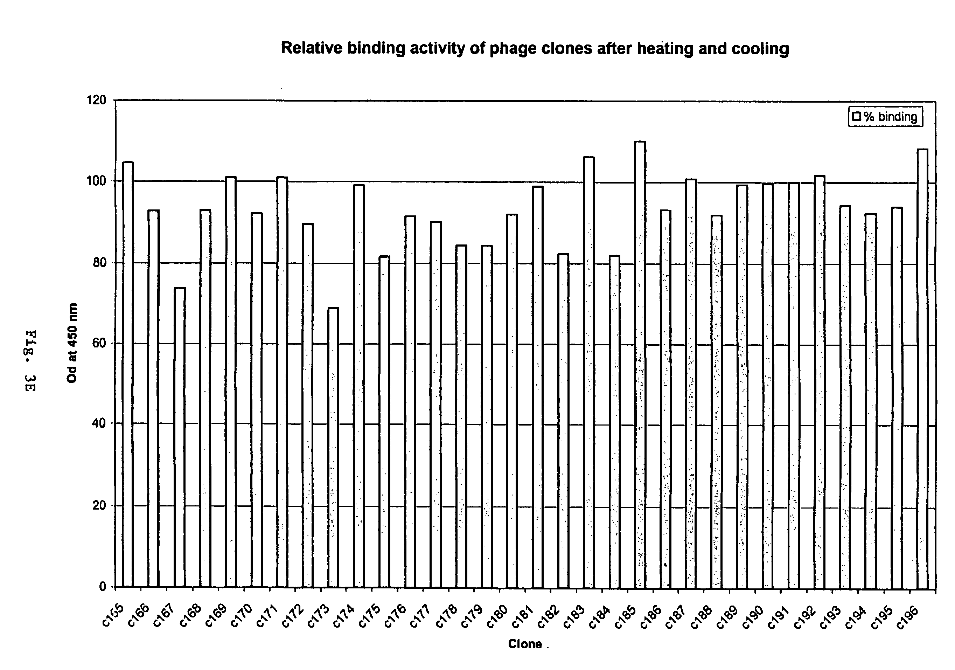

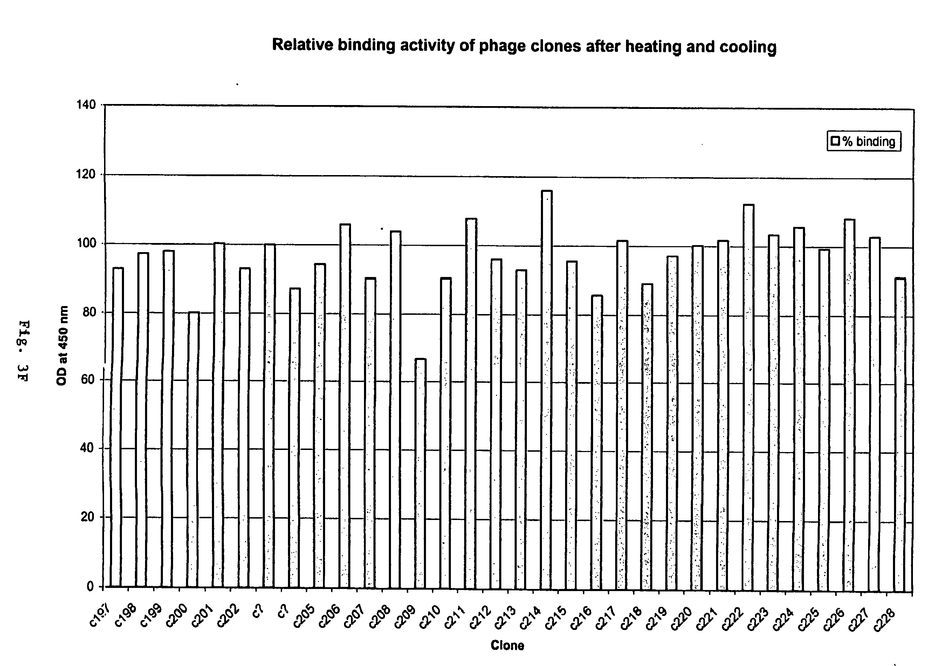

Figs. 3A-3F are histograms that illustrate the relative amount of protein A binding activity retained by phage clones displaying V_{H} domains based on a DP47d scaffold after heat-induced unfolding and refolding. A sample of each phage clone was heated to cause the displayed V_{H} domains to unfold and then cooled to cause refolding, and a sample was left unheated. Binding of the heat-treated and the untreated phage samples to protein A was assessed by ELISA. The binding activity of the heat-treated clones expressed as a percentage of the binding activity of the untreated clones is illustrated. The columns marked "dp47" refer to DP47d.

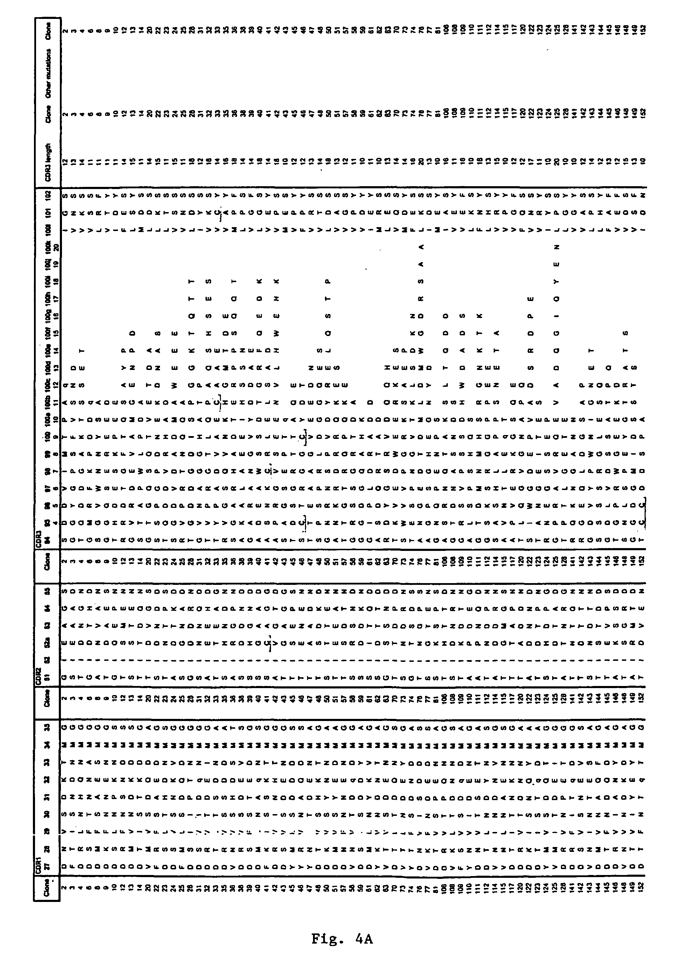

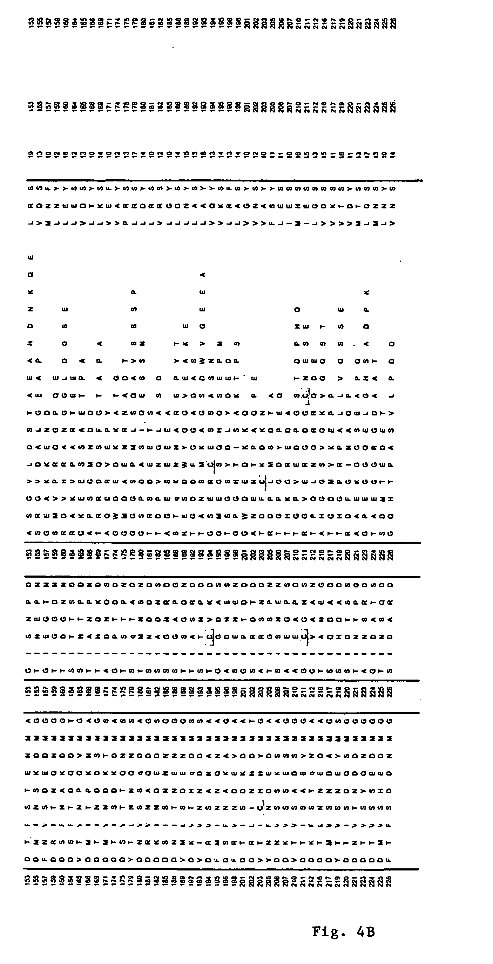

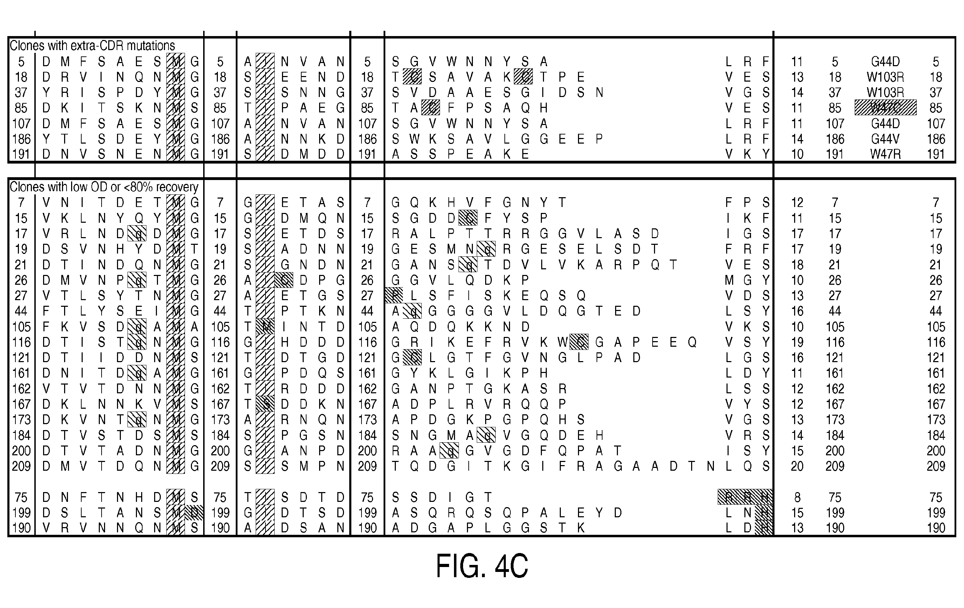

Figs. 4A-4C are panels of a table illustrating the amino acid sequences of the complementarity determining regions (CDR1, CDR2, CDR3) of several of the V_{H} domains displayed on the phage that retained greater than >60% relative binding to protein A in Figs 3A-3F. The sequences are identified in Figs 4A-4C by clone number. These clones are also referred to herein using a "pA-" prefix. For example, Clone 13 is also referred to herein as "pA-C 13." The first group of sequences presented in Fig. 4A and 4B are from clones that had a high degree of refolding as assessed by the results of protein A binding (group 1). The next group of sequences in Fig. 4C are from clones with good refolding. These clones also contain mutations outside the CDRs (group 2). The final group of sequences in Fig. 4C are from clones with lower refolding.

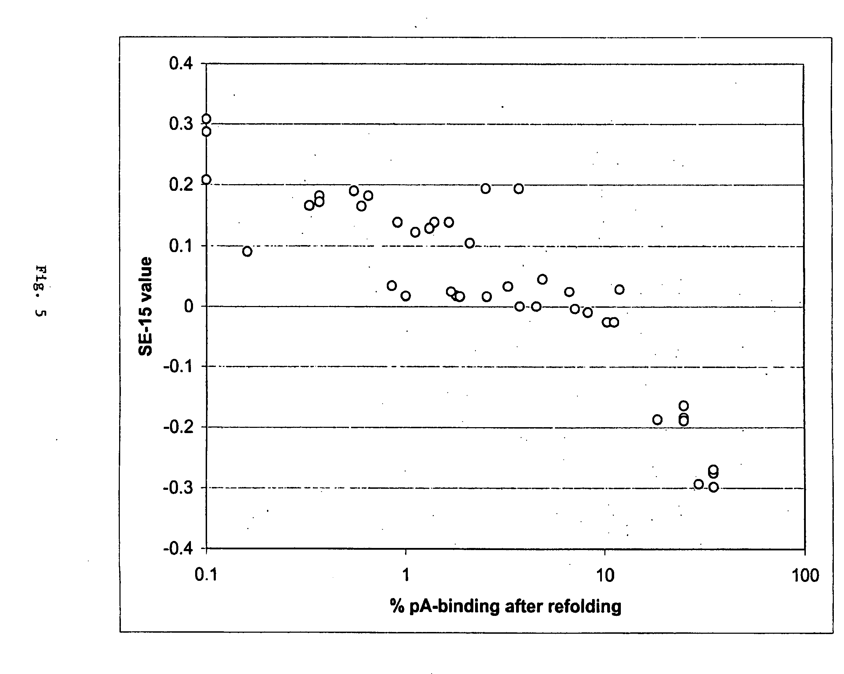

Fig. 5 is a graph illustrating the relationship between the capacity of a displayed V_{H} domain to undergo reversible heat unfolding and the hydrophobicity of the amino acid sequence from position 22 to position 36 of the V_{H} domain. The Sweet/Eisenberg hydrophobicity score (SE-15 value) for the sequence for position 22 to position 36 of several displayed V_{H} was determined using a window of 15 amino acids. The SE-15 value was plotted against the relative protein A binding activity (ELISA) for each clone after undergoing heat-induced unfolding and refolding. The graph illustrates that the ability of a displayed V_{H} to undergo heat-induced unfolding and refolding correlates with an SE-15 value of 0 or less for the amino acid sequence from position 22 to position 36. Amino acid positions are defined according to Kabat.

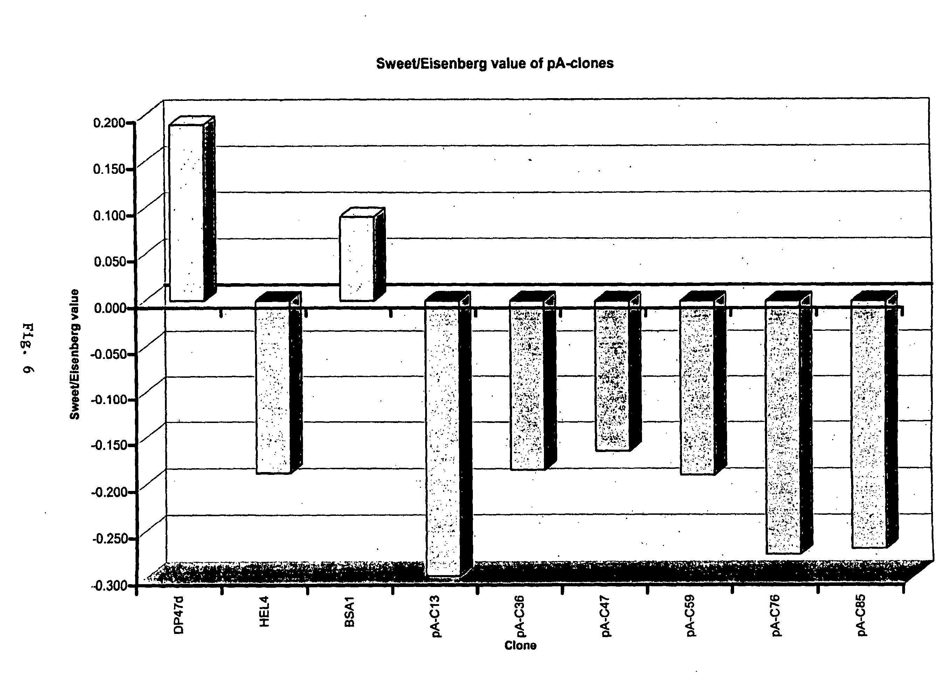

Fig. 6 is a histogram illustrating the Sweet/Eisenberg hydrophobicity score (Sweet/Eisenberg value) for the sequence for position 22 to position 36 of several displayed V_{H}. V_{H} domains DP47d and BSA1 have Sweet/Eisenberg values that are greater than 0, and do not undergo reversible heat-induced unfolding. V_{H} domains HEL4, pA-C13, pA-C36, pA-C47, pA-C59, pA-C76 and pA-C85 have Sweet/Eisenberg values that are less than 0, and the V_{H} domains of each of these clones undergo reversible heat-induced unfolding. Amino acid positions are defined according to Kabat.

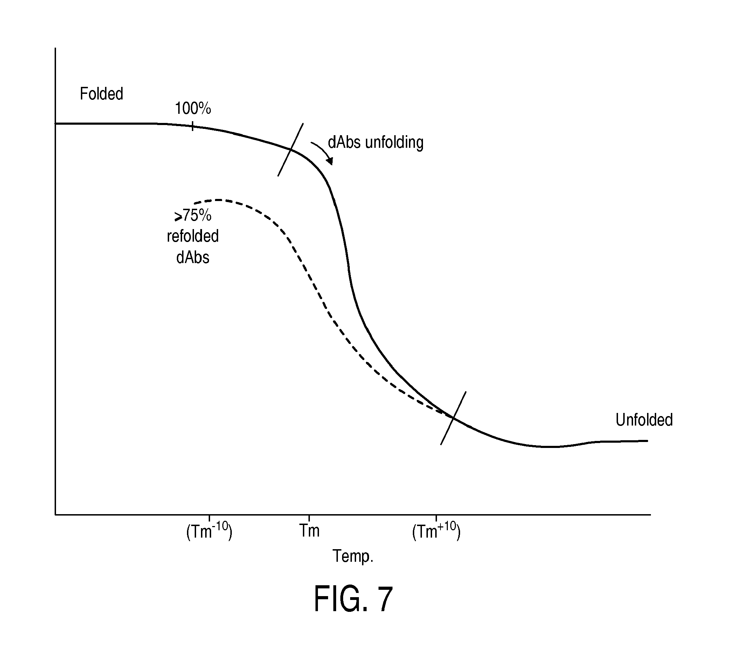

Fig. 7 is an illustration of the characteristic shape of an unfolding curve and a refolding curve. The curves are plotted using a measure of the concentration of properly folded polypeptide (*e.g.*, ellipticity or fluorescence) as the abscissa, and the unfolding agent (e.g., heat (temperature)) as the ordinate. The unfolding and refolding curves include a region in which the polypeptides are folded, an unfolding/refolding transition in which polypeptides are unfolded to various degrees, and a portion in which the polypeptides are unfolded. The y-axis intercept of the refolding curve is the relative amount of refolded protein recovered. In the illustrated plot, TM is the melting temperature of the polypeptide, and TM-10 and TM +10 are the melting temperature of the polypeptide minus 10 degrees and plus 10 degrees, respectively. The illustrated refolding curve indicates that greater than 75% of the polypeptides refolded.

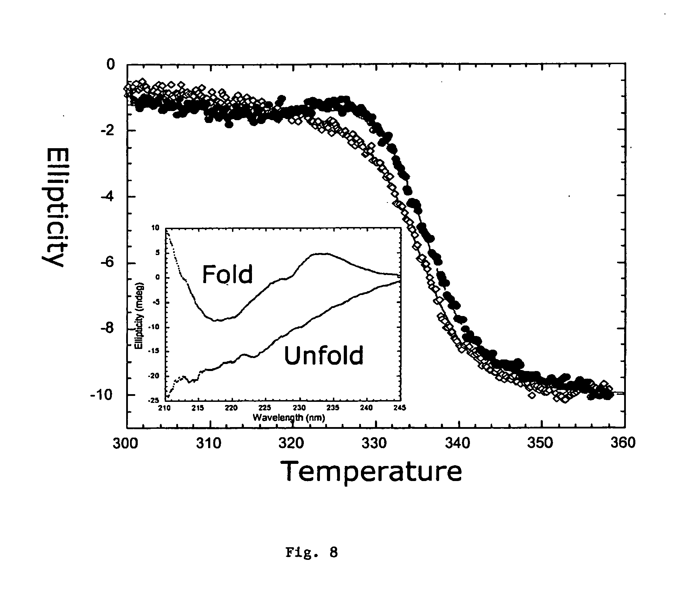

Fig. 8 is a graph showing heat-induced unfolding of dAb HEL4. dAb HEL4 was unfolded by heating and ellipticity assessed during heating (filled circles). The unfolded dAb was then refolded by decreasing the temperature. The refolded dAb was then again unfolded by heating and ellipticity assessed during heating (open diamonds). The graph shows that the unfolding curves of both heat-induced unfoldings are superimposable, demonstrating that dAb HEL4 undergoes reversible heat-induced unfolding. The inset shows the far-UV CD spectra for folded dAb HEL4 at 25°C (Fold) and for unfolded dAb HEL4 at 80°C (Unfold).

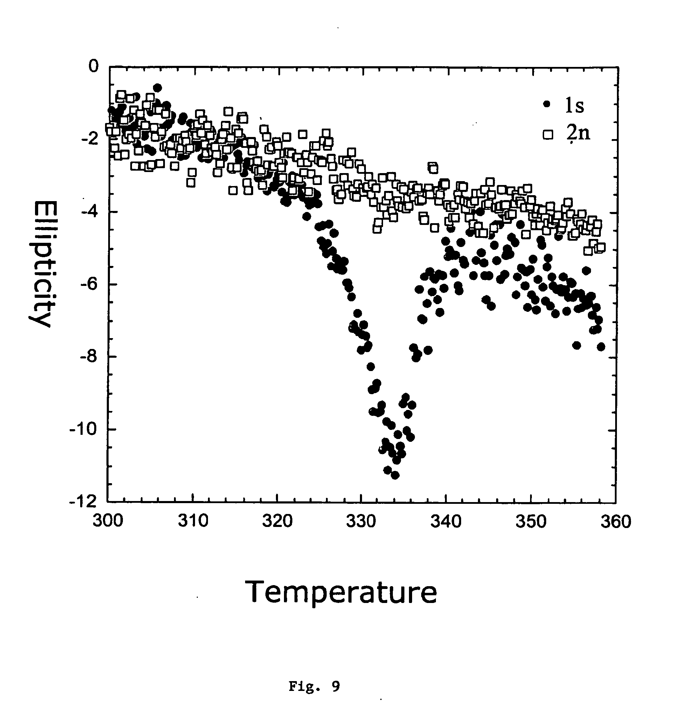

Fig. 9 is a graph showing that a dAb comprising DP47 variant in which Trp47 was replace with Arg (DP47-W47R) does not unfold reversibly upon heating. dAb DP47-W47R was unfolded by heating and ellipticity assessed during heating (filled circles). The unfolded dAb was then refolded by decreasing the temperature. The refolded dAb was then again unfolded by heating and ellipticity assessed during heating (open squares). The unfolding curves are not superimposable and have a shape characteristic of denatured polypeptides.

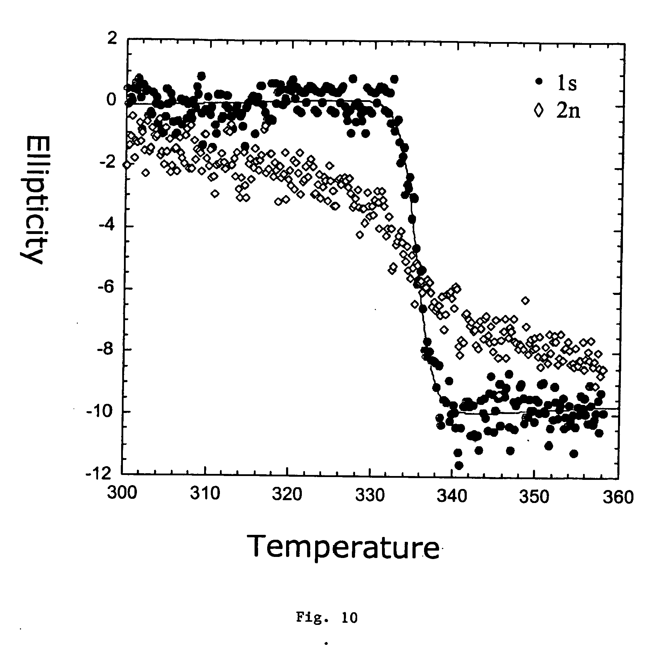

Fig. 10 is a graph showing that a dAb comprising DP47 variant in which Ser35 was replaced with Gly (DP47-S47G) unfolds reversibly upon heating to a limited extent. dAb DP47- S47G was unfolded by heating and ellipticity assessed during heating (filled circles). The unfolded dAb was then refolded by decreasing the temperature. The refolded dAb was then again unfolded by heating and ellipticity assessed during heating (open diamonds). The unfolding curves are not superimposable and reveal that a proportion of the ellipticity (and hence a portion of the original secondary structure) was recovered upon refolding, and that a melting transition is observed upon re-heating the sample.

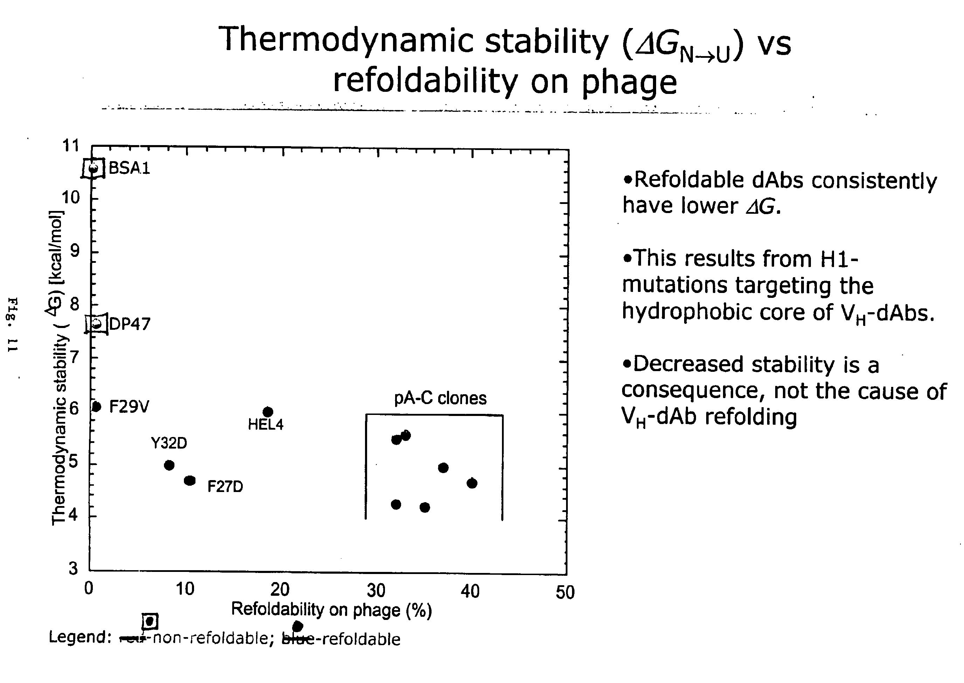

Fig. 11 is a graph illustrating the relationship between thermodynamic stability of polypeptides (ΔG folded→ unfolded) and reversible unfolding of polypeptides displayed on phage. The graph shows that non-refoldable polypeptides BSA1 and DP47d have high thermodynamic stability, and that refoldable polypeptide HEL4 and several refoldable mutants of DP47 have lower thermodynamic stability. Thermodynamic stability of refoldable polypeptides was determined from ellipticity data obtained during heat-induced unfolding. Thermodynamic stability of polypeptides that were not refoldable was determined by monitoring fluorescence during urea-induced unfolding.

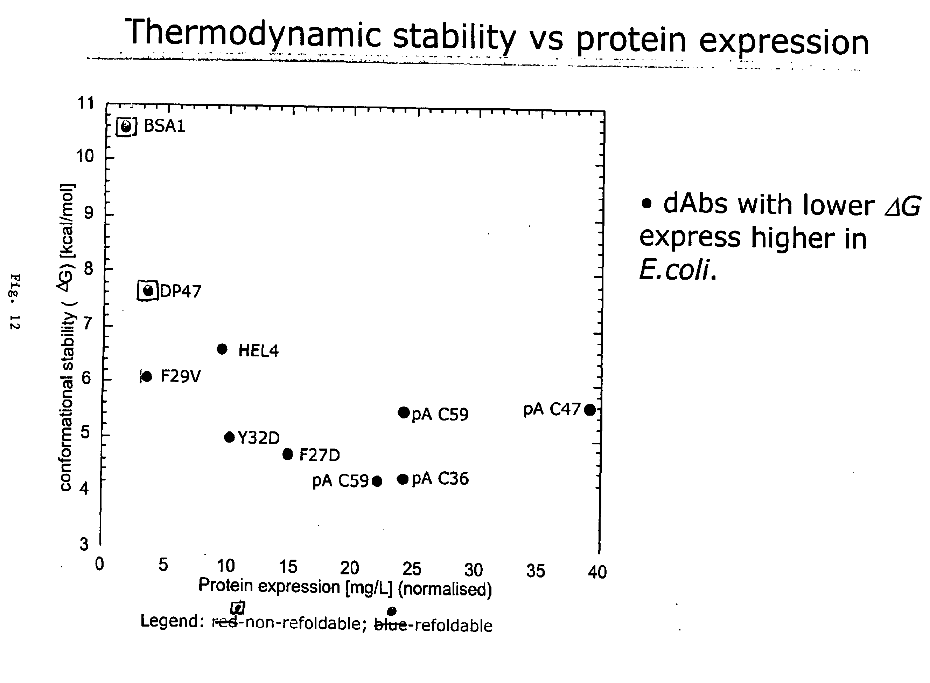

Fig. 12 is a graph illustrating the relationship between thermodynamic stability of polypeptides (ΔG folded→ unfolded) and protein expression level in E. coli supernatant. The graph shows that non-refoldable polypeptides BSA1 and DP47 have high thermodynamic stability but are expressed at relatively low levels, and that refoldable polypeptide HEL4 and several refoldable mutants of DP47d have lower thermodynamic stability but are expressed at relatively high levels. Thermodynamic stability of refoldable polypeptides was determined from ellipticity data obtained during heat-induced unfolding. Thermodynamic stability of polypeptides that were not refoldable was determined by monitoring fluorescence during urea-induced unfolding. Protein expression is the amount of polypeptide purified using protein A sepharose from a 1L culture of *E. coli,* normalized to cell density (OD600) of the culture.

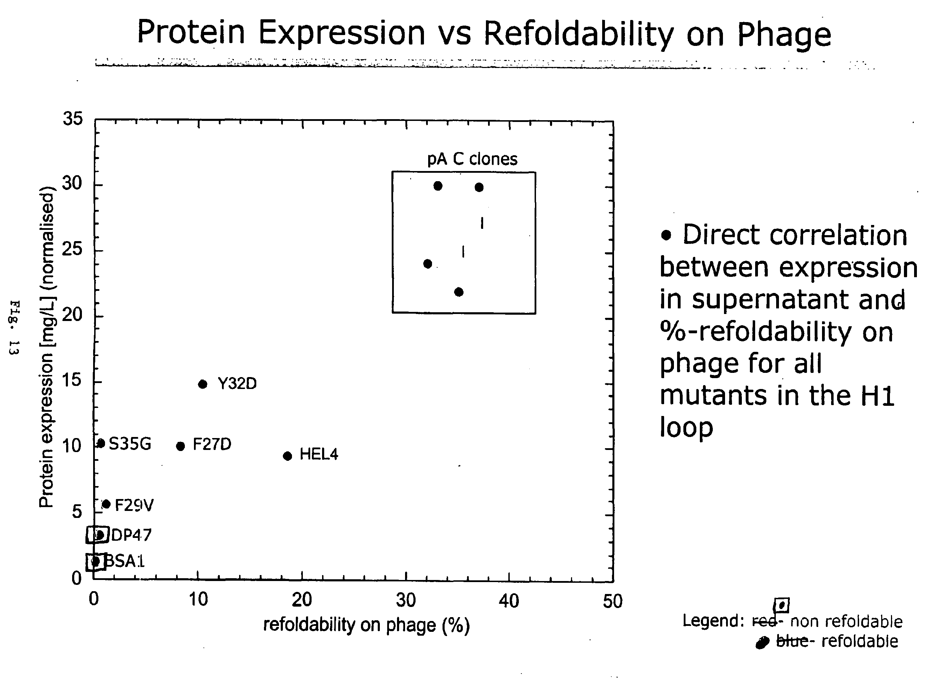

Fig. 13 is a graph illustrating the relationship between protein expression levels in *E*. *coli* supernatant and reversible unfolding of polypeptides displayed on phage. The graph shows that non-refoldable polypeptides BSA1 and DP47d are expressed at relatively low levels, and that refoldable polypeptide HEL4 and several refoldable mutants of DP47d have are expressed at relatively high levels. The graph reveals a direct correlation between reversible unfolding of polypeptides displayed on phage and the quantity of polypeptide in the supernatant of cultures of *E. coli* that express the polypeptide. Protein expression is the amount of polypeptide purified using protein A sepharose from a 1L culture of *E. coli,* normalized to cell density (OD600) of the culture.

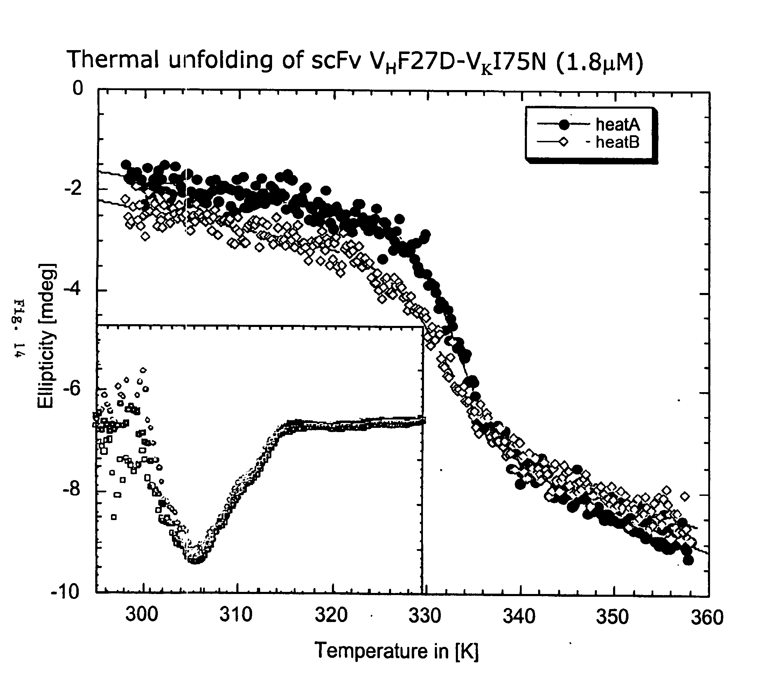

Fig. 14 is a graph showing heat-induced unfolding of a single chain Fv (scFv) containing a reversibly unfoldable Vκ (DPK9-I75N) and a reversibly unfoldable V_{H} (DP47-F27D). The scFv was unfolded by heating and ellipticity assessed during heating (filled circles). The unfolded scFv was then refolded by decreasing the temperature. The refolded scFv was then again unfolded by heating and ellipticity assessed during heating (open diamonds). The graph shows that the unfolding curves of both heat-induced unfoldings are superimposable, demonstrating that scFv undergoes reversible heat-induced unfolding. Other scFvs containing germline V_{H} and germline Vκ, germline V_{H} and unfoldable Vκ (DPK9-175N), or reversibly unfoldable V_{H} (DP47-F27D) and germline Vκ aggregated under the conditions used. The inset shows the far-UV CD spectra for folded scFv before heat induced unfolding (dark trace) and for folded scFV following heat induced unfolding and refolding (lighter trace). The spectra are superimposable indicating that the scFv regained all secondary structure following refolding.

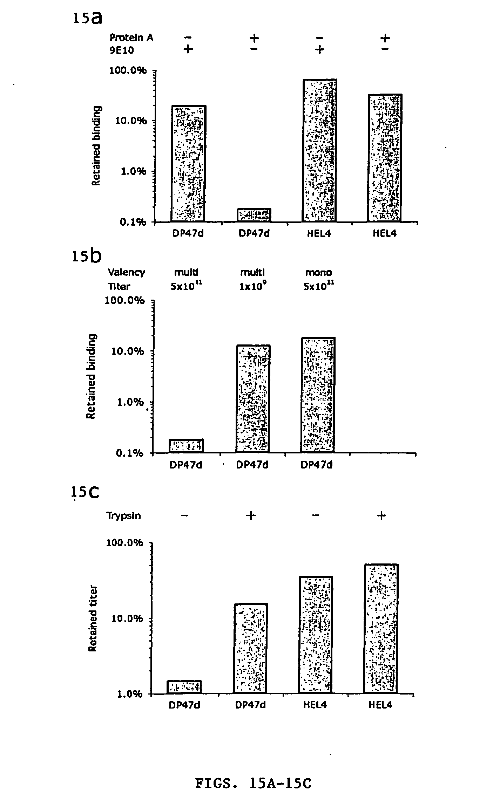

Figs. 15A and 15B are histograms showing the effect of heat-treatment of phage (80°C for 10 min then cooled to 4°C for 10 min) on binding of displayed dAbs to protein A or antibody. In FIG. 15A, phage displaying (5x10¹¹ TU/ml) either DP47d or HEL4 dAb multivalently were assayed for binding to either anti-c-myc antibody (9E10) or protein A by ELISA. 9E10 recognises the c-myc tag peptide tag appended to the dAb as a linear determinant. Using a dilution series of phage, the retained binding was calculated from the phage titers required for an ELISA signal of 0.5 OD₆₅₀ₙₘ-OD₄₅₀ₙₘ (titer untreated/titer heat treated). In FIG. 15B, phage displaying DP47d were assayed for binding to protein A by ELISA. Phage concentration was high (5x10¹¹ TU/ml) or low (1x10⁹ TU/ml) and DP47d was displayed in multivalent or monovalent states.
Fig.15C is a histogram showing the effect of heat-treatment of phage (80°C for 10 min then cooled to 4°C for 10 min) on infectivity. Phage displaying either DP47d or HEL4 multivalently were heated and then cooled, after 10 min a phage sample was treated with 0.9 mg/mL trypsin at 22°C for 10 min, and then used to infect *E. coli* TG1 cells.

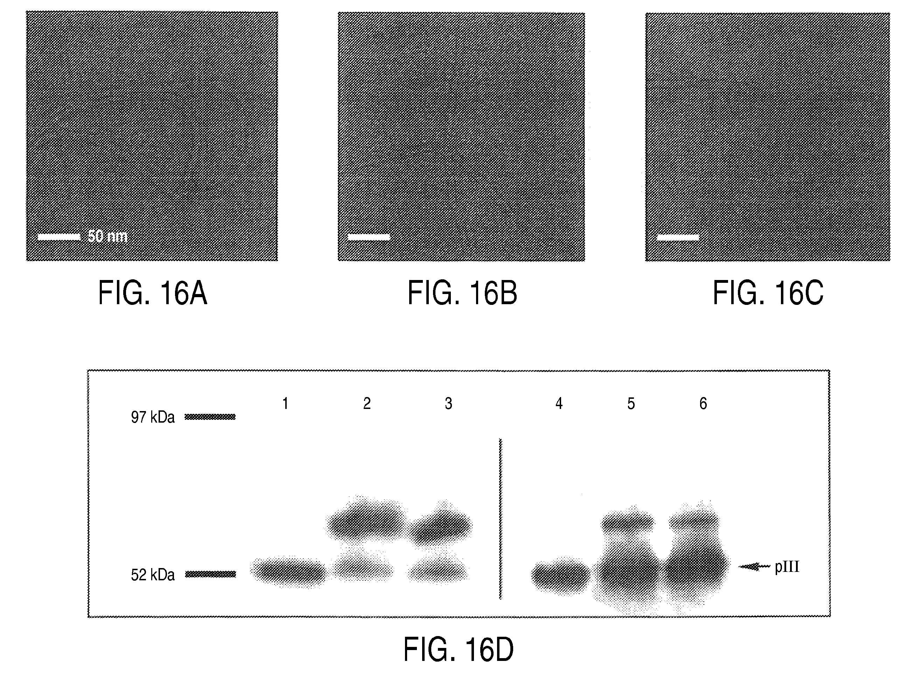

Figs. 16A - 16C are copies of transmission electron micrographs of negatively stained phage tips before and after heat treatment (10 min at 80°C). FIGS 16A and 16B show that heated DP47d phages form aggregates. However, as shown in FIG. 16C, heated HEL4 phages which display the HEL4 VH domain, which unfolds reversibly, did not form aggregates.
Fig. 16D is a copy of an image of a Western blot in which 10¹⁰ transducing units (TU) of phage per lane were separated and detected using an anti-pIII mouse monoclonal antibody. The phage loaded in lanes I to 6 were: fd, HEL4 (multivalent), DP47d (multivalent), M13, HEL4 (monovalent), and DP47d (monovalent), respectively.

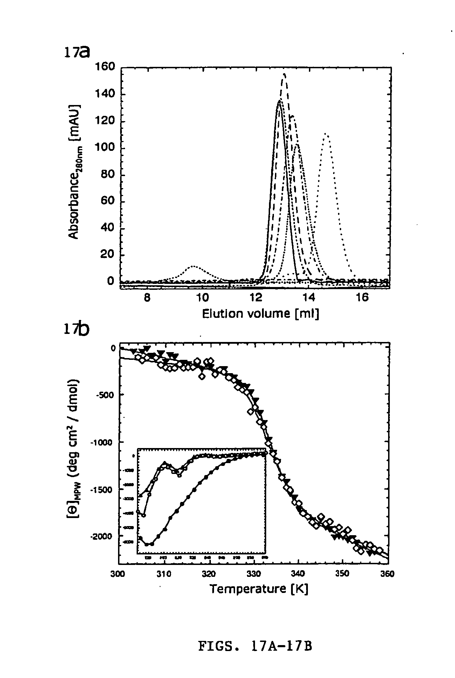

Fig. 17A is a representative analytical gel-filtration chromatagram of selected human V_{H}3 dAbs. The chromatograms for C13 (———), C36 (———), C47 (———), C59 (———), C76 (———)and C85 (.........) dAbs (10 µM in PBS) were obtained using a SUPERDEX-75 column (Amersham Biosciences), apparent Mr for C13, C36, C47, C59, C76 and C85 were 22 kDa, 17 kDa, 19 kDa, 10 kDa, 20 kDa, and 15 kDa, respectively.
Fig. 17B is a graph showing heat induced denaturation curves of the C36 dAb (5 µM in PBS) recorded by CD at 235 nm: ▼, mean residual ellipticity upon first heating; ◊, mean residual ellipticity upon second heating. Inset: CD spectra of dAb HEL4 (5 µM in PBS) in the far-UV region at different temperature: ▲ , 25°C before unfolding; ●, 85°C (unfolded polypeptide); O, 25°C after sample cooling.

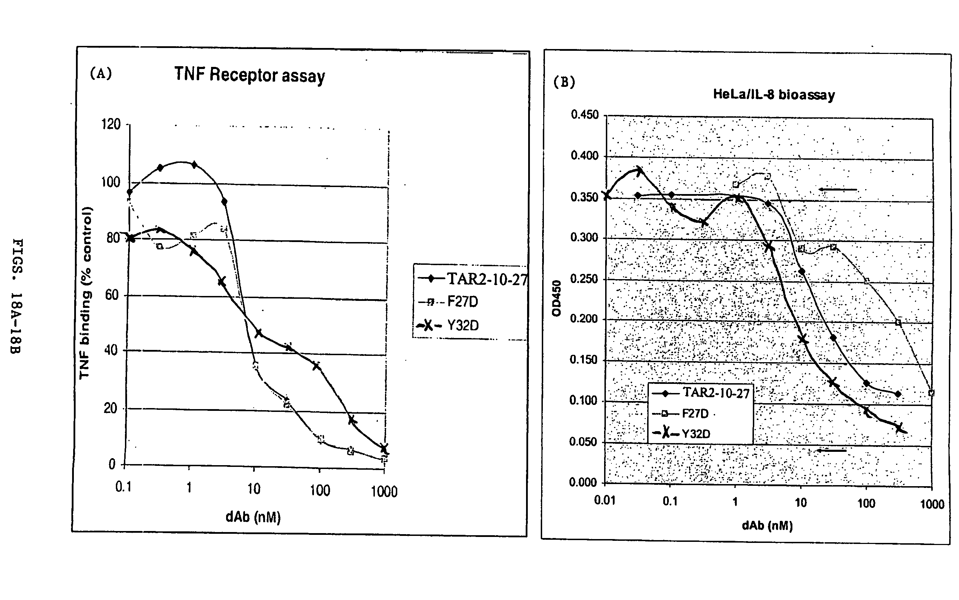

Fig. 18A is a graph showing that TAR2-10-27 and variants TAR2-10-27 F27D and TAR2-10-27 Y23D bind human Tumor Necrosis Factor Receptor 1 (TNF1) and inhibit binding of TNF to the receptor in a receptor binding ELISA. TNFR1 was immobilized on the plate and TNF was mixed with TAR2-10-27, TAR2-10-27 F27D or TAR2-10-27 Y23D and then added to the wells. The amount of TNF that bound the immobilized receptor was quantified using an anti-TNF antibody. TAR2-10-27, TAR2-10-27 F27D and TAR2-10-27 Y23D each bound human TNFR1 and inhibited the binding of TNF to the receptor.
Fig. 18B is a graph showing that TAR2-10-27 and variants TAR2-10-27 F27D and TAR2-10-27 Y23D bound human Tumor Necrosis Factor Receptor 1 (TNFR1) expressed on HeLa cells and inhibited TNF-induced production of IL-8 in an *in vitro* assay. HeLa cells were plated in microtitre plates and incubated overnight with TAR2-10-27, TAR2-10-27 F27D or TAR2-10-27 Y23D and 300 pg/ml TNF. Post incubation, the supernatant was aspirated off the cells and the amount of IL-8 in the supernatant was measured using a sandwich ELISA. TAR2-10-27, TAR2-10-27 F27D and TAR2-10-27 Y23D each bound human TNFR1 and inhibited TNF-induced IL-8 production.

### DETAILED DESCRIPTION OF THE INVENTION

The invention relates to polypeptides that unfold reversibly and to methods for selecting and/or designing such polypeptides. Polypeptides that unfold reversibly provide several advantages. Notably, such polypeptides are resistant to aggregation or do not aggregate. Due to this resistance to aggregation, polypeptides that unfold reversibly can readily be produced in high yield as soluble proteins by expression using a suitable biological production system, such as E. *coli.* In addition, polypeptides that unfold reversibly can be formulated and/or stored at higher concentrations than conventional polypeptides, and with less aggregation and loss of activity.

As described herein, polypeptides that unfold reversibly can be selected from a polypeptide display system in which the polypeptides have been unfolded *(e.g.,* by heating) and refolding (*e.g*., by cooling). The selection and design processes described herein yields polypeptides that unfold reversibly and are resistant to aggregation. These selection and design processes are distinct from methods that select polypeptides based on enhanced stability, such as polypeptides that remain folded at elevated temperature. (See, *e.g.,* Jung, S. et al., J. Mol. Biol. 294:163-180 (1999).)

As used herein, "polypeptide display system" refers to a system in which a collection of polypeptides are accessible for selection based upon a desired characteristic, such as a physical, chemical or functional characteristic. The polypeptide display system can be a suitable repertoire of polypeptides (*e*.*g*., in a solution, immobilized on a suitable support). The polypeptide display system can also be a biochemical system that employs a cellular expression system (*e.g.*, expression of a library of nucleic acids in, e.g., transformed, infected, transfected or transduced cells and display of the encoded polypeptides on the surface of the cells) or an acellular expression system (e.g., emulsion compartmentalization and display). Preferred polypeptide display systems link the coding function of a nucleic acid and physical, chemical and/or functional characteristics of a polypeptide encoded by the nucleic acid. When such a polypeptide display system is employed, polypeptides that have a desired physical, chemical and/or functional characteristic can be selected and a nucleic acid encoding the selected polypeptide can be readily isolated or recovered. A number of polypeptide display systems that link the coding function of a nucleic acid and physical, chemical and/or functional characteristics of a polypeptide are known in the art, for example, bacteriophage display (phage display), ribosome display, emulsion compartmentalization and display, yeast display, puromycin display, bacterial display, polypeptide display on plasmid and covalent display and the like. (See, e.g., EP 0436597 (Dyax), U.S. Patent No. 6,172,197 (McCafferty et al.*),* U.S. Patent No. 6,489,103 (Griffiths et al.).) The polypeptide display system can comprise a plurality of replicable genetic display packages, as described by McCafferty *et al.* (*e.g.,* WO 92/01047; U.S. Patent No. 6,172,197). A replicable genetic display package (RGDP) is a biological particle which has genetic information providing the particle with the ability to replicate. An RGDP can display on its surface at least part of a polypeptide. The polypeptide can be encoded by genetic information native to the RGDP and/or artificially placed into the RGDP or an ancestor of it. The RGDP can be a virus e.g., a bacteriophage, such as fd or M13. For example, the RGDP can be a bacteriophage which displays an antibody variable domain (e.g., V_{H}, V_{I},) at its surface. This type of RGDP can be referred to as a phage antibody (pAb).

As used herein, the terms "reversibly unfoldable" and "unfolds reversibly" refer to polypeptides that can be unfolded (e.g., by heat) and refolded in the method of the invention. A reversibly unfoldable polypeptide (a polypeptide that unfolds reversibly) loses function when unfolded but regains function upon refolding. Such polypeptides are distinguished from polypeptides that aggregate when unfolded or that improperly refold (misfolded polypeptides), *i.e*., do not regain function. Preferably, a polypeptide that unfolds reversibly can unfold reversibly when displayed in a polypeptide display system, for example, when display on bacteriophage. Particularly preferred polypeptide that unfold reversibly can unfold reversibly when displayed in a polypeptide display system and as a soluble polypeptide (e.g., an autonomous soluble polypeptide).

As used herein, "repertoire of polypeptides" refers to a collection of polypeptides that are characterized by amino acid sequence diversity. The individual members of a repertoire can have common features, such as common structural features (e.g., a common core structure) and/or common functional features (e.g., capacity to bind a common ligand *(e.g.,* a generic ligand or a target ligand)).

As used herein, "library" refers to a collection of heterogeneous nucleic acids that can be expressed and preferably are replicable. For example, a library can contain a collection of heterogeneous nucleic acids that are incorporated into a suitable vector, such as an expression plasmid, a phagemid and the like. Expression of such a library can produce a repertoire of polypeptides. "Library" also refers to a collection of heterogeneous polypeptides that are displayed in a polypeptide display system that links coding function of a nucleic acid and physical, chemical and/or functional characteristics of a polypeptide encoded by the nucleic acid, and can be selected or screened to provide an individual polypeptide (and nucleic acid encoding same) or a population of polypeptides (and nucleic acids encoding same) that have a desired physical, chemical and/or functional characteristic. A collection of phage that displays a collection of heterogeneous polypeptides is one example of such a library. A library that is a collection of heterogeneous polypeptides encompasses a repertoire of polypeptides.

As used herein, "functional" describes a polypeptide that is properly folded so as to have a specific desired activity, such as ligand-binding activity (*e.g*., binding generic ligand, binding target ligand), or the biological activity of native or naturally produced protein. For example, the term "functional polypeptide" includes an antibody or antigen-binding fragment thereof that binds a target antigen through its antigen-binding site, and an enzyme that binds its substrate(s).

As used herein, "generic ligand" refers to a ligand that binds a substantial portion (e.g., substantially all) of the functional members of a given repertoire. A generic ligand (e.g., a common generic ligand) can bind many members of a given repertoire even though the members may not have binding specificity for a common target ligand. In general, the presence of a functional generic ligand-binding site on a polypeptide (as indicated by the ability to bind a generic ligand) indicates that the polypeptide is correctly folded and functional. Accordingly, polypeptides that are correctly folded can be selected or recovered from a repertoire of polypeptides by binding to a generic ligand. Suitable examples of generic ligands include superantigens, antibodies that bind an epitope expressed on a substantial portion of functional members of a repertoire, and the like.

As used herein, "target ligand" refers to a ligand which is specifically or selectively bound by a polypeptide. For example, a target ligand can be a ligand for which a specific binding polypeptide or polypeptides in a repertoire are identified. For example, when a polypeptide is an antibody or antigen-binding fragment thereof, the target ligand can be any desired antigen or epitope, and when a polypeptide is an enzyme, the target ligand can be any desired substrate. Binding to the target antigen is dependent upon the polypeptide being functional, and upon the specificity of the target antigen-binding site of the polypeptide.

As used herein, "antibody polypeptide" is a polypeptide that is an antibody, a portion of an antibody, or a fusion protein that contains a portion of an antibody (e.g., an antigen-binding portion). Thus, "antibody polypeptides" include, for example, an antibody (e.g., an IgG), an antibody heavy chain, an antibody light chain, homodimers and heterodimers of heavy chains and/or light chains, and antigen-binding fragments or portions of an antibody, such as a Fv fragment (*e.g*., single chain Fv (scFv), a disulfide bonded Fv), a Fab fragment, a Fab' fragment, a F(ab')₂ fragment, a single variable domain (V_{H}, V_{L}), a dAb and the like.

As used herein, "antibody format" refers to any suitable polypeptide structure in which an antibody variable domain can be incorporated so as to confer binding specificity for antigen on the structure. A variety of suitable antibody formats are known in the art, such as, chimeric antibodies, humanized antibodies, human antibodies, single chain antibodies, bispecific antibodies, antibody heavy chains, antibody light chains, homodimers and heterodimers of antibody heavy chains and/or light chains, antigen-binding fragments of any of the foregoing (e.g., a Fv fragment (e.g., single chain Fv (scFv), a disulfide bonded Fv), a Fab fragment, a Fab' fragment, a F(ab')2 fragment), a single variable domain (*e.g*., V_{H}, V_{L}), a dAb, and modified versions of any of the foregoing (e.g., modified by the covalent attachment of polyethylene glycol or other suitable polymer).

"Superantigen" is a term of art that refers to generic ligands that interact with members of the immunoglobulin superfamily at a site that is distinct from the conventional ligand-binding sites of these proteins. Staphylococcal enterotoxins are examples of superantigens which interact with T-cell receptors. Superantigens that bind antibodies include Protein G, which binds the IgG constant region (Bjorck and Kronvall, J. Immunol., 133:969 (1984)); Protein A which binds the IgG constant region and V_{H} domains (Forsgren and Sjoquist, J. Immunol., 97:822 (1966)); and Protein L which binds V_{L} domains (Bjorck, J. Immunol., 140:1194 (1988)).

As used herein, "unfolding agent" refers to an agent (e.g., compound) or to energy that can cause polypeptide unfolding. When an unfolding agent is a compound, the compound can be added to a polypeptide display system in an amount sufficient to cause a desired degree of polypeptide unfolding. Examples of suitable compounds include, chaotropic agents (e.g., guanidine hydrochloride, urea), acids (e.g., hydrochloric acid, acetic acid), bases (e.g., sodium hydroxide, potassium hydroxide), and organic solvents (*e.g.*, an alcohol (e.g., methanol, ethanol), a ketone (*e.g*., methyl ethyl ketone), an aldehyde (e.g., formaldehyde, dimethylformaldehyde), tetrahydrofuran, dioxane, toluene and the like). The unfolding agent can be energy, such as heat and/or pressure. When the unfolding agent is heat, the polypeptide system is exposed to a sufficient amount of energy (e.g., thermal, electromagnetic) to impart enough heat to the polypeptide display system to raise the temperature of the system to a temperature that is sufficient to cause a desired degree of polypeptide unfolding. Preferred unfolding agents (or combinations of unfolding agents) do not substantially inhibit aggregation of unfolded polypeptides that do not unfold reversibly.

As used herein, "folding gatekeeper" refers to an amino acid residue that, by virtue of its biophysical characteristics and by its position in the amino acid sequence of a protein, prevents the irreversible formation of aggregates upon protein unfolding. A folding gatekeeper residue blocks off-pathway aggregation, thereby ensuring that the protein can undergo reversible unfolding. A folding gate keeper generally reduces the SE score (hydrophobicity score) of the amino acid sequence of the region in which it is found.

As used herein "secretable" or "secreted" means that when a polypeptide is produced by expression in E. *coli.,* it is produced and exported to the periplasmic space or to the medium.

### ASSESSING UNFOLDING AND REFOLDING

Polypeptide unfolding and refolding can be assessed, for example, by directly or indirectly detecting polypeptide structure using any suitable method. For example, polypeptide structure can be detected by circular dichroism (CD) (*e.g.*, far-UV CD, near-UV CD), fluorescence (*e.g*., fluorescence of tryptophan side chains), susceptibility to proteolysis, nuclear magnetic resonance (NMR), or by detecting or measuring a polypeptide function that is dependent upon proper folding. In one example, polypeptide unfolding is assessed using a functional assay in which loss of binding function (*e.g*., binding a generic and/or target ligand, binding a substrate) indicates that the polypeptide is unfolded.

The extent of unfolding and refolding of a soluble polypeptide can determined by using an unfolding or denaturation curve. An unfolding curve can be produced by plotting the unfolding agent (e.g., temperature, concentration of chaotropic agent, concentration of organic solvent) as the ordinate and the relative concentration of folded polypeptide as the abscissa. The relative concentration of folded polypeptide can be determined directly or indirectly using any suitable method *(e.g.,* CD, fluorescence, binding assay). For example, a polypeptide solution can be prepared and ellipticity of the solution determined by CD. The ellipticity value obtained represents a relative concentration of folded polypeptide of 100%. The polypeptide in the solution is then unfolded by incrementally adding unfolding agent (e.g., heat, a chaotropic agent) and ellipticity is determined at suitable increments (*e.g*., after each increase of one degree in temperature). The polypeptide in solution is then refolded by incrementally reducing the unfolding agent and ellipticity is determined at suitable increments. The data can be plotted to produce an unfolding curve and a refolding curve. As shown in Fig. 7, the unfolding and refolding curves have a characteristic shape that includes a portion in which the polypeptide molecules are folded, an unfolding/refolding transition in which polypeptide molecules are unfolded to various degrees, and a portion in which the polypeptide molecules are unfolded. The y-axis intercept of the refolding curve is the relative amount of refolded protein recovered. A recovery of at least about 50%, or at least about 60%, or at least about 70%, or at least about 75%, or at least about 80%, or at least about 85%, or at least about 90%, or at least about 95% is indicative that the polypeptide unfolds reversibly.

In a preferred embodiment, the soluble polypeptide unfolds reversibly when heated. Reversibility of unfolding of the soluble polypeptide is determined by preparing a polypeptide solution and plotting heat unfolding and refolding curves for the polypeptide. The peptide solution can be prepared in any suitable solvent, such as an aqueous buffer that has a pH suitable to allow the peptide to dissolve (*e.g*., pH that is about 3 units above or below the isoelectric point (pI)). The polypeptide solution is concentrated enough to allow unfolding/folding to be detected. For example, the polypeptide solution can be about 0.1 µM to about 100 µM, or preferably about 1 µM to about 10 µM.

If the melting temperature (Tm) of the soluble polypeptide is known, the solution can be heated to about ten degrees below the Tm (Tm-10) and folding assessed by ellipticity or fluorescence (*e.g*., far-UV CD scan from 200 nm to 250 nm, fixed wavelength CD at 235 nm or 225 nm; tryptophan fluorescent emission spectra at 300 to 450 nm with excitation at 298 nm) to provide 100% relative folded polypeptide. The solution is then heated to at least ten degrees above Tm (Tm+10) in predetermined increments (*e.g*., increases of about 0.1 to about 1 degree), and ellipticity or fluorescence is determined at each increment. Then, the polypeptide is refolded by cooling to at least Tm-10 in predetermined increments and ellipticity or fluorescence determined at each increment. If the melting temperature of the polypeptide is not known, the solution can be unfolded by incrementally heating from about 25°C to about 100°C and then refolded by incrementally cooling to at least about 25°C, and ellipticity or fluorescence at each heating and cooling increment is determined. The data obtained can be plotted to produce an unfolding curve and a refolding curve, in which the y-axis intercept of the refolding curve is the relative amount of refolded protein recovered.

Some polypeptides unfold reversibly as soluble polypeptides, but not as displayed polypeptides (e.g., displayed as phage coat protein fusion proteins on the surface of a bacteriophage). However, polypeptides that undergo reversible unfolding as displayed polypeptides generally also undergo reversible unfolding when prepared as soluble polypeptides. Thus, reversible unfolding in the context of a displayed polypeptide is highly advantageous and affords the ability to select polypeptides that are reversibly unfoldable as soluble polypeptides from a repertoire or library of displayed polypeptides.

Unfolding and refolding of polypeptides that are contained within a polypeptide display system, for example, polypeptides displayed on bacteriophage, can be assessed by detecting polypeptide function that is dependent upon proper folding. For example, a polypeptide display system comprising displayed polypeptides that have a common function, such as binding a common ligand (*e.g.*, a generic ligand, a target ligand, a substrate), can be unfolded and then refolded, and refolding can be assessed using a functional assay. For example, whether a polypeptide that has a binding activity unfolds reversibly can be determined by displaying the polypeptide on a bacteriophage and measuring or determining binding activity of the displayed polypeptide. The displayed polypeptide can be unfolded by heating the phage displaying the polypeptide to about 80°C, and then refolded by cooling the phage to about 20°C or about room temperature, and binding activity of the refolded polypeptide can be measure or determined. A recovery of at least about 50%, or at least about 60%, or at least about 70%, or at least about 75%, or at least about 80%, or at least about 85%, or at least about 90%, or at least about 95% of the binding activity is indicative that the polypeptide unfolds reversibly. In a preferred embodiment, the displayed polypeptide comprises an antibody variable domain, and binding to a generic ligand (*e.g*., protein A, protein L) or a target ligand is determined.

The polypeptides disclosed herein unfold reversibly in solution and/or when displayed in a suitable polypeptide display system at a polypeptide concentration of at least about 1 µM to about 1 mM, at least about 1 µM to about 500 µM, or at least about 1 µM to about 100 µM. For example, certain single human antibody variable domains can unfold reversibly when displayed in a multimeric phage display system that produces a local concentration (on the phage tip) of displayed antibody variable domain polypeptide of about 0.5 mM. In particular embodiments, the polypeptides unfold reversibly in solution or when displayed on the phage tip at a polypeptide concentration of about 10 µM, about 20 µM, about 30 pM, about 40 µM, about 50 µM, about 60 µM, about 70 µM, about 80 µM, about 90 µM, about 100 µM, about 200 µM, about 300 µM, about 400 µM or about 500 µM.

### SELECTION METHODS

In one aspect, the invention is a process for selecting, isolating and/or recovering a polypeptide that unfolds reversibly from a library or a repertoire of polypeptides (*e.g*., a polypeptide display system). In one embodiment, the method comprises unfolding a collection of polypeptides (e.g., the polypeptides in a library, a repertoire or a polypeptide display system), refolding at least a portion of the unfolded polypeptides, and selecting, isolating and/or recovering a refolded polypeptide. In another embodiment, the method comprises providing a collection of unfolded polypeptides (e.g., the polypeptides in a library, a repertoire or a polypeptide display system), refolding at least a portion of the unfolded polypeptides, and selecting, isolating and/or recovering a refolded polypeptide.

### Polypeptide Display Systems

Preferably, the polypeptide that unfolds reversibly is selected, isolated and/or recovered from a repertoire of polypeptides in a suitable polypeptide display system. For example, a polypeptide that unfolds reversibly can be selected, isolated and/or recovered from a repertoire of polypeptides that is in solution, or is covalently or noncovalently attached to a suitable surface, such as plastic or glass (e.g., microtiter plate, polypeptide array such as a microarray). For example an array of peptides on a surface in a manner that places each distinct library member (e.g., unique peptide sequence) at a discrete, predefined location in the array can be used. The identity of each library member in such an array can be determined by its spatial location in the array. The locations in the array where binding interactions between a target ligand, for example, and reactive library members occur can determined, thereby identifying the sequences of the reactive members on the basis of spatial location. (See, e.g., U.S. Patent No. 5,143,854, WO 90/15070 and WO 92/10092.)

Preferably, the method employs a polypeptide display system that links the coding function of a nucleic acid and physical, chemical and/or functional characteristics of the polypeptide encoded by the nucleic acid. Preferably, the polypeptide display system comprises a library, such as a bacteriophage display library. Bacteriophage display is a particularly preferred polypeptide display system.

A number of suitable bacteriophage display systems (e.g., monovalent display and multivalent display systems) have been described. (See, *e.g.,* Griffiths et al., U.S. Patent No. 6,555,313 B1 (incorporated herein by reference); Johnson et al., U.S. Patent No. 5,733,743 (incorporated herein by reference); McCafferty et al., U.S. Patent No. 5,969,108 (incorporated herein by reference); Mulligan-Kehoe, U.S. Patent No. 5,702,892 (incorporated herein by reference); Winter, G. et al., Annu. Rev. Immunol. 12:433-455 (1994); Soumillion, P. et al., Appl. Biochem. Biotechnol. 47(2-3):175-189 (1994); Castagnoli, L. et al., Comb. Chem. High Throughput Screen, 4(2):121-133 (2001).) The polypeptides displayed in a bacteriophage display system can be displayed on any suitable bacteriophage, such as a filamentous phage (e.g., fd, M13, F1), a lytic phage (*e*.*g*., T4, T7, lambda), or an RNA phage (*e.g*., MS2), for example.

Generally, a library of phage that displays a repertoire of polypeptides, as fusion proteins with a suitable phage coat protein, is produced or provided. Such a library can be produced using any suitable methods, such as introducing a library of phage vectors or phagemid vectors encoding the displayed polypetides into suitable host bacteria, and culturing the resulting bacteria to produce phage (e.g., using a suitable helper phage or complementing plasmid if desired). The library of phage can be recovered from such a culture using any suitable method, such as precipitation and centrifugation.

The polypeptide display system can comprise a repertoire of polypeptides that contains any desired amount of diversity. For example, the repertoire can contain polypeptides that have amino acid sequences that correspond to naturally occurring polypeptides expressed by an organism, group of organisms, desired tissue or desired cell type, or can contain polypeptides that have random or randomized amino acid sequences. If desired, the polypeptides can share a common core or scaffold. For example, all ploypeptides in the repertoire or library can be based on a scaffold selected from protein A, protein L, protein G, a fibronectin domain, an anticalin, CTLA4, a desired enzyme (e.g., a polymerase, a cellulase), or a polypeptide from the immunoglobulin superfamily, such as an antibody or antibody fragment (*e.g.,* a V_{H,} a V_{L}). The polypeptides in such a repertoire or library can comprise defined regions of random or randomized amino acid sequence and regions of common amino acid sequence. In certain embodiments, all or substantially all polypeptides in a repertoire are of a desired type, such as a desired enzyme (*e.g*., a polymerase) or a desired antigen-binding fragment of an antibody (e.g., human V_{H} or human V_{L}). In preferred embodiments, the polypeptide display system comprises a repertoire of polypeptides wherein each polypeptide comprises an antibody variable domain. For example, each polypeptide in the repertoire can contain a V_{H}, a V_{L} or an Fv (*e.g*., a single chain Fv).

Amino acid sequence diversity can be introduced into any desired region of a desired polypeptide or scaffold using any suitable method. For example, amino acid sequence diversity can be introduced into a target region, such as a complementarity determining region of an antibody variable domain or a hydrophobic domain, by preparing a library of nucleic acids that encode the diversified polypeptides using any suitable mutagenesis methods (*e.g*., low fidelity PCR, oligonucleotide-mediated or site directed mutagenesis, diversification using NNK codons) or any other suitable method. If desired, a region of a polypeptide to be diversified can be randomized.

The size of the polypeptides that make up the repertoire is largely a matter of choice and uniform polypeptide size is not required. Generally, the polypeptides in the repertoire contain at least about nine amino acid residues and have secondary structure. Preferably, the polypeptides in the repertoire have at least tertiary structure (form at least one domain).

The repertoires of polypeptides comprise polypeptides that unfold reversibly and can be unfolded using a suitable unfolding agent as described herein. A repertoire of polypeptides can be enriched in polypeptides that unfold reversibly and, for example, are secretable when expressed in *E*. *coli.* Generally, at least about 10% of the polypeptides contained in such an enriched repertoire unfold reversibly. More preferably, at least about 20%, or at least about 30%, or at least about 40%, or at least about 50%, or at least about 60%, or at least about 70%, or at least about 80%, or at least about 90% of the polypeptides in the enriched repertoire unfold reversibly. Preferred repertoires contain polypeptides that unfold reversibly when heated.

In certain embodiments, substantially all polypeptides in the repertoire share a common selectable characteristic (e.g., physical characteristic, chemical characteristic, functional characteristic). Preferably, the common selectable characteristic is dependent upon proper folding and distinguishes properly folded polypeptides from unfolded and misfolded polypeptides. For example, the common selectable characteristic can be a characteristic such as binding affinity which allows properly folded polypeptides to be distinguished from and selected over misfolded and unfolded polypeptides. In certain embodiments, the common selectable characteristic can be used to select properly folded polypeptides but is absent from unfolded and misfolded polypeptides. For example, a repertoire of polypeptides in which substantially all polypeptides in the repertoire have a common functional characteristic that distinguishes properly folded polypeptides from unfolded and misfolded polypeptides, such as a common binding function (e.g., bind a common generic ligand, bind a common target ligand, bind (or are bound by) a common antibody), a common catalytic activity or resistant to proteolysis (*e.g.,* proteolysis mediated by a particular protease) can be used in the method. In other embodiments, a repertoire of polypeptides in which substantially all polypeptides that unfold reversibly in the repertoire have a common selectable characteristic that distinguishes properly folded polypeptides from unfolded and misfolded polypeptides can be used in the method.

In particular embodiments, the polypeptide display system comprises a library of polypeptides that comprise immunoglobulin variable domains (*e.g.,* V_{H}, V_{L}). The variable domains can be based on a germline sequence (*e.g.,* DP47dummy (SEQ ID NO:3, DPK9 dummy (SEQ ID NO:6)) and if desired can have one or more diversified regions, such as the complementarity determining regions. Other suitable germline sequence for V_{H} include, for example, sequences encoded by the V_{H} gene segments DP4, DP7, DP8, DP9, DP10, DP31, DP33, DP45, DP46, DP49, DP50, DP51, DP53, DP54, DP65, DP66, DP67, DP68 and DP69, and the JH segments JH1, JH2, JH3, JH4, JH4b, JH5 and JH6. Other suitable germline sequence for V_{L} include, for example, sequences encoded by the Vκ gene segments DPK1, DPK2, DPK3, DPK4, DPK5, DPK6, DPK7, DPK8, DPK9, DPK10, DPK12, DPK13, DPK15, DPK16, DPK18, DPK19, DPK20, DPK21, DPK22, DPK23, DPK24, DPK25, DPK26 and DPK 28, and the Jκ segements Jκ 1, Jκ 2, Jκ 3, Jκ 4 and Jκ 5.

One or more of the framework regions (FR) of the variable domain can comprise (a) the amino acid sequence of a human framework region, (b) at least 8 contiguous amino acids of the amino acid sequence of a human framework region, or (c) an amino acid sequence encoded by a human germline antibody gene segment, wherein said framework regions are as defined by Kabat. In certain embodiments, the amino acid sequence of one or more of the framework regions is the same as the amino acid sequence of a corresponding framework region encoded by a human germline antibody gene segment, or the amino acid sequences of one or more of said framework regions collectively comprise up to 5 amino acid differences relative to the amino acid sequence of said corresponding framework region encoded by a human germline antibody gene segment.

In other embodiments, the amino acid sequences of FR1, FR2, FR3 and FR4 are the same as the amino acid sequences of corresponding framework regions encoded by a human germline antibody gene segment, or the amino acid sequences of FR1, FR2, FR3 and FR4 collectively contain up to 10 amino acid differences relative to the amino acid sequences of corresponding framework regions encoded by said human germline antibody gene segments. In other embodiments, the amino acid sequence of said FR1, FR2 and FR3 are the same as the amino acid sequences of corresponding framework regions encoded by said human germline antibody gene segment.

The polypeptides comprising a variable domain preferably comprise a target ligand binding site and/or a generic ligand binding site. In certain embodiments, the generic ligand binding site is a binding site for a superantigen, such as protein A, protein L or protein G.

The variable domain can be based on any desired variable domain, for example a human V_{H} (*e.g.*, V_{H} 1a, V_{H} 1b, V_{H} 2, V_{H} 3, V_{H} 4, V_{H} 5, V_{H} 6), a human Vλ (*e.g.*, VλI, VλII, VλII VλIV, VλV or VλVI) or a human Vκ (*e.g*., Vκ1, Vκ2, Vκ3, Vκ4, Vκ5, Vκ6, Vκ7, Vκ8, Vκ9 or Vκ10). Preferably, the variable domain is not a *Camelid* immunoglobulin domain, such as a V_{H} H, or contain one or more amino acids (e.g., frame work amino acids) that are unique to *Camelid* immunoglobulin variable domains encoded by germline sequences but not, for example, to human immunoglobulin variable domains. (See, e.g., Davies et al., Protein Engineering 9:531-537 (1996); Tanha et al., J. Biol. Chem. 276:24774-24780 (2001); Riechmann et al., J. Immunol. Methods 23:25-38 (1999).) In one embodiment, the V_{H} that unfolds reversibly does not comprise one or more amino acids that are unique to murine (e.g., mouse) germline framework regions. Preferably, the variable domain unfolds reversibly when heated.

The isolated polypeptide comprising a variable domain can be an antibody format Thus, in certain embodiments, the isolated polypeptide comprising a variable domain that unfolds reversibly can be a homodimer of variable domain, a heterodimer comprising a variable domain, an Fv, a scFv, a disulfide bonded Fv, a Fab, a single variable domain or a variable domain fused to an immunoglobulin Fc portion.

In one embodiment the polypeptide display system comprises polypeptides that are nucleic acid polymerases, such as variants of a thermostable DNA polymerase (e.g., Taq polymerase.)

### Unfolding and Refolding

The polypeptides (*e.g*., displayed polypeptides) can be unfolded using any desired unfolding agent. Suitable unfolding agents include, for example, heat and/or pressure, low or high pH, chaotropic agents (e.g., guanidine hydrochloride, urea and the like) and organic solvents (*e.g*., an alcohol (e.g., methanol, ethanol), a ketone (e.g., methyl ethyl ketone), an aldehyde (*e.g.,* formaldehyde, dimethylformaldehyde), tetrahydrofuran, dioxane, toluene and the like). In certain embodiments, the displayed polypeptides are unfolded using an unfolding agent, with the proviso that the unfolding agent is not a chaotropic agent. Generally, unfolding is effectuated by exposing the collection of polypeptides to an amount of unfolding agent (e.g., heat) that is sufficient to cause at least about 10% of the polypeptides in the collection to unfold. In particular embodiments, unfolding is effectuated by exposing the collection of polypeptides to an amount of unfolding agent (e.g., heat) that is sufficient to cause at least about 20%, or at least about 30%, or at least about 40%, or at least about 50%, or at least about 60% or at least about 70%, or at least about 80%, or at least about 90%, or at least about 95%, or at least about 98%, or at least about 99%, or substantially all of the polypeptides in the collection to unfold.

In practice, the polypeptides in the repertoire would have a range of melting temperatures (Tm). A Tm (e.g., for use in methods described herein) can be obtained for a repertoire by obtaining a random sample of about 10 to about 100 polypetides from the repertoire and determining the average Tm for the polypeptides in the sample.

Preferably, the displayed polypeptides are unfolded by heating the polypeptide display system to a suitable unfolding temperature (Ts), such as a temperature that is sufficient to cause at least about 10% of the displayed polypeptides to unfold. A temperature that is sufficient to cause a desired percentage of displayed polypeptides to unfold can readily determined using any suitable methods, for example by reference to an unfolding and/or refolding curve (as described herein). When it is desirable to unfold substantially all displayed polypeptides, the lowest temperature that falls within the unfolded portion of the unfolding and refolding curve for the polypeptide system will generally be sufficient. The temperature selected will be dependent upon the thermostability of the displayed polypeptides. For example, an unfolding temperature of 100°C or higher can be used if the displayed polypeptides are from, or are variants of polypeptides from, a thermophile or extreme thermophile (*e.g*., *Thermus aquaticus*). When the polypeptides are unfolded using heat, the polypeptides are generally unfolded by heating to temperature (Ts) that is at least about the melting temperature (Tm) of the polypeptide to be selected or greater that the Tm of the polypeptide to be selected.

In certain embodiments, the displayed polypeptides are unfolded by raising the temperature of the polypeptide display system to an unfolding temperature that is between about 25°C and about 100°C. When the polypeptide display system is phage display, it is preferred that the displayed polypeptides are unfolded by raising the temperature of the phage display system to about 80°C. Unfolding displayed or soluble polypeptides at high temperatures (e.g., at about 100°C) is also preferred and can be advantageous. For example, heat sterilizable polypeptides can be selected, isolated and/or sterilized by heating the polypeptide display or system or soluble polypeptide to about 100°C.

Once the desired unfolding temperature has been attained, the polypeptide display system can be maintained at that temperature for a period of time (*e.g*., up to about 10 hours), if desired. For example, the polypeptide display system can be maintained at the unfolding temperature for a period of about 100 milliseconds to about 10 hours. In particular embodiments, the polypeptide display system is maintained at the unfolding temperature for about 1 second to about 20 minutes.

The temperature of the polypeptide display system can be raised at any suitable rate, for example at a rate of about 1°C per millisecond to about 1°C per hour. In a particular embodiment, the temperature is preferably raised at a rate of about 1°C per second.

The unfolded polypeptides in the polypeptide display system, or a portion of the unfolded polypeptides, can be refolded by decreasing the amount or concentration of unfolding agent in the system. The amount or concentration of unfolding agent in the system can be decreased using any suitable method, for example, by dilution, dialysis, buffer exchange, titration or other suitable method. Heat can be reduced by cooling at room temperature or under refrigeration (*e.g*., in a refrigerated cooling block or bath), for example. Pressure can be reduced, for example, by venting.

It may be desirable to refold only a portion of the unfolded displayed polypeptides before selection. For example, highly stable polypeptides that unfold reversibly can be selected, isolated and/recovered when the unfolding agent is decreased minimally, such that only the most stable portion (e.g., about 0.00001% to about 1%) of the unfolded displayed polypeptides refold. Accordingly, refolding can be effectuated by decreasing the amount or concentration of unfolding agent in the polypeptide display system to an amount or concentration that results in the desired degree of refolding. The amount or concentration of unfolding agent that can remain in the polypeptide display system but permit the desired percentage of unfolded displayed polypeptides to refold can be readily determined using any suitable method, for example by reference to an unfolding and refolding curve (as described herein). When it is desirable to refold substantially all unfolded displayed polypeptides, the amount or concentration of unfolding agent can be reduced to the concentration or amount in the polypeptide display system before unfolding (e.g., the system is cooled to room temperature) or the unfolding agent can be substantially removed.

Generally, refolding is effectuated by decreasing the amount or concentration of unfolding agent (e.g., heat) so that at least about 0.00001% of the unfolded polypeptides refold. In particular embodiments, refolding is effectuated by decreasing the amount or concentration of unfolding agent (e.g., heat) so that at least about 0.0001%, or at least about 0.001%, or at least about 0.01%, or at least about 0.1 %, or at least about I %, or at least about 10%, or about 20%, or at least about 30%, or at least about 40%, or at least about 50%, or at least about 60% or at least about 70%, or at least about 80%, or at least about 90%, or at least about 95%, or at least about 98%, or at least about 99%, or substantially all of the unfolded polypeptides that can undergo refolding in the polypeptide display system refold.

As described herein, heat is a preferred unfolding agent. In certain embodiments where the displayed polypeptides are heat unfolded, the displayed unfolded polypeptides are refolded by lowering the temperature of the polypeptide display system to a refolding temperature (Tc) that is below about 99°C but above the freezing temperature of the polypeptide display system. As with unfolding, the refolding temperature selected will be dependent upon the thermostability of the displayed polypeptides. For example, a refolding temperature of 100°C or higher can be used if the displayed polypeptides are from, or are variants of polypeptides from, a thermophile or extreme thermophile (e.g., *Thermus aquaticus).* Preferably, the unfolding temperature and refolding temperature differ by at least about 10°C to at least about 120°C.

In one embodiment, the polypeptide display system is a phage display system, the displayed polypeptides are unfolded by heating the system to about 80°C, and the displayed unfolded polypeptides can be refolded by reducing the temperature of the phage display system to a temperature between about 1°C to about 70°C. In more particular embodiments, the displayed unfolded polypeptides are refolded by reducing the temperature of the phage display system to a temperature between about 1°C to about 60°C, or about 1°C to about 50°C, or about 1°C to about 40°C, or about 1°C to about 30°C, or about 1°C to about 20°C, or about 1°C to about 10°C.

Once the desired refolding temperature has been attained, the polypeptide display system can be maintained at that temperature for any desired period of time *(e.g.,* up to about 10 hours), if desired. For example, the polypeptide display system can be maintained at the refolding temperature for a period of about 100 milliseconds to about 10 hours. In particular embodiments, the polypeptide display system is maintained at the refolding temperature for about 1 minute to about 20 minutes.

The temperature of the polypeptide display system can be decreased at any suitable rate, for example at a rate of about 1°C per millisecond to about 1°C per hour. In a particular embodiment, the temperature is preferably decreased at a rate of about 1°C per 100 milliseconds or about 1°C per second.

The polypeptide display system can be maintained at atmospheric pressure (~1 atm) during unfolding and refolding of the displayed polypeptides. However, if desired, the polypeptide display system can be maintained at lower or higher pressure. In such situations, more or less unfolding agent may be required to obtain the desired degree of unfolding. For example, more or less heat may need to be applied to the polypeptide display system (relative to a system at 1 atm) in order to achieve the desired unfolding temperature because lowering or raising the pressure of the system can change the temperature of the system.

Unfolding and refolding can be carried out under suitable pH or buffer conditions. Generally, unfolding and refolding are carried out at a pH of about 1 to about 13, or about 2 to about 12. Preferred pH conditions for unfolding and refolding are a pH of about 5 to about 9, or about 6 to about 8, or about 7. Folding and unfolding the polypeptides under acidic or alkaline conditions can allow for selection of polypeptides that function under extreme acidic or alkaline conditions, such as polypeptide drugs that can be administered orally and/or have therapeutic action in the stomach.

### Selection/Isolation/Recovery

A polypeptide that unfolds reversibly (*e.g*., a population of polypeptides that unfold reversibly) can be selected, isolated and/or recovered from a repertoire or library (e.g., in a polypeptide display system) using any suitable method. A polypeptide can be recovered by selecting and/or isolating the polypeptide. Recovery can be carried out at a suitable recovery temperature (Tr). Generally, a suitable recovery temperature is any temperature that is less than the melting temperature (Tm) of the polypeptide but higher than the freezing temperature of the polypeptide display system. In certain embodiments, the recovery temperature (Tr) is substantially the same as the refolding temperature (Tc), (*e.g*., Tr=Tc).

In certain embodiments, a polypeptide that unfold reversibly is selected or isolated based on a selectable characteristic (e.g., physical characteristic, chemical characteristic, functional characteristic) that distinguishes properly folded polypeptides from unfolded and misfolded polypeptides. Examples of such selectable characteristics include fluorescence, susceptibility to fluorescence quenching and susceptibility to chemical modification (*e.g.*, with Iodoacetic acid, Iodoacetamide or other suitable polypeptide modifying agent). Preferably, a polypeptide that unfolds reversibly is selected, isolated and/or recovered based on a suitable selectable functional characteristic that distinguishes properly folded polypeptides from unfolded and misfolded polypeptides. Suitable functional characteristics for selecting or isolating a polypeptide that unfolds reversibly include any function that is dependent on proper folding of the polypeptide. Accordingly, a polypeptide that unfolds reversibly can have the function when properly folded, but the function can be lost or diminished upon unfolding and can be absent or diminished when the polypeptide is unfolded or misfolded. Suitable selectable functional characteristics include, for example, binding to a generic ligand (e.g., a superantigen), binding to a target ligand (*e.g*., an antigen, an epitope, a substrate), binding to an antibody (e.g., through an epitope expressed on the properly folded polypeptide), a catalytic activity and resistance to proteolysis. (See, *e.g*., Tomlinson et al., WO 99/20749; WO 01/57065; WO 99/58655.)

In preferred embodiments, the polypeptide that unfolds reversibly is selected and/or isolated from a library or repertoire of polypeptides in which substantially all polypeptides that unfold reversibly share a common selectable feature. For example, the polypeptide that unfolds reversibly can be selected from a library or repertoire of polypeptides in which substantially all polypeptides that unfold reversibly bind a common generic ligand, bind a common target ligand, bind (or are bound by) a common antibody, possess a common catalytic activity or are each resistant to proteolysis (e.g., proteolysis mediated by a particular protease). Selection based on binding to a common generic ligand can yield a population of polypeptide that contains all or substantially all polypeptides that unfold reversibly that were components of the original library or repertoire.

Any suitable method can be used to select, isolate and/or recover the polypeptides that unfold reversibly. For example, polypeptides that bind a target ligand or a generic ligand, such as protein A, protein L or an antibody, can be selected, isolated and/or recovered by panning or using a suitable affinity matrix. Panning can be accomplished by adding a solution of ligand (*e.g*., generic ligand, target ligand) to a suitable vessel (*e.g.*, tube, petri dish) and allowing the ligand to become deposited or coated onto the walls of the vessel. Excess ligand can be washed away and polypeptides (e.g., a phage display library) can be added to the vessel and the vessel maintained under conditions suitable for polypeptides to bind the immobilized ligand. Unbound polypeptide can be washed away and bound polypeptides can be recovered using any suitable method, such as scraping or lowering the pH, for example.

Suitable ligand affinity matrices generally contain a solid support or bead (e.g., agarose) to which a ligand is covalently or noncovalently attached. The affinity matrix can be combined with polypeptides (*e.g.*, a phage display library) using a batch process, a column process or any other suitable process under conditions suitable for binding of polypeptides to the ligand on the matix. Polypeptides that do not bind the affinity matrix can be washed away and bound polypeptides can be eluted and recovered using any suitable method, such as elution with a lower pH buffer, with a mild denaturing agent (e.g., urea), or with a peptide that competes for binding to the ligand.

In some embodiments, the generic or target ligand is an antibody or antigen binding fragment thereof. Antibodies or antigen binding fragments that bind structural features of polypeptides that are substantially conserved in the polypeptides of a library or repertoire are particularly useful as generic ligands. Antibodies and antigen binding fragments suitable for use as ligand for isolating, selecting and/or polypeptides that unfold reversibly can be monoclonal or polyclonal and can be prepared using any suitable method.

Polypeptides that unfold reversibly can also be selected, for example, by binding metal ions. For example, immobilized metal affinity chromatography (IMAC; Hubert and Porath, J. Chromotography, 98:247(1980)) takes advantage of the metal-binding properties of histidine and cysteine amino acid residues, as well as others that may bind metals, on the exposed surfaces of numerous proteins. It employs a resin, typically agarose, comprising a bidentate metal chelator (e.g. iminodiacetic acid, IDA, a dicarboxylic acid group) to which is complexed metallic ions. Such resins can be readily prepared, and several such resins are commercially available, such as CHELATING SEPHAROSE 6B (Pharmacia Fine Chemicals; Piscataway, NJ). Metallic ion that are of use include, but are not limited to, the divalent cations Ni²⁺, Cu²⁺, Zn²⁺, and Co²⁺. A repertoire of polypeptides or library can prepared in binding buffer which consists essentially of salt (typically, NaCl or KCl) at a 0.1- to 1.0M concentration and a weak ligand (*e.g*., Tris, ammonia), the latter of which has affinity for the metallic ions of the resin, but to a lesser degree than does a polypeptide to be selected according to the invention. Useful concentrations of the weak ligand range from 0.01- to 0.1M in the binding buffer.

The repertoire of polypeptides or library can contacted with the resin under conditions which permit polypeptides having metal-binding domains to bind. Non-binding polypeptides can be washed away, and the selected polypeptides are eluted with a buffer in which the weak ligand is present in a higher concentration than in the binding buffer, specifically, at a concentration sufficient for the weak ligand to displace the selected polypeptides, despite its lower binding affinity for the metallic ions. Useful concentrations of the weak ligand in the elution buffer are 10- to 50-fold higher than in the binding buffer, typically from 0.1 to 0.3 M. Preferably the concentration of salt in the elution buffer equals that in the binding buffer. According to the methods of the present invention, the metallic ions of the resin typically serve as the generic ligand; however, it is contemplated that they can also be used as the target ligand.

IMAC can be carried out using a standard chromatography apparatus (columns, through which buffer is drawn by gravity, pulled by a vacuum or driven by pressure), or by batch procedure, in which the metal-bearing resin is mixed, in slurry form, with the repertoire of polypeptides or library.

Partial purification of a serum T4 protein by IMAC has been described (Staples et al., U.S. Patent No. 5,169,936); however, the broad spectrum of proteins comprising surface-exposed metal-binding domains also encompasses other soluble T4 proteins, human serum proteins (e.g., IgG, haptoglobin, hemopexin, Gc-globulin, C1q, C3, C4), human desmoplasmin, *Dolichos biflorus* lectin, zinc-inhibited Tyr(P) phosphatases, phenolase, carboxypeptidase isoenzymes, Cu, Zn superoxide dismutases (including those of humans and all other eukaryotes), nucleoside diphosphatase, leukocyte interferon, lactoferrin, human plasma α₂-SH glycoprotein, *β*₂-macroglobulin, α₁-antitrypsin, plasminogen activator, gastrointestinal polypeptides, pepsin, human and bovine serum albumin, granule proteins from granulocytes, lysozymes, non-histone proteins, human fibrinogen, human serum transferrin, human lymphotoxin, calmodulin, protein A, avidin, myoglobins, somatomedins, human growth hormone, transforming growth factors, platelet-derived growth factor, α-human atrial natriuretic polypeptide, cardiodilatin and others. In addition, extracellular domain sequences of membrane-bound proteins may be purified using IMAC. Polypeptides that unfold reversibly and comprise a scaffold from any of the above proteins or metal-binding variants thereof can be selected, isolated or recovered using the methods described herein.

Polypeptides that unfold reversibly can also be selected from a suitable phage display system (or other suitable polypeptide display system), based on infectivity of phage following unfolding and refolding or based on aggregation of phage displaying polypeptides. A high local concentration of displayed polypeptide can be produced by displaying polypeptides on multivalent phage proteins (*e.g*., pIII protein of filamentous bacteriophage). The displayed polypeptides are adjacently located at the phage tip and can interact with each other and form aggregates if they do not unfold reversibly. Such aggregates can be intraphage aggregates which form between the polypeptides displayed on a particular phage and/or interphage aggregates which form between polypeptides displayed on two or more phage when the phage display system comprises a plurality of phage particles at a sufficient concentration. Accordingly, polypeptides that unfold reversibly can be selected from a multivalent phage display system by recovering displayed polypeptides that do not aggregate using any suitable method, such as centrifugation (*e.g*., ultracentrifugation), or by selecting based on function of the displayed polypeptide or infectivity of the phage.

For example, when heated to a suitable temperature (e.g., about 80°C) the displayed polypeptides unfold. However, heating filamentous phage to 80°C reduces infectivity of the phage only slightly (Holliger et al., J. Mol. Biol. 288:649-657 (1999)). The high local concentration of displayed polypeptides can lead to aggregation of unfolded polypeptides and thereby, substantially reduce the infectivity of phage that display polypeptides that do not unfold reversibly. For example, infectivity of phage that display polypeptides that do not unfold reversibly can be reduced by 70 fold or more. However, infectivity of phage displaying polypeptides that unfold reversibly is reduced to a lesser degree or substantially unchanged by heating, for example, at 80°C (relative to infectivity of phage that do not display polypeptides following heating at 80°C). Accordingly, in certain embodiments, polypeptides that unfold reversibly can be selected by unfolding and refolding the polypeptides in a suitable polypeptide display system (*e*.*g*., a filamentous bacteriophage display system), and selecting phage with infectivity that is not substantially reduced or that is substantially unchanged. The selected polypeptides that unfold reversibly can be further selected for any desired properties, such as binding a desired antigen, catalytic activity, and the like. Selection based on infectivity can also be employed to prepare a library or repertoire that is enriched in polypeptides that unfold reversibly or nucleic acids encoding polypeptides that unfold reversibly.

Polypeptides that unfold reversibly can also be selected from a suitable phage display system (or other suitable polypeptide display system), based on aggregation of phage displaying polypeptides that do not unfold reversibly. For example, a suitable polypeptide display system can be unfolded (e.g., by heating to about 80°C) and refolded by cooling under conditions in which at least a portion of polypeptides that do not unfold reversibly aggregate. The presence or degree of aggregation can be determined using any suitable method, such as electron microscopy or an infectivity assay. Polypeptides that unfold reversibly can be selected by recovering polypeptides (*e.g.*, displayed on phage) that do not aggregate using any suitable method, such as centrifugation (e.g., ultracentrifugation) or by infeceting suitable host bacteria (when the polypeptides are displayed on phage).

Other polypeptide display systems, including systems in which polypeptides are immobilized on a solid support, can be prepared in which displayed polypeptides that do not unfold reversibly can form aggregates. Generally, the displayed polypeptides in such a system are positioned in close proximity to each other. For example, the displayed polypeptides can separated by no more than about twice the length of linear amino acid sequence of the polypeptide. The displayed polypeptides are separated by no more that a distance that is determined by multiplying the number of amino acid residues in the polypeptide by the length of a peptide bond (3.8 Å) times 2. For example, the polypeptides can be separated by no more than about 200 Å to about 300 Å. In a particular example, the polypeptide contains 100 amino acids, and they are separated by no more that 760Å. The displayed polypeptides are spaced no closer than the distance between the centers of two adjacent identical globular polypetides. For example, two immunoglobulin variable domains that are tethered to a substrate by their C-termini should be no closer that 25Å.

Such polypeptide display systems can be prepared using any suitable method. For example, polypeptides can be concatenated (see, e.g., WO 02/30945) or produced as fusion proteins which bring the polypeptides together (*e*.*g*., by dimerizing or oligomerizing, by assembly into a viral coat or capsid). Such polypeptide display systems can also be prepared by immobilizing polypeptides on to a suitable solid support (e.g., a bead, plastic, glass) using any suitable method. Polypeptides that unfold reversibly can be selected from such a polypeptide display system by recovering displayed polypeptides that do not aggregate using any suitable method. For example, when the polypeptides are displayed on a mobile sold support (e.g., beads) interbead aggregates can form and be removed by as centrifugation or other suitable method.

Generally, the polypeptide display system comprises a plurality of polypeptide species and more than one copy (polypeptide molecule) of each species. Preferably, each polypeptide species is present in a concentration that is sufficient to permit aggregation of species that do not unfold reversibly. For example, in polypeptide display systems in which more than one copy of a displayed polypeptides species are located adjacent to each other or co-localized, such as phage display, each species of polypeptide is present in a concentration (e.g., local concentration such as on the phage tip) that is sufficiently high to permit aggregation of species that do not unfold reversibly.

In one embodiment, the process for selecting a polypeptide that binds a ligand and unfolds reversibly from a repertoire of polypeptides comprises, providing a polypeptide display system comprising a repertoire of polypeptides; heating the repertoire to a temperature (Ts) at which at least a portion of the displayed polypeptides are unfolded; cooling the repertoire to a temperature (Tc) wherein Ts>Tc, whereby at least a portion of the polypeptides have refolded and a portion of the polypeptides have aggregated; and recovering at a temperature (Tr) at least one polypeptide that unfolds reversibly and binds a ligand. Preferably, the ligand is bound by (binds) folded polypeptide but is not bound by (does not bind) aggregated polypeptides. The recovered polypeptide that unfolds reversibly has a melting temperature (Tm), and preferably, the repertoire was heated to Ts, cooled to Tc and the polypeptide that unfolds reversibly was isolated at Tr, such that Ts>Tm>Tc, and Ts>Tm>Tr. Preferably, the recovered polypeptide is not misfolded. Misfolded polypeptides can be identified based on certain selectable characteristic (e.g., physical characteristic, chemical characteristic, functional characteristic) that distinguishes properly folded polypeptides from unfolded and misfolded polypeptides as described herein.

In additional embodiments, the process further comprises confirming that the recovered polypeptide binds a ligand (e.g., target ligand, generic ligand). The process can further comprise confirming that the Tm of the recovered polypeptide is less than the temperature Ts to which the repertoire was heated. The Tm of a recovered polypeptide can be determined using any suitable method, e.g., by circular dichroism, change in fluorescence of the polypeptide with increasing temperature, differential scanning calorimetry (DSC). Preferably, the recovered polypeptide has a Tm that is equal to or greater than about 37°C, and more preferably greater that about 37°C, when rounded up to the nearest whole number. In other embodiments, the Tm of the recovered polypeptide is less than about 60°C. In other embodiments, the Tm of the recovered polypeptide is greater than about 37°C and less than about 60°C.

In one embodiment, the polypeptide is recovered at a temperature (Tr) that is substantially the same as Tc (*e.g,* Tc=Tr). In other embodiments, Ts is at least about 60°C. In certain embodiments, the Tm of the recovered polypeptide is less than about 60°C, and the repertoire was heated to a temperature (Ts) greater than about 60°C.

In certain embodiments, the polypeptide display system comprises a plurality of replicable genetic display packages. Preferably, the polypeptide display system is a phage display system, such as a multivalent phage display system. In some embodiments, the polypeptide display system, pre-selection repertoire and/or pre-selection library comprises at least about 10³ members (species), at least about 10⁴ members, at least about 10⁵ members, at least about 10⁶ members, or at least about 10⁷ members. In one embodiment, a phage display system is provided and at least a portion of the polypeptides that aggregate at Tc form interphage aggregates. In another embodiment, a phage display system is provided and at least a portion of the polypeptides that aggregate at Tc form interphage aggregates and intraphage aggregates.

### METHODS FOR DESIGNING POLYPEPTIDES

In another aspect, the invention relates to a method for designing and/or preparing a variant polypeptide that unfolds reversibly. The method comprises providing the amino acid sequence of a parental polypeptide, identifying one or more regions of said amino acid sequence that are hydrophobic, selecting one or more of the hydrophobic regions, and replacing one or more amino acids in a selected region to produce a variant amino acid sequence in which the hydrophobicity of the selected region is reduced. A polypeptide that comprises or consists of the variant amino acid sequence can be produced and, if desired, its ability to unfold reversibly can be assessed or confirmed using any suitable method. Preferably, the variant unfolds reversibly when heated and cooled, as described herein.

A variant polypeptide that unfolds reversibly can be prepared using any desired parental polypeptide. For example, the polypeptide that unfolds reversibly can be prepared using a parental polypeptide that is based on a scaffold selected from an enzyme, protein A, protein L, a fibronectin, an anticalin, a domain of CTLA4, or a polypeptide of the immunoglobulin superfamily, such as an antibody or an antibody fragment.

When the parental polypeptide is an antibody variable domain (e.g., a human V_{H}, a human V_{L}), it can comprise V and/or D (where the polypeptide comprises a V_{H} domain) and/or J segment sequences encoded by a germline V, D or J segment respectively, or a sequence that results from naturally occurring or artificial mutations (e.g., somatic mutation or other processes). For example, the parental polypeptide can be a parental immunoglobulin variable domain having an amino acid sequence that is encoded by germline gene segments, or that results from insertion and/or deletion of nucleotides during V(D)J recombination *(e.g.,* N nucleotides, P nucleotides) and/or mutations that arise during affinity maturation.

Similarly, the parental polypeptide can be a parental enzyme that has an amino acid sequence encoded by the germline or that results from naturally occurring or artificial mutation (e.g., somatic mutation).

Hydrophobic regions of the parental polypeptide can be identified using any suitable scale or algorithm. Preferably, hydrophobic regions are identified using the method of Sweet and Eisenberg. (Sweet, R.M. and Eisenberg, D., J. Mol. Biol. 171:479-488 (1983).) The Sweet and Eisenberg method can produce a hydrophobicity score (S/E score) for an amino acid sequence (e.g., an identified hydrophobic region) using a 9 to 18 amino acid window (e.g., a window of 9, 10, 11, 12, 13, 14, 15, 16, 17 or 18 amino acids). A 15 amino acid window is generally preferred. In some embodiments, a parental amino acid sequence is analyzed using the Sweet and Eisenberg method, and a hydrophobicity plot for a selected hydrophobic region is generated (ordinate is the S/E score using a suitable window, abscissa is the amino acid positions of the selected region). A variant amino acid sequence for the selected hydrophobic region is designed in which one or more of the amino acid residues in the parental sequence with a different amino acid residue, and analyzed using the Sweet and Eisenberg method. A hydrophobicity plot for the variant amino acid sequence is produces. A decrease in the area under the curve of the hydrophobicity plot of a selected hydrophobic region in the variant amino acid sequence relative to the area under the curve of the corresponding region in the parental amino acid sequence is indicative of a reduction in hydrophobicity of the selected region. The amino acid sequence of the variant V_{H} or variant V_{L} can comprise one or more of the amino acid replacements for V_{H} or V_{L} described herein. The amino acid sequence of the variant V_{H} or variant V_{L} can comprise one or more framework regions as described herein.

In a preferred embodiments, a polypeptide comprising a variant antibody variable domain that unfolds reversibly is designed and/or prepared. In one embodiment, the variant antibody variable domain is a variant V_{H} domain in which the hydrophobicity of the amino acid sequence from position 22 to position 36 is reduced (Kabat amino acid numbering system) relative to the corresponding amino acid sequence of the parental V_{H}. In another embodiment, the variant antibody variable domain is a variant V_{H} and the hydrophobicity of the H1 loop is reduced relative to the H1 loop of the parental V_{H} (H1 loop is as defined by the AbM amino acid numbering system). Preferably, hydrophobicity is determined using the method of Sweet and Eisenberg with a 15 amino acid window for V_{H}. In certain embodiments, the S/E score of the amino acid sequence from position 22 to position 36 of the variant V_{H} is 0.15 or less, or 0,1 or less for an S/E score rounded to one decimal place, or 0.09 or less, or 0.08 or less, or 0.07 or less, or 0.06 or less, or 0.05 or less, or 0.04 or less, or 0 or less. Preferably, the S/E score of the H1 loop of the variant V_{H} is 0 or less.

In another embodiment, the variant antibody variable domain is a variant V_{L} and the hydrophobicity of the FR2-CDR2 region and/or FR3 is reduced relative to the FR2-CDR2 region and/or FR3 of the parental V_{L} (FR2-CDR2 region and FR3 region are as defined by the Kabat amino acid numbering system). In another embodiment, the variant antibody variable domain is a variant V_{L} and the hydrophobicity of the amino acid sequence from position 44 to position 53 and/or position 73 to position 76 is reduced relative to the corresponding amino acid sequence of the parental V_{L} (Kabat amino acid numbering system). Preferably, hydrophobicity is determined using the method of Sweet and Eisenberg with an 11 amino acid window for V_{L}. In certain embodiments, the S/E score of the amino acid sequence from position 44 to position 53 that has a S/E score of less than 0.23. For example, the S/E score for position 44 to position 53 of the variant V_{L} can be than 0.2, less than 0.17, less than 0.15, less than 0.13, less than 0.10, or less than -0.1. In some embodiments, the S/E score for FR3 of the variant V_{L} is less than 0.35. For example, the S/E score for FR3 of the variant V_{L} can be less than 0.3, less than 0.25, less than 0.2, less than 0.17, less than 0.15, less than 0.13, less than 0.10, or less than -0.1.

A variant polypeptide that comprises a variant amino acid sequence and unfolds reversibly can be prepared using any suitable method. For example, the amino acid sequence of a parental polypeptide can be altered at particular positions (as described herein with respect to antibody variable domain polypeptides) to produce a variant polypeptide that unfolds reversibly.

### LIBRARIES/REPERTOIRES

In other aspects, the invention relates to repertoires of polypeptides that unfold reversibly, to libraries that encode polypeptides that unfold reversibly, and to methods for producing such libraries and repertoires.

The libraries and repertoires of polypeptides comprise polypeptides that unfold reversibly (and/or nucleic acids encoding polypeptides that unfold reversibly) using a suitable unfolding agent as described herein. Generally at least about 1% of the polypeptides contained in the repertoire or library or encoded by the library unfold reversibly. More preferably, at least about 10%, or at least about 20%, or at least about 30%, or at least about 40%, or at least about 50%, or at least about 60%, or at least about 70%, or at least about 80%, or at least about 90% of the polypeptides in the repertoire or library or encoded by the library unfold reversibly. Such libraries are referred to herein as being enriched in polypeptides that unfold reversibly or in nucleic acids encoding polypeptides that unfold reversibly. Preferred repertoires and libraries contain polypeptides that unfold reversibly when heated. Preferably, the libraries of the invention comprise heterogeneous nucleic acids that are replicable in a suitable host, such as recombinant vectors that contain nucleic acids encoding polypeptides (e.g., plasmids, phage, phagmids) that are replicable in *E. coli,* for example.

Libraries that encode and/or contain polypeptides that unfold reversibly can be prepared or obtained using any suitable method. The library of the invention can be designed to encode polypeptides that are based on a polypeptide of interest (e.g., a polypeptide selected from a library) or can be selected from another library using the methods described herein. For example, a library enriched in polypeptides that unfold reversibly can be prepared using a suitable polypeptide display system.

In one example, a phage display library comprising a repertoire of displayed polypeptides comprising an antibody variable domain (*e.g.,* V_{H}, Vκ, Vλ) is unfolded and refolded as described herein and a collection of refolded polypeptides is recovered thereby yielding a phage display library enriched in polypeptides that unfold reversibly. In another example, a phage display library comprising a repertoire of displayed polypeptides comprising an antibody variable domain (*e.g*., V_{H}, Vκ, Vλ) is first screened to identify members of the repertoire that have binding specificity for a desired target antigen. A collection of polypeptides having the desired binding specificity are recovered, the collection is unfolded and refolded, and a collection of polypeptides that unfold reversibly and have the desired binding specificity is recovered, yielding a library enriched in polypeptides that unfold reversibly.

In another example, a polypeptide of interest, such as an immunoglobulin variable domain, is selected (*e*.*g*., from a library) and the amino acid sequence of the polypeptide is analyzed to identify regions of hydrophobicity. Hydrophobicity can be determined using any suitable method, scale or algorithm. Preferably, hydrophobicity is determined using the method of Sweet and Eisenberg. (Sweet, R.M. and Eisenberg, D., J. Mol. Biol. 171:479-488 (1983).) A region of hydrophobicity in the polypeptide is selected and a collection of nucleic acids is prepared that contains sequence diversity targeted to the region encoding the hydrophobic region of the polypeptide. The collection of nucleic acids can be randomized in the target region or sequence diversity that results in reduced hydrophobicity of the selected region of the polypeptide (through amino acid replacement) can be introduced. The collection of nucleic acids can then be inserted into a suitable vector (*e.g*., a phage, a phagemid) to yield a library. The library can be expressed in a suitable polypeptide display system and the polypeptides in the display system can be unfolded, refolded and a collection of polypeptides that unfold reversibly can be selected or recovered as described herein to yield a library enriched in polypeptides that unfold reversibly.

When the library is based on a polypeptide of interest, it is preferred that the hydrophobic region targeted for sequence diversification is a hydrophobic region that is associated with aggregation of unfolded polypeptides. Such aggregation prone hydrophobic regions can be identified using any suitable method. For example, amino acids in the hydrophobic regions can be replaced with less hydrophobic residues (e.g., Tyr replaced with Asp or Glu). The resulting polypeptides can be unfolded and aggregation can be assessed using any suitable method. Decreased aggregation of polypeptide that contains an amino acid replacement indicates that the hydrophobic region that contains the amino acid replacement is an aggregation prone hydrophobic region.

A particular repertoire of polypeptides or library comprises V_{H} domains (e.g., based on a parental V_{H} domain comprising a germline V_{H} gene segment) containing at least one amino acid replacement that reduces the hydrophobicity of the amino acid sequence from position 22 to position 36, or position 26 to position 35, as defined by the Kabat amino acid numbering scheme. Another particular repertoire of polypeptides or library comprises VH domains wherein the hydrophobicity of the amino acid sequence from position 22 to position 36 (Kabat amino acid numbering scheme) has a Sweet/Eisenberg hydrophobicity score of 0 or less. Preferably, at least about 1% of the polypeptides contained in the repertoire or library have a Sweet/Eisenberg hydrophobicity score of 0 or less for the amino acid sequence from position 22 to position 36. More preferably, at least about 10%, or at least about 20%, or at least about 30%, or at least about 40%, or at least about 50%, or at least about 60%, or at least about 70%, or at least about 80%, or at least about 90% of the polypeptides have a Sweet/Eisenberg hydrophobicity score of 0 or less for the amino acid sequence from position 22 to position 36.

Another particular repertoire of polypeptides or library comprises V_{L} domains (*e*.*g*., based on a parental V_{L} domain comprising a germline V_{L} gene segment) containing at least one amino acid replacement that reduces the hydrophobicity of the amino acid sequence from position 44 to position 53, as defined by the Kabat amino acid numbering scheme. Another repertoire of polypeptides or library comprises V_{L} domains (*e*.*g*., based on a parental V_{L} domain comprising a germline V_{L} gene segment) containing at least one amino acid replacement that reduces the hydrophobicity of framework 3 (FR3), as defined by the Kabat amino acid numbering scheme. Other repertoires and libraries can be produced that contain polypeptides comprising an antibody variable region having amino acid sequence diversity at particular residues or regions as described herein.

The libraries and repertoires can be based on a polypeptide that unfolds reversibly (e.g., when heated), such as a polypeptide that unfolds reversibly that is selected or designed as described herein. Generally, one or more amino acid residues are identified in the amino acid sequence or the polypeptide that unfolds reversibly that prevent irreversible aggregation of the polypeptide upon unfolding. Such amino acid residues are referred to herein as folding gatekeepers. (See Examples, Sections 16-19) Folding gatekeepers can be identified, for example, by aligning the amino acid sequences of two or more polypeptides that unfold reversibly and that are based on a common amino acid sequence (polypeptide scaffold amino acid sequence) but contain some degree of sequence diversity. Such an alignment can be used to identify amino acid substitutions in the scaffold sequence that are common to polypeptides that unfold reversibly, A polypeptide based on the scaffold sequence but containing one or more the identified amino acid substitutions can be prepared and assessed for reversible unfolding, to confirm that the identified amino acid residues are folding gatekeepers. Folding gatekeeper residues can also be designed into a desired polypeptide, such as a human V_{H} as described herein. (See Examples, Sections 16-19)

The library can comprise a collection of nucleic acids that encode polypeptides that unfold reversibly and/or a collection of polypeptides that unfold reversibly in which each polypeptide contains a common folding gatekeeper residue (one or more common folding gatekeeper residues). For example, as described herein, a library comprising a collection of heterogeneous nucleic acids that each encode a human antibody variable domain containing a folding gatekeeper residue can be prepared. If desired, the members of the library can have sequence diversity in a particular region (e.g., in the CDRs). In certain embodiments, the library comprises a collection of heterogeneous nucleic acids encoding antibody variable domains (*e.g.,* V_{H} (human V_{H}), V_{L}, (human V_{L})) that contain folding gatekeeper residues. In one embodiment, the library comprises a collection of heterogeneous nucleic acids encoding V_{H}s (human V_{H}s) that contain folding gatekeeper residues in CDR1, CDR1 and CDR2 or CDR2. Preferably, the nucleic acids in such a library encode V_{H}s that contain diverse CDR3s (e.g., randomized CDR3s).

The gatekeeper residues can be designed into CDR1 and/or CDR2 using the methods described herein or other suitable methods, and nucleic acids produced that encode V_{H}s that contain those gatekeeper residues. V_{Hs} that unfold reversibly can be isolated or selected, using the methods described herein or other suitable method, and nucleic acid(s) encoding CDR1 and/or CDR2 from a V_{H} that unfolds reversibly (the same or different V_{H}s) can be obtained and ligated to one or more nucleic acids encoding suitable framework regions and CDR3.

In one embodiment, the library of nucleic acids encodes a polypeptide that unfolds reversibly, wherein each member of the library encodes a polypeptide comprising the amino acid sequence of a parental polypeptide in which at least one amino acid residue is replaced with a folding gatekeeper residue and at least one other amino acid residue is replaced, added or deleted. The parental polypeptide can be an antibody variable domain that comprises framework regions as described herein. In particular embodiments, the parental polypeptide is a human V_{H} and a folding gatekeeper is introduced into CDR1. In another particular embodiment, the parental polypeptide is a human V_{L} and a folding gatekeeper is introduced into the FR2-CDR2 region.

In another embodiment, the library of nucleic acids encodes an antibody variable domain that unfolds reversibly, wherein each member of the library comprising a first nucleic acid encoding CDR1 and optionally CDR2 of the variable domain or an antibody variable domain that unfolds reversibly, where said first nucleic acid is operably linked to one or more other nucleic acids to produce a construct that encodes antibody variable domains in which CDR1 and optionally CDR2 are encoded by said first nucleic acid. If desired, the members of the library encode an antibody variable domain in which CDR3 is diversified (e.g., at targeted positions) or randomized (*e.g.,* across the entire CDR3 sequence and/or has CDR3s of varying length).

Libraries that encode a repertoire of a desired type of polypeptides can readily be produced using any suitable method. For example, a nucleic acid sequence that encodes a desired type of polypeptide (e.g., a polymerase, an immunoglobulin variable domain) can be obtained and a collection of nucleic acids that each contain one or more mutations can be prepared, for example by amplifying the nucleic acid using an error-prone polymerase chain reaction (PCR) system, by chemical mutagenesis (Deng et al. J. Biol. Chem., 269:9533 (1994)) or using bacterial mutator strains (Low et al. J. Mol. Biol., 260:359 (1996)).

In other embodiments, particular regions of the nucleic acid can be targeted for diversification. Methods for mutating selected positions are also well known in the art and include, for example, the use of mismatched oligonucleotides or degenerate oligonucleotides, with or without the use of PCR. For example, synthetic antibody libraries have been created by targeting mutations to the antigen binding loops. Random or semi-random antibody H3 and L3 regions have been appended to germline immunoglobulin V gene segments to produce large libraries with unmutated framework regions (Hoogenboom and Winter (1992) supra; Nissim *et al.* (1994) supra; Griffiths *et al.* (1994) supra; DeKruif *et al.* (1995) supra). Such diversification has been extended to include some or all of the other antigen binding loops (Crameri et al. (1996) Nature Med., 2:100; Riechmann et al. (1995) Bio/Technology, 13:475; Morphosys, WO 97/08320, supra). In other embodiments, particular regions of the nucleic acid can be targeted for diversification by, for example, a two-step PCR strategy employing the product of the first PCR as a "mega-primer." (See, *e.g.,* Landt, O. et al., Gene 96:125-128 (1990).) Targeted diversification can also be accomplished, for example, by SOE PCR. (See, *e.g.,* Horton, R.M. et al., Gene 77:61-68 (1989).)

As described herein, sequence diversity at selected positions can be achieved by altering the coding sequence which specifies the sequence of the polypeptide such that a number of possible amino acids (e.g., all 20 or a subset thereof) can be incorporated at that position. Using the IUPAC nomenclature, the most versatile codon is NNK, which encodes all amino acids as well as the TAG stop codon. The NNK codon is preferably used in order to introduce the required diversity. Other codons which achieve the same ends are also of use, including the NNN codon, which leads to the production of the additional stop codons TGA and TAA. Such a targeted approach can allow the full sequence space in a target area to be explored.

Preferred libraries of polypeptides that unfold reversibly comprise polypeptides that are members of the immunoglobulin superfamily (e.g., antibodies or portions thereof). For example the libraries can comprise antibody polypeptides that unfold reversibly and have an known main-chain conformation. (See, *e.g.,* Tomlinson et al., WO 99/20749.)

Libraries can be prepared in a suitable plasmid or vector. As used herein, vector refers to a discrete element that is used to introduce heterologous DNA into cells for the expression and/or replication thereof. Any suitable vector can be used, including plasmids (e.g., bacterial plasmids), viral or bacteriophage vectors, artificial chromosomes and episomal vectors. Such vectors may be used for simple cloning and mutagenesis, or an expression vector can be used to drive expression of the library. Vectors and plasmids usually contain one or more cloning sites (e.g., a polylinker), an origin of replication and at least one selectable marker gene. Expression vectors can further contain elements to drive transcription and translation of a polypeptide, such as an enhancer element, promoter, transcription termination signal, signal sequences, and the like. These elements can be arranged in such a way as to be operably linked to a cloned insert encoding a polypeptide, such that the polypeptide is expressed and produced when such an expression vector is maintained under conditions suitable for expression (e.g., in a suitable host cell).

Cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV40, adenovirus) are useful for cloning vectors in mammalian cells. Generally, the origin of replication is not needed for mammalian expression vectors unless these are used in mammalian cells able to replicate high levels of DNA, such as COS cells.

Cloning or expression vectors can contain a selection gene also referred to as selectable marker. Such marker genes encodes a protein necessary for the survival or growth of transformed host cells gown in a selective culture medium. Host cells not transformed with the vector containing the selection gene will therefore not survive in the culture medium. Typical selection genes encode proteins that confer resistance to antibiotics and other toxins, *e.g.* ampicillin, neomycin, methotrexate or tetracycline, complement auxotrophic deficiencies, or supply critical nutrients not available in the growth media.

Suitable expression vectors can contain a number of components, for example, an origin of replication, a selectable marker gene, one or more expression control elements, such as a transcription control element (*e*.*g*., promoter, enhancer, terminator) and/or one or more translation signals, a signal sequence or leader sequence, and the like. Expression control elements and a signal or leader sequence, if present, can be provided by the vector or other source. For example, the transcriptional and/or translational control sequences of a cloned nucleic acid encoding an antibody chain can be used to direct expression.

A promoter can be provided for expression in a desired host cell. Promoters can be constitutive or inducible. For example, a promoter can be operably linked to a nucleic acid encoding an antibody, antibody chain or portion thereof, such that it directs transcription of the nucleic acid. A variety of suitable promoters for procaryotic (*e.g.,* the *β*-lactamase and lactose promoter systems, alkaline phosphatase, the tryptophan (trp) promoter system, lac, tac, T3, T7 promoters for E. *coli*) and eucaryotic (*e.g.,* simian virus 40 early or late promoter, Rous sarcoma virus long terminal repeat promoter, cytomegalovirus promoter, adenovirus late promoter, EG-1a promoter) hosts are available.

In addition, expression vectors typically comprise a selectable marker for selection of host cells carrying the vector, and, in the case of a replicable expression vector, an origin or replication. Genes encoding products which confer antibiotic or drug resistance are common selectable markers and may be used in procaryotic (e.g., *β*-lactamase gene (ampicillin resistance), *Tet* gene for tetracycline resistance) and eucaryotic cells (e.g., neomycin (G418 or geneticin), gpt (mycophenolic acid), ampicillin, or hygromycin resistance genes). Dihydrofolate reductase marker genes permit selection with methotrexate in a variety of hosts. Genes encoding the gene product of auxotrophic markers of the host (*e.g., LEU2, URA3, HIS3)* are often used as selectable markers in yeast. Use of viral (*e.g.,* baculovirus) or phage vectors, and vectors which are capable of integrating into the genome of the host cell, such as retroviral vectors, are also contemplated.

Suitable expression vectors for expression in prokaryotic (e.g., bacterial cells such as *E. coli*) or mammalian cells include, for example, a pET vector (e.g., pET-12a, pET-36, pET-37, pET-39, pET-40, Novagen and others), a phage vector (*e.g.,* pCANTAB 5 E, Pharmacia), pRIT2T (Protein A fusion vector, Pharmacia), pCDM8, pCDNA1.1/amp, pcDNA3.1, pRc/RSV, pEF-1 (Invitrogen, Carlsbad, CA), pCMV-SCRIPT, pFB, pSG5, pXT1 (Stratagene, La Jolla, CA), pCDEF3 (Goldman, L.A., et al., Biotechniques, 21:1013-1015 (1996)), pSVSPORT (GibcoBRL, Rockville, MD), pEF-Bos (Mizushima, S., et al., Nucleic Acids Res., 18:5322 (1990)) and the like. Expression vectors which are suitable for use in various expression hosts, such as prokaryotic cells (*E. coil*), insect cells (*Drosophila* Schnieder S2 cells, Sf9) and yeast (*P*. *methanolica, P. pastoris, S. cerevisiae)* and mammalian cells (eg, COS cells) are available.

Preferred vectors are expression vectors that enable the expression of a nucleotide sequence corresponding to a polypeptide library member. Thus, selection with generic and/or target ligands can be performed by separate propagation and expression of a single clone expressing the polypeptide library member. As described above, the preferred selection display system is bacteriophage display. Thus, phage or phagemid vectors may be used. The preferred vectors are phagemid vectors which have an *E. coli*. origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector can contain a *β*-lactamase gene to confer selectivity on the phagemid and a lac promoter upstream of an expression cassette that can contain a suitable leader sequence (e.g., pelB leader sequence), a multiple cloning site, one or more peptide tags, one or more TAG stop codons and the phage protein pIII. Thus, using various suppressor and non-suppressor strains of *E. coli* and with the addition of glucose, iso-propyl thio-*β-*D-galactoside (IPTG) or a helper phage, such as VCS M13, the vector is able to replicate as a plasmid with no expression, produce large quantities of the polypeptide library member only or product phage, some of which contain at least one copy of the polypeptide-pIII fusion on their surface.

The libraries and repertoires of the invention can contain antibody formats. For example, the polypeptide contained within the libraries and repertoires can be whole antibodies or fragments therefore, such as Fab, F(ab)2, Fv or scFv fragments, separate V_{H} or V_{L} domains, any of which is either modified or unmodified. scFv fragments, as well as other antibody polypeptides, can be readily produced using any suitable method. A number of suitable antibody engineering methods are well known in the art. For example, a scFv can be formed by linking nucleic acids encoding two variable domains with an suitable oligonucleotide that encodes an appropriately linker peptide, such as (Gly-Gly-Gly-Gly-Ser)₃ or other suitable linker peptide(s). The linker bridges the C-terminal end of the first V region and N-terminal end of the second V region. Similar techniques for the construction of other antibody formats, such as Fv, Fab and F(ab)₂ fragments. To format Fab and F(ab)₂ fragments, V_{H} and V_{L} polypeptides can combined with constant region segments, which may be isolated from rearranged genes, germline C genes or synthesized from antibody sequence data. A library or repertoire according to the invention can be a V_{H} or V_{L} library or repertoire.

In particular embodiments, the libraries and repertoires comprise immunoglobulin variable domains that unfold reversibly (e.g., V_{H}, V_{L}). The variable domains can be based on a germline sequence (*e.g.,* DP47dummy (SEQ ID NO:3, DPK9 dummy (SEQ ID NO:6)) and if desired can have one or more diversified regions, such as the complementarity determining regions.

One or more of the framework regions (FR) of the variable domains can comprise (a) the amino acid sequence of a human framework region, (b) at least 8 contiguous amino acids of the amino acid sequence of a human framework region, or (c) an amino acid sequence encoded by a human germline antibody gene segment, wherein said framework regions are as defined by Kabat. In certain embodiments, the amino acid sequence of one or more of the framework regions is the same as the amino acid sequence of a corresponding framework region encoded by a human germline antibody gene segment, or the amino acid sequences of one or more of said framework regions collectively comprise up to 5 amino acid differences relative to the amino acid sequence of said corresponding framework region encoded by a human germline antibody gene segment.

In other embodiments, the amino acid sequences of FR1, FR2, FR3 and FR4 are the same as the amino acid sequences of corresponding framework regions encoded by a human germline antibody gene segment, or the amino acid sequences of FR1, FR2, FR3 and FR4 collectively contain up to 10 amino acid differences relative to the amino acid sequences of corresponding framework regions encoded by said human germline antibody gene segments. In other embodiments, the amino acid sequence of said FR1, FR2 and FR3 are the same as the amino acid sequences of corresponding framework regions encoded by said human germline antibody gene segment.

The polypeptides comprising a variable domain that unfolds reversibly preferably comprise a target ligand binding site and/or a generic ligand binding site. In certain embodiments, the generic ligand binding site is a binding site for a superantigen, such as protein A, protein L or protein G..

The variable domains can be based on any desired variable domain, for example a human VH (e.g., V_{H} 1a, V_{H} 1b, V_{H} 2, V_{H} 3, V_{H} 4, V_{H} 5, V_{H} 6), a human Vλ (e.g., VλI, VλII, VλIII, VλIV, VλV or VλVI) or a human V_{K} (e.g., V_{K}1, V_{K}2, Vₖ3, Vₖ4, V_{K}5, V_{K}6, V_{K}7, V_{K}8, V_{K}9 or V_{K}10). Preferably, the variable domains are not a *Camelid* immunoglobulin domain, such as a V_{H} H, or contain one or more amino acids (e.g., framework amino acids) that are unique to *Camelid* immunoglobulin variable domains encoded by germline sequences but not, for example, to human immunoglobulin variable domains. (See, e.g., Davies et al., Protein Engineering 9:531-537 (1996); Tanha et al., J. Biol. Chem. 276:24774-24780 (2001); Riechmann et al., J. Immunol. Methods 23:25-38 (1999).) In one embodiment, the V_{H} that unfolds reversibly does not comprise one or more amino acids that are unique to murine (e.g., mouse) germline framework regions. Preferably, the variable domain is unfold reversibly when heated and cooled.

The isolated polypeptide comprising a variable domain can be an antibody format. Thus, in certain embodiments, the isolated polypeptide comprising a variable domain that unfolds reversibly can be a homodimer of variable domain, a heterodimer comprising a variable domain, an Fv, a scFv, a disulfide bonded Fv, a Fab, a single variable domain or a variable domain fused to an immunoglobulin Fc portion.

### POLYPEPTIDES

In one aspect, the invention is an isolated polypeptide that unfolds reversibly. As described herein, such polypeptides can be expressed in *E. coli* and recovered in high yield. As described herein, in preferred embodiments the polypeptides that unfold reversibly (e.g., when heated) are secreted when expressed in *E. coli,* and easily recovered as soluble polypeptides. Such polypeptides are also referred to as secretable when expressed in E. *coli.* In preferred embodiments, the polypeptide that unfolds reversibly is secreted in a quantity of at least about 0.5 mg/L when expressed in E. *coli.* In other preferred embodiments, the polypeptide that unfolds reversibly is secreted in a quantity of at least about 0.75 mg/L, at least about 1 mg/L, at least about 4 mg/L, at least about 5 mg/L at least about 10 mg/L, at least about 15 mg/L, at least about 20 mg/L, at least about 25 mg/L, at least about 30 mg/L, at least about 35 mg/L, at least about 40 mg/L, at least about 45 mg/L, or at least about 50 mg/L, or at least about 100 mg/L, or at least about 200 mg/L, or at least about 300 mg/L, or at least about 400 mg/L, or at least about 500 mg/L, or at least about 600 mg/L, or at least about 700 mg/L, or at least about 800 mg/L, at least about 900 mg/L, at least about 1g/L when expressed in *E. coli.* In other preferred embodiments, the polypeptide that unfolds reversibly is secreted in a quantity of at least about 1 mg/L to at least about 1g/L, at least about 1 mg/L to at least about 750 mg/L, at least about 100 mg/L to at least about 1 g/L, at least about 200 mg/L to at least about 1 g/L, at least about 300 mg/L to at least about 1 g/L, at least about 400 mg/L to at least about 1 g/L, at least about 500 mg/L to at least about 1g/L, at least about 600 mg/L to at least about 1 g/L, at least about 700 mg/L to at least about 1 g/L, at least about 800 mg/L to at least about 1 g/L, or at least about 900 mg/L to at least about 1g/L when expressed in E. *coli.* Although, the polypeptides described herein can be secretable when expressed in *E*. *coli,* they can be produced using any suitable method, such as synthetic chemical methods or biological production methods that do not employ E. *coli.* In particularly preferred embodiments, the polypeptide that unfolds reversibly is a human antibody variable domain (V_{H}, V_{L}) or comprises a human antibody variable domain that unfolds reversibly.

In some embodiments, the polypeptide that unfolds reversibly is a variant of a parental polypeptide that differs from the parental polypeptide in amino acid sequence (*e.g.,* by one or more amino acid replacements, additions and/or deletions), but qualitatively retains function of the parental polypeptide. Preferably, the activity of the variant polypeptide that unfolds reversibly is at least about 25%, at least about 30%, at least about 40%, at least about 50%, at least about 60%, at least about 70%, at least about 80%, at least about 90%, at least about 95%, or essentially the same as of the activity of the parental polypeptide. For example, if the parental polypeptide is an enzyme, the variant polypeptide can contain an amino acid sequence that differs from the parental enzyme (*e.g.,* by one to about ten amino acid substitutions, deletions and/or insertions) but will retain the catalytic activity of parental enzyme. Preferably, the variant enzyme that unfolds reversibly is characterized by a catalytic rate constant that is at least about 25% of the catalytic rate constant of the parental enzyme.

A variant polypeptide that unfolds reversibly can be prepared using any desired parental polypeptide. For example, the polypeptide that unfolds reversibly can be prepared using a parental polypeptide that is based on a scaffold selected from an enzyme, protein A, protein L, a fibronectin, an anticalin, a domain of CTLA4, or a polypeptide of the immunoglobulin superfamily, such as an antibody or an antibody fragment.

The parental polypeptide can comprise V and/or D (where the polypeptide comprises a V_{H} domain) and/or J segment sequences encoded by a germline V, D or J segment respectively, or a sequence that results from naturally occurring or artificial mutations (*e.g.,* somatic mutation or other processes). For example, the parental polypeptide can be a parental immunoglobulin variable domain having an amino acid sequence that is encoded by germline gene segments, or that results from insertion and/or deletion of nucleotides during V(D)J recombination (*e.g.,* N nucleotides, P nucleotides) and/or mutations that arise during affinity maturation. Similarly, the parental polypeptide can be a parental enzyme that has an amino acid sequence encoded by the germline or that results from naturally occurring or artificial mutation (e.g, somatic mutation).

A variant polypeptide that unfolds reversibly can be prepared using any suitable method. For example, the amino acid sequence of a parental polypeptide can be altered at particular positions (as described herein with respect to antibody variable domain polypeptides) to produce a variant polypeptide that unfolds reversibly. A variant polypeptide that unfolds reversibly can also be produced, for example, by providing a nucleic acid that encodes a parental polypeptide, preparing a library of nucleic acids that encode variants of the parental polypeptide (e.g., by error prone PCR or other suitable method) and expressing the library in a suitable polypeptide display system. A variant polypeptide that unfolds reversibly and retains a desired function of the parental polypeptide can be selected and isolated from such a polypeptide display system using the methods described herein or other suitable methods.

### Polypeptides Comprising V Domains That Unfold Reversibly

In preferred embodiments, the isolated polypeptide comprises an immunoglobulin variable domain that unfolds reversibly (*e.g.,* V_{H}, a variant V_{H}, V_{L} and/or variant V_{L}). In certain embodiment, the isolated polypeptide comprises a variant V_{H} and/or a variant V_{L} that unfolds reversibly. Preferably the variable domain that unfolds reversibly (e.g., V_{H}, variant V_{H}, V_{L}, variant V_{L}) has binding specificity for a target ligand and binds to the target ligand with a suitable dissociation constant (K_{d}) and suitable off rate (K_{off}). A suitable K_{d} can be about 50 nM to about 20 pM or less, for example about 50 nM, about 1 nM, about 900 pM, about 800 pM, about 700 pM, about 600 pM, about 500 pM, about 400 pM, about 300 pM, about 200 pM, about 100 pM or about 20 pM or less. A suitable K_{off} can be about 1 x 10⁻¹ s⁻¹ to about 1 x 10⁻⁷ s⁻¹ or less, for example about 1 x 10⁻¹ s⁻¹, about 1 x 10⁻² s⁻¹, about 1 x 10⁻³ s⁻¹, about 1 x 10⁻⁴ s⁻¹, about 1 x 10⁻⁵ s⁻¹, about 1 x 10⁻⁶ s⁻¹ or about 1 x 10⁻¹ s⁻¹ or less. Preferably, K_{d} and K_{off} are determined using surface plasmon resonance.

In certain embodiments, the isolated polypeptide comprises a V_{H} (e.g., variant V_{H}) that unfolds reversibly. In some embodiments, the amino acid sequence of the variant V_{H} that unfolds reversibly differs from the amino acid sequence of the parental V_{H} by at least one amino acid from position 22 to position 36, or differs from the amino acid sequence of the parental V_{H} by at least one amino acid in the H1 loop. The amino acid positions and CDR (H1, H2 and H3) and framework regions (FR1, FR2, FR3 and FR4) of the V_{H} can be defined using any suitable system, such as the systems of Kabat, Chothia or AbM. Preferably, positions 22 through 36 are defined according to the amino acid numbering system of Kabat, and the H1 loop is defined according to the amino acid numbering system used in the AbM software package (antibody analysis and structural modeling software; Oxford Molecular). The amino acid sequence of the variant V_{H} can contain at least one amino acid replacement from position 22 to position 36 relative to the parental V_{H}.

In certain embodiments, the amino acid sequence of the V_{H} that unfolds reversibly contains at least one Pro or Gly residue from position 22 to position 36. In other embodiments, the amino acid sequence of the V_{H} that unfolds reversibly contains at least one Pro or Gly residue in the H1 loop. In certain embodiments, the amino acid sequence of the variant V_{H} that unfolds reversibly contains at least one Pro or Gly replacement from position 22 to position 36 relative to the amino acid sequence of the parental V_{H}. In other embodiments, the amino acid sequence of the variant V_{H} that unfolds reversibly contains at least one Pro or Gly replacement in the H1 loop relative to the amino acid sequence of the parental V_{H}. In a particular embodiment, the variant V_{H} that unfolds reversibly comprises an amino acid sequence wherein the parental amino acid residue at position 29 is replaced with Pro or Gly.

In other embodiments, the variant V_{H} that unfolds reversibly contains at least one amino acid replacement from position 22 to position 36 relative to the parental V_{H} amino acid sequence, such that the hydrophobicity of the amino acid sequence from position 22 to position 36 of the variant V_{H} is reduced relative to the parental V_{H}. Hydrophobicity can be determined using any suitable scale or algorithm. Preferably, hydrophobicity is determined using the method of Sweet and Eisenberg, and the Sweet and Eisenberg hydrophobicity score (S/E score) of the amino acid sequence from position 22 to position 36 of the variant V_{H} is reduced relative to the parental V_{H}. The S/E method can use a 9 to 18 amino acid window (e.g., a window of 9, 10, 11, 12, 13, 14, 15, 16, 17 or 18 amino acids). Preferably, a 15 amino acid window is used for V_{H}.

In certain embodiments the S/E score of the amino acid sequence from position 22 to position 36 of the variant V_{H} is 0.15 or less, or 0.1 or less for an S/E score rounded to one decimal place, or 0.09 or less, or 0.08 or less, or 0.07 or less, or 0.06 or less, or 0.05 or less, or 0.04 or less. In preferred embodiments, the S/E score or the amino acid sequence from position 22 to position 36 of the variant V_{H} is 0.03 or less, 0.02 or less or 0.01 or less. In more preferred embodiments, the S/E score or the amino acid sequence from position 22 to position 36 of the variant V_{H} is 0 or less.

Producing V_{H} variants that have a reduced S/E score from position 22 to position 36 relative to a parental V_{H} can yield polypeptides that have several advantages. For example, it has been determined that a lower S/E score from position 22 to position 36 correlates with resistance to aggregation and enhanced yields from expression systems (e.g., bacterial expression systems). The capacity of V_{H} domains to resist aggregation at high protein concentrations is associated with a low S/E score. Thus, a variant V_{H} domain with an S/E score from position 22 to position 36 that is lower than the S/E score of the parental V_{H} domain can display enhanced resistance to aggregation, while lowering the S/E score of this region to 0 or less can produce variant V_{H} domains with superior aggregation resistance at high protein concentrations (*e.g.,* about 200 µM).

In other embodiments, the V_{H} that unfolds reversibly contains an amino acid sequence from position 22 to position 36 that has an S/E score of 0.15 or less, or 0.1 or less, or 0.09 or less, or 0.08 or less, or 0.07 or less, or 0.06 or less, or 0.05 or less, or 0.04 or less. In preferred embodiments, the V_{H} that unfolds reversibly contains an amino acid sequence from position 22 to position 36 that has an S/E score of 0.03 or less, 0.02 or less or 0.01 or less. In more preferred embodiments, the V_{H} that unfolds reversibly contains an amino acid sequence from position 22 to position 36 that has an S/E score of 0 or less.

In another embodiment, the amino acid sequence of the variant V_{H} differs from the amino acid sequence of the parental V_{H} by at least one amino acid in the H1 loop. Preferably, the variant V_{H} contains at least one amino acid replacement in the H1 loop, such that the hydrophobicity of H1 loop is reduced relative to the H1 loop of the parental V_{H}, Preferably, the S/E score of the H1 loop of the variant V_{H} is 0 or less.

In other embodiments, the V_{H} that unfolds reversibly contains an H1 loop that has an S/E score of 0 or less.

In particular embodiments, the V_{H} that unfolds reversibly (e.g., variant V_{H}) comprises an amino acid sequence in which one or more of the parental amino acid residues at position 27, 29, 30, 31, 32, 33 and 35 (Kabat numbering) is replaced with another amino acid residue as set forth in Table 1.

| Table 1 | |
|---|---|
| Position (Kabat numbering) | Amino Acid |
| 27 | Asp, Glu, His, Ala, Gln, Ser or Gly |
| 29 | Asp, Glu, Val, Ser, Pro, Gln or Gly |
| 30 | Asp, Pro, Gly, Thr, Leu, Gln or Val |
| 31 | Asp, Glu or Pro |
| 32 | Asp, Gln, Glu, Pro or Gly |
| 33 | Asp, Gly or Pro |
| 35 | Asp, Asn or Gly |

The amino acid sequence of the V_{H} that unfolds reversibly (*e.g.,* variant V_{H}) can comprise any one or any combination of amino acid replacements set forth in Table 1, and if desired, a variant V_{H} that unfolds reversibly can further differ from the parental V_{H} by replacement of one or more of the amino acid residues at parental position 22 through parental position 26, parental position 28 and parental position 36 (Kabat numbering).

In more particular embodiments, the variant V_{H} that unfolds reversibly comprises an amino acid sequence in which the parental amino acid residue at position 27 is replaced with Asp or Glu; the parental amino acid residue at position 29 is replaced with Asp, Glu, Pro or Gly; and/or the parental amino acid residue at position 32 is replaced with Asp or Glu (Kabat numbering). In one such embodiment, the variant V_{H} that unfolds reversibly comprises an amino acid sequence in which the parental amino acid residue at position 27 is replaced with Asp; the parental amino acid residue at position 29 is replaced with Asp, Pro or Gly; and/or the parental amino acid residue at position 32 is replaced with Glu (Kabat numbering).

In a specific embodiment, the variant V_{H} that unfolds reversibly comprises an amino acid sequence in which the parental amino acid residue at position 27 is replaced with Asp; the parental amino acid residue at position 29 is replaced with Val; and/or the parental amino acid residue at position 32 is replaced with Asp or Glu; and the parental residue at position 35 is replaced with Gly (Kabat numbering).

In preferred embodiments, the variant V_{H} that unfolds reversibly comprises an amino acid sequence in which the parental amino acid residue at position 27 is replaced with Asp and/or the parental amino acid residue at position 32 is replaced with Asp (Kabat numbering). If desired, the variant V_{H} that unfolds reversibly can further comprise amino acid replacements relative to the parental sequence at one or more of positions 22-26, 28-31 and 33-36 as described herein (Kabat numbering),e.g., to bring the S/E score of one or more of these regions to zero or less.

One or more of the framework regions (FR) of the V_{H} or variant V_{H} that unfold reversibly can comprise (a) the amino acid sequence of a human framework region, (b) at least 8 contiguous amino acids of the amino acid sequence of a human framework region, or (c) an amino acid sequence encoded by a human germline antibody gene segment, wherein said framework regions are as defined by Kabat. In certain embodiments, the amino acid sequence of one or more of the framework regions is the same as the amino acid sequence of a corresponding framework region encoded by a human germline antibody gene segment, or the amino acid sequences of one or more of said framework regions collectively comprise up to 5 amino acid differences relative to the amino acid sequence of said corresponding framework region encoded by a human germline antibody gene segment.

In other embodiments, the amino acid sequences of FR1, FR2, FR3 and FR4 are the same as the amino acid sequences of corresponding framework regions encoded by a human germline antibody gene segment, or the amino acid sequences of FR1, FR2, FR3 and FR4 collectively contain up to 10 amino acid differences relative to the amino acid sequences of corresponding framework regions encoded by said human germline antibody gene segments. In other embodiments, the amino acid sequence of said FR1, FR2 and FR3 are the same as the amino acid sequences of corresponding framework regions encoded by said human germline antibody gene segment. For example, the variant V_{L} can be a variant of human DP47 dummy (SEQ ID NO:3).

The isolated polypeptide comprising a V_{H} that unfolds reversibly (*e.g.,* variant V_{H}) comprises a target ligand binding site and/or a generic ligand binding site. In certain embodiments, the generic ligand binding site is a binding site for a superantigen, such as protein A, protein L or protein G.

The V_{H} that unfolds reversibly can be based on any desired parental V_{H}, for example a human V_{H} (e.g., V_{H} 1a, V_{H} 1b, V_{H} 2, V_{H} 3, V_{H} 4, V_{H} 5, V_{H} 6). Preferably, the V_{H} that unfolds reversibly is not a *Camelid* immunoglobulin domain, such as a V_{H}H, or does not contain one or more amino acids (*e.g.,* framework amino acids) that are unique to *Camelid* immunoglobulin variable domains encoded by germline sequences but not, for example, to human immunoglobulin variable domains. (See, *e*.*g*., Davies et al., Protein Engineering 9:531-537 (1996); Tanha et al., J. Biol. Chem. 276:24774-24780 (2001); Riechmann et al., J. Immunol. Methods 23:25-38 (1999).) In one embodiment, the V_{H} that unfolds reversibly does not comprise one or more amino acids that are unique to murine (e.g., mouse) germline framework regions. Preferably, the V_{H} unfolds reversibly when heated and cooled.

The isolated polypeptide comprising a V_{H} that unfolds reversibly (e.g. variant V_{H}) can be an antibody format. Thus, in certain embodiments, the isolated polypeptide comprising a V_{H} that unfolds reversibly can be a homodimer of V_{H}, a heterodimer comprising a V_{H}, an Fv, a scFv, a disulfide bonded Fv, a Fab, a V_{H} or a V_{H} fused to an immunoglobulin Fc portion.

The invention also relates to an isolated polypeptide comprising an immunoglobulin light chain variable domain (V_{L}) (*e.g.,* a variant V_{L}) that unfolds reversibly. In one embodiment, the isolated polypeptide comprises a V_{L} that unfolds reversibly and comprises an amino acid sequence from position 44 to position 53 that has a Sweet/Eisenberg hydrophobicity score (S/E score) of less than 0.23. In other embodiments, the S/E score for position 44 to position 53 can be less than 0.2, less than 0.17, less than 0.15, less than 0.13, less than 0.10, or less than -0.1 or less. The S/E method can use a 9 to 18 amino acid window (*e.g.,* a window of 9, 10, 11, 12, 13, 14, 15, 16, 17 or 18 amino acids). Preferably, an 11 amino acid window is used for V_{L}. The amino acid positions and CDR (L1, L2 and L3) and framework regions (FR1, FR2, FR3 and FR4) of the V_{L} can be defined using any suitable system, such as the systems of Kabat, Chothia or AbM. Preferably, the amino acid positions and CDR and framework regions are according to the amino acid numbering system of Kabat. In other embodiments, V_{L} that unfolds reversibly further comprises a FR3 that has a Sweet/Eisenberg hydrophobicity score (S/E score) of less than 0.35, and FR3 is as defined by the Kabat amino acid numbering system. In other embodiments, the S/E score for FR3 can be less than 0.3, less than 0.25, less than 0.2, less than 0.17, less than 0.15, less than 0.13, less than 0.10, or less than -0.1 or less.

In certain embodiments, the isolated polypeptide comprises a variant V_{L} that unfolds reversibly. In some embodiments, the amino acid sequence of the variant V_{L} that unfolds reversibly differs from the amino acid sequence of the parental V_{L} by at least one amino acid from position 44 to position 53 of said variant V_{L}, such that the variant V_{L} comprises the amino acid sequence of a parental V_{L} wherein at least one amino acid residue from position 44 to position 53 is replaced such that the Sweet/Eisenberg hydrophobicity score (S/E score) of the amino acid sequence from position 44 to position 53 of said variant V_{L} is less than 0.23. The amino acid sequence of the variant V_{L} that unfolds reversibly can contain at least one amino acid replacement from position 44 to position 53 relative to the parental V_{L}. In other embodiments, the amino acid sequence of the variant V_{L} that unfolds reversibly further differs from the amino acid sequence of the parental V_{L} by at least one amino acid in FR3 of said variant V_{L}. In these embodiments, the variant V_{L} that unfolds reversibly comprises FR3 having the amino acid sequence of a parental V_{L} FR3 wherein at least one amino acid residue is replaced such that the Sweet/Eisenberg hydrophobicity score (S/E score) of FR3 said variant V_{L} is less than 0.35.

In other embodiments, the amino acid sequence of the variant V_{L} that unfolds reversibly differs from the amino acid sequence of the parental V_{L} by at least one amino acid in FR3 of said variant V_{L}, such that the variant V_{L} comprises the amino acid sequence of a parental V_{L} wherein at least one amino acid residue in FR3 is replaced such that the Sweet/Eisenberg hydrophobicity score (S/E score) of the amino acid sequence of FRe of said variant V_{L} is less than 0.35. For example, the amino acid sequence of the variant V_{L} that unfolds reversibly can differ from the amino acid sequence of the parental V_{L} by at least one amino acid from position 73 to position 76 of said variant V_{L}, such that the variant V_{L} comprises the amino acid sequence of a parental V_{L} wherein at least one amino acid residue from position 73 to position 76 is replaced such that the Sweet/Eisenberg hydrophobicity score (S/E score) of the amino acid sequence of FR3 of said variant V_{L} is less than 0.35.

In particular embodiments, the V_{L} that unfolds reversibly comprises an amino acid sequence in which one or more of the amino acid residues at position 10, 13, 20, 23, 26, 27, 29, 31, 32, 35, 36, 39, 40, 42, 45, 46, 47, 48, 49, 50, 57, 59, 60, 68, 75, 79, 80, 83, 89, 90 and 92 (Kabat numbering) is replaced with another amino acid residue as set forth in Table 2. For example, when the isolated V_{L} that unfolds reversibly is a variant V_{L}, it can comprise an amino acid sequence in which one or more of the parental amino acid residues at position 10, 13, 20, 23, 26, 27, 29, 31, 32, 35, 36, 39, 40, 42, 45, 46, 47, 48, 49, 50, 57, 59, 60, 68, 75, 79, 80, 83, 89, 90 and 92 (Kabat numbering) is replaced with another amino acid residue as set forth in Table 2.

| Table 2 | | | |
|---|---|---|---|
| Position (Kabat numbering) | Amino Acid | Position (Kabat numbering) | Amino Acid |
| 10 | Phe | 46 | Asn, Phe, His or Pro |
| 13 | Gly | 47 | Pro |
| 20 | Ala or Ser | 48 | Asn, Pro, Asp, Thr, Gly or Val |
| 23 | Trp | 49 | Asn, Asp, Ser, Cys, Glu, Gly, Lys or Arg |
| 26 | Asn | 50 | Pro, Asp, Asn, Glu or Arg |
| 27 | Arg | 57 | Glu |
| 29 | Val | 60 | Pro |
| | | 68 | Glu |
| 31 | Gly | 69 | Glu |
| 32 | Ser or Phe | 75 | Asn or Met |
| 35 | Gly | 79 | Arg |
| 36 | His | 80 | Ala |
| 39 | Arg | 83 | Ala or Leu |
| 40 | Ser | 89 | Arg |
| 42 | Thr, Asn or Glu | 90 | GIn or Pro |
| 45 | Glu, Asp, Gln, Pro, Asn, His or Thr | 92 | His |

The amino acid sequence of the V_{L} or variant V_{L} that unfolds reversibly can comprise any one or any combination of amino acid replacements set forth in Table 2, and if desired can further differ from the parental V_{L} by replacement of the amino acid residue at parental position 26 with Asn and/or replacement of the amino acid residue at parental position 89 with Arg (Kabat numbering).

In more particular embodiments, the variant V_{L} that unfolds reversibly comprises an amino acid sequence in which the parental amino acid residue at position 45 is replaced with Glu, Asp, Gln, Pro, Asn, His or Thr; the parental amino acid residue at position 48 is replaced with Asn, Pro, Asp, Thr, Gly or Val; the parental amino acid residue at position 49 is replaced with Asn, Asp, Ser, Cys, Glu, Gly, Lys or Arg; the parental amino acid residue at position 50 is replaced with Pro, Asp, Asn, Glu or Arg; and/or the parental amino acid residue at position 75 is replaced with Asn or Met. (Kabat numbering).

In other particular embodiments, the V_{L} or variant V_{L} that unfolds reversibly comprises an amino acid sequence which contain one of the amino acid replacements set forth in Table 2 and has an amino acid sequences that comprises:
Gly at position 31 and Asn at position 49;
Ser at position 32 and Asn at position 75;
Ser at position 40 and Asp at position 49;
Arg at position 39 and Asn at position 49;
Glu at position 45 and Asn at position 75;
Pro at position 46 and Asp at position 50;
Asn at position 26, Thr at position 42 and Asp at position 50;
Phe at position 32, Glu at position 45 and Glu at position 57;
Asp at position 49, Ala at position 80 and Arg at position 89;
Asn at position 49, Glu at position 68 and Arg at position 79;
Ser at position 20, Trp at position 23, Phe at position 46 and Asn at position 49;
Val at position 29, Asn at position 42, Glu at position 45, Leu at position 83 and His at position 92;
Thr at position 35 and Pro at position 90;
Asp at position 45 and Pro at position 60;
Arg at position 49 and Phe at position 10;
Ser at position 49 and Ala at position 20;
Ser at position 49 and Arg at position 27;
Pro at position 50 and Val at position 48; and
Arg at position 50, Gly at position 13 and Glu at position 42, wherein amino acid positions are by Kabat numbering.

In certain embodiments, the amino acid sequence of the variant V_{L} that unfolds reversibly contains at least one Pro or Gly replacement from position 44 to position 53 and/or in FR3 (e.g., from position 73 to position 76) relative to the amino acid sequence of the parental V_{L}. In a particular embodiment, the amino acid sequence of the variant V_{L} comprises Pro at position 45, position 48 and/or position 50.

One or more of the framework regions (FR) of the V_{L} or variant V_{L} that unfolds reversibly can comprise (a) the amino acid sequence of a human framework region, (b) at least 8 contiguous amino acids of the amino acid sequence of a human framework region, or (c) an amino acid sequence encoded by a human germline antibody gene segment, wherein said framework regions are as defined by Kabat. In certain embodiments, the amino acid sequence of one or more of the framework regions is the same as the amino acid sequence of a corresponding framework region encoded by a human germline antibody gene segment, or the amino acid sequences of one or more of said framework regions collectively comprise up to 5 amino acid differences relative to the amino acid sequence of said corresponding framework region encoded by a human germline antibody gene segment.

In other embodiments, the amino acid sequences of FR1, FR2, FR3 and FR4 are the same as the amino acid sequences of corresponding framework regions encoded by a human germline antibody gene segment, or the amino acid sequences of FR1, FR2, FR3 and FR4 collectively contain up to 10 amino acid differences relative to the amino acid sequences of corresponding framework regions encoded by said human germline antibody gene segments. In other embodiments, the amino acid sequence of said FR1, FR2 and FR3 are the same as the amino acid sequences of corresponding framework regions encoded by said human germline antibody gene segment. For example, the variant V_{L} can a variant of human DPK9 dummy (SEQ ID NO:6).

The isolated polypeptide comprising a V_{L} that unfolds reversibly (e.g., variant V_{L}) comprises a target ligand binding site and/or a generic ligand binding site. In certain embodiments, the generic ligand binding site is a binding site for a superantigen, such as protein A, protein L or protein G.

The V_{L} that unfolds reversibly can be based on any desired parental V_{L}, for example a human Vλ (e.g., VλI, VλII, VλIII, VλIV, VλV or VλVI) or a human V_{K} (e.g., V_{K}1, Vₖ2, V_{K}3, V_{K}4, V_{K}5, V_{K}6, V_{K}7, V_{K}8, V_{K}9 or Vₖ10). Preferably, the V_{L} that unfolds reversibly is not a *Camelid* immunoglobulin domain, or contain one or more amino acids (e.g., framework amino acids) that are unique to *Camelid* immunoglobulin variable domains encoded by germline sequences but not, for example, to human immunoglobulin variable domains. (See, *e.g*., Davies et al., Protein Engineering 9:531-537 (1996); Tanha et al., J. Biol. Chem. 276:24774-24780 (2001); Riechmann et al., J. Immunol. Methods 23:25-38 (1999).) In one embodiment, the V_{H} that unfolds reversibly does not comprise one or more amino acids that are unique to murine (e.g., mouse) germline framework regions. Preferably, the V_{L} unfolds reversibly when heated.

The isolated polypeptide comprising a V_{L} that unfolds reversibly (*e.g.,* variant V_{L}) can be an antibody format. Thus, in certain embodiments, the isolated polypeptide comprising a V_{L} that unfolds reversibly can be a homodimer of V_{L}, a heterodimer comprising a V_{L}, an Fv, a scFv, a disulfide bonded Fv, a Fab, a V_{L} or a V_{L} fused to an immunoglobulin Fc portion.

### Isolated Polypeptides Comprising Disulfide Bonded V Domains

The invention also relates to an isolated polypeptide comprising an immunoglobulin variable domain (e.g, V_{H}, V_{L}), wherein said variable domain comprises a disulfide bond between a cysteine in CDR2 and a cysteine in CDR3, wherein CDR2 and CDR3 are defined using the Kabat amino acid numbering system. In some embodiments, the disulfide bond is between cysteine residues at position 52a and position 98; position 51 and position 98; or position 51 and position 100b, wherein said positions are defined using the Kabat amino acid numbering system. In other embodiments, the disulfide bond is between a cysteine at position 51 and a cysteine in CDR3; or a cysteine in CDR2 and a cysteine at position 98, wherein CDR2, CDR3 and said positions are defined using the Kabat amino acid numbering system.

The isolated polypeptide comprising a disulfide bonded variable domain can be an antibody format. Thus, in certain embodiments, the isolated polypeptide comprising a disulfide bonded variable domain can be a homodimer of variable domains, a heterodimer of variable domains, an Fv, a scFv, a Fab, a single variable domain or a single variable domain fused to an immunoglobulin Fc portion.

### NUCLEIC ACIDS, HOST CELLS AND METHODS FOR PRODUCING REFOLDABLE POLYPEPTIDES

The invention also relates to isolated and/or recombinant nucleic acids encoding polypeptides that unfold reversibly as described herein.

Nucleic acids referred to herein as "isolated" are nucleic acids which have been separated away from other material (e.g., other nucleic acids such as genomic DNA, cDNA and/or RNA) in its original environment (*e.g.,* in cells or in a mixture of nucleic acids such as a library). An isolated nucleic acid can be isolated as part of a vector (e.g., a plasmid). Nucleic acids can be naturally occurring, produced by chemical synthesis, by combinations of biological and chemical methods (*e.g.,* semisynthetic), and be isolated using any suitable methods.

Nucleic acids referred to herein as "recombinant" are nucleic acids which have been produced by recombinant DNA methodology, including methods which rely upon artificial recombination, such as cloning into a vector or chromosome using, for example, restriction enzymes, homologous recombination, viruses and the like, and nucleic acids prepared using the polymerase chain reaction (PCR). "Recombinant" nucleic acids are also those that result from recombination of endogenous or exogenous nucleic acids through the natural mechanisms of cells or cells modified to allow recombination (e.g., cells modified to express Cre or other suitable recombinase), but are selected for after the introduction to the cells of nucleic acids designed to allow and make recombination probable. For example, a functionally rearranged human-antibody transgene is a recombinant nucleic acid.

Nucleic acid molecules of the present invention can be used in the production of antibodies (*e.g.,* human antibodies, humanized antibodies, chimeric antibodies and antigen-binding fragments of the foregoing), *e.g*., antibodies that bind an αE integrin or integrin αE chain (CD103). For example, a nucleic acid (e.g., DNA) encoding an antibody of the invention can be incorporated into a suitable construct (*e*.*g*., an expression vector) for further manipulation or for production of the encoded polypeptide in suitable host cells.

Expression constructs or expression vectors suitable for the expression of a antibody or antigen-binding fragment are also provided. For example, a nucleic acid encoding all or part of a desired antibody can be inserted into a nucleic acid vector, such as a plasmid or virus, for expression. The vector can be capable of replication in a suitable biological system (e.g., a replicon). A variety of suitable vectors are known in the art, including vectors which are maintained in single copy or multiple copy, or which become integrated into the host cell chromosome. Suitable expression vectors can contain a number of components, for example, an origin of replication, a selectable marker gene, one or more expression control elements, such as a transcription control element (*e.g.,* promoter, enhancer, terminator) and/or one or more translation signals, a signal sequence or leader sequence, and the like. Expression control elements and a signal or leader sequence, if present, can be provided by the vector or other source. For example, the transcriptional and/or translational control sequences of a cloned nucleic acid encoding an antibody chain can be used to direct expression.

In another aspect, the invention relates to recombinant host cells and a method of preparing an polypeptide of the invention that unfolds reversibly. The polypeptide that unfolds reversibly can be obtained, for example, by the expression of one or more recombinant nucleic acids encoding a polypeptide that unfolds reversibly or using other suitable methods. For example, the expression constructs described herein can be introduced into a suitable host cell, and the resulting cell can be maintained (e.g., in culture, in an animal, in a plant) under conditions suitable for expression of the constructs. Suitable host cells can be prokaryotic, including bacterial cells such as E. *coli, B. subtilis* and/or other suitable bacteria; eucaryotic cells, such as fungal or yeast cells (*e.g., Pichia pastoris, Aspergillus sp., Saccharomyces cerevisiae, Schizosaccharomyces pombe, Neurospora crassa*), or other lower eukaryotic cells, and cells of higher eucaryotes such as those from insects (*e*.*g*., *Drosophila* Schnieder S2 cells, Sf9 insect cells (WO 94/26087 (O'Connor)), mammals (*e*.*g*., COS cells, such as COS-1 (ATCC Accession No. CRL-1650) and COS-7 (ATCC Accession No. CRL-1651), CHO (e.g., ATCC Accession No. CRL-9096), 293 (ATCC Accession No. CRL-1573), HeLa (ATCC Accession No. CCL-2), CV1 (ATCC Accession No. CCL-70), WOP (Dailey, L., et al., J. Virol., 54:739-749 (1985), 3T3, 293T (Pear, W. S., et al., Proc. Natl. Acad. Sci. U.S.A., 90:8392-8396 (1993)) NSO cells, SP2/0, HuT 78 cells and the like, or plants (e.g., tobacco). (See, for example, Ausubel, F.M. et al., eds. Current Protocols in Molecular Biology, Greene Publishing Associates and John Wiley & Sons Inc. (1993).)

The invention also relates to a recombinant host cell which comprises a (one or more) recombinant nucleic acid or expression construct comprising a nucleic acid encoding a polypeptide that unfolds reversibly. The invention also includes a method of preparing an polypeptide that unfolds reversibly, comprising maintaining a recombinant host cell of the invention under conditions appropriate for expression of a polypeptide that unfolds reversibly. The method can further comprise the step of isolating or recovering the polypeptide that unfolds reversibly, if desired.

For example, a nucleic acid molecule (*i.e.,* one or more nucleic acid molecules) encoding a polypeptide that unfolds reversibly, or an expression construct (*i.e.,* one or more constructs) comprising such nucleic acid molecule(s), can be introduced into a suitable host cell to create a recombinant host cell using any method appropriate to the host cell selected (*e.g.,* transformation, transfection, electroporation, infection), such that the nucleic acid molecule(s) are operably linked to one or more expression control elements (*e.g.,* in a vector, in a construct created by processes in the cell, integrated into the host cell genome). The resulting recombinant host cell can be maintained under conditions suitable for expression (e.g., in the presence of an inducer, in a suitable animal, in suitable culture media supplemented with appropriate salts, growth factors, antibiotics, nutritional supplements, etc.), whereby the encoded polypeptide(s) are produced. If desired, the encoded protein can be isolated or recovered (*e.g.,* from the animal, the host cell, medium, milk). This process encompasses expression in a host cell of a transgenic animal (see, *e.g.,* WO 92/03918, GenPharm International).

The polypeptides that unfolds reversibly described herein can also be produced in a suitable *in vitro* expression system, by chemical synthesis or by any other suitable method.

In certain embodiments, the invention does not include a polypeptide having a variable domain comprising a sequence encoded by a germline V_{H} or germline V_{L} gene segment, or consisting of or comprising SEQ ID NOS:7-60.

ASP ILE GLN MET THR GLN SER PRO SER SER LEU SER ALA
SER VAL GLY ASP ARG VAL THR ILE THR CYS GLN ALA SER
GLN ASP ILE SER ASN TYR LEU ALA TRP TYR GLN GLN LYS
PRO GLY LYS ALA PRO GLU LEU ARG ILE TYR ASP ALA SER
ASN LEU GLU THR GLY VAL PRO SER ARG PHE SER GLY SER
GLY SER GLY THR ASP PHE THR PHE THR ILE SER SER LEU
GLN PRO GLU ASP ILE ALA THR TYR TYR CYS GLN GLN TYR
GLN ASN LEU PRO LEU THR PHE GLY PRO GLY THR LYS VAL
ASP ILE LYS ARG THR VAL ALA ALA PRO SER VAL
SEQ ID NO:7

GLN ILE VAL LEU THR GLN SER PRO ALA ILE MET SER ALA
SER PRO GLY GLU LYS VAL THR MET THR CYS SER ALA SER
SER SER VAL TYR TYR MET TYR TRP TYR GLN GLN LYS PRO
GLY SER SER PRO ARG LEU LEU ILE TYR ASP THR SER ASN

LEU ALA SER GLY VAL PRO VAL ARG PHE SER GLY SER GLY
SER GLY THR SER TYR SER LEU THR ILE SER ARG MET GLU
ALA GLU ASP ALA ALA THR TYR TYR CYS GLN GLN TRP SER
SER TYR PRO PRO ILE THR PHE GLY VAL GLY THR LYS LEU
GLU LEU LYS ARG ALA ASP ALA ALA PRO THR VAL SER ILE
PHE PRO PRO SER SER GLU GLN LEU THR SER GLY GLY ALA
SER VAL VAL CYS PHE LEU ASN ASN PHE TYR PRO LYS ASP
ILE ASN VAL LYS TRP LYS ILE ASP GLY SER GLU ARG GLN
ASN GLY VAL LEU ASN SER TRP THR ASP GLN ASP SER LYS
ASP SER THR TYR SER MET SER SER THR LEU THR LEU THR
LYS ASP GLU TYR GLU ARG HIS ASN SER TYR THR CYS GLU
ALA THR HIS LYS THR SER THR SER PRO ILE VAL LYS SER
PHE ASN ARG ASN GLU CYS SEQ ID NO:8

ASP ILE VAL LEU THR GLN SER PRO ALA ILE MET SER ALA
SER PRO GLY GLU LYS VAL THR MET THR CYS SER ALA SER
SER SER VAL ASN TYR MET TYR TRP TYR GLN GLN LYS SER
GLY THR SER PRO LYS ARG TRP ILE TYR ASP THR SER LYS
LEU ALA SER GLY VAL PRO VAL ARG PHE SER GLY SER GLY
SER GLY THR SER TYR SER LEU THR ILE SER SER MET GLU
THR GLU ASP ALA ALA THR TYR TYR CYS GLN GLN TRP GLY
ARG ASN PRO THR PHE GLY GLY GLY THR LYS LEU GLU ILE
LYS ARG ALA ASP ALA ALA PRO THR VAL SER ILE PHE PRO
PRO SER SER GLU GLN LEU THR SER GLY GLY ALA SER VAL
VAL CYS PHE LEU ASN ASN PHE TYR PRO LYS ASP ILE ASN
VAL LYS TRP LYS ILE ASP GLY SER GLU ARG GLN ASN GLY

VAL LEU ASN SER TRP THR ASP GLN ASP SER LYS ASP SER
THR TYR SER MET SER SER THR LEU THR LEU THR LYS ASP
GLU TYR GLU ARG HIS ASN SER TYR THR CYS GLU ALA THR
HIS LYS THR SER THR SER PRO ILE VAL LYS SER PHE ASN
ARG ASN GLU CYS SEQ ID NO:9

GLN ILE VAL LEU THR GLN SER PRO ALA ILE MET SER ALA
SER PRO GLY GLU LYS VAL THR MET THR CYS SER ALA SER
SER SER VAL SER TYR MET HIS TRP TYR GLN GLN LYS SER
GLY THR SER PRO LYS ARG TRP ILE TYR ASP THR SER LYS
LEU ALA SER GLY VAL PRO ALA ARG PHE SER GLY SER GLY
SER GLY THR SER TYR SER LEU THR ILE SER SER MET GLU
ALA GLU ASP ALA ALA THR TYR TYR CYS GLN GLN TRP SER
SER ASN PRO TYR THR PHE GLY GLY GLY THR LYS LEU GLU
ILE LYS SEQ ID NO:10

GLN ILE VAL LEU THR GLN SER PRO ALA ILE MET SER ALA
SER PRO GLY GLU LYS VAL THR MET THR CYS SER ALA SER
SER SER VAL SER TYR MET HIS TRP TYR GLN GLN LYS SER
GLY THR SER PRO LYS ARG TRP ILE TYR ASP THR SER LYS
LEU ALA SER GLY VAL PRO ALA ARG PHE SER GLY SER GLY
SER GLY THR SER TYR SER LEU THR ILE SER SER MET GLU
ALA GLU ASP ALA ALA THR TYR TYR CYS GLN GLN TRP SER
SER ASN PRO TYR THR PHE GLY GLY GLY THR LYS LEU GLU
ILE LYS SEQ ID NO:11

GLU ILE GLN LEU THR GLN SER PRO SER SER LEU SER ALA
SER LEU GLY GLU ARG VAL SER LEU THR CYS ARG THR SER
GLN GLU ILE SER GLY TYR LEU SER TRP LEU GLN GLN LYS
PRO ASP GLY THR ILE LYS ARG LEU ILE TYR ASP ALA THR
LYS LEU ASP SER GLY ALA PRO LYS ARG PHE SER GLY SER
ARG SER GLY SER ASP TYR SER LEU THR ILE SER SER LEU
GLU SER GLU ASP PHE ALA ASP TYR TYR CYS LEU GLN TYR
ALA SER PHE PRO ARG THR PHE GLY GLY GLY THR LYS LEU
GLU ILE LYS ARG THR VAL ALA ALA PRO SER VAL PHE ILE
PHE PRO PRO SER ASP GLU GLN LEU LYS SER GLY THR ALA
SER VAL VAL CYS LEU LEU ASN ASN PHE TYR PRO ARG GLU
ALA LYS VAL GLN TRP LYS VAL ASP ASN ALA LEU GLN SER
GLY ASN SER GLN GLU SER VAL THR GLU GLN ASP SER LYS
ASP SER THR TYR SER LEU SER SER THR LEU THR LEU SER
LYS ALA ASP TYR GLU LYS HIS LYS VAL TYR ALA CYS GLU
VAL THR HIS GLN GLY LEU SER SER PRO VAL THR LYS SER
PHE ASN ARG GLY GLU CYS SEQ ID NO:12

ASP ILE GLN MET THR GLN SER PRO SER SER LEU SER ALA
SER VAL GLY ASP ARG VAL THR ILE THR CYS GLN ALA SER
GLN ASP ILE SER ASP TYR LEU ILE TRP TYR GLN GLN LYS
LEU GLY LYS ALA PRO ASN LEU LEU ILE TYR ASP ALA SER
THR LEU GLU THR GLY VAL PRO SER ARG PHE SER GLY SER
GLY SER GLY THR GLU TYR THR PHE THR ILE SER SER LEU
GLN PRO GLU ASP ILE ALA THR TYR TYR CYS GLN GLN TYR
ASP ASP LEU PRO TYR THR PHE GLY GLN GLY THR LYS VAL

GLU ILE LYS ARG   SEQ ID NO:13

ASP ILE GLN MET THR GLN SER PRO SER SER LEU SER ALA
SER VAL GLY ASP ARG VAL THR ILE THR CYS GLN ALA SER
GLN ASP ILE SER ASP TYR LEU ILE TRP TYR GLN GLN LYS
LEU GLY LYS ALA PRO ASN LEU LEU ILE TYR ASP ALA SER
THR LEU GLU THR GLY VAL PRO SER ARG PHE SER GLY SER
GLY SER GLY THR GLU TYR THR PHE THR ILE SER SER LEU
GLN PRO GLU ASP ILE ALA THR TYR TYR CYS GLN GLN TYR
ASP ASP LEU PRO TYR THR PHE GLY GLN GLY THR LYS VAL
GLU ILE LYS ARG SEQ ID NO:14

ASP ILE GLN MET THR GLN SER PRO SER SER LEU SER ALA
SER VAL GLY ASP ARG VAL THR ILE THR CYS GLN ALA SER
GLN ASP ILE SER ASP TYR LEU ILE TRP TYR GLN GLN LYS
LEU GLY LYS ALA PRO ASN LEU LEU ILE TYR ASP ALA SER
THR LEU GLU THR GLY VAL PRO SER ARG PHE SER GLY SER
GLY SER GLY THR GLU TYR THR PHE THR ILE SER SER LEU
GLN PRO GLU ASP ILE ALA THR TYR TYR CYS GLN GLN TYR
ASP ASP LEU PRO TYR THR PHE GLY GLN GLY THR LYS VAL
GLU ILE LYS ARG SEQ ID NO:15

ASP ILE GLN MET THR GLN SER PRO SER SER LEU SER ALA
SER VAL GLY ASP ARG VAL THR ILE THR CYS GLN ALA SER
GLN ASP ILE SER ASP TYR LEU ILE TRP TYR GLN GLN LYS
LEU GLY LYS ALA PRO ASN LEU LEU ILE TYR ASP ALA SER

THR LEU GLU THR GLY VAL PRO SER ARG PHE SER GLY SER
GLY SER GLY THR GLU TYR THR PHE THR ILE SER SER LEU
GLN PRO GLU ASP ILE ALA THR TYR TYR CYS GLN GLN TYR
ASP ASP LEU PRO TYR THR PHE GLY GLN GLY THR LYS VAL
SEQ ID NO:16

DIQMTQSPSTLSASVGDRVTITCRASQSISSWLAWYQQKPGKAPKLLIYDAS
SLESGVPSRFSGSGSGTEFTLTISSLQPDDFATYYCQQYNSYS
SEQ ID NO:17

DIVLTQAPPSLDVSQGRATISCRTSKSVRTSSYSYMHWYQQKPGQPPKLLNL
CASNQVSRVPARFSGSGSGTDFTLKIHPVEEEDAATYFCQQSNENP SEQ ID
NO:18

VIWMTQSPSLLSASTGDRVTISCRMSQGISSYLAWYQQKPGKAPELLIYAAS
TLQSGVPSRFSGSGSGTDFTLTISCLQSEDFATYYCQQYYSFP SEQ ID
NO:19

AIQLTQSPSSLSASVGDRVTITCRASQGISSALAYQQKPGKAPKLLIYDASSLE
SGVPSRFSGSGSGTDFTLTISSLQPEDFATYYCQQFNNYP SEQ ID NO:20

AIQLTQSPSSLSASVGDRVTITCRASQGISSALAWYQQKPGKAPKLLIYDASS
LESGVPSRFSGSGSGTDFTLTISSLQPEDFATYYCQQFNNYP SEQ ID NO:21

DIQMTQSPSSLSASVGDRVTITCQASQDISNYLNWYQQKPGKAPKLLIYDAS
NLETGVPSRFSGSGSGTDFTFTISSLQPEDIATYYCQQYDNLP SEQ ID
NO:22

DIQMTQSPSSLSASVGDRVTITCQASQDISNYLNWYQQKPGKAPKLLIYDAS
NLETGVPSRFSGSGSGTDFTFTISSLQPEDIATYYCQQYDNLP SEQ ID
NO:23

DIQMIQSPSFLSASVGDRVSIICWASEGISSNLAWYLQKPGKSPKLFLYDAKD
LHPGVSSRFSGRGSGTDFTLTIISLKPEDFAAYYCKQDFSYP SEQ ID NO:24

EIVLTQSPATLSLSPGERATLSCRASQSVSSYLAWYQQKPGQAPRLLIYDASN
RATGIPARFSGSGSGTDFTLTISSLEPEDFAVYYCQQRSNWP SEQ ID NO:25

EIVLTQSPATLSLSPGERATLSCRASQGVSSYLAWYQQKPGQAPRLLIYDAS
NRATGIPARFSGSGPGTDFTLTISSLEPEDFAVYYCQQRSNWH SEQ ID
NO:26

EIVLTQSPATLSLSPGERATLSCGASQSVSSSYLAWYQQKPGLAPRLLIYDAS
SRATGIPDRFSGSGSGTDFTLTISRLEPEDFAVYYCQQYGSSP SEQ ID
NO:27

QIVLTQSPAIMSASPGEKVTMTCSASSSVSYMYWYQQKPGSSPRLLIYDTSN
LASGVPVRFSGSGSGTSYSLTISRMEAEDAATYYCQQWSSYPP SEQ ID
NO:28

QIVLTQSPAIMSASPGEKVTMTCSASSSVSYMHWYQQKSGTSPKRWIYDTS
KLASGVPARFSGSGSGTSYSLTISSMEAEDAATYYCQQWSSNP SEQ ID
NO:29

QIVLTQSPAIMSASPGEKVTMTCSASSSVSYMYWYQQKPGSSPRLWIYDTSN
LVSGVPARFSGSRSGTSYSLTISSMEAEDAATYYCQQYSGYPS SEQ ID
NO:30

ENVLTQSPAIMSASPGEKVTMTCSASSSVSYMHWYQQKSSTSPKLWIYDTS
KLASGVPGRFSGSGSGNSYSLTISSMEAEDVATYYCFQGSGYPL SEQ ID
NO:31

QILLTQSPAIMSASPGEKVTMTCSASSSVSYMHWYQQKPGSSPKPWIYDTSN
LASGFPARFSGSGSGTSYSLIISSMEAEDAATYYCHQRSSYP SEQ ID NO:32

QIVLTQSPAIMSASPGEKVTMTCSASSSISYMHWYQQKPGTSPKRWIYDTSK
LASGVPARFSGSGSGTSYSLTISSMEAEDAATYYCHQRSSYP SEQ ID
NO:33

DIQMTQSPASLSASVGETVTITCQASENIASDLAWYQKQGKSPQLLVYDAR
NLADGVPSRFSGSGSGTHYSLNIHSLQSEDVARYYCQHYYGTP SEQ ID
NO:34

AQVLTQTESPVSAPVGGTVTINCQASQSVYDNNYLSWYQQKPGQPPKLLIY
DASKLASGVPSRFSGSGSGTQFTLTISGVQCDDAATYYCQGSYYSSGWY
SEQ ID NO:35

AQGPTQTPSSVSAAVGGTVTINCQTSESFYSNNILSWYQQKPGQPPKLLIYD
ASTLASGVPSRFKGSGSGTQFTLTISDVQCDDAATYYCQGSYHSSGWY
SEQ ID NO:36

AAVLTQTPSPVSAAVGGTVTIKCQSSQSVYNNNLLSWYQQKPGQPPKLLIY
DASNLASGVPDRFSGSGSGTQFTLTISGVQCDDAATYYCLGGYDDDAD
SEQ ID NO:37

AVVLTQTASPVSAAVGGTVTINCQASQSISTALAWYQQKPGQRPKLLIYDAS

KLASGVPSRFKGSGSGTQFTLTISGVQCDDAATYYCQQGYSSSSAD
SEQ ID NO:38

GLU VAL LYS VAL ILE GLU SER GLY GLY GLY LEU VAL GLN
PRO GLY GLY SER LEU LYS LEU SER CYS ALA ALA SER GLY
PHE ASP PHE SER ARG TYR TRP MET SER TRP VAL ARG GLN
ALA PRO GLY LYS GLY LEU GLU TRP ILE GLY GLU ILE ASN
PRO ASP SER SER THR ILE ASN TYR THR PRO SER LEU LYS
ASP LYS PHE ILE ILE SER ARG ASP ASN ALA LYS ASN THR
LEU TYR LEU GLN MET SER LYS VAL ARG SER GLU ASP THR
ALA LEU TYR TYR CYS ALA ARG LEU GLY GLY ASP LEU HIS
TYR TYR ALA MET ASP TYR TRP GLY GLN GLY THR SER VAL
THR VAL SER SER SEQ ID NO:39

GLU VAL LYS VAL ILE GLU SER GLY GLY GLY LEU VAL GLN
PRO GLY GLY SER LEU LYS LEU SER CYS ALA ALA SER GLY
PHE ASP PHE SER ARG TYR TRP MET SER TRP VAL ARG GLN
ALA PRO GLY LYS GLY LEU GLU TRP ILE GLY GLU ILE ASN
PRO ASP SER SER THR ILE ASN TYR THR PRO SER LEU LYS
ASP LYS PHE ILE ILE SER ARG ASP ASN ALA LYS ASN THR
LEU TYR LEU GLN MET SER LYS VAL ARG SER GLU ASP THR
ALA LEU TYR TYR CYS ALA ARG LEU GLY GLY ASP LEU HIS
TYR TYR ALA MET ASP TYR TRP GLY GLN GLY THR SER VAL
THR VAL SER SER  SEQ ID NO:40

GLU VAL LYS LEU LEU GLU SER GLY GLY GLY LEU VAL GLN

PRO GLY GLY SER LEU LYS LEU SER CYS ALA ALA SER GLY
PHE ASP PHE SER LYS TYR TRP MET SER TRP VAL ARG GLN
ALA PRO GLY LYS GLY LEU GLU TRP ILE GLY GLU ILE HIS
PRO ASP SER GLY THR ILE ASN TYR THR PRO SER LEU LYS
ASP LYS PHE ILE ILE SER ARG ASP ASN ALA LYS ASN SER
LEU TYR LEU GLN MET SER LYS VAL ARG SER GLU ASP THR
ALA LEU TYR TYR CYS ALA ARG LEU HIS TYR TYR GLY TYR
ASN ALA TYR TRP GLY GLN GLY THR LEU VAL THR VAL SER
ALA GLU SER ALA ARG ASN PRO THR ILE TYR PRO LEU THR
LEU PRO PRO ALA LEU SER SER ASP PRO VAL ILE ILE GLY
CYS LEU ILE HIS ASP TYR PHE PRO SER GLY THR MET ASN
VAL THR TRP GLY LYS SER GLY LYS ASP ILE THR THR VAL
ASN PHE PRO PRO ALA LEU ALA SER GLY GLY ARG TYR THR
MET SER ASN GLN LEU THR LEU PRO ALA VAL GLU CYS PRO
GLU GLY GLU SER VAL LYS CYS SER VAL GLN HIS ASP SER
ASN PRO VAL GLN GLU LEU ASP VAL ASN CYS SER GLY
SEQ ID NO:41

MET TYR ARG SER ALA PHE SER VAL GLY LEU GLU THR ARG
VAL THR VAL PRO ASN VAL PRO ILE ARG PHE THR LYS ILE
PHE TYR ASN GLN GLN ASN HIS TYR ASP GLY SER THR GLY
LYS PHE TYR CYS ASN ILE PRO GLY LEU TYR TYR PHE SER
TYR HIS ILE THR VAL TYR MET LYS ASP VAL LYS VAL SER
LEU PHE LYS LYS ASP LYS ALA VAL LEU PHE THR TYR ASP
GLN TYR GLN GLU LYS ASN VAL ASP GLN ALA SER GLY SER
VAL LEU LEU HIS LEU GLU VAL GLY ASP GLN VAL TRP LEU

GLN VAL TYR GLY ASP GLY ASP HIS ASN GLY LEU TYR ALA
ASP ASN VAL ASN ASP SER THR PHE THR GLY PHE LEU LEU
TYR HIS ASP THR ASN   SEQ ID NO:42

MET TYR ARG SER ALA PHE SER VAL GLY LEU GLU THR ARG
VAL THR VAL PRO ASN VAL PRO ILE ARG PHE THR LYS ILE
PHE TYR ASN GLN GLN ASN HIS TYR ASP GLY SER THR GLY
LYS PHE TYR CYS ASN ILE PRO GLY LEU TYR TYR PHE SER
TYR HIS ILE THR VAL TYR MET LYS ASP VAL LYS VAL SER
LEU PHE LYS LYS ASP LYS ALA VAL LEU PHE THR TYR ASP
GLN TYR GLN GLU LYS ASN VAL ASP GLN ALA SER GLY SER
VAL LEU LEU HIS LEU GLU VAL GLy ASP GLN VAL TRP LEU
GLN VAL TYR GLY ASP GLY ASP HIS ASN GLY LEU TYR ALA
ASP ASN VAL ASN ASP SER THR PHE THR GLY PHE LEU LEU
TYR HIS ASP THR ASN SEQ ID NO:43

MET TYR ARG SER ALA PHE SER VAL GLY LEU GLU THR ARG
VAL THR VAL PRO ASN VAL PRO ILE ARG PHE THR LYS ILE
PHE TYR ASN GLN GLN ASN HIS TYR ASP GLY SER THR GLY
LYS PHE TYR CYS ASN ILE PRO GLY LEU TYR TYR PHE SER
TYR HIS ILE THR VAL TYR MET LYS ASP VAL LYS VAL SER
LEU PHE LYS LYS ASP LYS ALA VAL LEU PHE THR TYR ASP
GLN TYR GLN GLU LYS ASN VAL ASP GLN ALA SER GLY SER
VAL LEU LEU HIS LEU GLU VAL GLY ASP GLN VAL TRP LEU
GLN VAL TYR GLY ASP GLY ASP HIS ASN GLY LEU TYR ALA
ASP ASN VAL ASN ASP SER THR PHE THR GLY PHE LEU LEU

TYR HIS ASP THR ASN SEQ ID NO:44

MET TYR ARG SER ALA PHE SER VAL GLY LEU GLU THR ARG
VAL THR VAL PRO ASN VAL PRO ILE ARG PHE THR LYS ILE
PHE TYR ASN GLN GLN ASN HIS TYR ASP GLY SER THR GLY
LYS PHE TYR CYS ASN ILE PRO GLY LEU TYR TYR PHE SER
TYR HIS ILE THR VAL TYR MET LYS ASP VAL LYS VAL SER
LEU PHE LYS LYS ASP LYS ALA VAL LEU PHE THR TYR ASP
GLN TYR GLN GLU LYS ASN VAL ASP GLN ALA SER GLY SER
VAL LEU LEU HIS LEU GLU VAL GLY ASP GLN VAL TRP LEU
GLN VAL TYR GLY ASP GLY ASP HIS ASN GLY LEU TYR ALA
ASP ASN VAL ASN ASP SER THR PHE THR GLY PHE LEU LEU
TYR HIS ASP THR ASN SEQ ID NO:45

MET TYR ARG SER ALA PHE SER VAL GLY LEU GLU THR ARG
VAL THR VAL PRO ASN VAL PRO ILE ARG PHE THR LYS ILE
PHE TYR ASN GLN GLN ASN HIS TYR ASP GLY SER THR GLY
LYS PHE TYR CYS ASN ILE PRO GLY LEU TYR TYR PHE SER
TYR HIS ILE THR VAL TYR MET LYS ASP VAL LYS VAL SER
LEU PHE LYS LYS ASP LYS ALA VAL LEU PHE THR TYR ASP
GLN TYR GLN GLU LYS ASN VAL ASP GLN ALA SER GLY SER
VAL LEU LEU HIS LEU GLU VAL GLY ASP GLN VAL TRP LEU
GLN VAL TYR GLY ASP GLY ASP HIS ASN GLY LEU TYR ALA
ASP ASN VAL ASN ASP SER THR PHE THR GLY PHE LEU LEU
TYR HIS ASP THR ASN SEQ ID NO:46

MET TYR ARG SER ALA PHE SER VAL GLY LEU GLU THR ARG
VAL THR VAL PRO ASN VAL PRO ILE ARG PHE THR LYS ILE
PHE TYR ASN GLN GLN ASN HIS TYR ASP GLY SER THR GLY
LYS PHE TYR CYS ASN ILE PRO GLY LEU TYR TYR PHE SER
TYR HIS ILE THR VAL TYR MET LYS ASP VAL LYS VAL SER
LEU PHE LYS LYS ASP LYS ALA VAL LEU PHE THR TYR ASP
GLN TYR GLN GLU LYS ASN VAL ASP GLN ALA SER GLY SER
VAL LEU LEU HIS LEU GLU VAL GLY ASP GLN VAL TRP LEU
GLN VAL TYR GLY ASP GLY ASP HIS ASN GLY LEU TYR ALA
ASP ASN VAL ASN ASP SER THR PHE THR GLY PHE LEU LEU
TYR HIS ASP THR ASN SEQ ID NO:47

ALA VAL GLN LEU GLU GLN SER GLY PRO GLY LEU VAL ARG
PRO SER GLN THR LEU SER LEU THR CYS THR VAL SER GLY
THR SER PHE ASP ASP TYR TYR TRP THR TRP VAL ARG GLN
PRO PRO GLY ARG GLY LEU GLU TRP ILE GLY TYR VAL PHE
TYR THR GLY THR THR LEU LEU ASP PRO SER LEU ARG GLY
ARG VAL THR MET LEU VAL ASN THR SER LYS ASN GLN PHE
SER LEU ARG LEU SER SER VAL THR ALA ALA ASP THR ALA
VAL TYR TYR CYS ALA ARG ASN LEU ILE ALA GLY GLY ILE
ASP VAL TRP GLY GLN GLY SER LEU VAL THR VAL SER SER
ALA SER THR LYS GLY PRO SER VAL PHE PRO LEU ALA PRO
SER SER LYS SER THR SER GLY GLY THR ALA ALA LEU GLY
CYS LEU VAL LYS ASP TYR PHE PRO GLU PRO VAL THR VAL
SER TRP ASN SER GLY ALA LEU THR SER GLY VAL HIS THR

PHE PRO ALA VAL LEU GLN SER SER GLY LEU TYR SER LEU
SER SER VAL VAL THR VAL PRO SER SER SER LEU GLY THR
GLN THR TYR ILE CYS ASN VAL ASN HIS LYS PRO SER ASN
THR LYS VAL ASP LYS LYS VAL GLU PRO SEQ ID NO:48

GLU VAL GLN LEU VAL GLU SER GLY GLY GLY LEU VAL GLN
PRO GLY ARG SER LEU ARG LEU SER CYS VAL THR SER GLY
PHE THR PHE ASP ASP TYR ALA MET HIS TRP VAL ARG GLN
SER PRO GLY LYS GLY LEU GLU TRP VAL SER GLY ILE SER
TRP ASN THR GLY THR ILE ILE TYR ALA ASP SER VAL LYS
GLY ARG PHE ILE ILE SER ARG ASP ASN ALA LYS ASN SER
LEU TYR LEU GLN MET ASN SER LEU ARG VAL GLU ASP THR
ALA LEU TYR TYR CYS ALA LYS THR ARG SER TYR VAL VAL
ALA ALA GLU TYR TYR PHE HIS TYR TRP GLY GLN GLY ILE
LEU VAL THR VAL SER SER GLY SER ALA SER ALA PRO THR
LEU PHE PRO LEU VAL SER CYS GLU ASN SER ASN PRO SER
SER THR VAL ALA VAL GLY CYS LEU ALA GLN ASP PHE LEU
PRO ASP SER ILE THR PHE SER TRP LYS TYR LYS ASN ASN
SER ASP ILE SER SER THR ARG GLY PHE PRO SER VAL LEU
ARG GLY GLY LYS TYR ALA ALA THR SER GLN VAL LEU LEU
PRO SER LYS ASP VAL MET GLN GLY THR ASN GLU HIS VAL
VAL CYS LYS VAL GLN HIS PRO ASN GLY ASN LYS GLU LYS
ASP VAL PRO LEU SEQ ID NO:49

ASP VAL GLN LEU GLN ALA SER GLY GLY GLY SER VAL GLN
ALA GLY GLY SER LEU ARG LEU SER CYS ALA ALA SER GLY

TYR THR ILE GLY PRO TYR CYS MET GLY TRP PHE ARG GLN
ALA PRO GLY LYS GLU ARG GLU GLY VAL ALA ALA ILE ASN
MET GLY GLY GLY ILE THR TYR TYR ALA ASP SER VAL LYS
GLY ARG PHE THR ILE SER GLN ASP ASN ALA LYS ASN THR
VAL TYR LEU LEU MET ASN SER LEU GLU PRO GLU ASP THR
ALA ILE TYR TYR CYS ALA ALA ASP SER THR ILE TYR ALA
SER TYR TYR GLU CYS GLY HIS GLY LEU SER THR GLY GLY
TYR GLY TYR ASP SER TRP GLY GLN GLY THR GLN VAL THR
VAL SER SER GLY ARG TYR PRO TYR ASP VAL PRO ASP TYR
GLY SER GLY ARG ALA SEQ ID NO:50

ASP VAL GLN LEU GLN GLN SER GLY PRO GLU LEU GLU LYS
PRO GLY ALA SER VAL LYS ILE SER CYS LYS ALA SER GLY
PHE SER LEU PRO GLY HIS ASN ILE ASN TRP ILE VAL GLN
ARG ASN GLY LYS SER LEU GLU TRP ILE GLY ASN ILE ASP
PRO TYR TYR GLY GLY THR ASN PHE ASN PRO LYS PHE LYS
GLY LYS ALA THR LEU THR VAL ASP LYS SER SER SER THR
LEU TYR MET HIS LEU THR SER LEU GLN SER GLU ASP SER
ALA VAL TYR TYR CYS ALA ARG ARG ARG ASP GLY ASN TYR
GLY PHE THR TYR TRP GLY GLN GLY THR LEU VAL THR VAL
SER ALA ALA LYS THR THR PRO PRO SER VAL TYR PRO LEU
ALA PRO GLY SER ALA ALA GLN THR ASN SER MET VAL THR
LEU GLY CYS LEU VAL LYS GLY TYR PHE PRO GLU PRO VAL
THR VAL THR TRP ASN SER GLY SER LEU SER SER GLY VAL
HIS THR PHE PRO ALA VAL LEU GLN SER ASP LEU TYR THR
LEU SER SER SER VAL THR VAL PRO SER SER THR TRP PRO

SER GLU THR VAL THR CYS ASN VAL ALA HIS PRO ALA SER
SER THR LYS VAL ASP LYS LYS ILE SEQ ID NO:51

QDTLKEYGPGKLPSQTFSLTCTFSGFSLSTYGMMVSWMCQPSGKGLVWLA
LIWCNNDKGYNPFLRSQLTISKDTSNNQVFLKITSVDPADTATYYCA
SEQ ID NO:52

QVQLQQSGPGLVKPSQTLSLTCAISGDSVSSNSAAWNWIRQSPSRGLEWLG
RTYYRSKWYNDYAVSVKSRITINPDTSKNQFSLQLNSVTPEDTAVYYCAR
SEQ ID NO:53

ASP ILE GLN MET THR GLN SER PRO ALA ILE MET SER ALA
SER PRO GLY GLU LYS VAL THR MET THR CYS SER ALA SER
SER SER VAL SER TYR MET TYR TRP TYR GLN GLN LYS PRO
GLY SER SER PRO ARG LEU LEU ILE TYR ASP SER THR ASN
LEU ALA SER GLY VAL PRO VAL ARG PHE SER GLY SER GLY
SER GLY THR SER TYR SER LEU THR ILE SER ARG MET GLU
ALA GLU ASP ALA ALA THR TYR TYR CYS GLN GLN TRP SER
THR TYR PRO LEU THR PHE GLY ALA GLY THR LYS LEU GLU
LEU LYS ARG ALA ASP ALA ALA PRO THR VAL SER ILE PHE
PRO PRO SER SER GLU GLN LEU THR SER GLY GLY ALA SER
VAL VAL CYS PHE LEU ASN ASN PHE TYR PRO LYS ASP ILE
ASN VAL LYS TRP LYS ILE ASP GLY SER GLU ARG GLN ASN
GLY VAL LEU ASN SER TRP THR ASP GLN ASP SER LYS ASP
SER THR TYR SER MET SER SER THR LEU THR LEU THR LYS
ASP GLU TYR GLU ARG HIS ASN SER TYR THR CYS GLU ALA
THR HIS LYS THR SER THR SER PRO ILE VAL LYS SER PHE
ASN ARG   SEQ ID NO:54

GLN ILE VAL SER THR GLN SER PRO ALA ILE MET SER ALA
SER PRO GLY GLU LYS VAL THR MET THR CYS SER ALA SER

SER SER ARG SER TYR MET GLN TRP TYR GLN GLN LYS PRO
GLY THR SER PRO LYS ARG TRP ILE TYR ASP THR SER LYS
LEU ALA SER GLY VAL PRO ALA ARG PHE SER GLY SER GLY
SER GLY THR SER TYR SER LEU THR ILE SER SER MET GLU
ALA GLU ASP ALA ALA THR TYR TYR CYS HIS GLN ARG SER
SER TYR THR PHE GLY GLY GLY THR LYS LEU GLU ILE LYS
ARG THR VAL ALA ALA PRO SER VAL PHE ILE PHE PRO PRO
SER ASP GLU GLN LEU LYS SER GLY THR ALA SER VAL VAL
CYS LEU LEU ASN ASN PHE TYR PRO ARG GLU ALA LYS VAL
GLN TRP LYS VAL ASP ASN ALA LEU GLN SER GLY ASN SER
GLN GLU SER VAL THR GLU GLN ASP SER LYS ASP SER THR
TYR SER LEU SER SER THR LEU THR LEU SER LYS ALA ASP
TYR GLU LYS HIS LYS VAL TYR ALA CYS GLU VAL THR HIS
GLN GLY LEU SER SER PRO VAL THR LYS SER PHE ASN ARG
GLY GLU SEQ ID NO:55

GLU LEU VAL MET THR GLN SER PRO SER SER LEU SER ALA
SER VAL GLY ASP ARG VAL ASN ILE ALA CYS ARG ALA SER
GLN GLY ILE SER SER ALA LEU ALA TRP TYR GLN GLN LYS
PRO GLY LYS ALA PRO ARG LEU LEU ILE TYR ASP ALA SER
ASN LEU GLU SER GLY VAL PRO SER ARG PHE SER GLY SER
GLY SER GLY THR ASP PHE THR LEU THR ILE SER SER LEU
GLN PRO GLU ASP PHE ALA ILE TYR TYR CYS GLN GLN PHE
ASN SER TYR PRO LEU THR PHE GLY GLY GLY THR LYS VAL
GLU ILE LYS ARG THR VAL ALA ALA PRO SER VAL PHE ILE
PHE PRO PRO SER ASP GLU GLN LEU LYS SER GLY THR ALA
SER VAL VAL CYS LEU LEU ASN ASN PHE TYR PRO ARG GLU
ALA LYS VAL GLN TRP LYS VAL ASP ASN ALA LEU GLN SER
GLY ASN SER GLN GLU SER VAL THR GLU GLN ASP SER LYS
ASP SER THR TYR SER LEU SER SER THR LEU THR LEU SER
LYS ALA ASP TYR GLU LYS HIS LYS VAL TYR ALA CYS GLU

VAL THR HIS GLN GLY LEU SER SER PRO VAL THR LYS SER
PHE ASN ARG GLY GLU CYS SEQ ID NO:56

GLU LEU VAL MET THR GLN SER PRO SER SER LEU SER ALA
SER VAL GLY ASP ARG VAL ASN ILE ALA CYS ARG ALA SER
GLN GLY ILE SER SER ALA LEU ALA TRP TYR GLN GLN LYS
PRO GLY LYS ALA PRO ARG LEU LEU ILE TYR ASP ALA SER
ASN LEU GLU SER GLY VAL PRO SER ARG PHE SER GLY SER
GLY SER GLY THR ASP PHE THR LEU THR ILE SER SER LEU
GLN PRO GLU ASP PHE ALA ILE TYR TYR CYS GLN GLN PHE
ASN SER TYR PRO LEU THR PHE GLY GLY GLY THR LYS VAL
GLU ILE LYS ARG THR VAL ALA ALA PRO SER VAL PHE ILE
PHE PRO PRO SER ASP GLU GLN LEU LYS SER GLY THR ALA
SER VAL VAL CYS LEU LEU ASN ASN PHE TYR PRO ARG GLU
ALA LYS VAL GLN TRP LYS VAL ASP ASN ALA LEU GLN SER
GLY ASN SER GLN GLU SER VAL THR GLU GLN ASP SER LYS
ASP SER THR TYR SER LEU SER SER THR LEU THR LEU SER
LYS ALA ASP TYR GLU LYS HIS LYS VAL TYR ALA CYS GLU
VAL THR HIS GLN GLY LEU SER SER PRO VAL THR LYS SER
PHE ASN ARG GLY GLU CYS  SEQ ID NO:57

GLU ILE VAL MET THR GLN SER PRO ALA SER LEU SER LEU
SER PRO GLY GLU ARG ALA THR LEU SER CYS ARG ALA SER
GLN SER VAL SER ASN TYR LEU ALA TRP TYR GLN GLN LYS
PRO GLY GLN ALA PRO ARG LEU LEU ILE HIS ASP ALA SER
GLY ARG ALA THR GLY ILE PRO ASP ARG PHE SER GLY SER
THR ASP PHE THR LEU THR ILE SER ARG LEU
GLU PRO GLU ASP PHE ALA VAL TYR TYR CYS GLN GLN ARG
ALA ASN TRP GLY THR TRP THR PHE GLY GLN GLY THR LYS
VAL GLU ILE LYS ARG THR SEQ ID NO:58

GLU ILE VAL LEU THR GLN SER PRO ALA THR LEU SER LEU

SER PRO GLY GLU ARG ALA THR LEU SER CYS GLY ALA SER
GLN SER VAL SER SER ASN TYR LEU ALA TRP TYR GLN GLN
LYS PRO GLY GLN ALA PRO ARG LEU LEU ILE TYR ASP ALA
SER SER ARG ALA THR GLY ILE PRO ASP ARG PHE SER GLY
SER GLY SER GLY THR ASP PHE THR LEU THR ILE SER ARG
LEU GLU PRO GLU ASP PHE ALA VAL TYR TYR CYS GLN GLN
TYR GLY SER SER PRO LEU THR PHE GLY GLY GLY THR LYS
VAL GLU ILE LYS ARG THR VAL ALA ALA PRO SER VAL PHE
ILE PHE PRO PRO SER ASP GLU GLN LEU LYS SER GLY THR
ALA SER VAL VAL CYS LEU LEU ASN ASN PHE TYR PRO ARG
GLU ALA LYS VAL GLN TRP LYS VAL ASP ASN ALA LEU GLN
SER GLY ASN SER GLN GLU SER VAL THR GLU GLN ASP SER
LYS ASP SER THR TYR SER LEU SER SER THR LEU THR LEU
SER LYS ALA ASP TYR GLU LYS HIS LYS VAL TYR ALA CYS
GLU VAL THR HIS GLN GLY LEU SER SER PRO VAL THR LYS
SER PHE ASN ARG GLY GLU CYS SEQ ID NO:59

ASP ILE GLN MET THR GLN SER PRO ALA ILE MET SER ALA
SER PRO GLY GLU LYS VAL THR MET THR CYS SER ALA SER
SER SER VAL SER TYR MET TYR TRP TYR GLN GLN LYS PRO
GLY SER SER PRO ARG LEU LEU ILE TYR ASP SER THR ASN
LEU ALA SER GLY VAL PRO VAL ARG PHE SER GLY SER GLY
SER GLY THR SER TYR SER LEU THR ILE SER ARG MET GLU
ALA GLU ASP ALA ALA THR TYR TYR CYS GLN GLN TRP SER
THR TYR PRO LEU THR PHE GLY ALA GLY THR LYS LEU GLU
LEU LYS ARG ALA ASP ALA ALA PRO THR VAL SER ILE PHE
PRO PRO SER SER GLU GLN LEU THR SER GLY GLY ALA SER
VAL VAL CYS PHE LEU ASN ASN PHE TYR PRO LYS ASP ILE
ASN VAL LYS TRP LYS ILE ASP GLY SER GLU ARG GLN ASN
GLY VAL LEU ASN SER TRP THR ASP GLN ASP SER LYS ASP
SER THR TYR SER MET SER SER THR LEU THR LEU THR LYS

ASP GLU TYR GLU ARG HIS ASN SER TYR THR CYS GLU ALA
THR HIS LYS THR SER THR SER PRO ILE VAL LYS SER PHE
ASN ARG SEQ ID NO:60

### TARGET LIGANDS, COMPOSITIONS AND METHODS

The polypeptides that unfold reversibly described herein can have binding specificity for a target ligand. For example, a polypeptide that comprises an antibody variable region that unfolds reversibly and has binding specificity for a particular target ligand can be selected, isolated and/or recovered using any suitable method, such as the binding methods described herein. Exemplary target ligands that polypeptides that unfold reversibly (*e.g.,* polypeptides comprising a reversibly unfoldable V_{H} or V_{K}) can have binding specificity for include, human or animal proteins, cytokines, cytokine receptors, enzymes, co-factors for enzymes and DNA binding proteins. Suitable cytokines and growth factors, cytokine and growth factor receptor and other target ligands include but are not limited to: ApoE, Apo-SAA, BDNF, Cardiotrophin-1, CEA, CD40, CD40 Ligand, CD56, CD38, CD138, EGF, EGF receptor, ENA-78, Eotaxin, Eotaxin-2, Exodus-2, FAPα, FGF-acidic, FGF-basic, fibroblast growth factor-10, FLT3 ligand, Fractalkine (CX3C), GDNF, G-CSF, GM-CSF, GF-β1, human serum albumin, insulin, IFN-γ, IGF-I, IGF-II, IL-1α, IL-1β, IL-1 receptor, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF A, VEGF B, VEGF C, VEGF D, VEGF receptor 1, VEGF receptor 2, VEGF receptor 3, GCP-2, GRO/MGSA, GRO-β, GRO-γ, HCC1, 1-309, HER 1, HER 2, HER 3 and HER 4. It will be appreciated that this list is by no means exhaustive.

In some embodiments, the invention is an isolated polypeptide that comprises an antibody variable domain that unfolds reversibly and binds a target ligand. Preferably, the antibody variable domain that unfolds reversibly binds a target ligand that is a cytokine, growth factor, cytokine receptor or growth factor receptor (*e.g.,* a human cytokine, human growth factor, human cytokine receptor or human growth factor receptor). More preferably, the antibody variable domain that unfolds reversibly neutralizes the activity of the a cytokine, growth factor, cytokine receptor or growth factor receptor with a neutralized dose 50 (ND50) of about 1 µM or less, or 500 nM or less, in a standard cellular assay, such as the assay that measures TNF-induced IL-8 secretion by HeLa cells described herein. In particular embodiments, the antibody variable domain that unfolds reversibly neutralizes the activity of the a cytokine, growth factor, cytokine receptor or growth factor receptor with a ND50 of about or about 400 nM or less, or about 300 nM or less, or about 200 nM or less, or about 100 nM or less, or about 1 nM or less, or about 100 pM or less, or about 10 pM or less.

In other embodiments, the antibody variable domain that unfolds reversibly binds a cytokine or growth factor, and inhibits the interaction of the cytokine or growth factor with a cognate cytokine receptor or growth factor receptor with an inhibitory concentration 50 (IC50) of about 1 µM or less, or about 500 nM or less, in a standard receptor binding assay, such as the assay TNF Receptor I (p55) assay described herein. In particular embodiments, the antibody variable domain that unfolds reversibly inhibits the interaction of the cytokine or growth factor with a cognate cytokine receptor or growth factor receptor with IC50 of about 400 nM or less, or about 300 nM or less, or about 200 nM or less, or about 100 nM or less, or about I nM or less, or about 100 pM or less, or about 10 pM or less. In other embodiments, the antibody variable domain that unfolds reversibly binds a cytokine receptor or growth factor receptor, and inhibits the interaction of the cytokine receptor or growth factor receptor with a cognate cytokine or growth factor with an inhibitory concentration 50 (IC50) of about 1 µM or less, or about 500 nM or less, in a standard receptor binding assay, such as the assay TNF Receptor 1 (p55) assay described herein. In particular embodiments, the antibody variable domain that unfolds reversibly inhibits the interaction of the cytokine receptor or growth factor receptor with a cognate cytokine or growth factor with IC50 of about or about 400 nM or less, or about 300 nM or less, or about 200 nM or less, or about 100 nM or less, or about 1 nM or less, or about 100 pM or less, or about 10 pM or less.

Compositions comprising a polypeptide that unfolds reversibly, including pharmaceutical or physiological compositions are provided. Pharmaceutical or physiological compositions comprise one or more polypeptide that unfolds reversibly and a pharmaceutically or physiologically acceptable carrier. Typically, these carriers include aqueous or alcoholic/aqueous solutions, emulsions or suspensions, including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates. Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The compositions can comprise a desired amount of polypeptide that unfolds reversibly. For example the compositions can comprise about 5% to about 99% polypeptide that unfolds reversibly by weight. In particular embodiments, the composition can comprise about 10% to about 99%, or about 20% to about 99%, or about 30% to about 99% or about 40% to about 99%, or about 50% to about 99%, or about 60% to about 99%, or about 70% to about 99%, or about 80% to about 99%, or about 90% to about 99%, or about 95% to about 99% polypeptide that unfolds reversibly by weight.

In one example, the composition is a biological washing powder comprising a polypeptide that unfolds reversibly (*e.g.,* a polypeptide comprising an immunoglobulin variable domain that unfolds reversibly). In another embodiment, the composition is freeze dried (lyophilized).

The invention also provides a sealed package (e.g., a sealed sterile package) comprising a polypeptide that unfolds reversibly (e.g., when heated (*e.g*., a polypeptide comprising an immunoglobulin variable domain that unfolds reversibly)). In some embodiments, the sealed package further comprises a sterile instrument. In particular embodiments, the sterile instrument is a medical instrument, such as a surgical instrument.

The polypeptides that unfold reversibly described herein will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of polypeptides that unfold reversibly in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton, New York, pp. 179-213; McFarlin et al. (1973) Science, 179: 478: and Satoh et al. (1987) J. Immunol., 138: 179).

The selected polypeptides of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the selected antibodies, receptors or binding proteins thereof of the present invention, or even combinations of selected polypeptides according to the present invention having different specificities, such as polypeptides selected using different target ligands, whether or not they are pooled prior to administration.
The route of administration of pharmaceutical compositions according to the invention may be any of those commonly known to those of ordinary skill in the art. For therapy, including without limitation immunotherapy, the selected antibodies, receptors or binding proteins thereof of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. The dosage and frequency of administration will depend on the age, sex and condition of the patient, concurrent administration of other drugs, counterindications and other parameters to be taken into account by the clinician. A therapeutically effective amount of a polypeptide that unfolds reversibly (*e.g.,* an antibody variable domain that unfolds reversibly) is administered. A therapeutically effective amount is an amount sufficient to achieve the desired therapeutic effect, under the conditions of administration.

The invention also provides a kit use in administering a polypeptide that unfold reversibly to a subject (e.g., patient), comprising a polypeptide that unfolds reversibly, a drug delivery device and, optionally, instructions for use. The polypeptide that unfolds reversibly can be provided as a formulation, such as a freeze dried formulation. In certain embodiments, the drug delivery device is selected from the group consisting of a syringe, an inhaler, an intranasal or ocular administration device (*e.g.,* a mister, eye or nose dropper) a needleless injection device.

The selected polypeptides of this invention can be lyophilised for storage and reconstituted in a suitable carrier prior to use. Any suitable lyophilization method (e.g., spray drying, cake drying) and/or reconstitution techniques can be employed. It will be appreciated by those skilled in the art that lyophilisation and reconstitution can lead to varying degrees of antibody activity loss (*e.g.,* with conventional immunoglobulins, IgM antibodies tend to have greater activity loss than IgG antibodies) and that use levels may have to be adjusted upward to compensate. In a particular embodiment, the invention provides a composition comprising a lyophilized (freeze dried) polypeptide that unfolds reversibly as described herein. Preferably, the lyophilized (freeze dried) polypeptide loses no more than about 20%, or no more than about 25%, or no more than about 30%, or no more than about 35%, or no more than about 40%, or no more than about 45%, or no more than about 50% of its activity when rehydrated. Activity is the amount of polypeptide required to produce the effect of the polypeptide before it was lyophilized. For example, the amount of rehydrated enzyme needed to produce half maximal conversion of a substrate into a product in a give time period, or the amount of a binding polypeptide needed to achieve half saturation of binding sites on a target protein. The activity of the polypeptide can be determined using any suitable method before lyophilization, and the activity can be determined using the same method after rehydration to determine amount of lost activity.

Compositions containing the present selected polypeptides or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose." Amounts needed to achieve this dosage will depend upon the severity of the disease and the general state of the patient's own immune system, but generally range from 0.005 to 5.0 mg of selected antibody, receptor (*e.g*., a T-cell receptor) or binding protein thereof *per* kilogram of body weight, with doses of 0.05 to 2.0 mg/kg/dose being more commonly used. For prophylactic applications, compositions containing the present selected polypeptides or cocktails thereof may also be administered in similar or slightly lower dosages.

A composition containing a selected polypeptide according to the present invention may be utilised in prophylactic and therapeutic settings to aid in the alteration, inactivation, killing or removal of a select target cell population in a mammal. In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the selected antibodies, cell-surface receptors or binding proteins thereof whereby the undesired cells are killed or otherwise removed from the blood for return to the mammal in accordance with standard techniques.

### EXEMPLIFICATION

### Section 1: EP- V_{H} library and EP- V_{L} library

Error-prone PCR is a random mutagenesis strategy that introduces mutations into a DNA segment. Hence, it is a useful tool for preparing nucleic acids encoding diversified proteins that contain random amino acid substitutions. Error-prone PCR can be carried out in a number of ways, generally by altering buffer conditions (*e.g.,* varying dNTP concentrations) to reduce the fidelity of nucleotide incorporation. In this study, an error-prone PCR library was constructed using the GENEMORPH PCR Mutagenesis Kit (Stratagene). The kit incorporates the MUTAZYME DNA polymerase that has a high intrinsic error rate of nucleotide incorporation compared to Taq polymerase. Mutation frequency using this system can be altered by manipulating the starting concentration of template (V_{H} and Vκ coding sequence) in the reaction. The starting concentration was altered accordingly to give a varying rate of mutational frequency. Initially, two libraries were constructed for each V_{H} and Vκ template and cloned into a phagemid vector pR2. The V_{H} template was V3-23/DP47 and J_{H}4b, and the V_{L} template was 012/02/DPK9 and Jκ1. The pR2 vector is derived from pHEN1. (Hoogenboom, HR et al., Nucleic Acids Res. 19:4133-4137 (1991).) pR2 contains a lac promoter, a leader sequence upstream of the cloning site which is followed by His6 and VSV tags, an amber stop condon and the gene encoding the pIII phage coat protein. These consisted of one having a low mutational frequency (∼1bp change/template) and the other with a medium mutational frequency (∼2bp changes/template). These libraries were combined to give a diversity of 1-2 x 10⁵.

### Subcloning the Error-Prone PCR Library into Phage Fd-Myc

The strategy involved the PCR amplification of the error-prone PCR libraries from the pR2 phagemid vector followed by subcloning the amplified product into the phage Fd-Myc. The error-prone PCR libraries, in the pR2 phagemid in the *E*. *coli* host HB2151, were plated out to give confluent growth on large plates (22x22 cm). The estimated total number of colonies was 10⁷- 10⁸, ensuring that the diversity of the error-prone libraries was well covered. The colonies were scraped from the plates and the DNA of the phagemid library subsequently isolated. The isolated phagemid DNA library was used as the template for PCR amplification of the error-prone library.

The library was PCR amplified using synthetic oligonucleotide primers that contained the restriction sites ApaL 1 and Not 1. Thus facilitating subcloning of the amplified products into the corresponding sites in phage Fd-Myc.
V_{H} Fd-Myc PCR Primers:
5' GAG CGC CGT GCA CAG GTG CAG CTG TTG 3' (SEQ IS NO:61)
5' GAG TCG ACT TGC GGC CGC GCT CGA GAC GGT GAC 3' (SEQ ID NO:62)
Vκ Fd-Myc PCR Primers:
5' GAG CGC CGT GCA CAG ATC CAG ATG ACC CAG TCT CC 3' (SEQ ID NO:63)
5' GAG TCG ACT TGC GGC CGC CCG TTT GAT TTC CAC CTT GG 3' (SEQ ID NO:64)

Restriction sites ApaL I (GTGCAC) and Not 1 (GCGGCCGC) are underlined. The primers were biotinylated at the 5' terminus. Incorporating the ApaL1 site into nucleic acids encoding V_{H} or Vκ causes the first amino acid of both V_{H} and Vκ to become a glutamine.

The PCR products of the amplified library were purified from an agarose gel and then restriction enzyme digested with ApaL 1 and Not 1. The digested product was purified by phenol/chloroform extraction, treated with streptavidin DYNABEADS (superparamagnetic monodisperse polymer beads; Dynal Biotech) to remove cut 5' ends and undigested product, and then subjected to QIAQUICK PCR purification kit (Qiagen). The product was ligated into the ApaL 1/Not 1 sites in phage Fd-Myc and transformed into *E*. *coli* TG1 giving a library size of 10⁶-10⁷.

### Section 2: Library 3.25G

### Construction of Fd-myc Vector

The Fd-myc vector was assembled from Fd-tet-Dog1 (McCafferty et al. (Nature) 1989) by cutting the vector at ApaL1 and Not1 and ligating a synthetic double-stranded DNA cassette composed of 5'-end phosphorylated oligos LJ1012 and LJ1013 (SEQ ID NO:65 and SEQ ID NO:66, respectively) which encode a myc tag and a trypsin cleavage site. The resulting vector Fd-myc is very similar to Fd-Tet-Dogl in that ApaL1 and Not1 sits are present for the cloning of insert in between the leader sequence and gene III. The additional feature is the presence of a myc-tag in between the Not1 site and gene III which allows for immunological detection of encoded gene III fusion protein, and also allows bound phage (*e.g.,* selected using anti-myc antibody) to be eluted by digestion with trypsin since there is a trypsin cleavage site in the myc-tag.

LJ1012: P-TGCACAGGTCCACTGCAGGAGGCGGCCGCAGAACAAAA
ACTCATCTCAGAAGAGGATCTGAATTC (SEQ ID NO:65)

LJ1013: P- GGCCGAATTCAGATCCTCTTCTGAGATGAGTTTTTGTTCTGCG
GCCGCGAGGACGTCACCTGCTG (SEQ ID NO:66)

### Preparation of the 3.25G Insert and Ligation

Eleven PCRs using the ligated DNAs of libraries pR3-7781, pR3-7782, pR3-7783, pR3-7784, pR3-7785, pR3-7786, pR3-7787, pR3-7788, pR3-7789, pR3-77890 or pR3-7791 as template were performed. These libraries are based on V_{H} -DP47 and contain diversified CDR1, CDR2, and CDR3 (CDR3 varying from 10 to 20 amino acids in length). The PCRs were performed on each sub-library using primers LJ1011 and LJ1027, to append an ApaL1 site at the 5'-end and a Not1 site at the 3'-end. The resulting amplified fragments were purified, digested consecutively with ApaL1 and with Not1, re-purified and then ligated into the corresponding sites of Fd-myc.
LJ1011: GAGTCGACTTGCGGCCGCGCTCGAGACGGTGACCAG (SEQ ID NO:67)
LJ1027: GAGCGCCGTGCACAGGTGCAGCTGTTGGAGTCTGGG (SEQ ID NO:68)

### Electroporation of Library 3.25G and Storage

After purification, the 11 ligations were pooled and electro-transformed into *E. coli* TG1 cells. After electroporation, the cells were resuspended in 2XTY and incubated for 1 hour at 37°C for phenotypic expression. The resulting library was then plated on TYE plates supplemented with 15 µg/ml tetracycline for overnight growth, and an aliquot was taken for titration on TYE plates supplemented with 15 µg/ml tetracycline. The size of the library was 1.6 x 10⁹ clones. Aliquots of the library were prepared by resuspending the bacteria at an OD600 of 40, diluting with an equivalent volume of glycerol (final OD600 = 20), and aliquoting as 1 ml samples that were frozen and stored at -80°C until use.

### Phage Production and Purification

A 1 ml sample of library 3.25G was thawed and used to inoculate 500 ml of 2XTY supplemented with 15 µg/ml of tetracycline, in a 2.5 L shaker flask. The culture was incubated for 20 hours at 30°C for phage production. The cells were pelleted by centrifugation at 5,500 g for 15 min to remove bacteria. To precipitate phage, 90 ml PEG/NaCl (20 % Polyethylene glycol 8000 [Sigma; formally sold as PEG 6000], 2.5 M NaCl) were added to 450 ml of culture supernatant, and after mixing, the solution was incubated for 1 hour on ice. Phage were collected from the resulting mixture by centrifugation at 5,500 g for 30 min at 4°C. The supernatant was discarded, the tubes were re-centrifuged briefly, and the remaining PEG/NaCl was carefully removed. The pellet was resuspended in 10 ml of PBS, centrifuged at 3,300 g for 15 min to remove remaining bacteria, and then filtrated through a 0.45 µm disposable filter. Phage titer was estimated by spectroscopy: a 100-dilution in PBS was prepared and the absorbance at 260 nm is measured. The phage titer (in TU per ml) was calculated using the formula: OD₂₆₀ x 10¹³ x 2.214. The phage were stored at -20°C after adding 10-15% glycerol (final concentration).

### Section 3: Phage Selection

### Coating/Blocking of Immunotube

Immunotubes (Nunc) were coated overnight (about 18 hours) at room temperature with either 4 ml of PBS containing 10 µg/ml of protein A, or 4 ml of PBS containing 10 µg/ml of protein L. In the morning, the solutions were discarded and the tubes were blocked with PBS supplemented with 2% v/v TWEEN 20 (Polyoxyethylensorbitan monolaurate; for protein A-coated immunotubes) or 2% w/v BSA (for protein L-coated immunotubes). The tubes were incubated for 1 hour at 37°C, then washed three times with PBS, before use for phage selection.

### Heat Unfolding and Refolding of Fd-phage Displayed Polypeptides

Approximately 5x10¹⁰ TU of domain antibody phage library was diluted into 200 µl of PBS, and aliquoted in two thin-walled PCR tubes. The tubes were then placed in a PCR apparatus for heating at 80°C for 10 minutes (temperature of cover lid: 85°C). After heating, the solutions were rapidly cooled down to 4°C in the PCR apparatus to produce refolded phage solutions.

### Selection of Refolded Fd-phage Displayed Polypeptides

The refolded phage solutions were pooled and added to 4 ml of PBS supplemented with either 2% v/v TWEEN 20 (Polyoxyethylensorbitan monolaurate; for selection on protein A) or 2% w/v BSA (for selection on protein L). The resulting phage solutions were added to Immunotubes coated with protein A or Immunotubes coated with protein L, after sealing the tubes were rotated end-over-end 30 min at room temperature, and then held on the bench at room temperature for 1.5 hours. Unbound phage were removed by washing the tubes ten times with PBS supplemented with 0.1% TWEEN 20 (Polyoxyethylensorbitan monolaurate), and ten times with PBS. Bound phage were eluted by adding 1 ml of PBS supplemented with trypsin (1 mg/ml) and incubating for 10-15 min while gently rotating. The solution containing the eluted phage was then transferred to a fresh microcentrifuge tube and stored on ice.

### E. coli Infection of Selected fd-Phage

From an overnight culture of *E. coli* TG1 cells in 2xTY at 37°C, a 100-fold dilution was prepared in 25 ml of fresh 2XTY medium, and the culture was incubated at 37°C with shaking (250 rpm) until the optical density at 600 nm (OD₆₀₀) was 0.5-0.7 (mid-log phase). Ten milliliters of this culture was then incubated with 500 µl of the eluted phage sample (the remaining 500 µl were kept at 4C) at 37°C for 30 min without shaking to allow for phage infection. After phage infection, a 100 µl aliquot was taken for titration of the phage: 10 µl of a 1:10² dilution and 10 µl of a 1:10⁴ dilution in 2xTY were spotted on TYE plates containing 15 µg/ml tetracycline and grown overnight at 37°C. The titre was determined by multiplying the number of colonies by the dilution factor (i.e., 100 or 10,000) then multiplying by 1000 (10 µl spotted from 10 ml infected culture), to gives the titre for 500 µl of eluted phage. The total number of eluted phage was determined by multiplying by 2 (1 ml total eluate). The remaining infected *E. coli* TG1 culture (9.9 ml) was transferred to a disposable 14 ml tube, and centrifuged at 3,300 g in for 10 min. The cell pellet was resuspended in 2 ml of fresh 2xTY, plated on a large 22 cm² dish containing TYE, 15 µg/ml tetracycline, and incubated overnight at 37°C

### Amplification of Selected fd-phage Vectors

The next day, 10 ml of 2xTY supplemented with 15% glycerol were added to the 22 cm² dish dish, the cells were loosened using a glass spreader, and the resulting mixure was transferred to a fresh 50 ml disposable tube. Fifty microliters of the cell suspension were then used to inoculate 100 ml of 2xTY containing 15 µg/ml tetracycline, whilst 1 ml of bacterial suspension was diluted with 1 ml sterile glycerol and stored at -70°C. The 100 ml culture was grown with shaking at 37°C overnight.

### Purification of Selected fd-phage Vectors

The next day, the 100 ml overnight culture was centrifuged at 3,300 g for 15 min to remove bacteria. The supernatant was filtered through a 0.45 um disposable filter. To precipitate phage, 20 ml PEG/NaCl (20 % Polyethylene glycol 8000; Sigma [formally sold as PEG 6000], 2.5 M NaCl) was added to 80 ml of supernatant, and after mixing, the solution was incubated for 1 hour on ice. Phage were collected from the resulting mixture by centrifugation at 3,300 g for 30 min at 4°C. The supernatant was discarded, the tubes were re-centrifuged briefly, and the remaining PEG/NaCl was carefully removed. The pellet was resuspended in 1 ml of PBS, then transferred to a fresh micro-centrifuge tube. Remaining bacterial debris were removed by centrifuging the micro-centrifuge tube at 11,600 g for 10 min. The supernatant was transferred to a fresh micro-centrifuge tube, and stored at 4°C, until the next selection round. Phage titer was estimated by spectroscopy: a 100-dilution in PBS was prepared and the absorbance at 260 nm was measured. The phage titer (in TU per ml) was calculated using the formula: OD₂₆₀ x 10¹³ x 2.214.

### Results

Using this protocol and 3.25G phage library (Section 2), three rounds of selection for binding to protein A-coated immunotubes were conducted. After round 1, the phage titer was below 10⁷ TU, whilst after the third selection round, the phage titer had risen to be greater than 10⁹ TU. A sample of the bacterial suspension obtained from phage amplification after round 3, was serially diluted (10-fold series) and plated on TYE plates supplemented with 15 µg/ml tetracycline. After overnight incubation at 37°C, individual colonies were picked for screening (see section 4).

Using this protocol and EP- V_{H} phage library (Section 1), three selection rounds (for protein A binding) were performed to produce a high titer preparation. A sample of the bacterial suspension obtained from phage amplification after round 3, was serially diluted (10-fold series) and plated on TYE plates supplemented with 15 µg/ml tetracycline. After overnight incubation at 37°C, individual colonies were picked for screening (see section 4).

Using this protocol and EP- V_{K} phage library (Section 2), three selection rounds (for protein L binding) were performed to produce a high titer preparation. A sample of the bacterial suspension obtained from phage amplification after round 3, was serially diluted (10-fold series) and plated on TYE plates supplemented with 15 µg/ml tetracycline. After overnight incubation at 37°C, individual colonies were picked for screening (see section 4).

### Section 4: Phage Screening 1

### Growing Phage Clones and Preparation

A 96-well culture plate (flat bottom, with evaporation lid, Corning) was used for individual phage growth: each well was filled with 175 µl of 2xTY containing 15 µg/ml tetracycline, and inoculated with a single colony from the selected bacterial clones obtained using the Phage Selection protocol. The plate was incubated overnight (about 18 hours) with shaking at 37°C. The next day, the cells were pelleted by plate centrifugation for 20 min at 2,000 rpm at room temperature. One hundred microliters (100 µl) of each culture supernatant (containing the phage) were transferred to a fresh 96-well culture plate.

To facilitate detection of bound phage by ELISA, phage were chemically derivatized with a biotinylated reagent. This procedure allowed bound phage to be detected using a conjugate of streptavidin and horseradish peroxydase (Str-HRP, Sigma). This strategy was chosen to decrease the possibility of cross reactivity between antibodies used to detect bound phage and immobilized protein A or protein L in the wells. Thus, each 100 µl sample of culture supernatant was reacted with 100 µl of PBS containing a 500 µM concentration of EZ-link Sulfo-NHS biotin (Perbio), for 1 hour at room temperature (with medium agitation on a rotating plate).

### Phage Thermodenaturation (Heat Induced Unfolding of Phage Displayed Polypeptide)

After biotinylation of the phage, 80 µl of each 200 µl sample was transferred to a THERMOWELL 96-well plate (Costar). This step was repeated with another 96-well THERMOWELL 96-well plate (Costar). The first plate was covered with a lid and placed in a PCR apparatus for incubation at 80°C during 10 min (temperature of cover lid: 85°C). After heating, the plate was rapidly cooled down to 4°C in the PCR apparatus. The second plate was kept on ice. Both plates were then treated in parallel: to each well (containing 80 µl of heated or non-heated phage supernatant), 20 µl of PBS supplemented with 10% v/v TWEEN 20 (Polyoxyethylensorbitan monolaurate;for protein A-based ELISA) or 10% w/v BSA (for protein L-based ELISA) was added and mixed. The samples were then assayed by ELISA.

### Coating and Blocking of ELISA Plates

MAXISORB 96-well plates (Nunc) were coated overnight (about 18 hours) at room temperature with either 100 µl of PBS containing 10 µg/ml of protein A, or 100 µl of PBS containing 10 µg/ml of protein L, per individual well. The next day, the plates were emptied, and the wells were blocked with 200 µl of PBS supplemented with 2% v/v TWEEN 20 (Polyoxyethylensorbitan monolaurate; for protein A-coated wells) or 2% w/v BSA (for protein L-coated wells). The plates are incubated for 1h at 37°C, then washed three times with PBS, before use for screening.

### Phage ELISA

Heat treated or control phage samples were transferred to the empty wells of coated/blocked ELISA plates, and incubated for 2 hours at room temperature. Unbound phage was then removed by washing the wells 6-times with PBS. For detection, Str-HRP (from a 1 mg/ml stock in PBS) is diluted 1/2000 in PBS supplemented with 2% v/v TWEEN 20 (Polyoxyethylensorbitan monolaurate; for protein A-based ELISA) or 2% w/v BSA (for protein L-based ELISA), and 100 µl of this solution was added to each ELISA well. After incubation for 1 hour at room temperature, the unbound Str-HRP was removed by washing the wells 6-times with PBS. For colorimetric reaction, a 10 mg/ml solution of TMB (tetramethylbenzidine) was diluted 100-fold in a buffer of 0.1 M sodium acetate, pH 6.0. Next, hydrogen peroxyde was added (0.4 µl per ml of buffer/TMB), and 100 µl of the resulting solution was added to each ELISA well. After color (blue) had developed, the reaction was stopped by adding 50 µl of 1 M sulfuric acid per well (color turns to yellow). The optical density or each well at 450 nm was measured.

### Results

The described method was used for screening phage in five separate studies:
Clones from the 3.25G phage library (Section 2) selected for refolding
Clones from the EP-V_{H} phage library (Section 1) selected for refolding
Clones from the EP- Vκ phage library (Section 1) selected for refolding
Clones from the ten mini-phage libraries in V_{H}-DP47d (Section 10)
Clones from the five mini-phage libraries in Vκ-DPK9d (Section 10)

For each screening, positive and negative controls were used in order to establish the % refolding range: thus for ELISA on protein A, negative control was phage displaying V_{H} -DP47, and positive control was phage displaying HEL*4*. As shown herein V_{H} -DP47 (also referred to as V_{H} -DP47 dummy) does not unfold reversibly when heated and cooled; while HEL4 (a single V_{H} that binds hen egg white lysozyme that was selected from a library based on a V_{H} 3 scaffold (DP47 germline + JH4 segment) with randomised CDR 1, 2 and 3) does unfold reversibly under these conditions. Thus for ELISA on protein L, negative control was phage displaying Vκ-DPK9 which does not unfold reversibly when heated and cooled, and positive control was phage displaying Vκ-DPK9-A50P (which was obtained after screening the EP Vκ library for Vκ domains that unfold reversibly when heated).

For each series of phage clones, the un-heated and the heat-treated phage were tested for binding in an ELISA. This approach provides a semi-quantitative measure of refoldability of the antibody polypeptide displayed on the phage. For example, if an un-heated phage clone produces an OD450 of say 1.5 by ELISA (1.45 when the value of the background using a blank supernatant is substracted), and the same phage clone produces an OD450 of say 0.6 by ELISA (and 0.55 when the value of the background using a blank supernatant is substracted) after heat treatment, then the "percentage refolding" (which is assimilated to the percentage of protein A-binding activity that remains after heating/cooling) of this particular clone is (0.55/1.45)*100 = 29.0%.

Phage clones which yielded significant % refolding were then submitted to DNA sequencing of the V_{H} gene or Vκ gene insert (see section 6). In addition, selected clones were submitted to a second ELISA (see section 7) to further quantify refolding.

### Section 5: Phage Screening 2

### Growing Phage Clones and Preparation

Fifty milliliter disposable tubes (Coming) were used for individual phage growth: each tube was filled with 11 ml of 2xTY containing 15 µg/ml tetracycline, and inoculated with a single colony from the selected bacterial clones obtained using the Phage Selection protocol (Section 3) or after Phage Screening 1 (Section 4). The tubes were incubated overnight (∼18 hours) with shaking at 37°C. In the morning, the cells were pelleted by centrifugation for 20 min at 3,300 g at 4°C to remove bacteria. The supernatant was filtered through a 0.45 um disposable filter. To precipitate phage, 2 ml PEG/NaCl (20 % Polyethylene glycol 8000 [Sigma, formally sold as PEG 6000], 2.5 M NaCl) was added to 80 ml supernatant, and after mixing, the solution was incubated for 1 hour on ice. Phage were collected by centrifuging the resulting mixture at 3,300 g for 30 min at 4°C. The supernatant was discarded, the tubes were recentrifuged briefly, and the remaining PEG/NaCl was carefully removed. The pellet was resuspended in 0.2 ml of PBS, then transferred to a fresh micro-centrifuge tube. Remaining bacterial debris was removed by centrifuging the micro-centrifuge tube at 11,600 g for 10 min. The supernatant was transferred to a fresh micro-centrifuge tube, and stored on ice. Phage titer was estimated by spectroscopy: a 100-dilution in PBS was prepared and the absorbance at 260 nm was measured. The phage titer (in TU per ml) was calculated with the formula: OD₂₆₀ x 10¹³x2.214.

To facilitate detection of bound phage by ELISA, phage were chemically derivatized with a biotinylated reagent. This procedure allowed bound phage to be detected using a conjugate of streptavidin and horseradish peroxydase (Str-HRP, Sigma). This strategy was chosen to decrease the possibility of cross reactivity between antibodies used to detect bound phage and immobilized protein A or protein L in the wells. Thus, 4 x 10¹⁰ TU phage in 100 µl of PBS was reacted with 100 µl of PBS containing a 50 µM concentration of EZ-link Sulfo-NHS biotin (Perbio), for 1 hour at room temperature (with medium agitation on a rotating plate) or overnight at 4°C.

### Phage Thermodenaturation (Heat Induced Unfolding of Phage Displayed Polypeptide)

After biotinylation of the phage, for each clone, a 100 µl sample of phage was transferred to a thin-wall PCR tube (and the remaining biotinylated phage was kept on ice). The tube was placed in a PCR apparatus for incubation at 80°C during 10 min (temperature of cover lid: 85°C). After heating, the tube was rapidly cooled down to 4°C in the PCR apparatus. Both tubes (heated sample and sample kept on ice) were then treated in parallel: first eight 4-fold dilutions were prepared for both heat-treated and nonheat-treated samples of the same clone, in the appropriate buffer (PBS supplemented with 2% v/v TWEEN 20 (Polyoxyethylensorbitan monolaurate) for V_{H} -DP47 displaying phage; or PBS supplemented with 2% w/v BSA for Vκ-DPK9 displaying phage. Thus, in the tubes with the highest phage concentration, the titer was 10¹¹ TU per ml. The samples were then ready for assay by ELISA.

### Phage ELISA

ELISA plates were coated and blocked as described in Section 4. The samples were transferred to the empty wells of coated/blocked ELISA plates, and incubated for 2 hours at room temperature. Unbound phage was then removed by washing the wells 6-times with PBS. For detection, Str-HRP (from a 1 mg/ml stock in PBS) were diluted 1/2000 in PBS supplemented with 2% v/v TWEEN 20 (Polyoxyethylensorbitan monolaurate; for protein A-based ELISA) or 2% w/v BSA (for protein L-based ELISA), and 100 µl of the resulting solution was added to each ELISA well. After incubation for 1 hour at room temperature, the unbound conjugate was removed by washing the wells 6-times with PBS. For colorimetric reaction, a 10 mg/ml solution of TMB (tetramethylbenzidine)-was diluted 100-fold in a buffer of 0.1 M sodium acetate, pH 6.0. Next, hydrogen peroxyde was added (0.4 ul per ml of buffer/TMB, and 100 ul of this solution was added to each ELISA well. After color (blue) developed, the reaction was stopped by adding 50 µl of 1 M sulfuric acid per well (color turns to yellow). The optical density was recorded at 450 nm.

### Results

This method was used for screening clones in five experiments:
Clones with individual or multiple amino acid mutations in V_{H} -DP47;
Clones with individual or multiple amino acid mutations in Vκ-DPK9;
Clones such as DP47, BSA1, HEL4, pA-C (13, 36, 47, 59, 76, 85), Vκ-DPK9; and
A subset of clones from the ten mini-phage libraries in V_{H} -DP47, that showed promising % refolding.

BSA1 is a single V_{H} that binds bovine serum albumin and does not unfold reversibly when heated and cooled, that was selected from a library based on a V_{H} 3 scaffold (DP47 germline + JH4 segment) with randomised CDRs 1, 2 and 3.

For each screening, positive and negative controls were used in order to establish the % refolding range: thus for ELISA on protein A, negative control was phage displaying V_{H} -DP47, and positive control was phage displaying HEL4. Thus for ELISA on protein L, negative control was phage displaying Vκ-DPK9, and positive control was phage displaying Vκ-DPK9-A50P.

The scoring was done as follows: for each clone, the phage were tested as un-heated sample and as heat-treated sample in an eight-point dilution series. This approach permitted quantitative deductions about the refoldability of the domain antibody displayed on phage to be made. Thus, the OD450 were plotted onto a semilog graph (on the X-axis, concentration of phage (in TU) per well according to a semi-log scale; on the Y-axis, OD450 observed at each phage concentration), and linked together by simple linear interpolation between each data points.

The percent refolding was calculated as illustrated in the following example. For each clone, the phage concentration that produced a particular OD450 (*e.g.,* 0.2) was calculated (one value for the phage concentration of the non-treated sample, and one value for the heat-treated sample). Assume the concentration that produced that OD450 was 2x10⁸ for the non-treated phage, and 5 x 10⁹ for the heat-treated phage. The percent refolding would then be calculated using the formula : (2 x 10⁸ / 5 x 10⁹) *100 = 4% refolding. Using this system, we repeatedly observed a percent refolding of approx 0.5% for V_{H} -DP47 on phage, and about 18% refolding for phage displaying HEL4.

### Section 6: DNA Sequencing

DNAs encoding variable domains from selected clones that display reversible heat unfolding were sequenced as follows:
PCR reaction mix:
   5 µl 10x Buffer
   1 µl Primer LJ212 (20pmol/ul)
   1 µl Primer LJ006 (24pmol/ul)
   1 µl 20mM dNTPs
   0.5 µl Taq DNA polymerase
   41.5 µl H20
50 µl of the PCR reaction mix was aliquoted into each well of a 96 well PCR microplate. A colony (Fd-Myc/TG1) was gently touched with a sterile toothpick and transferred into the PCR mix. The toothpick was twisted about 5 times in the mixture. The mix was overlayed with mineral oil. The PCR parameters were: 94°C for 10min, followed by 30 cycles of: 94°C 30sec, 50°C 30sec, 72°C 45sec; and a final incubation at 72°C 5min. The amplified samples were purified using a QIAQUICK PCR product purification kit (Qiagen). Sequencing was carried out using either of the original PCR primers (LJ212 and/or LJ006).

Mutations that were detected in the sequencing of clones that encode immunoglobulin variable domains that unfold reversibly are presented in Tables 3 and 4.
Primer Sequences:
LJ006 5' ATGGTTGTTGTCATTGTCGGCGCA 3' (SEQ ID NO:69)
LJ212 5' ATGAGGTTTTGCTAAACAACTTTC 3' (SEQ ID NO:70)

### Section 7: List of Selected EP V_{H}

**Table 3 Mutations Found in Clones From EP- V_{H} Library Selected for Reversible Heat Unfolding**

| Mutation | Frequency | Elisa Signal* |
|---|---|---|
| Y32D | 5 | m |
| S30N/A33D | 1 | H |
| S31N/A84D | 1 | m |
| V12E/A33P/G55D | 1 | m |
| A23V/F27S/G54D | 2 | m |
| F27S/A33D/T87S | 3 | m |
| S30G/G54D/A98D | 1 | m |
| M34L/G52aD/K94E | 1 | m |
| L11S/S30G/Y32D/T77M | 1 | m |
| S31N/S62P/E85D/ Y96N/W103G | 8 | 6m/2H |

| | | |
|---|---|---|
| *m indicates medium ELISA signal and H indicates high ELISA signal. | | |

**Table 4 Frequency of Mutation in Clones From EP- V_{H} Library Selected for Reversible Heat Unfolding**

| Position of mutation | Overall Frequency of selection |
|---|---|
| 27 | 5 |
| 30 | 3 |
| 31 | 9 |
| 32 | 6 |
| 33 | 5 |
| 34 | 1 |

### Section 8: List of Selected EP V_{K}

**Table 5 Mutations Found in Clones From EP- Vκ Library Selected for Reversible Heat Unfolding**

| Mutation | Frequency |
|---|---|
| K45E | 1 |
| I48N | 2 |
| Y49N | 3 |
| Y49D | 4 |
| A50P | 1 |
| 175N | 4 |
| S31G/Y49N | 1 |
| Y32S/I75N | 1 |
| P40S/Y49D | 1 |
| K39/Y49N | 1 |
| K45E/I75N | 1 |
| L46P/A50D/W35G | 1 |
| S26N/K42T/A50D | 1 |
| Y32F/K45E/G57E | 1 |
| Y49D/P80A/Q89R | 1 |
| Y49N/G68E/Q79R | 1 |
| T20S/C23W/L46F/Y49N | 1 |
| I29V/K42N/K45E/F83L/ Y92H | 2 |

All clones selected gave a high ELISA signal.

**Table 6 Frequency of Mutation in Clones From EP- Vκ Library Selected for Reversible Heat Unfolding**

| Position of mutation | Overall Frequency of Selection |
|---|---|
| 45 | 5 |
| 48 | 2 |
| 49 | 13 |
| 50 | 2 |
| 75 | 6 |

### Section 9: List of Selected 3.25G V_{H}

After selecting library 3.25G with the heat/cool phage selection (see section 3), a large scale screening (see section 4) was performed to identify V_{H} clones that refolded when displayed on phage after thermodenaturation.

Clones were analyzed by assessing binding of heat treated and control phage to protein A in an ELISA as described herein. (Figs. 3A-3F) Clones that had above 60% refolding were further analyzed by sequencing. Figs. 4A-4C present the sequence of may of the clones that had above 60% refolding. The first set of sequences present in Figs. 4A-4C are from clone giving a high OD450 in the ELISA and a high % refolding. These sequences do not contain cysteines. This group of sequences forms the dataset for the analysis of amino acid preferences at all positions of CDR1, CDR2 and CDR3.

The next set of sequences in Figs. 4A-4C are from clones with excellent ELISA signals and good refolding. These clones contain mutations outside the CDRs.

The final group of sequences in Figs. 4A-4C are from with relatively low ELISA signals and/or refolding.

From the sequences presented in Figs. 4A-4C, six clones were selected and further analyzed in detail on phage (as displayed polypeptide) and as soluble proteins. The six clones selected are referred to as pA-C 13, pA-C36, pA-C47, pA-C59, pA-C76 and pA-C85. (Clones are identified in Figs. 4A-4C using the suffix only, *i.e.,* "pA-C13" is "C13.")

In addition, individual mutants were designed into DP47 and analyzed for refolding on phage (as displayed polypeptide) as for some of these mutations, as soluble protein. The particular mutations were: F27D, F29V, F27D/F29V, Y32E, S35G, F27D/F29V/Y32D/S35G, S53P, G54D, S53P/G54D, W47R, F100nV, Y102S, F100nV/Y102S, W103R

These clones were characterized for refoldability on phage (according to the protocol of Section 5, but with three data points being taken) and in solution. For the refolding in solution, thermodenaturation was followed by CD (Section 12). The results of these studies is presented in Table 7.

**Table 7**

| Clone | Refoldability on phage (%) | Refoldability in solution* |
|---|---|---|
| HEL4 | 82 | Y |
| BSA1 | 0 | N |
| pA-C36 | 80 | Y |
| DP47d | 0 | N |
| F27D | 65 | Y |
| F29V | 35 | N |
| F27D/F29V | 62 | nd |
| S30N/A33D | 81 | N |
| Y32D | 75 | Y |
| S35G | 15 | N |
| F27D/F29V/Y32D/S35G | 80 | Y |
| W47R | 5 | N |
| S53P | 2 | nd |
| G54D | 22 | N |
| S53P/G54D | 19 | nd |
| F100nV | 2 | nd |
| Y102S | 4 | N |
| F100nV/Y102S | 2 | nd |
| W103R | 3 | N |

| | | |
|---|---|---|
| *Y indicates refoldablility in solution, N indicates that the variable domain was not refoldable in solution, nd = not determined | | |

### Section 10: Mini-library Protocol

The positions of amino acid substitutions found in clones from the temperature selected libraries (the Error-Prone and G3.25 libraries) were analysed so that the entire sequence space at that particular site could be investigated.

Error-prone PCR samples a limited sequence space. For example, the A50P substitution selected from the error-prone Vκ library that has a frequency of 1bp change/ template would sample only 6 additional amino acids in total. Alanine at position 50 is encoded by the codon GCT. Changing this codon by 1 base gives the following codon permutations and amino acids:

| | |
|---|---|
| Alanine | = GCT, GCA, GCG, GCC |
| Aspartate | = GAT |
| Glycine | = GGT |
| Proline | = CCT |
| Serine | = TCT |
| Threonine | = ACT |
| Valine | = GTT |

Oligonucleotides randomized at a particular site by the codon NNK (N = A, G, C or T; K = G or T; M = A or C) were used in a PCR strategy to sample the entire sequence space (see Table 8). For Vκ a two-step PCR strategy was used. (See, Landt, O. et al. Gene 96:125-128 (1990)). The first PCR product (also referred to as "mega-primer") was generated using the oligonucleotide carrying the NNK changes (see Tables 8 and 9) together with one of the forward or reverse Fd-Myc primers carrying the ApaL 1 or Not 1 restriction site for subcloning into Fd-Myc as described previously:
Vκ Fd-Myc PCR Primers:
   5' GAG CGC CGTGCA CAG ATC CAG ATG ACC CAG TCT CC 3' (SEQ ID NO:71)
   5' GAG TCG ACT TGC GGC CGC CCG TTT GAT TTC CAC CTT GG 3'(SEQ ID NO:72)
Restriction sites ApaL 1 (GTGCAC) and Not 1 (GCGGCCGC) are underlined. The primers were biotinylated at the 5' terminus. Incorporating the ApaL1 site causes the first amino acid of both V_{H} and Vκ to become a glutamine.

DPK9 was used as a DNA template. The first PCR product or mega-primer was subsequently used in a second PCR reaction together with the second Fd-Myc primer not used in the first reaction. This PCR gave a product containing Apal1/Not1 restriction sites suitable for subcloning into the respective sites in Fd-Myc.

For V_{H}, SOE PCR was employed. (See, Horton, R.M. et al. Gene 77:61-68 (1989)). The primers used to generate the substitution by SOE PCR are shown in the Table 9. The additional primers necessary for amplification of the mutagenised product and subsequent subcloning into the ApaL 1 and Not 1 sites in Fd-Myc are:
5' GAG CGC CGT GCA CAG GTG CAG CTG TTG 3' (SEQ ID NO:73)
5' GAG TCG ACT TGC GGC CGC GCT CGA GAC GGT GAC 3' (SEQ ID NO:74)

**TABLE 8 Vκ Oligonucleotides containing the NNK codon**

| Position of randomisation in Vκ^{a} | 5'->3' Oligonucleotide sequence containing the codon NNK |
|---|---|
| 45 | GCAGCATAGATCAGGAGKNNAGGGGCTTTCCCTGG (SEQ ID NO:75) |
| 48 | GCAAACTGGATGCAGCATAKNNCAGGAGCTTAGG (SEQ ID NO:76) |
| 49 | GCAAACTGGATGCAGCKNNGATCAGGAGCTTAGG (SEQ ID NO:77) |
| 50 | GCAAACTGGATGCKNNATAGATCAGGAGCTTAGG (SEQ ID NO:78) |
| 75 | TTCACTCTCACCNNKAGCAGTCTGCAACCTG (SEQ ID NO:79) |

| | |
|---|---|
| ^{a}Primers for positions 45, 48, 49 and 50 are to the reverse/antisense strand, hence the reversal of NNK to KNN, and were used in the first PCR reaction with the Vκ Fd-Myc primer designed to the coding strand containing the ApaL 1 site. Conversely, position 75 was designed to the coding strand and used with the antisense FD-Myc primer containing the Not 1 site. | |

**TABLE 9 V_{H} Oligonucleotides for SOE PCR**

| Position of randomisation in V_{H} | 5'->3' Oligonucleotide sequences for SOE PCR |
|---|---|
| 27 | (F) CCGGAGGCTGCACAGGAGAGACGCAGGG (SEQ ID NO:80) (R) CCCTGCGTCTCTCCTGTGCAGCCTCCGGANNKACCTTTAGCAGCT ATGCCATG (SEQ ID NO:81) |
| 29 | (F) GAATCCGGAGGCTGCACAGGAGAGACGC (SEQ ID NO:82) (R) CTCTCCTGTGCAGCCTCCGGATTCACCNNKAGCAGCTATGCCATG AGCTGGGC (SEQ ID NO:83) |
| 30 | (F) GGCGGACCCAGCTCATGGCATAGCTMNNAAAGGTGAATCCGGAG GCTGCACAG (SEQ ID NO:84) (R) ATGCCATGAGCTGGGTCCGCCAGGCTC (SEQ ID NO:85) |
| 31 | (F) GGAGCCTGGCGGACCCAGCTCATGGCATAMNNGCTAAAGGTGAA |
| | (R) ATGCCATGAGCTGGGTCCGCCAGGCTCCAG (SEQ ID NO:87) TCCGGAGGCTGCAC (SEQ ID NO:86) |
| 32 | (F) GCTAAAGGTGAATCCGGAGGCTGCACAG (SEQ ID NO:88) (R) GCAGCCTCCGGATTCACCTTTAGCAGCNNKGCCATGAGCTGGGTC CGCCAGGCTC (SEQ ID NO:89) |
| 33 | (F) AGCTGCTAAAGGTGAATCCGGAGGCTG (SEQ ID NO:90) (R) CCGGATTCACCTTTAGCAGCTATNNKATGAGCTGGGTCCGCCAGG CTCCAGG (SEQ ID NO:91) |
| 35 | (R) GGGTCCGCCAGGCTCCAGGGAAGGGTCTAG (SEQ ID NO:93) (F) AGACCCTTCCCTGGAGCCTGGCGGACCCAMNNCATGGCATAGCT GCTAAAGGTGAATC (SEQ ID NO:92) |
| 54 | (F) ACCACTAATAGCTGAGACCCACTCTA (SEQ ID NO:94) (R) AGAGTGGGTCTCAGCTATTAGTGGTAGTNNKGGTAGCACATACTA CGCAGACTCCGTG (SEQ ID NO:95) |
| 84 | (F) CGCAGGCTGTTCATTTGCAGATACAGCG (SEQ ID NO:96) (R) GTATCTGCAAATGAACAGCCTGCGANNKGAGGACACCGCGGTAT ATTACTGTGCG (SEQ ID NO:97) |
| 85 | (F) GCACGCAGGCTGITCATITGCAGATA (SEQ ID NO:98) (R) CTGCAAATGAACAGCCTGCGTGCANNKGACACCGCGGTATATTA CTGTGCG (SEQ ID NO:99) |

Once assembled and digested, the PCR fragments were ligated into the ApaL1 and Not1 sites of Fd-myc. The ligated DNA was used to transform *E. coli* TG1 cells by electroporation. After one hour of phenotypic expression at 37°C, the cells were plated on TYE plates supplemented with 15 ug/ml of tetracycline.

For the screening of the mini-libraries, cultures were made in 96-well cell culture plates as described in Section 4. For the appropriate controls, three wells (usually A1, D6 and H11) were inoculated with a positive control phage (eg. Fd-myc-HEL4) and three wells were inoculated with a negative control phage (eg. Fd-myc-DP47d). The remaining 90 wells were inoculated with 90 different clones from a mini-library. This plate preparation procedure was repeated with each mini-library.

The use of 90 clones from each mini-library is sufficient to cover the encoded diversity. Indeed, since a NNK codon was used to diversify each position, there are 32 different codons possible. The screening of 90 clones ensures that there is over 90% chance that every possible codon (and hence amino acid) will be present at least once in the screened mini-library. This ensures that the screening covers all possible amino acid substitutions at the explored (randomized) positions. The procedures for biotinylation of phage, and detection/selection by ELISA are described in Section 4.
Several phage were obtained from the V_{H} -mini libraries that showed good refolding. These phage were then subjected to the Phage Screening 2 procedure (Section 5) to obtain more quantitative data on the refolding.

### Section 11: Subcloning, Expression and Purification of dAbs

### Subcloning

Selected substitutions were subcloned into an expression plasmid for expression in *E. coli* BL21(DE3)(pLysS). DNA from selected Fd-Myc phage clones was amplified by PCR using primers containing Sal 1 and BamH 1 restriction sites. The protocol was essentially as that described for DNA sequencing, isolating V_{H} or Vκ DNA from Fd-Myc/TG1 colonies by PCR. The primers used are shown in Table 10. The forward primers introduced an additional two amino acids (Ser and Thr) to the N-terminus. This is a result of creating a Sal 1 restriction site. The reverse primers were designed to have two consecutive stop codons (TAA) at the end of the coding region.

**TABLE 10 Oligonucleotides for Subcloning**

| Primer | Sequence 5' to 3' |
|---|---|
| V_{H} forward (Sal 1) | ACGCGTCGACGCAGGTGCAGCTGTTGG (SEQ ID NO:100) |
| V_{H} reverse (BamH 1) | TTAGGATCCTTATTAGCTCGAGACGGTGACCAG (SEQ ID NO:101) |
| V_{K} forward (Sal 1) | ACGCGTCGACGCAGATCCAGATGACCCAG (SEQ ID NO:102) |
| V_{K} reverse (BamH 1) | TTAGGATCCTTATTACCGTTTGATTTCCACCTTGG (SEQ ID NO:103) |

### Expression and Purification

A colony, from freshly transformed *E.coli* strain BL21(DE3)pLysS containing the expression plasmid clone, was grown overnight in 2xTY (ampicillin/chloramphenicol) at 37°C, 250rpm. A 1/100 aliquot of the overnight culture was then used to inoculate a larger volume of the same media and allowed to grow under the same conditions until the OD₆₀₀=0.9. 1mM IPTG (isopropyl ß,D-thiogalactisidase) was then added to the culture, and the culture allowed to grow overnight at 30°C, 250rpm. The culture was then centrifuged at 3300g for 20min at 4°C. The supernatant was then filtered through a 0.45µm filter.

Soluble V_{H} or V_{K} dAb were then captured on a protein A or protein L matrix (protein A agarose or protein L agarose). Depending on the culture volume, the supernatant was either loaded directly onto a prepacked protein A or L matrix column, or the matrix was added directly to the supernatant (batch binding). Elution from batch binding can be accomplished directly from collected matrix, or the matrix can be packed into a suitable column. Elution from protein A or protein L matrix was carried out at low pH. The dAbs can be further purified by gel filtration using Superdex75 (Amersham Pharmacia Biotech).

### Section 12: CD Analysis

Purified dAbs were dialysed overnight in PBS at 4°C, and concentrated (if needed) by centrifugation using Millipore 5K Molecular Weight Cut Off centrifugation concentrator tubes (at 20 °C). One and a half ml of dAb at 1-5 µM in PBS was transferred to a CD cuvette (1 cm pathlength) and introduced in the Jasco J-720 spectropolarimeter. Spectra at room temperature (25°C) or at high temperature (85°C) were recorded in the far-UV from 200 nm to 250 nm (four accumulations followed by averaging) at a scan speed of 12 nm min⁻¹, with a 2-nm bandwith and a 1 second integration time.

Heat-induced unfolding curves were recorded at fixed wavelength (usually 235 nm, sometimes at 225 nm) using a 2 nm bandwith. The temperature in the cuvette was raised at a rate of 50 °C per hour, from 25 °C to 85 °C. Data were acquired with a reading frequency of 1/20 sec⁻¹, a 1 second integration time and a 2 nm bandwith. After unfolding, the sample was rapidly cooled down to 25 °C (15°C min⁻¹), a spectrum was recorded, and a new heat-induced unfolding curves was recorded.

The ability of a dAb molecule to unfold reversibly following to heat-induced denaturation was evaluated by comparing the first and the second heat-induce unfolding curve. Super imposable first and second heat-induced unfolding curves indicate that the dAb underwent thermal-unfolding reversibly in PBS at 1-5 µM (e.g., pA-C36, pA-C47). The same holds true for the far-UV spectra: super imposable first and second spectra recorded at 25°C indicated that the dAb unfolds reversibly (Fig. 8).

If a dAb aggregates upon thermal unfolding, the first unfolding curve is characterized by a steep transition upon melting and a "noisy" post-transition line (due to the accumulation of aggregates). Moreover, the second unfolding curve is radically different from the first one: a melting transition is barely detectable because no, or very few, unfolded molecules properly refold upon cooling the sample. As a result, the first and second far-UV spectra differ considerably, the latter being more akin to that observed using a denaturated molecule. A typical example of an aggregating dAb is DP47 dummy, or DP47-W47R (Fig. 9).

Some dAbs do exhibit partial refolding at 1-5 µM (*e.g*., DP47-S35G): i.e., upon cooling, a proportion of the ellipticity (and hence a portion of the original secondary structure) is recovered, and a melting transition is observed upon re-heating the sample (Fig. 10). To calculate the percentage of refolding, the amount of ellipticity recovered after the first thermal denaturation is divided by the amount of ellipticity of the sample before the first thermal denaturation, and multiplied by 100.

Using this assay, the following isolated human V_{H} dAbs were shown to undergo irreversible thermal unfolding: DP47 dummy, BSA1, DP47-F29V, DP47-W47R, DP47-G54D, DP47-W103R.

Fully reversible unfolding was demonstrated with the following isolated human V_{H} dAbs: HEL4, pA-C13, pA-C36, pA-C47, pA-C59, pA-C76, pA-C85, DP47-F27D, DP47-Y32D, DP47-F27D/F29V/Y32E/S35G. While DP47-S35G demonstrate reversible unfolding to a lesser degree.

### Section 13: Mini-library in V_{κ} results

Phage ELISA and scoring of the clones were done according to protocol of Section 4. DNA sequencing of selected clones that showed reversible heat unfolding was done according to protocol of Section 6.

**Table 11 Mutations Found in Clones From Vκ Mini Library Selected for Reversible Heat Unfolding**

| | |
|---|---|
| 148 | 3P, 2D, 2T, 1G, 1N |
| Y49 | 5S, 1C, 1E, 1G, 1K, 1N, 1R |
| A50 | 3P,2N,1D,1E |
| 175^{c} | 2N, 2M |

**Table 12 Substitutions at Position 45 that Gave >70% Retention of Protein L Binding Activity After Heat Treatment**

| Position^{a} | Substitution Giving >70% Retention of Protein L Binding Activity After Heat Treatment^{b} |
|---|---|
| K45 | 4D, 2Q, 1P, 1N,1H |

| | |
|---|---|
| ^{a}The single substitutions at K45E, I48N, Y49D/N, A50P and I7SN were previously temperature selected from an error-prone phage display library (Section 1). Error-prone PCR samples a limited sequence space. Hence, these sites were randomised by NNK oligonucleotide mutagenesis so that the global sequence space was investigated. ^{b}Phage from 94 clones from each mini-library were screened for the retention of protein L binding activity after heating to 80°C for 10min. Phage were biotinylated prior to heat treatment and subsequently caputured on protein L coated plates and detected with streptavidin HRP. Approximately 10 clones, with an activity greater than 70%, from each library were subjected to sequencing. The total number of sequences is sometimes less than 10 due to second site substitutions as well as poor sequence signals. (E.g. Multiple substitutions found giving a high retention of activity were: K45T/Q90P; K45D/S60P; Y49R/S10F; Y49S/T20A; Y49S/Q27R; A50P/I48V; A50R/A13G/K42E). Note that five clones from the A50X mini-library with an activity in the 0-10% range gave the following sequences: 2A, 1T, 1V, 1Y. ^{c}Substitutions at position 175 represent partial results. | |

### Section 14: Mini-library in V_{H} results

Phage ELISA and scoring of the clones were done according to protocol of Section 4. DNA sequencing of selected clones that unfold reversibly when heated and coold was done according to protocol of Section 6.

**Table 13**

| Position | Selected amino acids | Best % refolding (ie pA-ELISA) |
|---|---|---|
| Phe27 | Gln(5), Ala(2), His(2), Asp(1), Ser(1), Gly(1), Cys(1) | Cys(56%), Asp(32%), His(30%) |
| Phe29 | Gly(4), Ser(2), Asp(2), Pro(1), Gln(1), His(1) | Asp(55%), Pro(50%) |
| Ser30 | Pro(4), Asp(2), Gly(1), Thr(1), Leu(1), Val(1) | Pro(46%), Asp(48%) |
| Ser31 | Pro(6), Asp(1) | Pro(43%) |
| Tyr32 | Gly(9), Gln(2), Glu(1), Pro(1) | Pro(100%), Gly(56) |
| Ala33 | Pro(3), Asp(1), Gly(1) | Pro(37%), Asp(34%) |
| *Ser35* | Asn(3), Asp(3) | Asp(56%) |
| Gly54 | Arg(2), Trp(1), Ser(1), Pro(1), Ala(1), Gly(1), Val(1) | Pro (28%) |
| Ala84 | None | |
| Glu85 | None | |

In the selected amino acids columns, the number in ( ) corresponds to the number of clones carrying this mutation, that were picked as positive by the phage ELISA screening. In the Best % refolding (i.e.. protein A ELISA), the number in ( ) corresponds to the mean % of refolding observed for all clones carrying the particular mutation.

A spurious mutation (Ser25 to Pro) was found in clones carrying the following mutations: A84E, or A33V, or A33E, or A33Q. It is possible that the S25P mutation has a positive effect on refolding, that is most likely surpassing the effect of the mutations at the intended positions.

A number of positive clones were then further analyzed for refolding on phage by following the protocol described in Section 5. The results are presented in Table 14.

**Table 14**

| clone | Ref | SE-15 |
|---|---|---|
| DP47d | 0.55 | 0.19 |
| HEL4 | 18.48 | -0.19 |
| BSA1 | 0.16 | 0.091 |
| pA-C13 | 35 | -0.3 |
| pA-C36 | 25 | -0.18 |
| pA-C47 | 25 | -0.16 |
| pA-C59 | 25 | -0.19 |
| pA-C76 | 35 | -0.28 |
| pA-C85 | 35 | -0.27 |
| F27D | 10.34 | -0.03 |
| F27H | 1.8 | 0.019 |
| F27A | 0.85 | 0.035 |
| F27Q | 3.75 | 0.001 |
| F27S | 1.7 | 0.025 |
| F27G | 2.57 | 0.017 |
| F29D | 11.2 | -0.03 |
| F29V | 1.12 | 0.123 |
| F29S | 6.66 | 0.025 |
| F29P | 11.9 | 0.029 |
| F29Q | 4.54 | 0.001 |
| F29G | 37.5 | 0.017 |
| S30D | 1.66 | 0.139 |
| S30P | 2.54 | 0.194 |
| S30G | 0.37 | 0.182 |
| S30T | 0.1 | 0.208 |
| S30L | 0.1 | 0.308 |
| S30Q | 0.33 | 0.166 |
| S30V | 0.1 | 0.287 |
| S31D | 1.4 | 0.139 |
| S31P | 3.72 | 0.194 |
| Y32D | 8.25 | -0.01 |
| Y32Q | 1 | 0.018 |
| Y32E | 7.11 | -0 |
| Y32P | 4.87 | 0.046 |
| Y32G | 3.28 | 0.034 |
| A33D | 1.32 | 0.129 |
| A33G | 0.37 | 0.172 |
| A33P | 1.88 | 0.017 |
| S35D | 0.91 | 0.139 |
| S35N | 0.6 | 0.165 |
| S35G | 0.65 | 0.182 |
| QUAD | 29.46 | -0.29 |
| A33D/S30N | 2.11 | 0.105 |

Ref. means % refolding on phage as determined by proteinA-binding according to protocol of Section 5. SE-15 represents the S/E hydrophobicity score of the segment of sequence from Cys 22 (included) to Trp36 (included): the hydrophobicity scores of each amino acid of the particular clone in that segment are added and then divided by 15. Quad means quadruple mutant (i.e., F27D/F29V/S35G/Y32E)

### Section 15: Aggregation resistant domain antibodies selected on phage by heat denaturation

Protein aggregation is a problem in biotechnology. Here we describe a method for selecting aggregation resistant polypeptides by heat denaturation. This is illustrated with antibody heavy chain variable domains (dAbs) which are prone to aggregate (Ward, E.S., et al. Nature 341, 544-546 (1989); Ewert, S., et al. Biochemistry 41, 3628-3636 (2002)). The dAbs were displayed multivalently at the infective tip of filamentous bacteriophage, and heated transiently to induce unfolding and to promote aggregation of the dAbs. After cooling, the dAbs were selected for binding to protein A (a common generic ligand that binds the folded dAbs). Phage displaying dAbs that unfolded reversibly were thereby enriched with respect to those that did not. From a repertoire of phage dAbs, six dAbs were characterised after selection; all resisted aggregation, were soluble, well expressed from bacteria, and were purified in high yields. These results demonstrate that the methods described herein can be used to produce aggregation resistant polypeptides, and to identify amino acid residues, sequences or features that promote or prevent protein aggregation, including those responsible for protein misfolding diseases (Dobson, C.M. Trends Biochem Sci 24, 329-3 32 (1999); Rochet, J.C. & Lansbury, P.T., Jr. Curr Opin Struct Biol 10, 60-68 (2000)).

In contrast to human V_{H} dAbs, those of camels and Ilamas have been shown to resist aggregation, even on heating at temperatures as high as 90ºC (Ewert, S., et al. Biochemistry 41, 3628-3636 (2002); Dumoulin, M. et al. Protein Sci 11, 500-515 (2002); van der Linden, R.H. et al. Biochim Biophys Acta 1431, 37-46 (1999)). This remarkable property has been attributed to reversible unfolding, and a series of highly-conserved, predominantly hydrophilic mutations in the β-sheet scaffold have been proposed to account for this behaviour (Ewert, S., et al. Biochemistry 41, 3628-3636 (2002); Dumoulin, M. et al. Protein Sci 11, 500-515 (2002)). As described herein, a human V_{H} dAb referred to as HEL4 has biophysical properties that are similar to those of camels and Ilamas (see also, Jespers, L., et al. J Mol Biol 337, 893-903 (2004)). For example, the HEL4 dAb unfolded reversibly above 62.1°C (*T*ₘ) at concentrations as high as 56 µM. In contrast heating a 5.0 µM solution of the DP47d dAb (a typical human V_{H} dAb encoded by the same germ-line gene as the HEL4 dAb) above 55°C led to irreversible unfolding and formation of aggregates (Jespers, L., et al. J Mol Biol 337, 893-903 (2004)). The human HEL4 dAb is devoid of mutations in the β-sheet scaffold and differs from the DP47d dAb only by mutations in the loops comprising the complementarity determining regions (CDRs) (Table 15). We used the HEL4 and DP47d dAbs to develop a method for the selection of human V_{H} dAbs that unfold reversibly from those that aggregate irreversibly.

The HEL4 and DP47d dAbs were displayed in a multivalent state on the surface of filamentous bacteriophage, thereby providing a link between antibody phenotype and genotype and a powerful means of selection (McCafferty, J., et al. Nature 348, 552-554 (1990)). To induce denaturation, the fusion phage (5x10¹¹ transducing units per ml (TU/ml) were heated to 80°C for 10 min; the phage capsid withstands this temperature (Holliger, P., et al. J Mol Biol 288, 649-657 (1999)) but not the dAbs, which unfold above 60°C (Ewert, S., et al. Biochemistry 41, 3628-3636 (2002)). After cooling, the phage-displayed dAbs were assayed for refolding by phage ELISA on protein A (a generic ligand common to these folded dAbs). Binding was reduced 3-fold for the HEL4 phage but 560-fold for the DP47d phage (FIG. 15A). This suggested that the HEL4 dAb had reversibly unfolded on the phage tip to a significant degree, and that the DP47d dAb had not.

### DP47d dAb aggregates upon heating

By transmission electron microscopy of negatively stained phage, it was observed that >90% of the heated DP47d phage (FIGS. 16A and 16B) were joined together via their tips whereas no clustering was seen with the untreated DP47d phage or heated HEL4 phage (FIG. 16C). These clusters of phage provide direct evidence of DP47d aggregation after heat denaturation. The appearance of clusters requires both high concentration of phage and high local concentration of the dAb at the phage tip (number of dAbs displayed per tip). Western blot analysis shows that for multivalent phage ∼80% of the five pIII coat proteins carry a fused dAb and for monovalent phage ∼20% (FIG. 16D). Thus no phage clusters were observed on heating multivalent DP47d phage at titers of 1x10⁹ TU/ml or monovalent DP47d phage at titers of 5x10¹¹ TU/ml, and in both cases the binding to protein A was only reduced 8-fold and 6-fold respectively (FIG. 15B). Without wishing to be bound by any particular theory, it appears that upon phage heating to 80°C, aggregation of DP47d dAb is nucleated by the formation of a micro-aggregate at the phage tip (intra-phage step) which then grows into oligomeric aggregates by phage clustering (inter-phage step), and hence that aggregation in our phage system follows a two-step process, as noted for other proteins (Dobson, C.M. Trends Biochem Sci 24, 329-332 (1999)).

### Heating phage displaying dAbs that do not unfold reversibly reduces infectivity

Heating at 80°C slightly reduced (3-fold down) the infectivity of the HEL4 phage, as previously observed with wild-type filamentous phage (Holliger, P., et al. J Mol Biol 288, 649-657 (1999)), but considerably reduced the infectivity of the DP47d phage (≥70-fold down) (FIG. 15C). The infectivity of heated DP47d phage could be partly restored by addition of trypsin which presumably cleaves within the dAb and/or the peptide linker connecting the dAb to the pIII protein. These observations are consistent with a model wherein intra- and inter-phage aggregates of DP47d dAb prevent the N-terminal domains of the pIII protein from binding to the bacterial pilus and/or to the TolA receptor (Holliger, P., et al J Mol Biol 288, 649-657 (1999)).

A selection in which phage displaying the HEL4 and DP47d dAbs were mixed in 1:10⁶ ratio respectively, incubated at 80°C for 10 min, cooled and selected on immobilized protein A was performed. Bound phage were eluted with trypsin, and used to re-infect bacteria. After two rounds of such selection, supernatants from infected colonies (n=86) were tested by ELISA on immobilized hen egg lysozyme (HEL) to distinguish the HEL-specific HEL4 phage from the DP47d phage. Phage from twelve colonies bound to hen egg lysozyme (corresponding to a 360-fold enrichment of the HEL4 phage per selection round). None of the 86 tested colonies secreted the HEL4 phage when the selection on protein A was repeated without the heat step. Thus, by two rounds of heat denaturation and biopanning on protein A, phage dAbs that resisted thermal aggregation were selected from those that did not.

A repertoire of human V_{H} dAbs (1.6x10⁹ clones) was prepared by diversification of the loops comprising the CDRs in the DP47d dAb, and displayed multivalently on phage. After three rounds of heat denaturation followed by selection on protein A, 179 out of 200 colonies secreted dAb phage that retained more than 80% of protein A-binding activity after heating. Twenty clones were sequenced and revealed as many unique dAb sequences with a large variability in the CDR sequences and lengths (Table 15). The diversity shows that, as with HEL4, mutations located entirely in the loops comprising the CDRs are sufficient to confer resistance to aggregation. Eighteen of these dAbs had an acidic isoelectric-point (5.1±1.1, mean±SD), consistent with earlier proposals of a direct correlation between net protein charge and resistance to aggregation (Wilkinson, D.L. & Harrison, R.G. Nature 341, 544-546 (1991); Chiti, F. et al. Proc Natl Acad Sci USA. 99, 16419-16426 (2002)).

Six dAbs with CDR3s comprising 10 to 20 amino acids were chosen for further characterization (C13, C36, C47, C59, C76, and C85). These proteins were well secreted from bacteria, and the C36, C47 and C59 dAbs were recovered in yields greater than 20 mg/L in bacterial supernatants compared to only 2.9 mg/L for the DP47d dAb (Table 16). After purification on immobilized protein A, the dAbs were subjected to size-exclusion chromatography on a SUPERDEX-75 column (gel filtration column; Amersham Biosciences). The dAbs eluted as mono-disperse symmetric peaks and the recoveries were nearly quantitative, indicating that in contrast to other human dAbs (Ewert, S., et al. Biochemistry 41, 3628-3636 (2002); Ewert, S., et al. J Mol Biol 325, 531-553 (2003)) they did not stick to the column matrix. The dAbs eluted at a mean apparent molecular mass (*M*_{*r*-app}) of 17 kDa (range 10 to 22 kDa), similar to the calculated molecular weight (*M*_{*r*-calc}) of 13-14 kDa for a monomeric dAb species (FIG. 17A). Variation in *M*_{*r*-app} has been observed for other dAbs (Ewert, S., et al. Biochemistry 41, 3628-3636 (2002); Ewert, S., et al. J Mol Biol 325, 531-553 (2003)) and may result from weak transient interactions with the column matrix or monomer/dinner equilibria (Sepulveda, J., et al. J Mol Biol 333, 355-365 (2003)). Importantly, at 5 µM, each selected dAb unfolded reversibly and cooperatively upon repeated cycles of thermodenaturation (FIG. 17B). Thus the selected dAbs not only resisted aggregation, but as reported for camel and Ilama dAbs (Ewert, S., et al. Biochemistry 41, 3628-3636 (2002); Arbabi Ghahroudi, M., et al. FEBS Lett 414, 521-526 (1997)), were mainly monomeric, well expressed, and purified in good yield by gel filtration.

### Selection on Antigen

In the work described above, protein A, a generic ligand that binds each member of the repertoire, was used for selection. However, any desired antigen can be used to select dAbs that combine the properties of reversible unfolding with a desired antigen specificity. This was demonstrated by selection of a synthetic human V_{H} repertoire for binding to human serum albumin (HSA), with and without a heat denaturation step, and followed by screening of 44 clones for binding to HSA after two rounds of selection. Without the heat step, six unique dAb clones (Table 15) that bound HSA were selected. When the heating step was employed, a single dAb clone (Clone #10, Table 15) was recovered. Only Clone #10 exhibited the properties of reversible unfolding (100% of HSA binding signal was retained after heating Clone #10 phage, compared with less than 10% retained binding signal the others) despite the close similarity in sequence to three of the other clones (#2, #5, #6 and #10 share 92% identity).

### Discussion

Protein aggregation is an off-pathway process that competes with the folding pathway, and usually involves association of the unfolded states, or partially unfolded states. Resistance to aggregation can be achieved by introducing mutations that stabilize the native state (increasing *ΔG*_{N-U}, the free energy of folding) and/or that reduce the propensity of the unfolded or partially unfolded states to aggregate (for example by increasing the solubility of these states). Several selection strategies have been devised to select for protein variants with improved stability: (i) by linking the infectivity of the phage to the proteolytic resistance of the displayed protein (Kristensen, P. & Winter, G. Fold Des 3, 321-328 (1998); Sieber, V., et al. Nat Biotechnol 16, 955-960 (1998); Martin, A., et al. J Mol Biol 309, 717-726 (2001)) and/or (ii) by challenging the displayed protein with elevated temperatures or denaturants (Shusta, E.V., et al. Nat Biotechnol 18, 754-759 (2000); Jung, S., et al. J Mol Biol 294,163-180 (1999))*.* The focus until now has been to destabilize (and promote elimination of) all but the most stable protein variants. For example, by heating a phage antibody library to 60°C, Jung et al. (J Mol Biol 294, 163-180 (1999)) selected a variant of the 4D5Flu antibody fragment, with an improved *ΔG*_{N-U} (+3.7 kcal/mol) and which remained folded at 60°C. Beyond this temperature, unfolding of the antibody fragment resulted in aggregation and loss of infectivity when phage displaying the antibody fragment was heated.

By contrast, in the studies described herein, selection included inducing unfolding of all the dAbs in the repertoire, stable and unstable alike. This selection process operates on the ability of the unfolded dAbs to avoid irreversible aggregation at the phage tip upon heating at 80°C and cooling. The folding properties of the selected dAbs cannot be attributed to stabilization of the native state, because biophysical analysis of thermodynamic stabilities of the selected dAbs indicates that the selected domains are less stable than typical aggregation-prone human dAbs. Thus the free energies of folding (*ΔG*_{N-U}) at 25°C (from 14 to 23 kJ/mol) (Table 16) are lower than those of the DP47d dAb (35 kJ/mol) and other aggregation-prone dAbs based on the same human DP47/3-23 germ-line segment (from 39.7 to 52.7 kJ/mol) (Tomlinson, I.M., et al. J Mol Biol 227, 776-798 (1992); Ewert, S., et al. Biochemistry 41, 3628-3636 (2002)). It appears that the described selection process using heat denaturation selects favorable properties of the unfolded or partially unfolded states.

Other beneficial properties of these selected dAbs appear to follow directly from their resistance to aggregation. For example, the high level of expression obtain for these aggregation-resistant dAbs is consistent with the identification of periplasmic aggregates as the major yield-limiting factor for the production of recombinant antibody fragments in *E. coli* (Wörn, A. & Plückthun, A. J Mol Biol 305, 989-1010 (2001)). In addition, the dAbs selected here were uniformly "non-sticky" upon gel filtration.

The methods described herein can be used to produce improved versions of other polypeptides (e.g., polypeptides expressed in the bacterial periplasm) that can be functionally displayed on the surface of filamentous bacteriophage (e.g., in a multivalent state) and bound by a ligand that recognizes only the properly folded state of the polypeptide (e.g., an antibody that binds properly folded polypeptide, a receptor that binds properly folded polypeptide). Such polypeptides can be diversified (e.g., by engineering random mutations) displayed on phage, denatured, and selected by bio-panning (or other suitable methods) after returning to conditions permissive to the native state (refolding). The methods described herein also provide an analytical tool to identify amino acid residues or polypeptide segments involved in off-pathway aggregation of proteins upon folding, including those involved in diseases of protein misfolding (Dobson, C.M. Trends Biochem Sci 24, 329-332 (1999); Rochet, J.C. & Lansbury, P.T., Jr. Curr Opin Struct Biol 10, 60-68 (2000)).

**Table 15. Sequences of loops comprising the CDRs of dAbs described in this study**

| | H1-CDR1¹ | H2-CDR2¹ | H3-CDR3¹ | |
|---|---|---|---|---|
| Clone | 26 to 35² | 50---a---------60------² | 94-100abcdefghij102² | L⁴ |
| DP47d | GFTFSSYAMS (SEQ ID NO: 104) | AISGSGGSTYYADSVKG (SEQ ID NO: 105) | K SYGA----------FDY (SEQ ID NO: 106) | 7 |
| HEL4 | GFRISDEDMG (SEQ ID NO: 107) | S1YGPSGSTYYADSVKG (SEQ ID NO: 108) | S ALBPLSEP-LGF (SEQ ID NO:109) | 11 |
| a)³C13 | GDMVNDKDMS (SEQ ID NO:110) | SISTENGSTYYADSVKG (SEQ ID NO:111) | G VRDEVAMGENPD------LSY (SEQ ID NO:112) | 15 |
| C22 | GFRFSAEDMG (SEQ ID NO:113) | SIDNDDGSTYYADSVKG (SEQ ID NO:114) | S SPGPDNEKDNAS-----LKS (SEQ ID NO:115) | 15 |
| C24 | GDSVSNKVMG (SEQ ID NO:116) | AIDTKDGSTYYADSVKG (SEQ ID NO:117) | S GDVDADMAWEEE------VSS (SEQ ID NO:118) | 15 |
| C33 | GDTLTSDNMA (SEQ ID NO: 119) | TTTEAGGSTYYADSVKG (SEQ ID NO:120) | T YPADVAECAAE-VCY (SEQ ID NO:121) | 14 |
| C36 | GVNVSHDSMT (SEQ ID NO:122) | AIRGPNGSTYYADSVKG (SEQ ID NO:123) | S GARHADTERPPSQQT-MPF (SEQ ID NO:124) | 18 |
| C37 | GYRISPDYMG (SEQ ID NO:125) | SISNNGGSTYYADSVKG (SEQ ID NO:126) | S VDAAESGIDSN------VGS (SEQ ID NO:127) | 14 |
| C46 | GYRVNAQDMS (SEQ ID NO:128) | TIENENGSTYYADSVKG (SEQ ID NO:129) | CTRGGCYDT------FPY (SEQ ID NO: 130) | 12 |
| C47 | GYN1TDENMA (SEQ ID NO:131) | TIAADNGSTYYADSVKG (SEQ ID NO:132) | T TEAAGVEEDN-----VRS (SEQ ID NO:133) | 13 |
| C50 | GDKVSYNNMA (SEQ ID NO:134) | SITTENGSTYYADSVKG (SEQ ID NO:135) | G NRNSPVDYRELQSTP-----LDS (SEQ ID NO:136) | 18 |
| C57 | GDNFNNENMG (SEQ ID NO:137) | TISDTNGSTYYADSVKG (SEQ ID NO: 138) | T TGTRQPQKE--------VGS (SEQ ID NO:139) | 12 |
| C58 | GVNVTDQDMG (SEQ ID NO:140) | SIRSNDGSTYYADSVKG (SEQ ID NO:141) | G RSSGRTDA-------VPY (SEQ ID NO:142) | 11 |
| C59 | GDSISDDYMA (SEQ ID NO:143) | SIDDKNGSTYYADSVKG (SEQ ID NO:144) | G GDGQAHK--------VDY (SEQ ID NO:145) | 10 |
| C61 | GDMLNYKVMG (SEQ ID NO:146) | STITQDGSTYYADSVKG (SEQ ID NO:147) | G IPLDRADD------IEY (SEQ ID NO:148) | 11 |
| C62 | GYKVNDNTMA . (SEQ ID NO:149) | SIDTTDGSTYYADSVKG (SEQ ID NO:150) | A SDQRTAD------MRS (SEQ ID NO:151) | 10 |
| C63 | GVTVSDENMG (SEQ ID NO:152) | GISSNDGSTYYADSVKG (SEQ ID NO:153) | R DYGSRVDQQH-----LES (SEQ ID NO:154) | 13 |
| C73 | GVTLNDEYMG (SEQ 1D NO:155) | SINDRNGSTYYADSVKG (SEQ ID NO:156) | S WVVPGRKSAEP---MDY (SEQ ID NO: 157) | 14 |
| C74 | GYTFSDNDMA (SEQ ID NO:158) | GITSDSGSTYYADSVKG (SEQ ID NO:159) | T ESPNGVTKLSDKN---FES (SEQ ID NO:160) | 16 |
| C76 | GDNVISDDMS (SEQ ID NO:161) | TINGPSGSTYYADSVKG (SEQ ID NO:162) | A NGEDTDMLDMWGDRSAALKS (SEQ ID NO:163) | 20 |
| C77 | GVKFNDEDMS (SEQ ID NO:164) | SIGTENGSTYYADSVKG (SEQ ID NO:165) | A GPSGHEGNYD-----IDS (SEQ ID NO:166) | 13 |
| C85⁴ | GDKITSKNMS (SEQ ID NO:167) | TIPAEGGSTYYADSVKG (SEQ ID NO:168) | T ACFPSAQH------VES (SEQ ID NO:169) | 11 |
| b)³ #1 | GFTFDLYDMS (SEQ ID NO:170) | SLVNSGVRTYYADSVKG (SEQ ID NO:171) | KLNQSYHWD----FDY (SEQ ID NO: 172) | 11 |
| #2 | GFTFSDYRMS (SEQ ID NO: 13) | TIISNGKFTYYADSVKG (SEQ ID NO:174) | K QDWMYM -----FDY (SEQ ID NO:175) | 9 |
| #3 | GFTFSKYWMS (SEQ ID NO:176) | SIDFMGPHTYYADSVKG (SEQ ID NO:177) | K GRTSMLPMKGK-----FDY (SEQ ID NO:178) | 14 |
| #4 | GFTFYDYNMS (SEQ ID NO:179) | TITHTGGVTYYADSVKG (SEQ ID NO:180) | K QNPSYQ PDY (SEQ ID NO:181) | 9 |
| #6 | GFTPHRYSMS (SEQ ID NO:182) | TILPGODVTYYADSVKG (SEQ ID NO:183) | K QTPDYM FDY (SEQ ID NO:184) | 9 |
| #7 | GFTFWKYNMA (SEQ ID NO:185) | TILGEGNNTYYADSVKG (SEQ ID NO:186) | KTMDYK FDY (SEQ ID NO:187) | 8 |
| #10 | GFTFDEYNMS (SEQ ID NO:188) | TILPHGDRTYYADSVKG (SEQ ID NO:189) | K QDPLYR FDY (SEQ ID NO:190) | 9 |

| | | | | |
|---|---|---|---|---|
| ¹ CDR1, 2 and 3 defined according to Kabat *et al.* and structural loops H1, 2 and 3 according to Chothia *et al*.. Sequences given include both these regions and residue 94 of FR3. ² Residue numbering according to Kabat *et al*. ³ Sequences of clones selected on protein A (a) and human serum albumin (b). ⁴ L: amino acid length of H3-CDR3. ⁴ This clone also contains a mutation of Trp47 to Cys in framework 2. | | | | |

**Table 16. Biophysical and expression data of selected dAbs**

| dAb clone | *T*ₘ¹ (°C) | *ΔG*_{N-U}² (kJ/mol) | % recovery⁴ on Superdex-75 | Yield⁵ (mg) |
|---|---|---|---|---|
| | | | | |
| DP47d | 61.4 | 35³ | ≤5 | 2.9 |
| HEL4 | 62.1 | 28 | ≥90 | 9.5 |
| C13 | 54.1 | 14 | 88 | nd⁷ |
| C36 | 59.9 | 23 | 95 | 24 |
| C47 | 60.7 | 23 | 100 | 39 |
| C59 | 55.9 | 18 | 94 | 22 |
| C76 | 54.5 | 21 | 100 | nd |
| C85 | 61.2 | 20 | 86⁶ | nd |
| | | | | |

| | | | | |
|---|---|---|---|---|
| ¹ Temperature of mid-point transition upon reversible unfolding (or aggregation for DP47d dAb). ² Thermodynamic stability value obtained from thermo-denaturation curves. ³ Thermodynamic stability value obtained from urea-induced denaturation curves at 25°C (unpublished data, L.J., O.S., G.W. and L.C. James). ⁴ Obtained by integrating the areas of the peak(s) eluted from Superdex G75. ⁵ Yield of purified protein obtained from a 1 L supernatant of bacterial culture normalised to 5.0 OD₆₀₀ₙₘ. ⁶ A peak corresponding 7% of the sample migrated as multimeric species. ⁷ nd: not determined. | | | | |

### Methods

*Phage display of a human V_{H} dAb library.* The dAb repertoire was created in two-steps by oligonucleotide-mediated diversification of several codons in the sequence of the DP47d dAb as follows (Kabat numbering for the amino acid positions and IUPAC-IUB code for the nucleotides): 27, KWT; 28, ANS; 29, NTT; 30, ANC; 31, NMT; 32, NAS; 33, DHT; 35, RSC; 50, RSC; 52, NNK; 52a, RNS; 53, VVW; 54, CGT; 94, RSW; 101, NVS; 102, THT; and NNK codons for all CDR3 positions from 95 to 100x (where x ranges alphabetically from a to k). The DNA inserts were flanked with *ApaL*I (at 5'-end) and *Not*I (at 3'-end) sites by PCR, digested and ligated into the corresponding sites of fd-myc, a multivalent phage vector derived from fdCAT1 (McCafferty, J., et al Nature 348, 552-554 (1990)) that contains a c-*myc* tag between the *Not*I site and *gene III*. The ligation products were transformed by electroporation into *E. coli* TG1 cells, and plated on 2xTY plates supplemented with 15 µg/ml of tetracycline (2xTY-Tet), yielding a library of 1.6x10⁹ clones. For monovalent display, dAb genes (as *Nco*I*-Not*I DNA fragments) were ligated into the corresponding sites of pR2, a phagemid vector derived from pHENI (Hoogenboom, H.R. et al. Nucleic Acids Res 19, 4133-4137 (1991)) that contains the (His)₈ and VSV tags between the *Not*I site and *gene III.* Phage were prepared, purified and stored as described (McCafferty, J., et al. Nature 348, 552-554 (1990); Hoogenboom, H.R. et al. Nucleic Acids Res 19, 4133-4137 (1991)). For the analysis of phage proteins, 1x10¹⁰ transducing units (TU) was subjected to SDS PAGE (4-12% Bis-Tris gel, Invitrogen), and transferred to a PVDF Immobilon-P membrane (Millipore) for detection; the blocked membrane was incubated with murine anti-pIII antibody (MoBiTec), then anti-murine horseradish peroxidase conjugate (Sigma-Aldrich), and electro-chemiluminescence reagents (Amersham Biosciences).

*Phage ELISA assays.* The ELISA wells were coated overnight at 4°C with one of the following ligands: 10 µg/ml of protein A in PBS, 10 µg/ml of mAb 9E 10 in PBS, or 3 mg/ml of HEL in 0.1 M NaHCO₃ buffer, pH 9.6. After blocking the wells with PBS containing 2% Tween-20 (PBST), a dilution series of phage in PBST was incubated for 2h. After washing with PBS, bound phage were detected as follows. In assays for binding to HEL, phage was detected directly using a conjugate of horseradish peroxidase with an anti-M13 monoclonal antibody (Amersham) using 3,3',5,5'-tetramethylbenzidine as substrate. In assays for binding to protein A, the phage (4x10¹⁰ TU/ml in PBS) was first biotinylated at 4°C with biotin-NHS (Perbio) (50 µM final concentration) and detected by sequential addition of streptavidin-horseradish peroxidase conjugate (1 µg/ml) (Sigma-Aldrich) in PBST, and substrate as above. In assays for binding to human serum albumin (HSA) (Sigma, coating at 10 µg/ml in PBS), the ELISA was performed in PBS supplemented with 2% skim-milk powder (PBSM) and bound phage were detected in two steps using a rabbit anti-fd bacteriophage monoclonal antibody (Sigma, 1/1000 dilution) and goat anti-rabbit IgG serum conjugated with horseradish peroxidase (Sigma, 1/1000 dilution) and substrate as above.

*Electron microscopy.* Phage (1x10¹² TU/ml in PBS) were heated at 80°C for 10 min and then cooled at 4°C for 10 min or left untreated as control. After dilution to 1x10¹⁰ TU/ml in PBS, phage were adsorbed on glow discharged carbon coated copper grids (S160-3, Agar Scientific), washed with PBS and then negatively stained with 2% uranyl acetate (w/v). The samples were studied using a FEI Tecnai 12 transmission electron microscope operating at 120 kV and recorded on film with calibrated magnifications.

*Phage selection.* Immunotubes (Nunc) were coated overnight with protein A, and blocked with PBST. Purified phage (1x10¹¹ TU/ml in PBS) were heated as described above, diluted with 3 ml of PBST, and incubated for 2h in the immunotubes. After 10 washes with PBS, protein A-bound phage were eluted in 1 ml of 100 µg/ml trypsin in PHS during 10 min, then used to infect 10 ml of log-phase *E*. *coli* TG1 cells at 37°C during 30 min. Serial dilutions (for phage titer) and library plating were performed on 2xTY-Tet agar plates. For the next selection round, cells were scraped from the plates and used to inoculate 200 ml of 2xTY-Tet at 37°C for phage amplification (McCafferty, J., et al. Nature 348, 552-554 (1990)). For selection on HSA, a similar synthetic human VH repertoire (Domantis Ltd) was used, the antigen (10 µg/ml in PBS) was coated in immunotubes, the blocking agent was PBSM, and the phage library (1x10¹² TU in 1 ml PBS) was heat-treated or left untreated before each round of biopanning.

*Protein expression and purification*. The genes encoding for the various dAbs were subcloned into pET-12a (Novagen) followed by the addition *of Sal*I and Bam*H*I sites by PCR. After transformation of *E*. *coli* BL21(DE3)pLysS cells (Novagen), dAb expression (1 L scale) was induced in the presence of 1 mM IPTG (final concentration) at 30°C during 16 hours. After centrifugation, the supernatants were filtered (0.22 µM) and each incubated overnight with 5 mL of STREAMLINE-protein A beads (Amersham Biosciences) at 4°C. The beads were packed into a column, washed with PBS, and bound dAbs were eluted in 0.1 M glycine-HCI, pH 3.0. After neutralisation to pH 7.4, the protein samples were dialyzed in PBS and concentrated before storage at 4°C. Protein purity was estimated by visual inspection after SDS-PAGE on 12% Bis-Tris gel (Invitrogen). To obtain expression yields from normalized cultures, five individual colonies from freshly transformed bacteria were grown overnight at 37°C, and induced for expression as described above (50 ml scale). Following to overnight expression, the cultures were combined and a culture volume corresponding to 600xOD₆₀₀ₙₘ (120 to 135 ml) was processed for dAb purification as described. The amount of purified protein was extrapolated to the protein yield per litre of bacterial culture, corrected for a final absorbance of 5.0 at OD₆₀₀ₙₘ. For analytical gel-filtration, 500 µl of a 7 µM solution of dAb were loaded on a SUPERDEX-75 (Amersham Biosciences). The *M*ᵣ of each of the peaks on the chromatograms were assigned using a scale calibrated with markers, and the yields calculated from the areas under the curve.

*Circular dichroism measurements.* CD cuvettes (1 cm path-length) were filled with a 5 µM solution of dAb in PBS and transferred to a J-720 polarimeter (Jasco). CD spectra at 25°C and 85°C were recorded in the far-UV (200 nm to 250 nm) with a 2 nm bandwidth and a 1 second integration time. Unfolding curves from 25°C to 85°C were monitored at 235 nm and repeated twice for each dAb. The unfolding curves were assumed to be two-state and fitted as described (Pace, C.N. & Scholtz, J.M. in Protein Structure, A Practical Approach, Edn. 2. (ed. T.E. Creighton) 299-321 (Oxford University Press, New York; 1997)) using *a ΔCₚ* contribution of 12 cal per amino acid residue (Myers, J.K., et al. Protein Sci 4, 2138-2148 (1995)). The values obtained for *T*ₘ (midpoint transition temperature in Kelvin), Δ*H*ₘ (enthalpy change for unfolding at *T*ₘ) were then used to calculate the thermodynamic stability (*ΔG*_{N-U}) of the protein at 25°C as described (Pace, C.N. & Scholtz, J.M. in Protein Structure, A Practical Approach, Edn. 2. (Ed. T.E. Creighton) 299-321 (Oxford University Press, New York; 1997)).

### Section 16. Introducing "folding gatekeeper" residues into single variable domain with defined specificity.

A "folding gatekeeper" is an amino acid that, by the virtue of its biophysical characteristics and by its position in the primary sequence of a protein, prevents the irreversible formation of aggregates upon protein unfolding. A folding gatekeeper residue blocks off-pathway aggregation, thereby ensuring that the protein can undergo reversible unfolding. The effectiveness of folding gatekeepers (determined by the position(s) and the biophysical characteristics) influence the maximal concentration at which the unfolded protein can remain in solution without forming aggregates. As described herein, folding gatekeepers have been introduced into single variable domains (DP47d, or VKdummy) lacking antigen-specificity.

TAR2-10-27 is a human V_{H}3 domain which has binding specificity for human tumor necosis factor receptor 1 (TNFRI, also referred to as "TAR2"). The amino acid sequence of TAR2-10-27 is: 
EVQLLESGGGLVQPGGSLRLSCAASGFTFEWYWMGWVRQAPGKGLEWVS
AISGSGGSTYYADSVKGRFTISRDNSKNTLYLQMNSLRAEDAAVYYCAKVK
LGGGPNFGYRGQGTLVTVSS (SEQ ID NO:191)
 The results of this *rational-design* approach complements another approach in which folding gatekeepers are introduced *ab initio* in dAb libraries displayed on phage (see Section 18)..

### Choosing the folding gatekeepers for TAR2-10-27

In the human DP-47-V_{H}3 domain, aggregation is mediated by the hydrophobic segment encompassing the H1-loop. Site-directed mutagenesis and biophysical analysis have shown that even a single point mutation that lower the SE-score over residue 22 to 36 in DP-47 to below zero, can confer proper folding in this variable domain at concentration up to 5 uM. The preferred positions for single point mutations are residues Phe27 and Tyr32 and, the preferred substitutions are the hydrophilic Asp, Glu, Gln, Asn residues, or the beta-strand breaking residue Pro. As shown in Table 17, four single mutants in the H1-loop of TAR2-10-27 significantly lower the SE-15 score, and may therefore confer high folding yield. Combinations of mutations would further lower the SE-15 score below zero.

**Table 17**

| Clone | HI sequence | SE-15 score |
|---|---|---|
| TAR2-10-27 | CAASGFTFEWYWMGW (SEQ ID NO:192) | 0.267 |
| TAR2-10-27-F27D | CAASGDTFEWYWMGW (SEQ ID NO:193) | 0.051 |
| TAR2-10-27-F27Q | CAASGQTFEWYWMGW (SEQ ID NO:194) | 0.079 |
| TAR2-10-27-Y32D | CAASGFTFEWDWMGW (SEQ ID NO:195) | 0.068 |
| TAR2-10-27-Y32Q | CAASGFTFEWQWMGW (SEQ ID NO:196) | 0.095 |

The F27D, F27Q, Y32D or Y32Q point mutations were introduced into the DP-47-V_{H}3 domain using SOE-PCR, and the nucleic acids encoding the mutated VH3 domains were subcloned, expressed and the encoded proteins were purified

The PCR-amplified genes encoding these proteins were subcloned into a derivative of the pET-12A vector in which the *OmpT* leader sequence was replaced with an eukaryotic leader sequence, using the *SalI* and *NotI* restriction sites. The ligated DNAs were used to transform *E. coli* BL21(DE3)plysS, which were then plated on agar plates supplemented with TYE-agar+5%glucose+100µg/ml ampicillin and incubated at 37°C overnight.

A 10mL 2xTY culture was inoculated with a single bacterial colony from the plates and grown overnight at 37°C. The 2xYT culture contained ampicillin (100 µg/mL and glucose (5%). A 500 mL 2xTY culture in a 2.5L culture flask was inoculated with 5 mL of the over-night culture and grown at 37°C with shaking (250 rpm) until an OD₆₀₀ₙₘ of 0.5-0.6. The media also contains ampicilin (50 µg/mL) and glucose (0.1%). When the OD₆₀₀ₙₘ reached 0.5-0.6, the culture flasks were further incubated at 30°C with shaking. After about 20 minutes, protein production was induced by the addition of 1mM IPTG (final concentration) and the cultures were grown overnight.

Then the culture media was transferred to cylindrical 0.5 or 1 L bottles (glass or plastic). 0.5-1 mL of a 1:1 slurry of protein A-beads (Amersham Biosciences) was added, and the mixture was rolled on horizontal flask rollers at 4°C overnight. Then the bottles were left upright allowing the resin to settle. The resin (beads) was repeatedly washed with 1xPBS, 500 mM NaCl (2x), and the washed resin was loaded into a drip column. The resin wass washed again with 5 column volumes of 1xPBS, 500 mM NaCl. After the resin had almost run dry, 0.1 M glycine pH 3.0 was applied to the column and 1ml fractions are collected. The collection tubes were filled prior to the elution with 200 µL 1M Tris, pH 7.4 in order to neutralise the solution. The OD₂₈₀ₙₘ was checked for each fraction and the protein elution monitored. The fractions that contained protein were combined and dialysed against an appropriate buffer (1x PBS in most cases). The protein concentration was again determined (using a calculated exctinction coefficient) and purity was evaluated by SDS-PAGE analysis.

The protein yields per litre of culture supernatant are shown in Table 18. As previously described herein, recombinant expression of the DP-47-V_{H}3 domain in the pET/BL21(DE3)pLysS system was increased when folding gatekeepers were inserted into the protein sequence.

**Table 18**

| Clone | mg protein per Litre |
|---|---|
| TAR2-10-27 | 0.35 |
| TAR2-10-27 F27Q | nd |
| TAR2-10-27 F27D | 4.6 |
| TAR2-10-27 Y32Q | nd |
| TAR2-10-27 Y32D | 5.3 |

Aggregation of heated and cooled proteins was assessed at different concentrations. The proteins were heated at different concentrations (1-100µM) for 10 minutes at 80°C. Then, the protein was allowed to refold at either room temperature or 4°C for 10 minutes. Protein A beads were added to the refolded protein solution, and the mixture was rolled until all refolded protein has been captured by the beads. The beads were washed repeatedly and equal amounts of beads were loaded onto a SDS-PAGE gel for visualisation. Densitometry was used to quantify the amount/concentration of protein recovered, and to calculate the concentration at which half the protein properly refolded after exposure to high temperature ([protein]_{50%}).

Samples of each protein at different concentrations ranging from 1-100µM were prepared. The samples were heated for 10 minutes at 80°C in a PCR block using 0.5 mL thin-walled tubes. The ramping was ∼10°C/min. Duplicate samples were prepared and processed in parallel but omitting the heating step.

After heating, the samples were kept at room temperature or 4°C for a few minutes. The samples were transferred to 1.5 mL micro tubes and centrifuged at 4°C for 10 minutes at high speed. The supernatant was recovered without disturbing any pellets that formed. 20 µL of a 1:1 slurry of protein A-beads was added to each tube, the tube was topped up to 1mL of PBS and rolled for 1-2 hours on an overhead roller at room temperature. When the protein A beads had settled, the supernatant was removed without disturbing the beads. The beads were washed three times with 1 mL PBS and resuspended in 40 µL of SDS-loading buffer, including DTT. The SDS-loading buffer contained a known concentration of BSA which served as a standard to normalise for possible error the loading volume. The samples were heated at 90°C for 10 minutes, centrifuged for 10 minutes at 14,000 rpm and then equal amounts of each sample were loaded and separated on a 12% Bis/Tris SDS-NuPage gel. Equal loading, background and intensity of bands were quantified using densitometry. The densitometry data was plotted against the concentration of initial protein to produce a sigmoidal curve that was used to determine the protein concentration at which 50% of the protein refolded after heat-unfolding ([protein]_{50%}). The [protein]_{50%} are shown in Table 19.

**Table 19**

| Clone | [protein]_{50%} (µM) |
|---|---|
| TAR2-10-27 | 2-4 |
| TAR2-10-27 F27Q | nd |
| TAR2-10-27 F27D | 5-10 |
| TAR2-10-27 Y32Q | 5-10 |
| TAR2-10-27 Y32D | 25-50 |

Introducing folding gatekeeper mutations had a beneficial effect on TAR2-10-27 by conferring the ability to resist aggregation at higher protein concentration: 2- to 4-fold higher for TAR210-27 F27D and TAR2-10-27 Y32Q, and 7 to 25-fold higher for TAR210-27 Y32D, relative to the parental TAR2-10-27.

### Functional Assays

TNFR1 binding activity of the VH domains was assessed in a receptor binding assay and a cell-based assay.

In the receptor bindng assay, anti-TNFR1 VH domains were tested for the ability to inhibit the binding of TNF to recombinant TNF receptor 1 (p55). Briefly, MAXISORP plates were incubated overnight with 30µg/ml anti-human Fc mouse monoclonal antibody (Zymed, San Francisco, USA). The wells were washed with phosphate buffered saline (PBS) containing 0.05% TWEEN-20 and then blocked with 1% BSA in PBS before being incubated with 100ng/ml TNF receptor 1 Fc fusion protein (R&D Systems, Minneapolis, USA). Anti-TNFR1 VH domain was mixed with TNF which was added to the washed wells at a final concentration of 10ng/ml. TNF binding was detected with 0.2µg/ml biotinylated anti-TNF antibody (HyCult biotechnology, Uben, Netherlands) followed by 1 in 500 dilution of horse radish peroxidase labelled streptavidin (Amersham Biosciences, UK), and then incubation with TMB substrate (KPL, Gaithersburg, USA). The reaction was stopped by the addition of HCl and the absorbance was read at 450nm. Anti-TNFRI VH domain binding activity lead to a decrease in TNF binding to receptor and therefore a decrease in absorbance compared with the TNF only control. (FIG. 18A)

In the cell-based assay, anti-TNFR1 VH domains were tested for the ability to neutralise the induction of IL-8 secretion by TNF in HeLa cells (method adapted from that of Akeson, L. et al. Journal of Biological Chemistry 271, 30517-30523 (1996), describing the induction of IL-8 by IL-1 in HUVEC). Briefly, HeLa cells were plated in microtitre plates and incubated overnight with VH domain proteins and 300 pg/ml TNF. Post incubation, the supernatant was aspirated off the cells and the amount of IL-8 in the supernatant was measured using a sandwich ELISA (R&D Systems). Anti-TNFR1 VH domain activity lead to a decrease in IL-8 secretion into the supernatant compared with the TNF only control. (FIG. 18B)

The results of both assays demonstrate that TAR2-10-27 F27D and TAR2-10-27 Y32D retained biological activity. The activity of TAR2-10-27 Y32D was about equivalent to that of the parental dAb TAR2-10-27, while the activity of TAR2-10-27 was slightly reduced relative to TAR2-10-27. (FIGS. 18A and 18B) For both the receptor-binding assay and the cell based assay, IC50 values can be calculated from the sigmoidal curves with an average precision within a factor of two.

### Conclusion

This section shows that a folding gatekeeper residue can be introduced into a VH domain of pre-defined specificity such that the resulting variant combines increased resistance to aggregation with antigen-binding activity. This was achieved without knowing the functional epitope that is bound by the VH domain. One variant in particular (TAR2-1027-Y32D) retained full biological activity and had a 10-fold increase in resistance to aggregation ([protein]_{50%}). Another benefit of introducing folding gatekeepers, is the increased expression level of the proteins (e.g., by >10-fold in the pETBL21(DE3)pLysS system). The SE-score of TAR2-10-27-Y32D (0.068) is still above 0 and above the value obtained for HEL4 (-0.187). Thus, if desired, additional folding gatekeepers could be introduced into TAR2-10-27-Y32D for further improvement of its biophysical properties.

### Section 17. Selection of an antigen-specific VH domain that contains a folding gatekeepers from a synthetic library of VH domains.

Using phage display technology, antibody variable domains can be isolated from a synthetic repertoire based on binding to an antigen (*e.g.,* by binding antigen immobilized on in a Petri dish and recovering adherent phage, "biopanning'). This approach was used to select a human V_{H} that binds human serum albumin (HSA) from a synthetic human VH repertoire following a heat denaturation step (or without the heat denaturation step for negative control).

The phage library of synthetic VH domains was the libray 4G, which is based on a human V_{H}3 comprising the DP47 germline gene and the J_{H}4 segment. The diversity at the following specific positions was introduced by mutagenesis (using NNK codons; numbering according to Kabat) in CDR1: 30, 31, 33, 35; in CDR2: 50, 52, 52a, 53, 55, 56; and in CDR3: 4-12 diversified residues: e.g. H95, H96, H97, and H98 in 4G H11 and H95, H96, H97, H98, H99, H100, H100a, H100b, H100c, H100d, H100e and H100f in 4G H19. The last three CDR3 residues are FDY so CDR3 lengths vary from 7-15 residues. The library comprises >1x10¹⁰ individual clones.

### Protocol

The 4G library was divided into two sub-libraries of equal number of transducing units (1x10¹¹). One was used as such for panning against HSA, and the other was heat-unfolded at 80°C for 10 minutes before each panning step. For the heating step, the phage sample was dispensed as 100 µL fractions in a PCR tube. After heating, the fractions were combined. Both samples (heat treated and unheated control) were centrifuges for 10 minutes at 14,000 rpm at 4°C. The supernatants were taken further into the selections on immuno-tubes. The antigen (HSA, Sigma-Aldrich) was coated in immuno-tubes (at 100 ug/mL in PBS), stored at 4°C. The tubes were blocked with 2% skim-milk in PBS (2MPBS).

### Results

After the first round of selection (R1), approx. 3000 phage were eluted from the tubes (both from the heated phage library and from the un-heated library). After a second round of selection (R2), a confluent lawn of colonies was obtained from the selection with un-heated phage, whereas the enrichment was less pronounced for the heated phage library (approx. 2 to 5-fold increase in titer). Individual colonies (n=44) were randomly picked from both libraries (heat treated and control) after R2 and separately grown overnight to secrete phage in the supernatant (using a 96-well microtiter cell culture plate).

A phage ELISA was performed according to the protocol of Section 4, with some modifications. The microtiter wells were coated overnight with HSA (10µg/mL in PBS) and blocked with 2MPBS. Each of the samples was washed three times with 0.1% TWEEN-20 in PBS. The detection reagents were rabbit-anti-bacteriophage fd full IgG (Sigma) at 1:1000 dilution followed by goat-anti-rabbit conjugated with horseradish peroxidase (full IgG) (Sigma) at 1:1000 dilution. All individual clones were tested against HSA with heat-step or without heat step. They were also tested against BSA (10ug/mL) and plastic. Only 2/37 positive signals were unspecific.

As predicted, all colonies obtained from the unheated control selection secreted phage VH domains that did not sustain heat-treatment In contrast, all colonies from the heated-selection secreted phage VH domains that refold upon cooling. Thirty-six positive clones in ELISA were sequenced, and 6 unique sequences were obtained for clones from the unheated panning and 1 unique sequence was obtained from the heat-unfolding panning (clone #10) (Table 15 b) #1-#4, #6, #7 and #10).

Clone 10, which has two acidic residues at positions 30 and 31 in the CDR1, exhibits the lowest SE-score (0.06) (15 amino acid window). In contrast, the mean SE score obtained from all sequences from the unheated phage selection (Clones #1-7) was above 0.15 (was 0.203±0.020; mean±standar deviation), thereby explaining their greater propensity to aggregate. (Interestingly, clone #10 shares 92% identity with clones #2, #5, and #6, suggesting a common binding epitope on HSA for these four VH domains. But only clone #10 exhibits the refolding property and has folding gatekeepers at positions 30 and 31.

The behaviour of the phage-displayed VH domains was further analysed with purified phage. A phage ELISA was performed according to the protocol of Section 5 with the following modifications. 1x10¹⁰ phage were either heat-treated (10 minutes at 80°C in 100 µL aliquots) or left untreated. The samples were centrifuged for 10 minutes at 14,000 rpm at 4°C and the supernatants were added to ELISA wells coated with HSA (with a dilution series of 1/3). After developing the ELISA, the OD450nm values (y-axis) for each heat treated/unheated phage pair were plotted against the phage number (x-axis) and the %-refoldability was calculated. The results are shown in Table 20.

**Table 20**

| clone | + Heat | - Heat | Refoldability [%] |
|---|---|---|---|
| #1 | 10¹⁰ | 1.5x10⁸ | 1.5 |
| #2 | 10¹⁰ | 1.3x10⁹ | n.d. |
| #3 | 10¹⁰ | 5.5x10⁷ | 0.55 |
| #4 | 10¹⁰ | 5.7x10⁸ | 9.1 |
| #6 | 9.75x10⁹ | 2.1x10⁷ | <0.2 |
| #7 | nd | nd | Nd |
| #10 | 10¹⁰ | 10¹⁰ | ∼100 |

### Conclusion

The results show that the VH domain clone selected on HSA (clone #10) following a heat unfolding step is fully resistant to aggregation when expressed on phage, whereas none of the VH domain clones selected without the heat unfolding step exhibited greater than 10% refolding after heating. This study demonstrates that antigen-specific variable domains that unfold reversibly upon heating can be selected from a repertoire of aggregation-prone VH domains (>98% of the clones from the primary library 4G have been shown to aggregate upon heating).

### Section 18. Creation of phage libraries of synthetic VH domains that include folding gatekeeper residues.

Section 16, demonstrated that a VH domain with good folding properties can be isolated from a repertoire of aggregation-prone VH domains in which the CDR1 loop was diversified and hence, a small percentage of the VH domains in the primary repertoire had folding gatekeepers in the CDR1 loop. This section describers the preparation of a phage repertoire in which the majority of VH domains unfold reversibly.

### Methods and results

Diversity was introduced by randomly combining DNA fragments encoding diversified CDR1, CDR2 and CDR3 using assembly PCR. The resulting fragments were cloned into a phage vector (Fd-myc in which the leader sequence was replaced with an eukaryotic leader sequence; see Section 2), to yield a primary library (VH-6G). The V_{H} library is based on a single human framework (V3-23/DP-47 and J_{H}4b). The canonical structures (V_{H}: 1-3) encoded by this frameworks are the most common in the human antibody repertoire. Side chain diversity was incorporated using NNK diversified codons at positions in the antigen binding site that make contacts with antigen in known structures, and are highly diverse in the mature repertoire. Diversity was incorporated at the following positions (Kabat numbering):
V_{H} CDR1: H30, H31, H32, H33, H35.
V_{H} CDR2: H50, H52, H52a, H53, H55 and H56.
V_{H} CDR3: 4-12 diversified residues: e.g. H95, H96, H97, and H98 in 4G H11 and H95, H96, H97, H98, H99, H100, H100a, H100b, H100c, H100d, H100e and H100f in 4G H19. The last three CDR3 residues are FDY so CDR3 lengths vary from 7-15 residues.

The primary library was then used to produce phage stock. The phage stock was split in 2: one stock was used as such for panning on immobilised protein A (which binds correctly folded VH domains). Bound phages were eluted, used to infect E. *coli* and library DNA was prepared from the pooled infected cells (DNA pool "E").

The second stock was first heated at 80°C for 10 minutes, and subsequently panned on protein A. The library of phage propagated after panning was enriched for VH domains that refold after heat denaturation. The DNA pool obtained from these phages is referred to as DNA pool "H".

Individual colonies from the primary library and from the selected libraries were separately grown for phage production. The phages were tested for their ability to bind to protein A (to assess production as well as correct folding) when untreated or after the heat treatment at 80°C. The results are shown in Table 21.

**Table 21**

| Pool | % positive clones on pA ELISA (no pre-heating) | % positive on pA ELISA (with heating at 80°C for 10 min) |
|---|---|---|
| VH-6G | 1 | nd |
| VH-6G-E | 91 | 24 |
| VH-6G-H | 82 | 51 |

Next a DNA fragment comprising the pooled CDR1 and CDR2 regions was amplified from DNA pool H. From DNA pool E, only the pooled CDR3s were amplified. Finally, a pool of CDR3s was PCR amplified from a naive passage (nP) with the 6G primary library. The naïve passage pool was produced by growing phage from the primary library, purifying the phage by precipitation, infecting E. *coli* with the purified phage and recovering DNA from the bacteria. Using SOE-PCR, the pools of CDR1 and CDR2s were randomly recombined with the pool of CDR3s, to recreate full VH domain genes. The following combinations were prepared (Table 22).

**Table 22**

| Code | CDR1/CDR2s | CDR3s |
|---|---|---|
| VH-6G-H+E | H | E |
| VH-6G-H+nP | H | nP |

The CDR1 and CDR2s were systematically taken from the DNA pool H, because the CDR1 segment contains the main determinants of aggregation. In contrast, the CDR3s were from the 'E' and 'nP' pools, which are less important for aggragation.

After SOE-PCR, the fragments were cloned into the phage vector (Fd-myc in which the leader sequence was replaced with an eukaryotic leader sequence; see Section 2), and used to transform *E. coli* by electroporation in order to obtain large repertoires (>10¹⁰ clones). Individual colonies (n=44) from the 6G-H+E and 6G-H+nP V_{H} libraries were separately grown for phage production. The phages were tested for their ability to bind to protein A (to assess production as well as correct folding) when untreated or after the heat treatment at 80°C. The results are shown in Table 23.

**Table 23.**

| Code | % positive clones on pA ELISA (no pre-heating) | % positive on pA ELISA (with heating at 80°C for 10 min) |
|---|---|---|
| VH-6G-H+E | 68 | 57 |
| VH-6G-H+nP | 70 | 61 |
| VH-4G-H20 | 50 | 6 |

About 85% of the phage that displayed a functional VH domain containing heat selected CDR1 and CDR2 loops, retained protein A binding activity after the heating step. Thus most of the V_{H} domains in library VH-6G include folding gatekeepers. In contrast, only 12% of the V_{H} domain clones in the unoptimised library 4G (VH-4G-H20) resist aggregation upon heating and retain protein A binding activity. These results demonstrate that adding a single heating step at the library clean-up stage (on protein A), resulted in enrichment of the VH domain library (by 7-fold) with DNA segments encoding VH domains that contain folding gatekeepers and that unfold reversibly upon heating.

Fourteen randomly picked clones from libraries VH-6G-H+E and VH-6G-H+nP were picked and sequenced. The sequences of the loop comprising the CDR1 are listed below as well as their SE-score (using a window of 15 amino acids). The diversified amino acids are underlined.

| Clone | Sequence | SE-score |
|---|---|---|
| 1. | CAASGFTFQYGPMSW (SEQ ID NO:197) | 0.152 |
| 2. | CAASGFTFAADNMDW (SEQ ID NO:198) | -0.074 |
| 3. | CAASGFTFPTNEMSW (SEQ ID NO:199) | -0.015 |
| 4. | CAASGFTFGNASMDW (SEQ ID NO:200) | -0.041 |
| 5. | CAASGFTFEDDLMNW (SEQ ID NO:201) | -0.021 |
| 6. | CAASGFTFGGDEMTW (SEQ ID NO:202) | -0.061 |
| 7. | CAASGFTFDDSTMQW (SEQ ID NO:203) | -0.075 |
| 8. | CAASGFTFDNSVMGW (SEQ ID NO:204) | 0.046 |
| 9. | CAASGFTFDQTAMHW (SEQ ID NO:205) | -0.021 |
| 10. | CAASGFTFPELPMGW (SEQ ID NO:206) | 0.105 |
| 11. | CAASGFTFTPGKMNW (SEQ ID NO:207) | 0.013 |
| 12. | CAASGFTFEHRTMGW (SEQ ID NO:208) | -0.011 |
| 13. | CAASGFTFPNSDMVW (SEQ ID NO:209) | 0.058 |
| 14. | CAASGFTFGKSTMAW (SEQ ID NO:210) | 0.044 |

Overall, the mean (±SD) SE-score for this region of the 14 Clones is 0.007 (±0.018) which is well below the mean SE-score of VH dummy (DP47d) (0.190) and the mean SE score (0.220) calculated for the encoded diversity (NNK codons) for that region. The diversity at each position was relatively large: 7, 11, 8, 10 and 9 different amino acids were counted in the dataset, at position 30, 31, 32, 33 and 35, respectively. Strongly hydrophilic and *β-*sheet breakers such as Asp, Glu, Pro and Gly accounted for 43% of the amino acids (versus 19% based on the encoded genetic diversity).

### Conclusion

A synthetic library of human V_{H}3 domain was created by recombination of PCR fragments and displayed on the surface of filamentous phage. A majority of the V_{H}3 dAbs (85%) unfolded reversibly upon heating at 80°C. This library was produced by (1) diversification of the CDR1 region (at position 30, 31, 32, 33 and 35), (2) heat-treatment of the primary phage library before clean-up on protein A, and (3) PCR-mediated recombination of heat-selected CDR1-CDR2-encoding genes and naïve passage- (or simply protein A-cleaned-up) CDR3-encoding genes. In the resulting library, the CDR1 sequences were diverse, but enriched for folding gatekeeper residues such as Asp, Glu, Pro and Gly.

### Section 19. Additional studies introducing folding gatekeepers into dAbs with predefined specificity

This section describes the implementation of folding gatekeepers into a dAb with pre-defmed specificity. TAR1-5-19 is a V_{K}1 domain which is specific for TNF-α Its sequence is:

DIQMTQSPSSLSASVGDRVTITCRASQSIDSYLHWYQQKPGKAPKLLIYSASE
LQSGVPSRFSGSGSGTDFTLTISSLQPEDFATYYCQQVVWRPFTFGQGTKVEI
KR (SEQ ID NO:211)

The results of this *rational-design* work complements another approach wherein 'folding gatekeepers' are introduced *ab initio* in dAb libraries displayed on phage.

### Choosing the folding gatekeepers for TAR1-5-19:

As described and demonstrated herein, folding gatekeepers can be introduced into the region comprising the H1 loop of DP47d, or the region comprising FR2-CDR2 of V_{K} dummy (DPK9). For TAR1-5-19, we selected position 49, a solvent-exposed residue at the boundary between FR2 and CDR2. It should be noted that in the absence of structural and mutagenesis data on the dAb, mutations within (or close to) the paratope may be likely to affect binding activity.

As shown in Table 28, two single mutants in PEP1-5-19 lower the SE-15 score (using the Sweet-Eisenberg (SE) scale over a window of 15 amino acids - Sweet R.M. & Eisenberg D., J. Mol. Biol. 171:479-488 (1983)), and therefore indicate that the mutant polypeptide would have high folding yield. It should be also noted that within that 15 aa region, the SE-score of PEP1-5-19 is already fairly low.

**Table 28**

| Clone | H1 sequence | SE-15 score |
|---|---|---|
| TAR1-5-19 | KAPKLLIYSASELQS | 0.011 |
| TAR1-5-19-Y49D | KAPKLLIDSASELQS | -0.187 |
| TAR1-5-19-Y49N | KAPKLLINSASELQS | -0.161 |

Aggregation assay of phage-displayed TAR1-5-19 and mutants thereof:
Using the methods described in Section 5, Phage Screening 2, monoclonal phages displaying TAR1-5-19 and variants were heated for 10 min at 80°C or left un-heated, and then incubated in TNF-α coated wells for ELISA. The % refolding data were calculated as described in Section 5, Phage Screening 2, and the results are shown in Table 30.

**Table 30**

| Clone | % refolding |
|---|---|
| TAR1-5-19 | 80 |
| TAR1-5-19-Y49N | 98 |
| TAR1-5-19-Y49D | 93 |

The folding gatekeepers have a beneficial effect on TAR1-5-19 refolding, and the variants that contained folding gatekeepers resisted aggregation when heated to 80°C even when displayed on phage. However, it should be noted that parental TAR1-5-19 also showed good refolding ability due to its low SE-score (0.011).

### Receptor binding assay of TAR1-5-19 and mutants thereof:

The activity assay was a RBA-ELISA (receptor-binding assay where increasing amount of dAb is used to compete with soluble TNF-α binding to immobilised TNF- α-receptor. Bound TNF-α was then detected with a biotinylated non-competing antibody, and streptavidin-HRP conjugate. The IC₅₀ (in nM) is the dAb dose require to produce a 50% reduction of a non-saturating amount of TNF-α to the receptor. The results are shown in Table 31.

**Table 31**

| Clone | IC₅₀ (nM) |
|---|---|
| TAR1-5-19 | 30 |
| TAR1-5-19-Y49N | 900 |
| TAR1-5-19-Y49D | 200 |

Here, the introduction of the folding gatekeeper into TAR1-5-19 affected the *in-vitro* activity of TAR1-5-19. This suggests that residue Y49 in TAR1-5-19 may be involved (directly or indirectly) in specific contact with TNF-α. For TAR1-5-19, Y49 does not appear to be the best choice for introducing a folding gatekeeper (although the mutants are still moderately active). Other positions within the 15 amino acid segment centered around Y49 could be considered to achieve a reduction of the SE-score without reducing the activity of the dAb. For example, TAR1-5-19 variants in which I48 is replaced with P, D, T, G or N, Y49 is replaced with S, C, E, G, K, N or R and/or 175 is replaced with N or M can be produced and characterized as described herein. Such a TAR1-5-19 variant can readily be produce using the methods demonstrated herein.

### Conclusion

In this study, folding gatekeepers were introduced into a dAb of pre-defined specificity, without prior knowledge of structure of the antigen-dAb complex. Binding of variants of TAR1-5-19 was reduced by 6- to 20-fold, but the variants still bound the target with moderate activity, demonstrating that the variants have biological activity. It should be noted that only a two single point mutants were created and studied. Further benefits of introducing folding gatekeepers would be realized by creating and screening additional variants that contain single point mutations or combinations of mutations, for example by positional diversification or by library generation.

**Table 24 Sweet-Eisenberg (S/E) values of DP47- V_{H} (with window of 11 amino acids)**

| Position | amino acid | S/E | Position | amino acid | S/E | Position | amino acid | S/E |
|---|---|---|---|---|---|---|---|---|
| 1 | E | 0.000 | 40 | A | -0.211 | 79 | L | -0.004 |
| 2 | V | 0.000 | 41 | P | -0.272 | 80 | Y | 0.030 |
| 3 | Q | 0.000 | 42 | G | -0.272 | 81 | L | 0.191 |
| 4 | L | 0.000 | 43 | K | -0.272 | 82 | Q | 0.198 |
| 5 | L | 0.000 | 44 | G | -0.268 | 83 | M | 0.245 |
| 6 | E | -0.122 | 45 | L | -0.222 | 84 | N | 0.160 |
| 7 | s | 0.072 | 46 | E | -0.072 | 85 | S | -0.070 |
| 8 | G | -0.094 | 47 | W | -0.077 | 86 | L | -0.247 |
| 9 | G | -0.055 | 48 | V | -0.077 | 87 | R | -0.395 |
| 10 | G | -0.227 | 49 | S | -0.066 | 88 | A | -0.229 |
| 11 | L | -0.399 | 50 | A | -0.066 | 89 | E | -0.170 |
| 12 | V | -0.338 | 51 | I | -0.238 | 90 | D | 0.065 |
| 13 | Q | -0.177 | 52 | S | -0.177 | 91 | T | 0.131 |
| 14 | P | -0.170 | 53 | G | -0.248 | 92 | A | -0.016 |
| 15 | G | 0.002 | 54 | S | -0.179 | 93 | V | -0.024 |
| 16 | G | 0.013 | 55 | G | 0.023 | 94 | Y | -0.037 |
| 17 | S | -0.083 | 56 | G | 0.023 | 95 | Y | 0.225 |
| 18 | L | -0.202 | 57 | s | -0.210 | 96 | C | 0.284 |
| 19 | R | -0.155 | 58 | T | -0.210 | 97 | A | 0.273 |
| 20 | L | -0.161 | 59 | Y | -0.066 | 98 | K | 0.484 |
| 21 | s | -0.161 | 60 | Y | -0.077 | 99 | S | 0.282 |
| 22 | C | 0.075 | 61 | A | -0.077 | 100 | Y | 0.282 |
| 23 | A | 0.099 | 62 | D | -0.070 | 101 | G | 0.175 |
| 24 | A | 0.163 | 63 | S | 0.155 | 102 | A | 0.099 |
| 25 | S | 0.166 | 64 | V | 0.155 | 103 | F | 0.053 |
| 26 | G | 0.005 | 65 | K | 0.116 | 104 | D | 0.053 |
| 27 | F | 0.207 | 66 | G | -0.085 | 105 | Y | 0.077 |
| 28 | T | 0.155 | 67 | R | -0.103 | 106 | W | 0.036 |
| 29 | F | 0.285 | 68 | F | -0.103 | 107 | G | 0.180 |
| 30 | s | 0.271 | 69 | T | -0.136 | 108 | Q | 0.191 |
| 31 | S | 0.366 | 70 | I | -0.269 | 109 | G | 0.099 |
| 32 | Y | 0.510 | 71 | S | -0.269 | 110 | T | 0.168 |
| 33 | A | 0.282 | 72 | R | -0.292 | 111 | L | 0.000 |
| 34 | M | 0.225 | 73 | D | -0.264 | 112 | V | 0.000 |
| 35 | S | 0.014 | 74 | N | -0.327 | 113 | T | 0.000 |
| 36 | W | 0.019 | 75 | S | -0.150 | 114 | V | 0.000 |
| 37 | V | 0.008 | 76 | K | -0.153 | 115 | S | 0.000 |
| 38 | R | -0.205 | 77 | N | -0.185 | 116 | S | 0.000 |
| 39 | Q | -0.229 | 78 | T | -0.039 | | | |

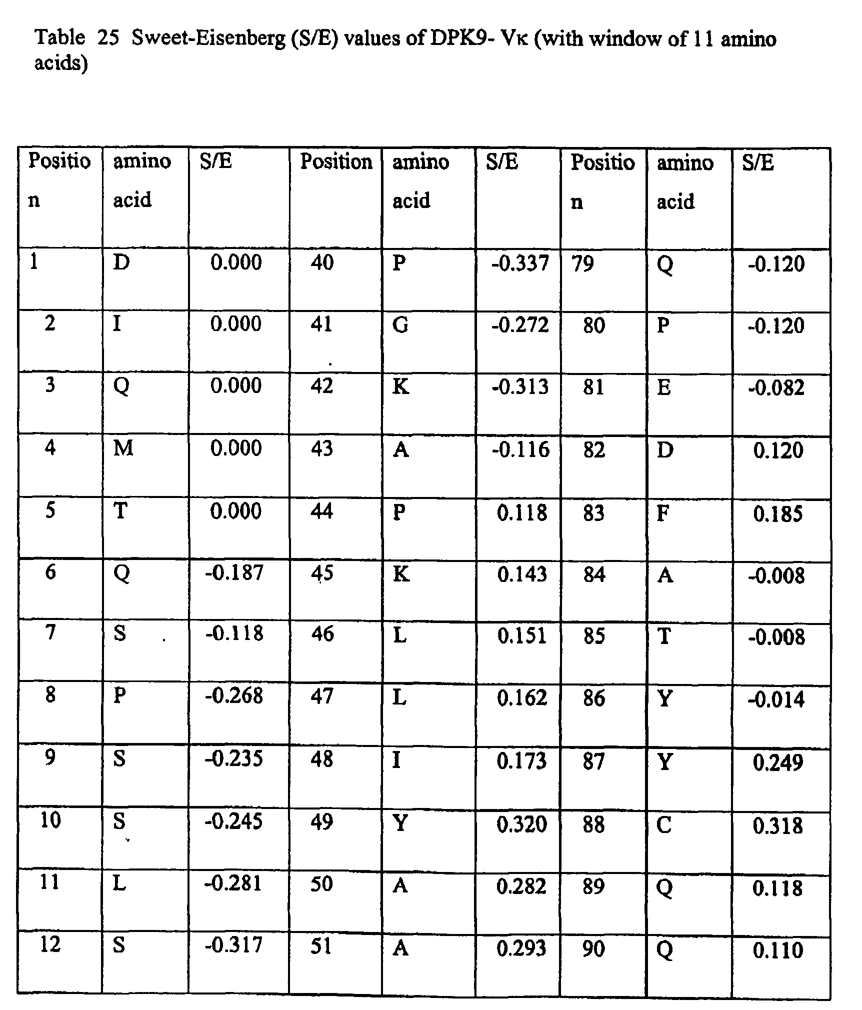

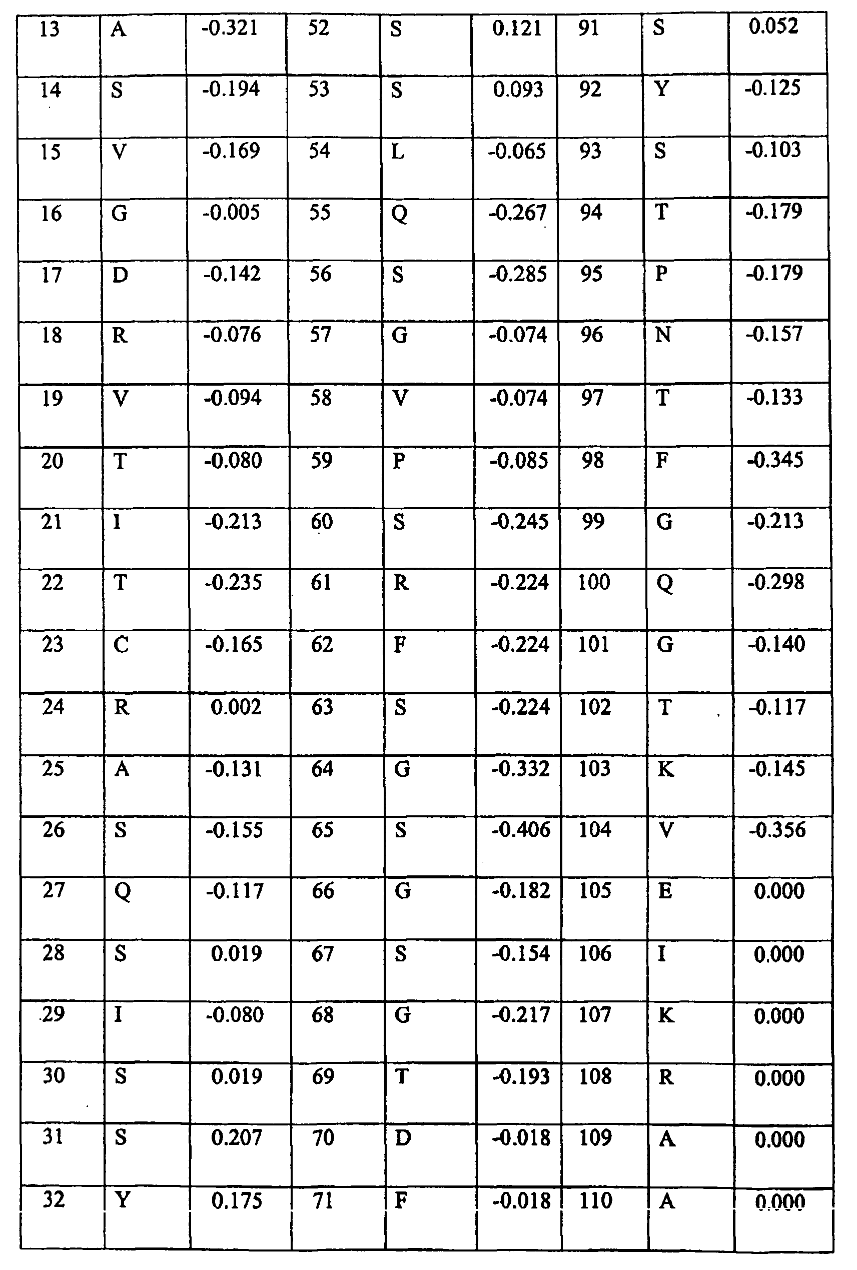

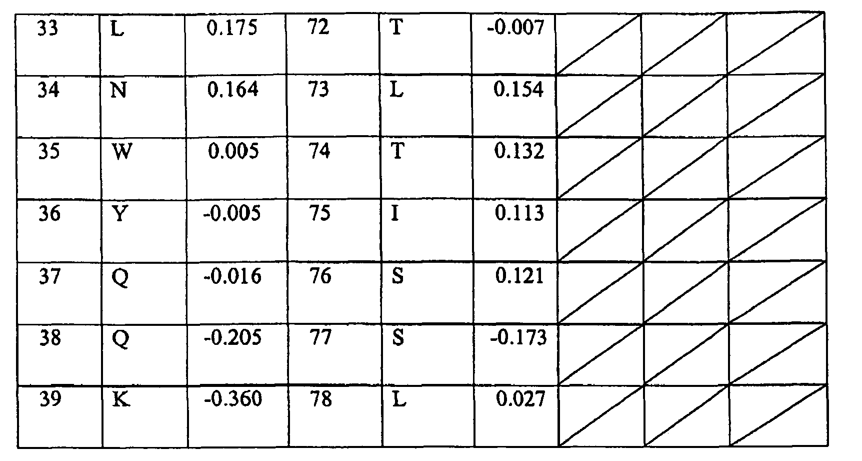

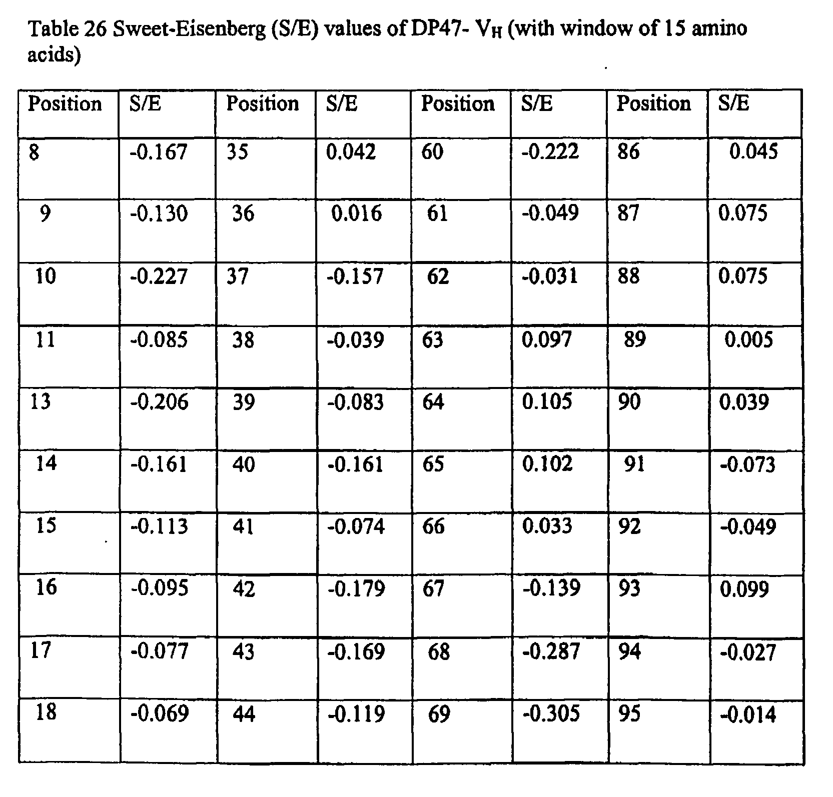

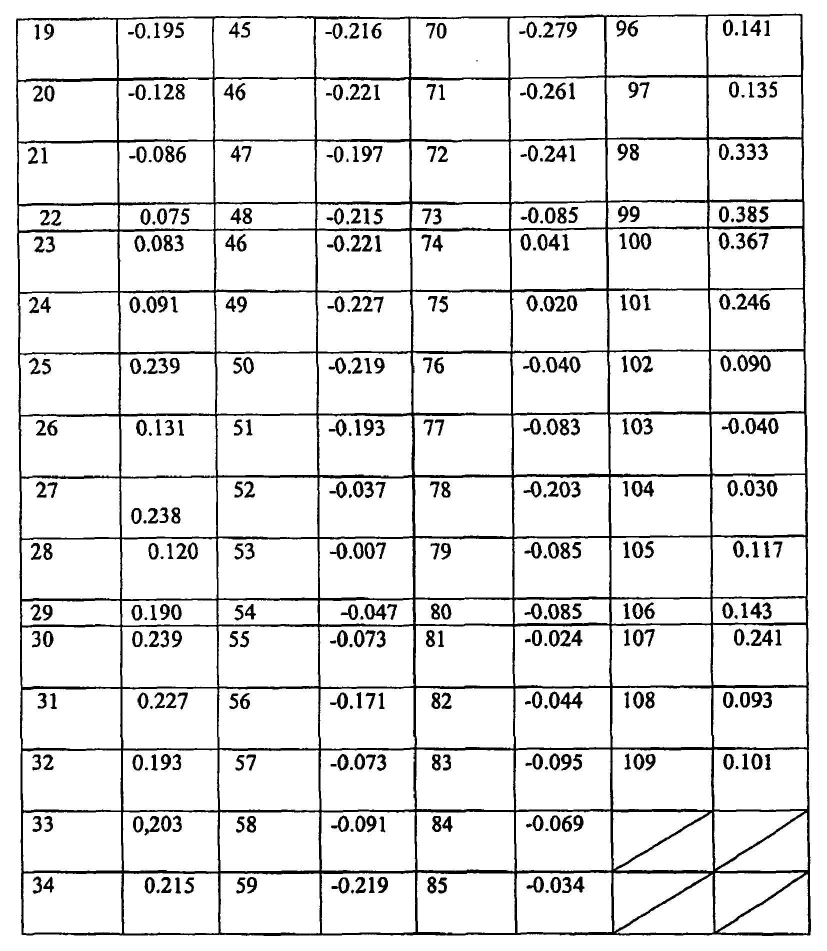

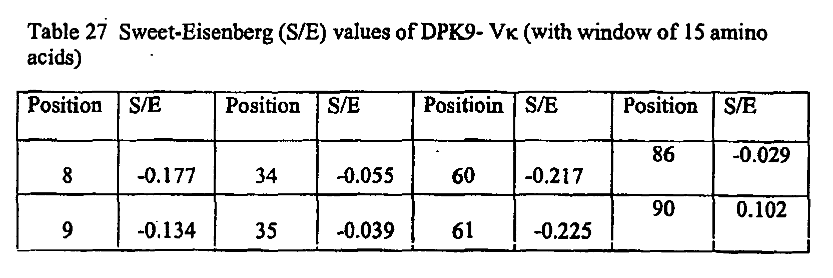

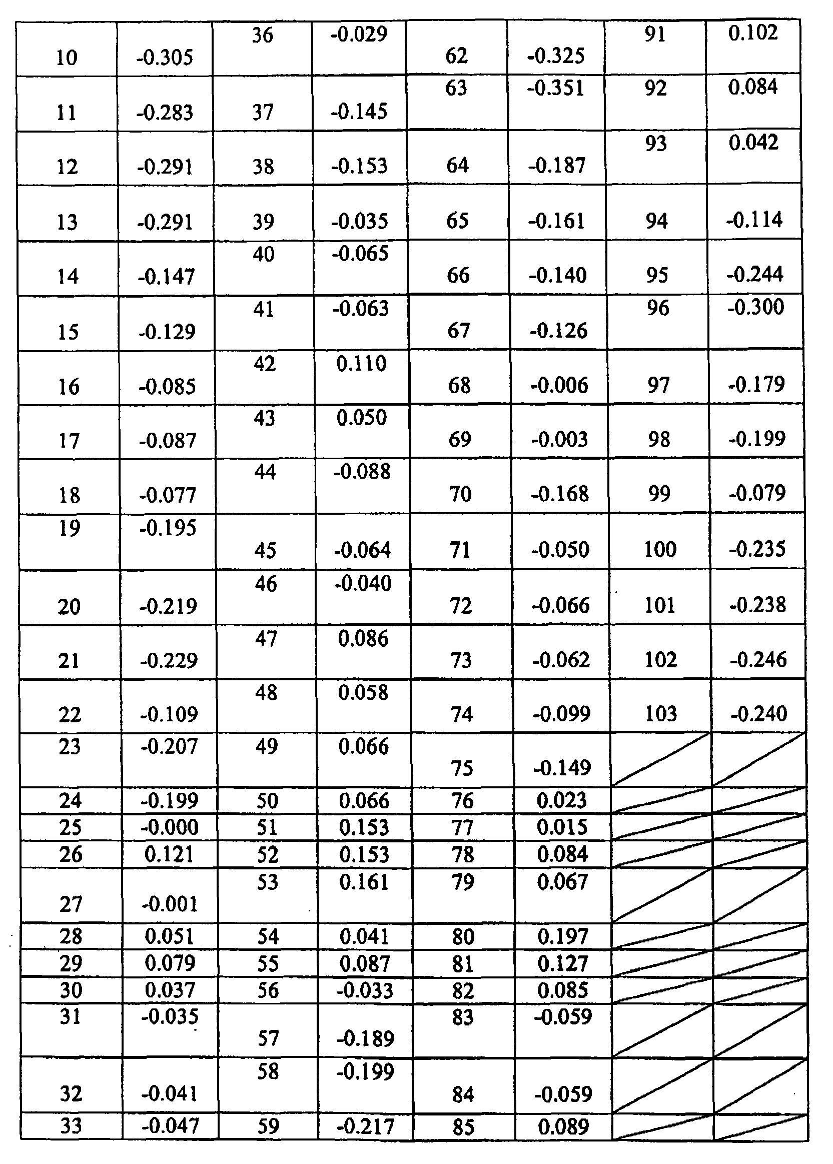

While this invention has been particularly shown and described with references to preferred embodiments thereof, it will be understood by those skilled in the art that various changes in form and details may be made therein without departing from the scope of the invention encompassed by the appended claims.

## Claims

1. An isolated polypeptide comprising a variant immunoglobulin light chain variable domain (VI) that unfolds reversibly, wherein
said variant VL comprises the amino acid sequence of a parental VL wherein at least one amino acid residue in the FR2-CDR2 region is replaced such that the Sweet/Eisenberg hydrophobicity score (S/E score) of the FR2- CDR2 region is reduced relative to the S/E score of the FR2-CDR2 region of the parental VL ;
said variant VL comprises the amino acid sequence of a parental VL wherein at least one amino acid residue in FR3 is replaced such that the S/E score of FR3 is reduced relative to the S/E score of FR3 of the parental VL ;
said variant VL comprises the amino acid sequence of a parental VL wherein at least one amino acid residue from position 44 to position 53 is replaced such that the S/E score is reduced relative to the S/E score of the amino acid sequence from position 44 to position 53 of the parental V ; and/or
said variant VI, comprises the amino acid sequence of a parental VL wherein at least one amino acid residue from 73 to position 76 is replaced such that the S/E score is reduced relative to the S/E score of the amino acid sequence from position 73 to position 76 of the parental VL ;
wherein the FR2-CDR2 region, FR3, positions 44-53 and positions 73-76 are assigned in accordance with the Kabat amino acid numbering system.

2. The isolated polypeptide of Claim 1, wherein the S/E score of the FR2-CDR2 region of said variant Vi. is 0.23 or less; the S/E score of FR3 of said variant VL is 0.35 or less; the S/E score of the amino acid sequence from position 44 to position 53 of said variant VL is 0. 23 or less; and/or the S/E score of the amino acid sequence from position 73 to position 76 of said variant VL is 0. 35 or less.

3. An isolated polypeptide of Claim 1, comprising a variant immunoglobulin light chain variable region domain (VL) that unfolds reversibly, wherein said variant VL comprises the amino acid sequence of a parental VL in which at least one amino acid residue in FR3 is replaced with a Pro or Gly residue; and/or said variant VL comprises the amino acid sequence of a parental VL in which at least one amino acid residue from position 44 to position 53 is replaced with a Pro or Gly residue, wherein FR3 and position 44 to position 53 are assigned in accordance with the Kabat amino acid numbering system.

4. The isolated polypeptide of any one of the preceding claims, wherein said variant VL unfolds reversibly when heated to a temperature greater than its melting temperature (Tm).

5. The isolated polypeptide of any one the preceding claims, wherein said variant VL unfolds reversibly when heated to about 80°C.

6. The isolated polypeptide of any one of the preceding claims, wherein said isolated polypeptide is an antibody format.

7. The isolated polypeptide of any one of any one of the preceding claims, wherein one or more of the framework regions (FR) in said variant VL comprise (a) the amino acid sequence of a human framework region, (b) at least 8 contiguous amino acids of the amino acid sequence of a human framework region, or (c) an amino acid sequence encoded by a human germline antibody gene segment, wherein said framework regions are as defined by Kabat.

8. The isolated polypeptide of any one of any one of the preceding claims, wherein the amino acid sequences of one or more framework regions in said variant VL are the same as the amino acid sequence of a corresponding framework region encoded by a human germline antibody gene segment, or the amino acid sequences of one or more of said framework regions collectively comprise up to 5 amino acid differences relative to the corresponding framework regions encoded by a human germline antibody gene segment.

9. The isolated polypeptide of any one of the preceding claims, wherein the amino acid sequences of FR1, FR2, FR3 and FR4 in said variant VL are the same as the amino acid sequences of corresponding framework regions encoded by a human germline antibody gene segment, or the amino acid sequences of FR1, FR2, FR3 and FR4 collectively contain up to 10 amino acid differences relative to the corresponding framework regions encoded by a human germline antibody gene segment.

10. The isolated polypeptide of any one of the preceding claims, wherein the variant VL domain comprises FR1, FR2 and FR3 regions, and the amino acid sequence of said FR1, FR2 and FR3 are the same as the amino acid sequences of corresponding framework regions encoded by a human germline antibody gene segment.

11. The isolated polypeptide of any one of the preceding claims, wherein said human germline antibody gene segment comprises a germline VL, gene segment selected from the group consisting of DPK1, DPK2, DPK3, DPK4, DPK5, DPK6, DPK7, DPK8, DPK9, DPK10, DPK12, DPK13, DPK15, DPK16, DPK18, DPK19, DPK20, DPK21, DPK22, DPK23, DPK24, DPK25, DPK26 and DPK 28 131.

12. The isolated polypeptide of Claim 11, wherein said human germline antibody gene segment further comprises a human germline L gene segment selected from the group consisting of Jxl, Jx2, Jx3, Jx4, and J, 5.

13. An isolated nucleic acid encoding a polypeptide of any one of the preceding claims.

14. A library enriched in a polypeptide of any one of Claims 1-12.

15. The library of Claim 14, wherein said library comprises a collection of heterogeneous nucleic acids that encode said polypeptides that unfold reversibly.

16. A library of nucleic acids encoding the polypeptides according to claim 14.

17. composition comprising a polypeptide according to any one of Claims 1-12.

18. The composition of Claim 17, wherein said polypeptide is present in an amount in the range of about 5% to about 99% by weight.

19. The composition of Claim 17, wherein said composition is a solution.

20. The composition of Claim 17, wherein said composition is a pharmaceutical composition.

21. The composition of Claim 17, wherein said composition is a biological washing powder.

22. A composition comprising a freeze dried polypeptide according to any one of Claims 1-12.

23. The composition of Claim 22, wherein said freeze dried polypeptide that unfolds reversibly loses no more than about 20% of its activity when rehydrated.

24. A sealed package comprising a polypeptide according to any one of Claims 1-12.

25. A kit for use in administering a polypeptide that unfolds reversibly to a subject, comprising a polypeptide that according to any one of Claims 1-12, a drug delivery device and, optionally, instructions for use.

26. The kit of Claim 25, wherein said drug delivery device is selected from the group consisting of a syringe, an inhaler, an intranasal administration device, an ocular administration device and a needleless injection device.

27. A method of treating or diagnosing a subject having a disease or disorder, comprising administering to a subject in need thereof a therapeutically effective amount of a polypeptide according to any one of Claims 1-12.

28. Use of a polypeptide according to any one of Claims 1-12 in therapy or diagnosis.

29. Use of polypeptide according to any one of Claims 1-12 for the manufacture of a medicament for treating a disease.

30. A polypeptide according to any one of Claims 1-12 for use in therapy or diagnosis.

31. A polypeptide according to any one of Claims 1-12, for the manufacture of a medicament for the treatment and/or prophylaxis and/or suppression of a disease or medical condition mediated by a cytokine, cytokine receptor, enzyme, enzyme co-factor, or DNA binding protein,

32. A polypeptide according to any one of Claims 1-12, wherein the polypeptide comprises an immunoglobulin variable domain, for targeting a cytokine receptor, enzyme, enzyme co- factor, or DNA binding protein, for treating and/or preventing and/or suppressing a disease or medical condition mediated by said cytokine receptor, enzyme, enzyme co-factor, or DNA binding protein.

33. The polypeptide of Claims 31-32, wherein the disease or medical condition is selected from an inflammatory state, allergic hypersensitivity, cancer, bacterial or viral infection, and an autoimmune disorder.

34. The polypeptide of Claims 31-32, wherein the disease or medical condition is selected from asthma, psoriasis, Type I diabetes, multiple sclerosis, rheumatoid arthritis, systemic lupus erythematosus, Crohn's disease, myasthenia gravis, a leukaemia or a solid tumour.

35. A recombinant host cell comprising a recombinant nucleic acid encoding a polypeptide according to any one of Claims 1-12, wherein said recombinant nucleic acid is operably linked to an expression control sequence.

36. A method for producing a polypeptide that unfolds reversibly, comprising maintaining the host cell of Claim 35, under conditions suitable for expression of said recombinant nucleic acid, whereby said recombinant nucleic acid is expressed and said polypeptide that unfolds reversibly is produced.

37. Use of a library or repertoire according to any one of Claims 14-16, for selecting a polypeptide that refolds reversibly,

38. Use of Claim 37, wherein said polypeptide that refolds reversibly comprises an antibody variable domain that unfolds reversibly.

39. Use of Claim 37, wherein said polypeptide that refolds reversibly comprises an antibody format.
